(19) Europäisches Patentamt
European Patent Office
Office européen des brevets

(11) **EP 4 345 096 B1**

(12) **EUROPEAN PATENT SPECIFICATION**

(45) Date of publication and mention of the grant of the patent:
**18.02.2026 Bulletin 2026/08**

(21) Application number: **22841467.8**

(22) Date of filing: **14.07.2022**

(51) International Patent Classification (IPC):
***C07D 235/26*** *(2006.01)* ***A61K 31/4184*** *(2006.01)*
***A61P 25/20*** *(2006.01)* ***A61P 25/32*** *(2006.01)*
***A61P 25/34*** *(2006.01)* ***A61P 25/36*** *(2006.01)*

(52) Cooperative Patent Classification (CPC):
**A61P 29/00; A61P 25/04; A61P 25/20; A61P 25/22; A61P 25/24; A61P 25/30; A61P 25/32; A61P 25/34; A61P 25/36; C07D 401/04; C07D 401/14; C07D 405/14; C07D 409/14; C07D 491/107**

(86) International application number:
**PCT/CN2022/105776**

(87) International publication number:
**WO 2023/284834 (19.01.2023 Gazette 2023/03)**

(54) **PIPERIDINE DERIVATIVE, AND PHARMACEUTICAL COMPOSITION THEREOF, PREPARATION METHOD THEREFOR, AND USE THEREOF**

PIPERIDINDERIVAT UND PHARMAZEUTISCHE ZUSAMMENSETZUNG DAVON, HERSTELLUNGSVERFAHREN DAFÜR UND VERWENDUNG DAVON

DÉRIVÉ DE PIPÉRIDINE, ET COMPOSITION PHARMACEUTIQUE DE CELUI-CI, PROCÉDÉ DE PRÉPARATION CORRESPONDANT ET UTILISATION ASSOCIÉE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **14.07.2021 CN 202110797657**

(43) Date of publication of application:
**03.04.2024 Bulletin 2024/14**

(73) Proprietors:
- **Yichang Humanwell Pharmaceutical Co., Ltd.**
  **Yichang, Hubei 443005 (CN)**
- **Tsinghua University**
  **Beijing 100084 (CN)**

(72) Inventors:
- **WANG, Jian**
  **Beijing 100084 (CN)**
- **SHENG, Xijun**
  **Yichang, Hubei 443005 (CN)**
- **LI, Lie**
  **Yichang, Hubei 443005 (CN)**
- **TIAN, Luanyuan**
  **Yichang, Hubei 443005 (CN)**
- **LV, Jinliang**
  **Yichang, Hubei 443005 (CN)**
- **WANG, Miao**
  **Yichang, Hubei 443005 (CN)**
- **ZHOU, Hao**
  **Yichang, Hubei 443005 (CN)**
- **YANG, Xiaoqing**
  **Yichang, Hubei 443005 (CN)**

(74) Representative: **HGF**
**HGF Limited**
**4th Floor, 1 City Square**
**Leeds LS1 2ES (GB)**

(56) References cited:
**WO-A1-03/082333** **WO-A1-2017/096323**
**WO-A2-2008/050698**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- GIOVANNA BRUNI ET AL: "Structure and properties of domperidone and its succinate salt", ACTA CRYSTALLOGRAPHICA. SECTION B: STRUCTURAL SCIENCE, CRYSTAL ENGINEERING AND MATERIALS (PRINT), WILEY-BLACKWELL PUBLISHING, INC, US, vol. 69, no. 4, 26 February 2016 (2016-02-26), pages 362 - 370, XP072459667, ISSN: 2052-5192, DOI: 10.1107/S2052519213016989
- PALIN, R. ; CLARK, J.K. ; EVANS, L. ; FEILDEN, H. ; FLETCHER, D. ; HAMILTON, N.M. ; HOUGHTON, A.K. ; JONES, P.S. ; MCARTHUR, D. ; : "Rapid access towards follow-up NOP receptor agonists using a knowledge based approach", BIOORGANIC & MEDICINAL CHEMISTRY LETTERS, ELSEVIER, AMSTERDAM NL, vol. 19, no. 22, 15 November 2009 (2009-11-15), Amsterdam NL , pages 6441 - 6446, XP026703825, ISSN: 0960-894X, DOI: 10.1016/j.bmcl.2009.09.028
- PALIN, R. ET AL.: "Structure-activity Relationships and CoMFA of N-3 Substituted Phenoxypropyl Piperidine Benzimidazol-2-one Analogues as NOP Receptor Agonists with Analgesic Properties.", BIOORGANIC & MEDICINAL CHEMISTRY, vol. 16, 10 January 2008 (2008-01-10), XP022558519, DOI: 10.1016/ j.bmc.2008.01.005
- DATABASE REGISTRY ANONYMOUS : "-2(1H)-Quinazolinone, 3,4-dihydro-1-[1-[[4-(3-phenyl-2- quinoxalinyl)phenyl]methyl]-4-piperidinyl]-(CA INDEX NAME) ", XP093024591, retrieved from STN
- PALIN, R. BARN, D.R. CLARK, J.K. COTTNEY, J.E. COWLEY, P.M. CROCKATT, M. EVANS, L. FEILDEN, H. GOODWIN, R.R. GRI: "Synthesis and SAR studies of 3-phenoxypropyl piperidine analogues as ORL1 (NOP) receptor agonists", BIOORGANIC & MEDICINAL CHEMISTRY LETTERS, ELSEVIER, AMSTERDAM NL, vol. 15, no. 3, 1 February 2005 (2005-02-01), Amsterdam NL , pages 589 - 593, XP004719973, ISSN: 0960-894X, DOI: 10.1016/j.bmcl.2004.11.049
- PALIN, R. BOM, A. CLARK, J.K. EVANS, L. FEILDEN, H. HOUGHTON, A.K. JONES, P.S. MONTGOMERY, B. WESTON, M.A. WISHA: "Synthesis and evaluation of N-3 substituted phenoxypropyl piperidine benzimidazol-2-one analogues as NOP receptor agonists with analgesic and sedative properties", BIOORGANIC & MEDICINAL CHEMISTRY, ELSEVIER, AMSTERDAM, NL, vol. 15, no. 4, 14 January 2007 (2007-01-14), AMSTERDAM, NL, pages 1828 - 1847, XP005830444, ISSN: 0968-0896, DOI: 10.1016/j.bmc.2006.11.030

## Description

### Technical Field

The present application belongs to the field of medicinal chemistry, in particular to a piperidine derivative, and a pharmaceutical composition, a preparation method and use thereof.

### Background

Pain is the most common physical sensation in people's daily life, affecting even more people than diabetes, cardiovascular diseases and cancers combined. The pathogenesis of pain is diverse, and it accompanies almost all diseases. There is a huge clinical need for anti-inflammatory analgesia. At present, opioid analgesics and non-steroidal anti-inflammatory drugs (NSAIDs) dominate the market of pain treatment and are widely used in areas such as surgical analgesia, advanced cancer analgesia and long-term chronic pain. Opioid analgesics are the main therapeutic drugs for moderate to severe pain in clinical practice, as they take effect quickly, have strong analgesic effect, etc. However, the side effects such as addiction, respiratory depression, constipation, tolerance, and itching limit the use, which also bring forth social problems such as drug dependence and death from abuse. It is a very urgent social need to study and develop a safe and effective analgesic drug without side effects.

For many years, scientists have been trying to find new analgesics for clinical selection, which have the analgesic effect of opioids without their side effects. Medicinal chemists have used a variety of strategies to develop safe and effective drugs to treat pain and opioid addiction. Many new target mechanisms have been discovered and applied to the research and development of novel analgesics. Numerous studies have shown that the analgesic effect of μ-receptor agonists is mediated through activation of $G_i$ signals, whereas side effects such as addiction, respiratory depression, and constipation are regulated by downstream β-arrestin signaling pathway. Therefore, G protein-biased μ-opioid receptor agonists have been an important direction of research in this field in recent years. Biased agonists of μ-receptors, which selectively activate $G_i$ pathway without affecting β-arrestin signaling pathway, provide possibility of developing desirable opioid drugs.

WO2017/161017A1 discloses a class of G protein-biased MOP receptor agonists of benzimidazolone piperidine compounds, which exhibit good analgesic activity in rodent analgesic models, with less associated side effects than morphine. However, the compounds themselves may have deficiencies in terms of druggability, and no further entry into clinical studies has been reported.

Selective KOP receptor agonists and DOP receptor agonists have their own side effects, although they have no side effects mediated by MOP receptor agonists. KOP receptor agonists may mediate the production of side effects such as restlessness, sedation, and polyuria, but they have also been found to be able to reduce the rewarding effect of addictive drugs, and therefore have the potential to be developed as analgesics which overcome opioid dependence. After being activated by agonists, DOP receptors will produce convulsive effect, but also show good analgesic effect on chronic pain. Compared with MOP receptor agonists, after being agonized, DOP receptors are not physiologically dependent and are less abuse-dependent, and they also have anti-anxiety and anti-depression effects. However, up to now, ligand compounds of selective KOP receptors or DOP receptors have not been successfully developed in clinical practice because of their limited analgesic efficacy.

The analgesic effect mediated by NOP receptors is much more complex than that of other opioid receptor family members. The nociceptin/orphanin FQ-NOP receptor system not only inhibits opioid receptor-mediated analgesic effect, but also mediates analgesic effect by reducing hyperalgesia. Studies have shown that NOP receptor agonists have analgesic effect and the effect of regulating addiction caused by MOP agonists, and it is found in non-human primate tests that selective NOP agonists do not cause respiratory depression and potential addiction. RO 64-6198, the first reported small molecule NOP receptor selective agonist, was developed by Roche and exhibited good analgesic effect in non-human primate models and rodent model experiments, without classical opioid-mediated side effects. Schering discloses in WO01/07050A1 a class of piperidinyl-derived compounds that are used as NOP receptor (ORL-1 receptor) agonists for the treatment of cough. Due to the close interaction between nociceptin/orphanin FQ receptors and classical opioid receptors, in recent years, people's attention has gradually shifted to the study of ligand compounds that act simultaneously on double targets.

BU08028 is an analog of buprenorphine. This molecule has relatively high affinity for all four typical kinds of opioid receptors. Results of in vitro experiments have shown that BU08028 is a partial agonist of MOP/NOP receptor, and compared with buprenorphine, BU08028 has higher biological activity for NOP receptor; and in in vivo experiments in non-human primates, compared with buprenorphine and remifentanil, BU08028 shows very high analgesic and anti-anxiety effects, with lower addiction, and more importantly, it does not show remarkable physiological dependence. Nalorphine is a kind of opioids with bifunctional effects of MOP receptor antagonist and KOP receptor agonist, which can inhibit the effect of morphine in vivo and show analgesic activity. Bifunctional molecules with high activity for KOP receptors and low or

moderate activity for MOP receptors are less addictive than MOP receptor agonists alone, but they have the side effects of making the patients irritable and being hallucinogenic. Cebranopadol is a MOP/NOP receptor bifunctional agonist. Preclinical data show that Cebranopadol also has partial agonist activity for KOP receptors. Cebranopadol has recently been reported to show reduced tolerance, physiological dependence, and respiratory depression compared with morphine. Patent WO2017/096323A1 discloses AT-121, which is a kind of piperidinyl spirocyclic compound with high affinity for both MOP and NOP receptors, and is a partial agonist of the two receptors. In non-human primate experiments, this molecule shows good analgesic and reinforcing effect of inhibitory efficacy, and has no side effects such as respiratory depression, opioid-induced hypersensitivity and drug dependence.

As can be seen from the existing reports, different opioid receptors have their own pharmacological functional characteristics, and the molecules with bifunctional or multifunctional effects on opioid receptors show unique pharmacological effects. While producing good analgesic efficacy, they can effectively reduce the side effects of traditional opioids such as morphine, such as respiratory depression, constipation, tolerance, addiction, etc., have anti-depression and anti-anxiety effects, and are potentially effective in the treatment of alcohol addiction. However, at present, they are all in the early stage of research, with few successes. On the other hand, there is an urgent social need for analgesic drugs without side effects. Therefore, there is a necessity to conduct more extensive research in the field of new analgesic drugs with multifunctional or bifunctional opioid receptor effects.

Summary

The invention is set out in the appended set of claims. Embodiments of the invention are those whose scope is within that of the appended claims. Other passages stating embodiments which do not fall under the scope of the appended claims are to be considered as examples.

The following is a summary of the subject matter described in detail herein. This summary is not intended to limit the protection scope of the claims.

The present application provides a piperidine derivative, a pharmaceutical composition thereof, a preparation method and a use thereof. This kind of compounds are structurally novel, and have high affinity and agonistic bioactivity for MOP receptor, NOP receptor (ORL-1 receptor) and (or) KOP receptor, high selectivity with bifunctional effect for MOP receptor and KOP receptor, or high selectivity with bifunctional effect for MOP receptor and NOP receptor (ORL-1 receptor); and some of the compounds have a high bias for G protein, and thus have a unique mechanism of pharmacological action. They can be developed into a new kind of opioid receptor drugs for the treatment or prevention of acute and chronic pain, anxiety, depression, alcohol addiction, and substance abuse/dependence.

In an embodiment of the present application, the present application provides a compound shown in Formula (I), or a stereoisomer, a pharmaceutically acceptable salt, a solvate, a deuterate, a metabolite, or a prodrug thereof;

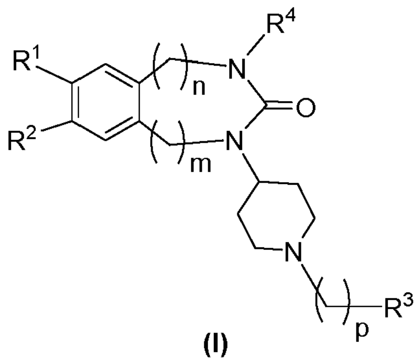

**(I)**

wherein,

n is 0 or 1;
m is 0 or 1;
p is 0, 1, or 2;
$R^1$ and $R^2$ are each independently selected from: hydrogen, halogen, $C_{1-3}$ alkyl, and $C_{1-3}$ alkoxy; provided that $R^1$ and $R^2$ are not hydrogen at the same time;
$R^3$ is selected from unsubstituted aryl, substituted aryl, unsubstituted heteroaryl, substituted heteroaryl, unsubstituted $C_{3-8}$ cycloalkyl, substituted $C_{3-8}$ cycloalkyl, unsubstituted $C_{4-6}$ heterocycloalkyl, and substituted $C_{4-6}$ heterocycloalkyl;
$R^4$ is selected from hydrogen, -$C_{1-3}$ alkyl-unsubstituted heterocycloalkyl, -$C_{1-3}$ alkyl-substituted heterocycloalkyl,

$-C_{1-3}$ alkyl-substituted spiroheterocyclic alkyl, $-C_{1-3}$ alkyl-C(O)$NR^5R^6$, $-C_{1-3}$ alkyl-$NR^7R^8$, $-C_{1-4}$ alkyl-unsubstituted-heteroaryl, and $-C_{1-4}$ alkyl-substituted heteroaryl; herein, $R^5$ and $R^6$ may be independently hydrogen or $C_{1-3}$ alkyl, or $R^5$ and $R^6$ together form unsubstituted $C_{4-6}$ heterocycloalkyl, or $R^5$ and $R^6$ together form $C_{4-6}$ substituted heterocycloalkyl; $R^7$ and $R^8$ may be independently hydrogen or $C_{1-3}$ alkyl, or $R^7$ and $R^8$ together form unsubstituted $C_{4-6}$ heterocycloalkyl, or $R^7$ and $R^8$ together form $C_{4-6}$ substituted heterocycloalkyl; provided that when $R^4$ is hydrogen, m is 0, and n is 1.

In another aspect, the present application provides a pharmaceutical composition, including the aforementioned piperidine derivative, or a stereoisomer, a pharmaceutically acceptable salt, a solvate, a deuterate, a metabolite, or a prodrug thereof, and a pharmaceutically acceptable carrier.

In a third aspect, the present application provides bioactivity of the aforementioned piperidine derivative or a pharmaceutical composition thereof as a MOP receptor and NOP receptor (ORL-1 receptor) agonist.

In a fourth aspect, the present application provides a preparation method for the aforementioned piperidine derivative, the preparation method including the following steps:

(1) coupling a compound of Formula (I-1) with a compound of Formula (I-2) to obtain a compound of Formula (I-3);

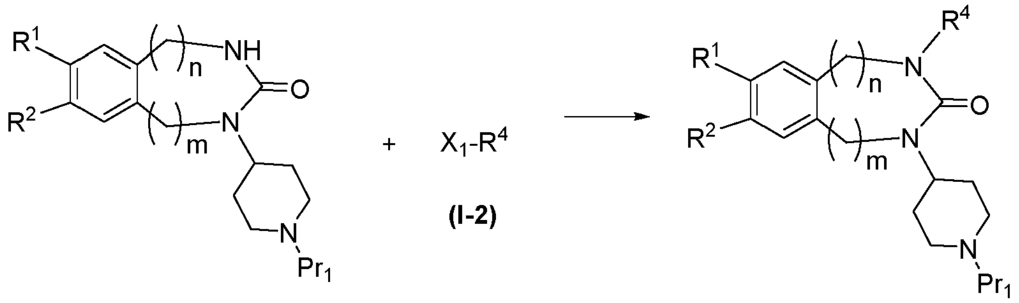

(I-1) (I-2) (I-3)

(2) subjecting the compound of Formula (I-3) to a deprotection reaction to obtain Formula (I-4), and then being coupled with a compound of Formula (I-5) to obtain a compound of Formula (I);

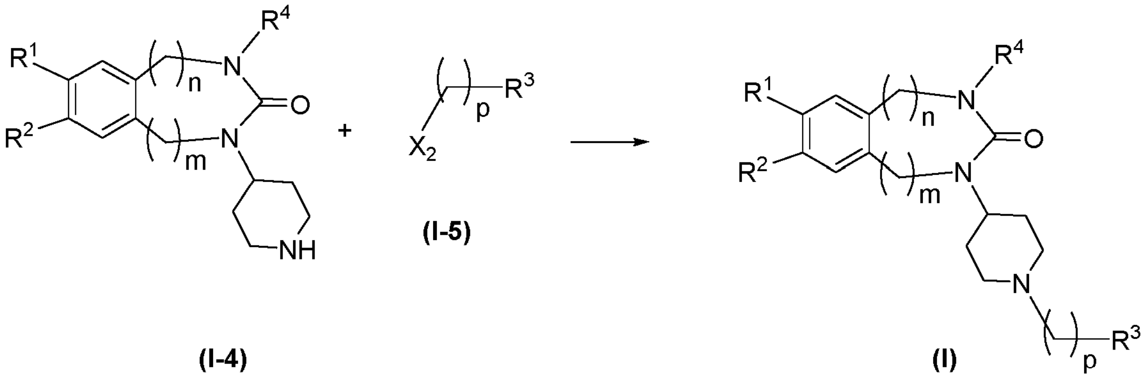

(I-4) (I-5) (I) ;

or,

(1') coupling a compound of Formula (I-6) with a compound of Formula (I-5) to obtain a compound of Formula (I-7);

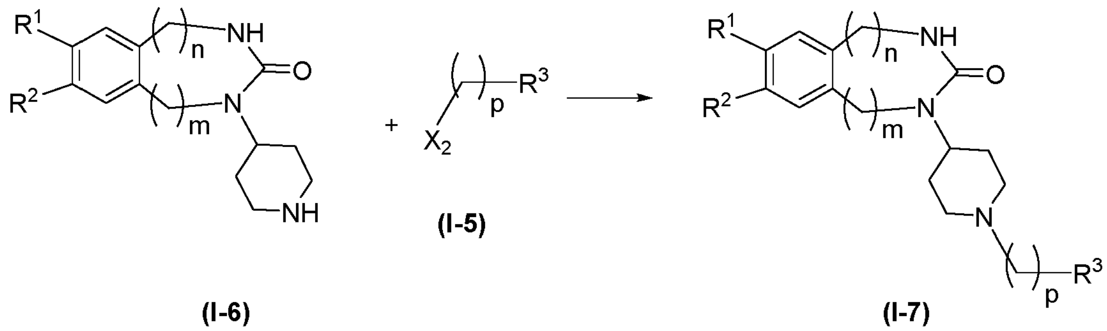

(I-5)

(I-6) (I-7)

(2) coupling the compound of Formula (I-7) with the compound of Formula (I-2) to obtain a compound of Formula (I);

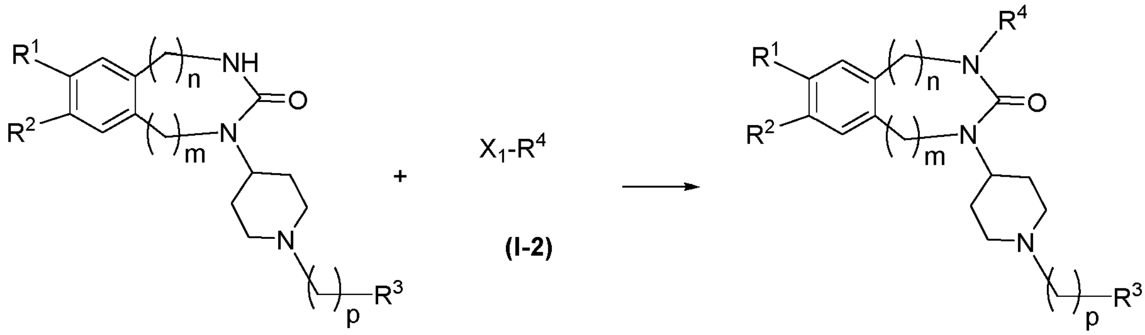

(I-7) (I-2) (I) ;

in the above preparation method, $X_1$ and $X_2$ each represent a leaving group, such as bromine, or the compound of Formula (I-5) is aldehyde, ketone, or boric acid; $Pr_1$ represents an amino protection group, such as tert-butyl oxycarbonyl, benzyloxycarbonyl, etc.

Brief Description of Drawings

The accompanying drawings are used to provide an understanding of the technical solutions of the present application, and constitute a part of the specification. They are used to explain the technical solutions of the present application together with the examples of the present application, and do not constitute a limitation to the technical solutions of the present application.

FIG. 1A is an analgesic time-effect curve of compound EX3 of the present application, in which experimental results are expressed as mean±standard deviation, compared with a control group, $^{###}p<0.001$, compared with a model group, $^{*}p<0.05$, $^{***}p<0.001$, differences between groups are compared by one-way analysis of variance Dunnett's, NS, not significant.

FIG. 1B is an analgesic dose-effect graph of compound EX3 of the present application, in which experimental results are expressed as mean±standard deviation, compared with a control group, $^{###}p<0.001$, compared with a model group, $^{*}p<0.05$, $^{***}p<0.001$, differences between groups are compared by one-way analysis of variance Dunnett's, NS, not significant.

FIG. 2 shows results of von Frey hairs and foot weight bearing experiment of compound EX3 of the present application; mechanical pain threshold (Von Frey) versus time curve (A) and relative analgesia rate (C); foot weight bearing difference versus time curve (B) and relative analgesia rate (D); in which results of the experiment are expressed as mean±standard deviation, compared with a control group (G1), $^{*}p<0.05$ means that the difference is significant, and $^{\&\&, \$\$, ##, **}p<0.01$ means that the difference is highly significant.

Detailed Description

In some embodiments, the present application provides a compound shown in Formula (I), and a stereoisomer, a

pharmaceutically acceptable salt, a solvate, a deuterate, a metabolite, or a prodrug thereof,

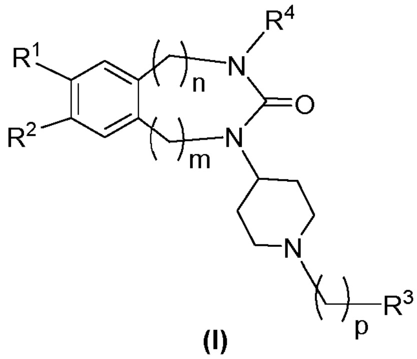

**(I)**

wherein,

n is 0 or 1;
m is 0 or 1;
p is 0, 1, or 2;
$R^1$ and $R^2$ are each independently selected from: hydrogen, halogen, $C_{1-3}$ alkyl, and $C_{1-3}$ alkoxy; provided that $R^1$ and $R^2$ are not hydrogen at the same time;
$R^3$ is selected from unsubstituted aryl, substituted aryl, unsubstituted heteroaryl, substituted heteroaryl, unsubstituted $C_{3-8}$ cycloalkyl, substituted $C_{3-8}$ cycloalkyl, unsubstituted $C_{4-6}$ heterocycloalkyl, and substituted $C_{4-6}$heterocycloalkyl; herein, substituted aryl, substituted heteroaryl, substituted $C_{3-8}$ cycloalkyl, or substituted $C_{4-6}$ heterocycloalkyl is substituted by 1-3 substituents independently selected from the following groups: halogen, $C_{1-4}$ alkyl, halogenated $C_{1-4}$ alkyl, $C_{1-4}$ alkoxy, halogenated $C_{1-4}$ alkoxy, aryl, and heteroaryl;
$R^4$ is selected from hydrogen, -$C_{1-3}$ alkyl-unsubstituted heterocycloalkyl, -$C_{1-3}$ alkyl-substituted heterocycloalkyl, -$C_{1-3}$ alkyl-substituted spiroheterocyclic alkyl, -$C_{1-3}$ alkyl-C(O)$NR^5R^6$, -$C_{1-3}$ alkyl-$NR^7R^8$, -$C_{1-4}$ alkyl unsubstituted-heteroaryl, and -$C_{1-4}$ alkyl-substituted heteroaryl; herein, $R^5$ and $R^6$ may be independently hydrogen or $C_{1-3}$ alkyl, or $R^5$ and $R^6$ together form $C_{4-6}$ unsubstituted heterocycloalkyl, or $R^5$ and $R^6$ together form $C_{4-6}$ substituted heterocycloalkyl; $R^7$ and $R^8$ may be independently hydrogen or $C_{1-3}$ alkyl, or $R^7$ and $R^8$ together form $C_{4-6}$ unsubstituted heterocycloalkyl, or $R^7$ and $R^8$ together form $C_{4-6}$ substituted heterocycloalkyl; provided that when $R^4$ is hydrogen, m is 0, and n is 1.

In some embodiments, in Formula (I), n is 0;

m is 0;

p is 0, 1, or 2.

In some embodiments, in Formula (I), n is 1;

m is 0;

p is 0, 1, or 2.

In some embodiments, in Formula (I), n is 0;

m is 1;

p is 0, 1, or 2.

In some embodiments, in Formula (I), $R^1$ and $R^2$ are each independently selected from: hydrogen, halogen, $C_{1-3}$ alkyl; provided that $R^1$ and $R^2$ are not hydrogen at the same time.
In some embodiments, in Formula (I), $R^1$ and $R^2$ are each independently selected from: hydrogen, chlorine, fluorine, $C_{1-3}$ alkyl; provided that $R^1$ and $R^2$ are not hydrogen at the same time.
In some embodiments, in Formula (I), $R^1$ and $R^2$ are each independently selected from: hydrogen, chlorine,

fluorine, methyl; provided that $R^1$ and $R^2$ are not hydrogen at the same time; optionally, $R^1$ and $R^2$ are both chlorine, or $R^1$ is chlorine and $R^2$ is fluorine, or $R^1$ is fluorine and $R^2$ is chlorine, or $R^1$ and $R^2$ are both fluorine, or $R^1$ is fluorine, methyl, or chlorine and $R^2$ is hydrogen, or $R^1$ is hydrogen and $R^2$ is chlorine or fluorine.

In some embodiments, in Formula (I), $R^3$ is selected from unsubstituted aryl, substituted aryl, unsubstituted heteroaryl, and substituted heteroaryl, herein unsubstituted aryl is phenyl or naphthyl; unsubstituted heteroaryl is furyl, thienyl, pyridyl, pyrrolyl, N-alkylpyrrolyl, pyrimidinyl, pyrazinyl, pyridazinyl, imidazolyl, pyrazolyl, triazolyl, or tetrazolyl; substituted aryl is phenyl or naphthyl substituted by 1-3 groups independently selected from the following: halogen, $C_{1-4}$ alkyl, halogenated $C_{1-4}$ alkyl, $C_{1-4}$ alkoxy, halogenated $C_{1-4}$ alkoxy, aryl, and heteroaryl; substituted heteroaryl is furyl, thienyl, pyridyl, pyrrolyl, N-alkylpyrrolyl, pyrimidinyl, pyrazinyl, pyridazinyl, imidazolyl, pyrazolyl, triazolyl, or tetrazolyl substituted by 1-2 groups independently selected from the following: halogen, $C_{1-4}$ alkyl, halogenated $C_{1-4}$ alkyl, $C_{1-4}$ alkoxy, halogenated $C_{1-4}$ alkoxy, aryl, and heteroaryl.

In some embodiments, in Formula (I), $R^3$ is phenyl, or phenyl substituted by 1-3 groups independently selected from the following: halogen, $C_{1-4}$ alkyl, halogenated $C_{1-4}$ alkyl, $C_{1-4}$ alkoxy, and halogenated $C_{1-4}$ alkoxy; or phenyl substituted by 1-3 groups independently selected from the following: fluorine, chlorine, bromine, iodine, $C_{1-4}$ alkyl, halogenated $C_{1-4}$ alkyl, $C_{1-4}$ alkoxy, and halogenated $C_{1-4}$ alkoxy.

In some embodiments, in Formula (I), $R^3$ is phenyl, 3,4-dichlorophenyl, 3,4-difluorophenyl, 2-chloro-4-fluorophenyl, 2-fluoro-4-chlorophenyl, 4-trifluoromethylphenyl, 4-trifluoromethoxyphenyl, 2,4-dichlorophenyl, 2,6-dichlorophenyl, 2,4-difluorophenyl, 2-chloro-4-methylphenyl, 2-methyl-4-fluorophenyl, 2-methyl-4-chlorophenyl, 2-methoxy-4-chlorophenyl, 2,4-dimethylphenyl, 2,6-dimethylphenyl, 2,4,6-trimethylphenyl, 4-tert-butylphenyl, 2-chlorophenyl, 2-methylphenyl, 2-fluorophenyl, 2-methoxyphenyl, 4-chlorophenyl, 4-fluorophenyl, or 4-methoxyphenyl.

In some embodiments, in Formula (I), $R^3$ is furyl, thienyl, pyridyl, pyrrolyl, N-alkylpyrrolyl, pyrimidinyl, pyrazinyl, pyridazinyl, imidazolyl, pyrazolyl, triazolyl, or tetrazolyl, optionally substituted by 1-2 groups independently selected from the following: fluorine, chlorine, bromine, iodine, $C_{1-4}$ alkyl, halogenated $C_{1-4}$ alkyl, $C_{1-4}$ alkoxy, and halogenated $C_{1-4}$ alkoxy; preferably, $R^3$ is 5-trifluoromethylpyridin-2-yl, or 5-chlorothiophen-2-yl.

In some embodiments, in Formula (I), $R^3$ is unsubstituted $C_{3-8}$ cycloalkyl, or substituted $C_{3-8}$ cycloalkyl, herein, $C_{3-8}$ cycloalkyl in unsubstituted $C_{3-8}$ cycloalkyl, substituted $C_{3-8}$ cycloalkyl is cyclopropyl, cyclobutyl, cyclopentyl, cyclohexyl, cycloheptyl, or cyclooctyl; substituted $C_{3-8}$ cycloalkyl is cyclopropyl, cyclobutyl, cyclopentyl, cyclohexyl, cycloheptyl, or cyclooctyl substituted by 1-3 groups independently selected from the following: fluorine, chlorine, bromine, iodine, $C_{1-4}$ alkyl, halogenated $C_{1-4}$ alkyl, $C_{1-4}$ alkoxy, halogenated $C_{1-4}$ alkoxy, and phenyl; preferably, substituted $C_{3-8}$ cycloalkyl is 4-tert-butylcyclohexyl, 4-isopropylcyclohexyl, 4-ethylcyclohexyl, 4-methylcyclohexyl, 4-trifluoromethylcyclohexyl, 2,3-dihydro-1H-inden-2-yl, or 2-chlorocyclohexyl.

In some embodiments, in Formula (I), $R^3$ is cyclopropyl, cyclopentyl, cyclohexyl, cycloheptyl, 4-tert-butylcyclohexyl, 4-isopropylcyclohexyl, 4-ethylcyclohexyl, 4-methylcyclohexyl, 4-trifluoromethylcyclohexyl, 2,3-dihydro-1H-inden-2-yl, or 2-chlorocyclohexyl.

In some embodiments, in Formula (I), $R^3$ is unsubstituted $C_{4-6}$ heterocycloalkyl or substituted $C_{4-6}$ heterocycloalkyl, herein, $C_{4-6}$ heterocycloalkyl in unsubstituted $C_{4-6}$ heterocycloalkyl or substituted $C_{4-6}$ heterocycloalkyl is tetrahydrofuranyl, tetrahydropyrrolyl, tetrahydrothienyl, piperidinyl, morpholinyl, or piperazinyl; substituted $C_{4-6}$ heterocycloalkyl is tetrahydrofuranyl, tetrahydropyrrolyl, tetrahydrothienyl, piperidinyl, morpholinyl, or piperazinyl substituted by 1-3 groups independently selected from the following: fluorine, chlorine, bromine, iodine, $C_{1-4}$ alkyl, halogenated $C_{1-4}$ alkyl, $C_{1-4}$ alkoxy, halogenated $C_{1-4}$ alkoxy, and phenyl; preferably, substituted $C_{4-6}$ heterocycloalkyl is *N*-isopropylpiperidin-4-yl.

In some embodiments, in Formula (I), $R^4$ is selected from: hydrogen, -$C_{1-3}$ alkyl-unsubstituted heterocycloalkyl selected from at least one of oxa and thia, -$C_{1-3}$ alkyl-substituted heterocycloalkyl selected from at least one of oxa and thia, -$C_{1-3}$ alkyl-substituted spiroheterocyclic alkyl, -$C_{1-3}$ alkyl-C(O)$NR^5R^6$, -$C_{1-3}$ alkyl-$NR^7R^8$, -$C_{1-4}$ alkyl-unsubstituted heteroaryl selected from at least one of aza, oxa, and thia, and -$C_{1-4}$ alkyl-substituted heteroaryl selected from at least one of aza, oxa, and thia; herein, $R^5$ and $R^6$ may be independently hydrogen or $C_{1-3}$ alkyl, or $R^5$ and $R^6$ and N connected thereto together form $C_{4-6}$ unsubstituted azacycloalkyl, or $R^5$ and $R^6$ and N connected thereto together form $C_{4-6}$ substituted azacycloalkyl; $R^7$ and $R^8$ may be independently hydrogen or $C_{1-3}$ alkyl, or $R^7$ and $R^8$ and N connected thereto together form $C_{4-6}$ unsubstituted azacycloalkyl, or $R^7$ and $R^8$ and N connected thereto together form $C_{4-6}$ substituted azacycloalkyl; provided that when $R^4$ is hydrogen, m is 0 and n is 1; the substitution means substitution by one or more of the following groups: C1-C4 alkyl, C1-C4 haloalkyl, C1-C4 alkoxy, C1-C4 haloalkoxy, C1-C4 alkanoyl, C1-C4 alkanoyloxy, hydroxyl, nitro, halogen, oxo and cyano.

In some embodiments, in Formula (I), $R^4$ is selected from hydrogen, m is 0, and n is 1.

In some embodiments, in Formula (I), $R^4$ is selected from: -$C_{1-3}$ alkyl-unsubstituted $C_{2-6}$ heterocycloalkyl selected from at least one of oxa and thia, -$C_{1-3}$ alkyl-substituted $C_{2-6}$ heterocycloalkyl selected from at least one of oxa and thia, -$C_{1-3}$ alkyl-substituted spiroheterocyclic alkyl, -$C_{1-3}$ alkyl-C(O)$NR^5R^6$, -$C_{1-3}$ alkyl-$NR^7R^8$, -$C_{1-4}$ alkyl-unsubstituted $C_{3-6}$ heteroaryl selected from at least one of aza, oxa, and thia, and -$C_{1-4}$ alkyl-substituted $C_{3-6}$ heteroaryl selected from at least one of aza, oxa and thia; herein, $R^5$ and $R^6$ may be independently hydrogen or $C_{1-3}$ alkyl, or $R^5$ and $R^6$ and N

connected thereto together form $C_{4-6}$ unsubstituted azacycloalkyl, or $R^5$ and $R^6$ and N connected thereto together form $C_{4-6}$ substituted azacycloalkyl; $R^7$ and $R^8$ may be independently hydrogen or $C_{1-3}$ alkyl, or $R^7$ and $R^8$ and N connected thereto together form $C_{4-6}$ unsubstituted azacycloalkyl, or $R^7$ and $R^8$ and N connected thereto together form $C_{4-6}$ substituted azacycloalkyl; the substitution means substitution by one or more of the following groups: C1-C4 alkyl, C1-C4 haloalkyl, C1-C4 alkoxy, C1-C4 haloalkoxy, C1-C4 alkanoyl, C1-C4 alkanoyloxy, hydroxyl, nitro, halogen, oxo and cyano.

In some embodiments, in Formula (I), $R^4$ is hydrogen, 2-(morpholinyl)ethyl, 2-(1,1-dioxothiomorpholine)ethyl, 2-(4-methylpiperazin-1-yl)ethyl, 2-(4-acetylpiperazin-1-yl)ethyl, 2-(3-oxopiperazin-1-yl)ethyl, 2-(pyrrolidin-1-yl)ethyl, 2-(piperidin-1-yl)ethyl, 2-(N,N'-dimethylamino)ethyl, 2-(2-oxopyrrolidin-1-yl)ethyl, *N,N'*-dimethylacetamide, oxiran-2-ylmethyl, or 2-(2-oxa-6-azaspiro[3.3]heptan-6-yl)ethyl; provided that when $R^4$ is hydrogen, m is 0, and n is 1.

In some embodiments, in Formula (I), $R^4$ is -$C_{1-3}$ alkyl-substituted spiroheterocyclic alkyl, and the substituted spiroheterocyclic alkyl may be 0 or 1 oxygenated azaspiro[3.3]heptane, azaspiro[3.5]nonane, azaspiro[3.4]octane, azaspiro[5.5]undecane, or azaspiro[4.5]decane.

Further, the compound of Formula (I) is a compound shown below, and a stereoisomer, pharmaceutically acceptable salt, solvate, deuterate, metabolite, or prodrug thereof:

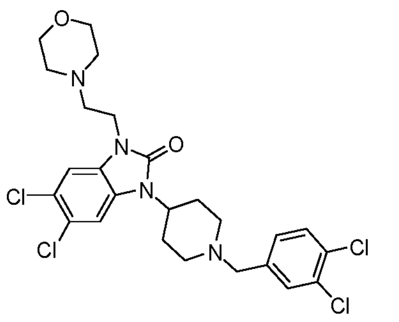

**EX1**

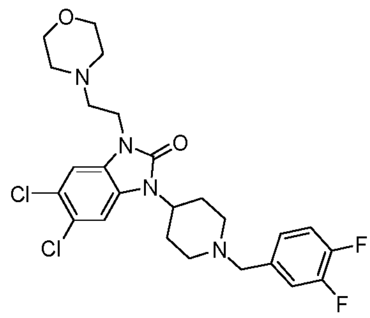

**EX2**

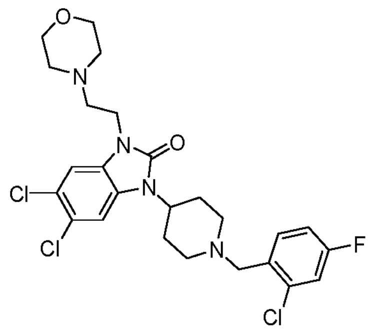

**EX3**

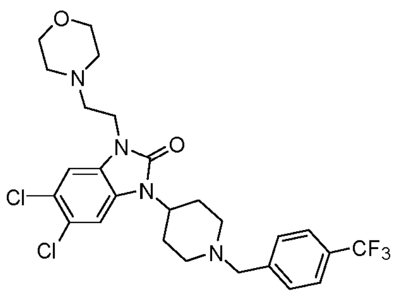

**EX4**

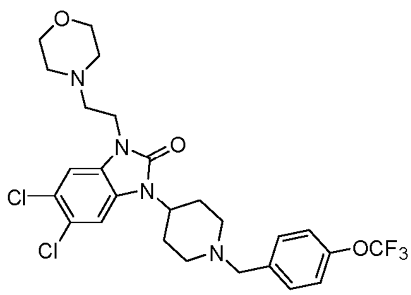

**EX5**

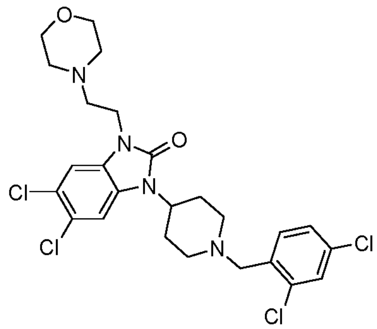

**EX6**

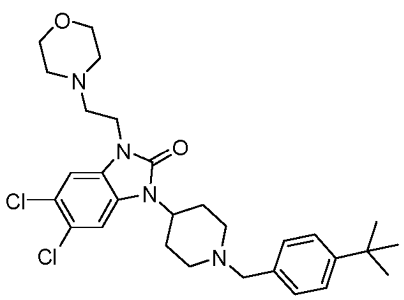

**EX7**

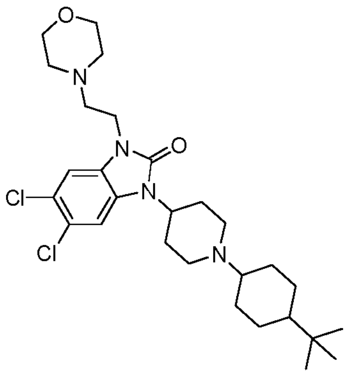

**EX8**

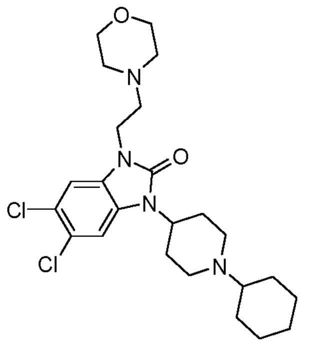

**EX9**

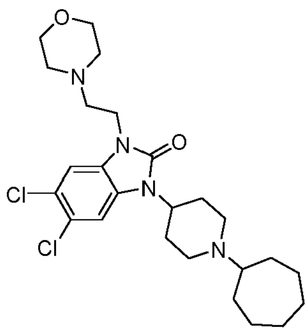

**EX10**

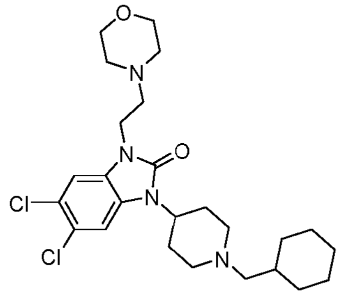

**EX11**

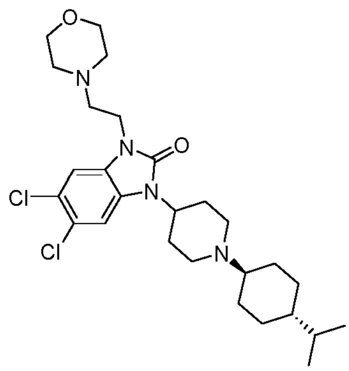

**EX12A**

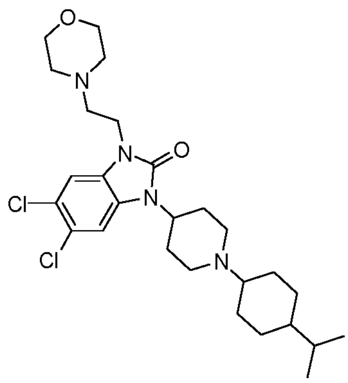

**EX12**

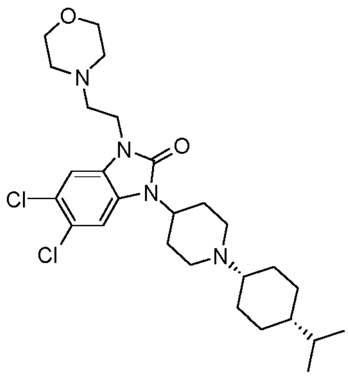

**EX12B**

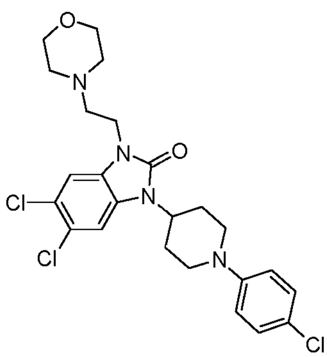

**EX13**

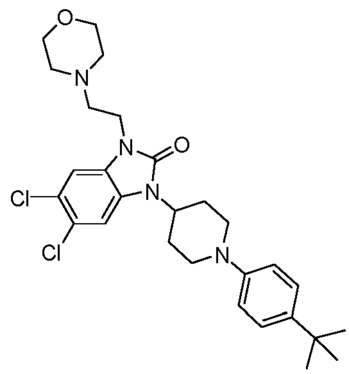

**EX14**

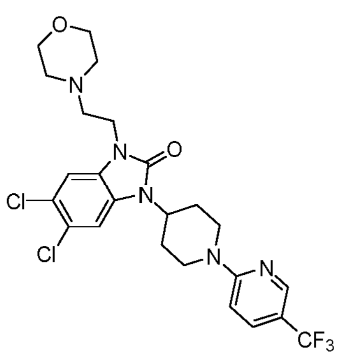

**EX15**

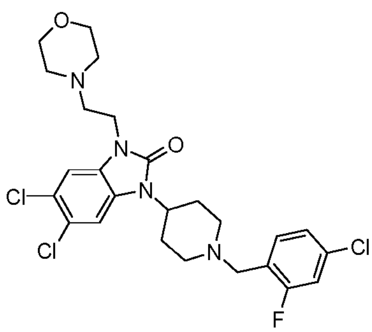

**EX16**

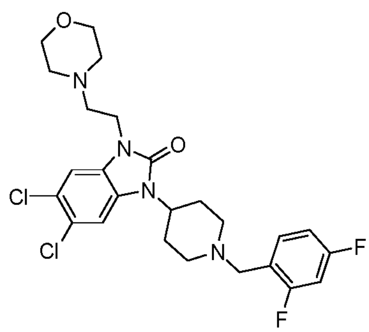

**EX17**

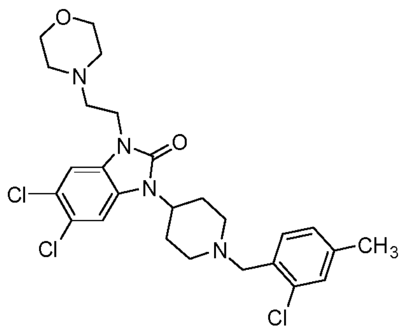

**EX18**

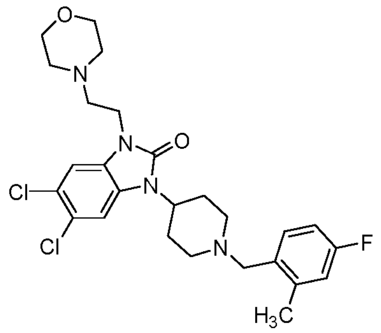

**EX19**

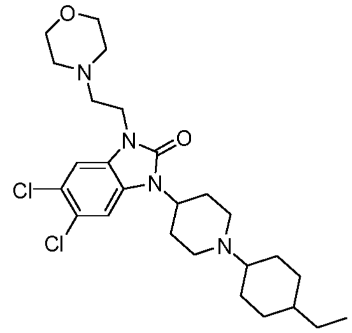

**EX20**

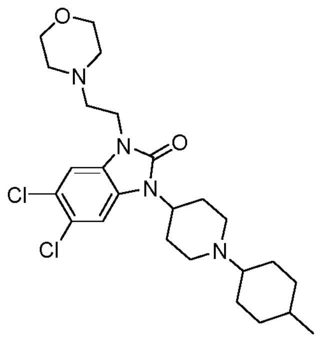

**EX21**

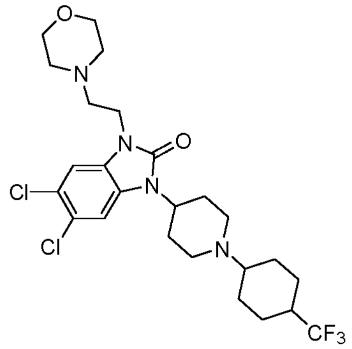

**EX22**

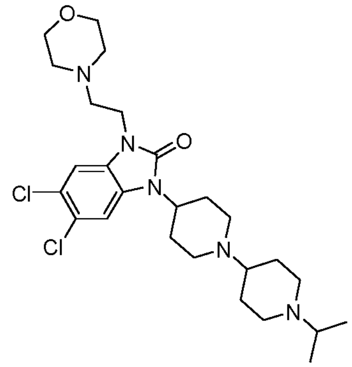

**EX23**

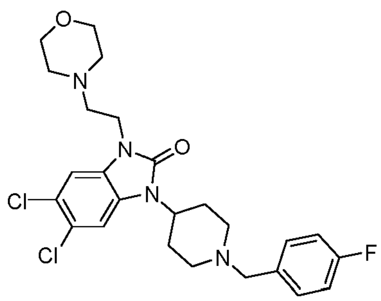

**EX24**

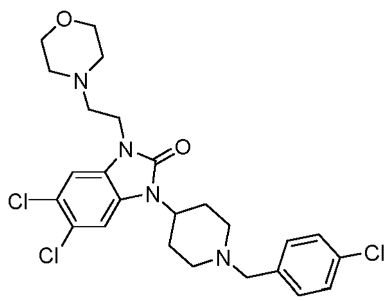

**EX25**

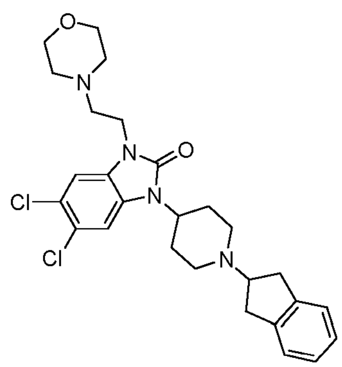

**EX26**

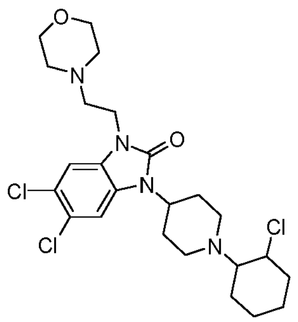

**EX27**

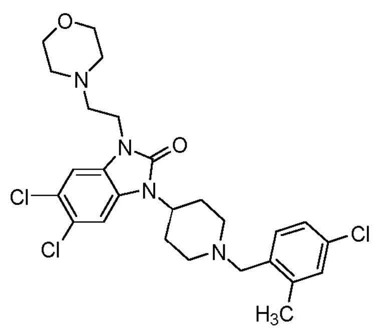

**EX28**

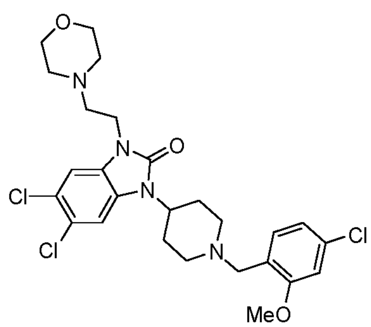

**EX29**

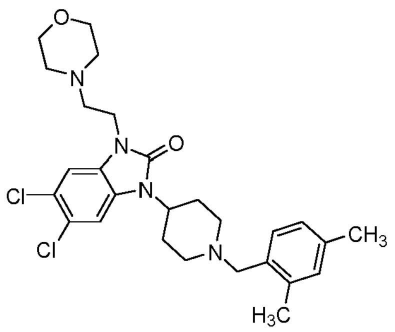

**EX30**

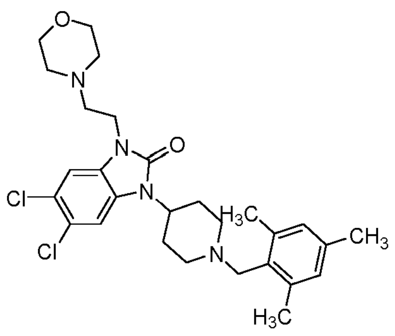

**EX31**

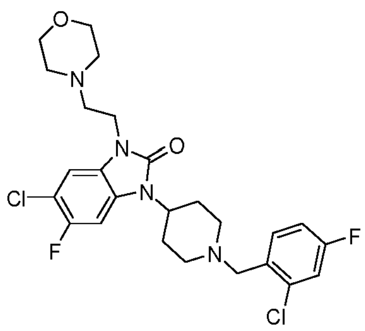

**EX33**

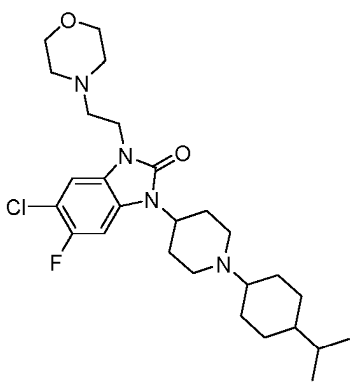

**EX34**

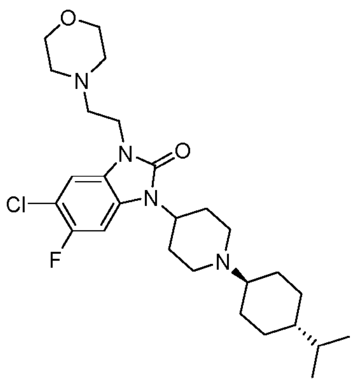

**EX34A**

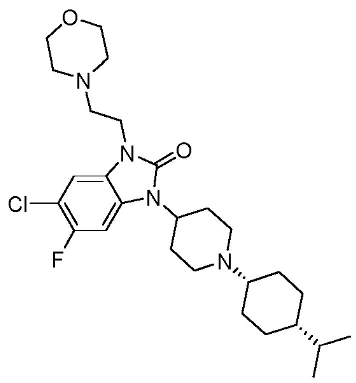

**EX34B**

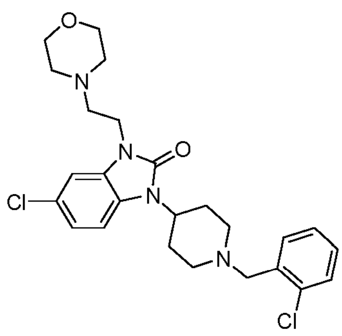

**EX32**

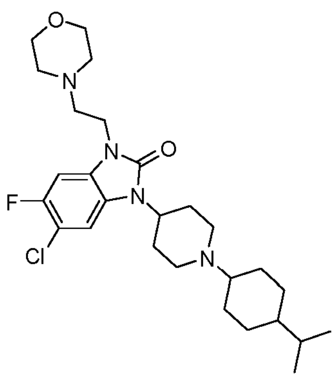

**EX36**

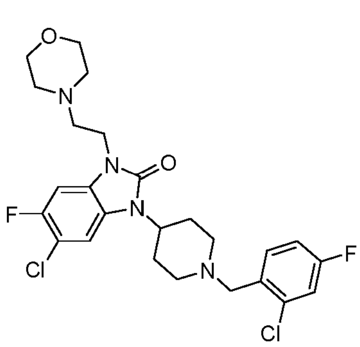

**EX35**

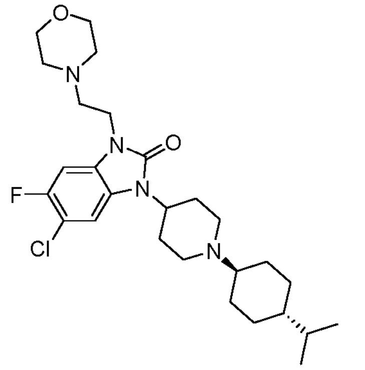

**EX36A**

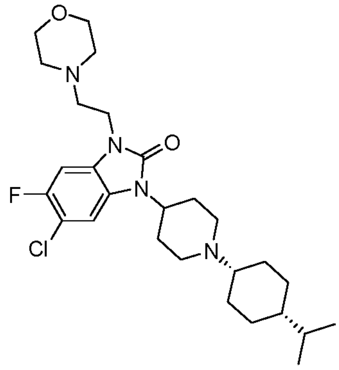

**EX36B**

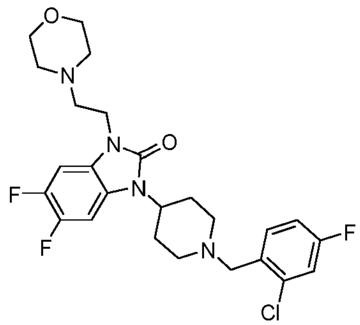

**EX37**

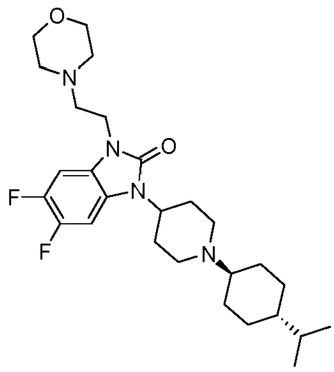

**EX38A**

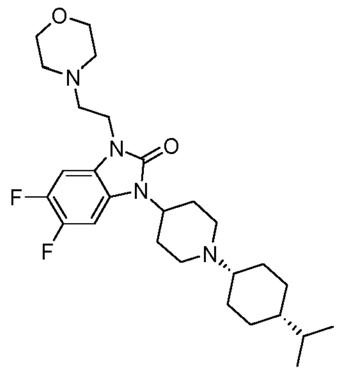

**EX38B**

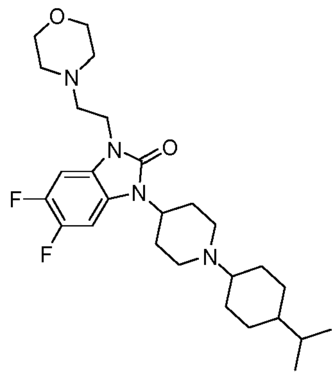

**EX38**

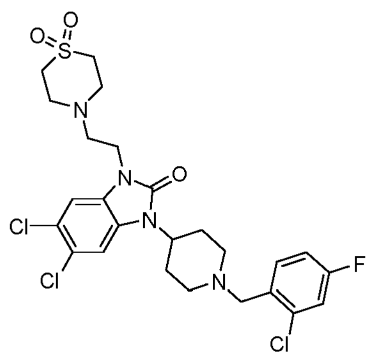

**EX39**

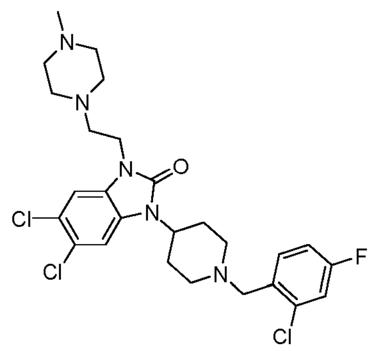

**EX40**

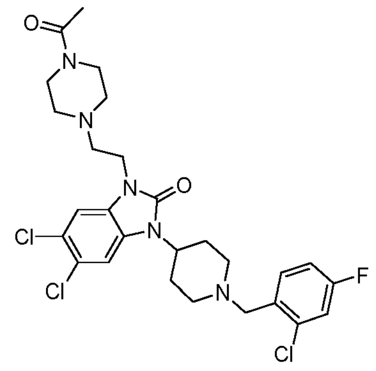

**EX41**

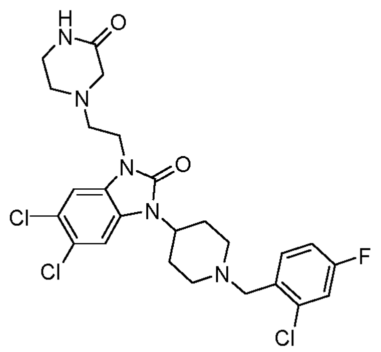

**EX42**

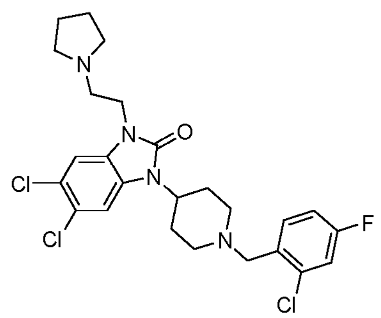

**EX43**

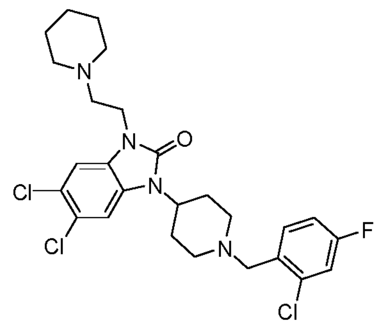

**EX44**

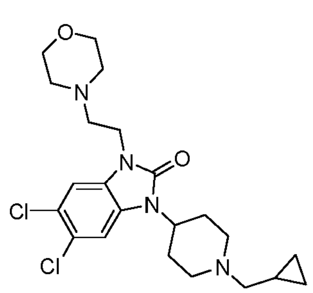

**EX45**

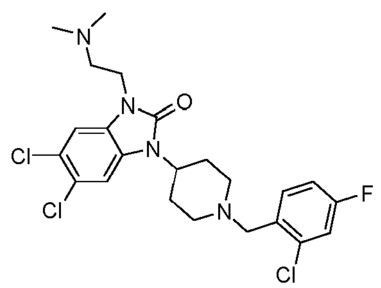

**EX46**

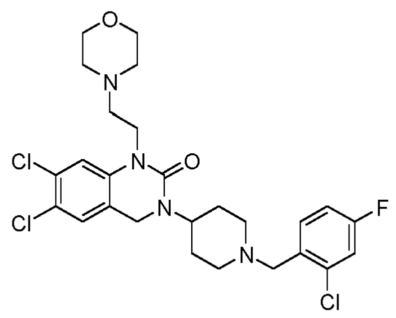

**EX47**

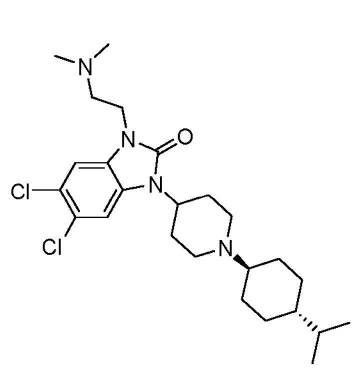

**EX48A**

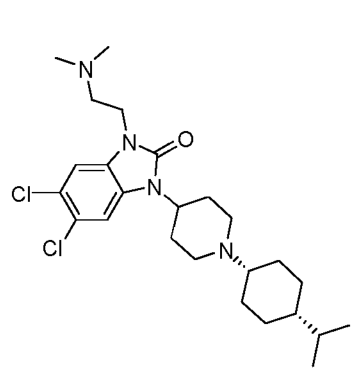

**EX48B**

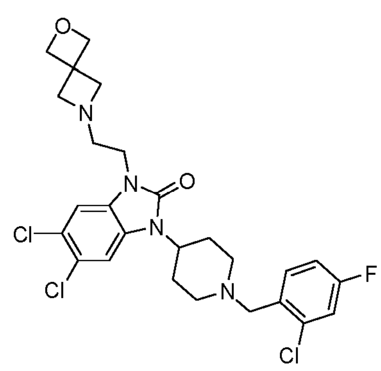

**EX49**

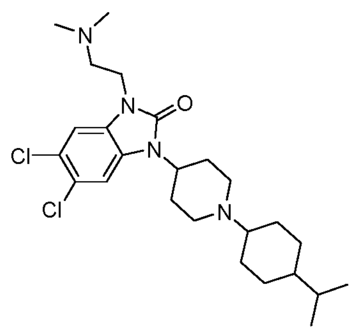

**EX48**

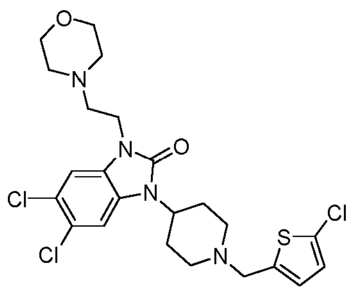

**EX50**

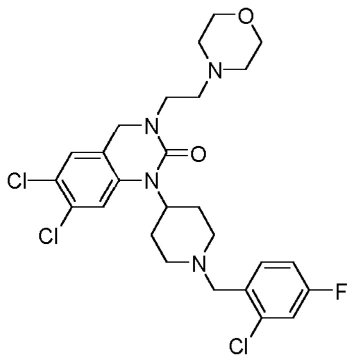

**EX51**

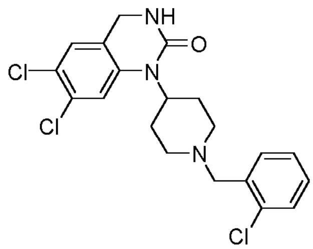

**EX52**

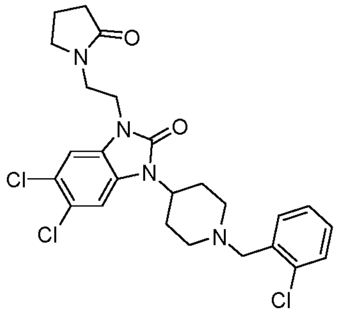

**EX53**

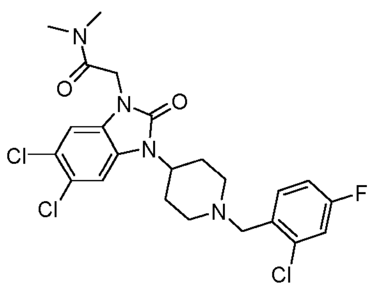

**EX54**

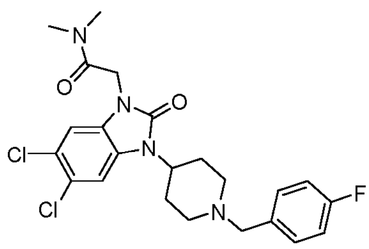

**EX55**

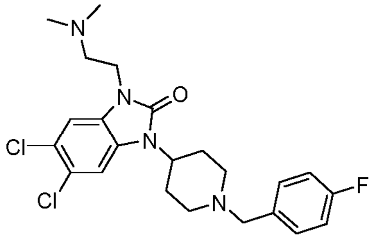

**EX56**

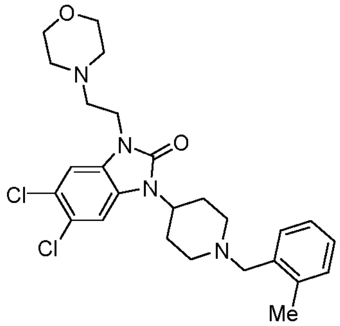

**EX57**

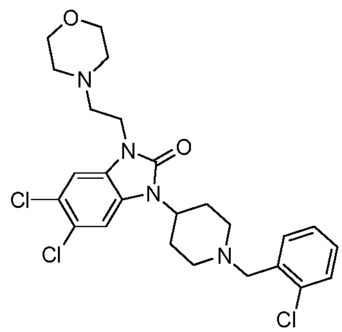

**EX58**

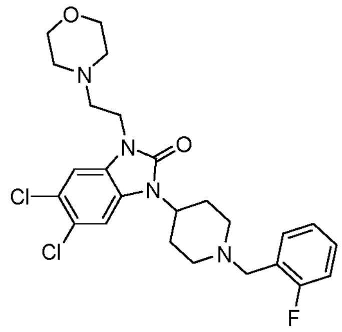

**EX59**

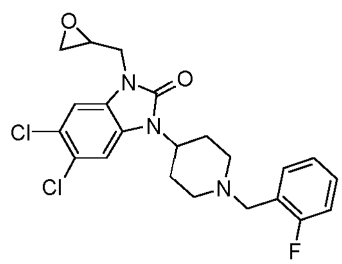

**EX60**

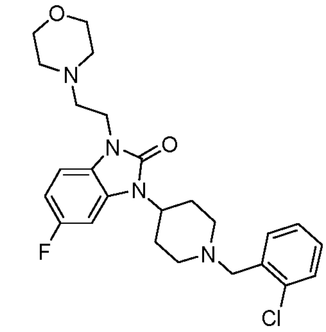

**EX61**

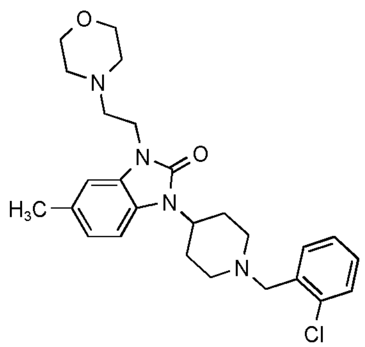

**EX62**

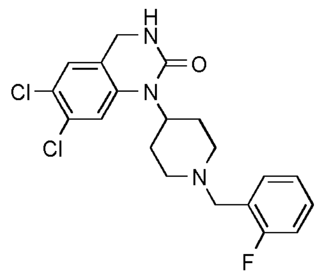

**EX63**

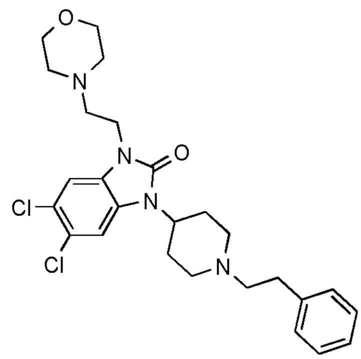

**EX64**

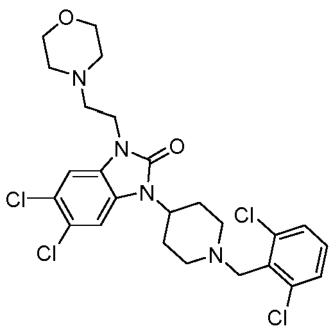

**EX65**

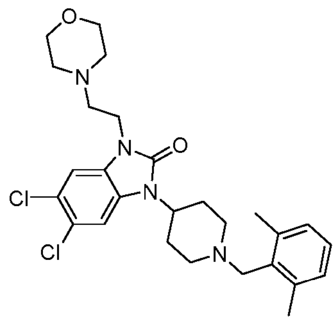

**EX66**

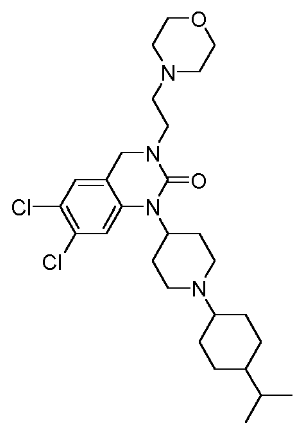

**EX67**

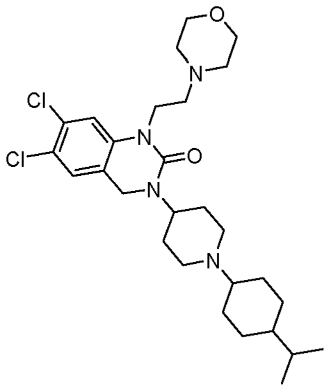

**EX68**

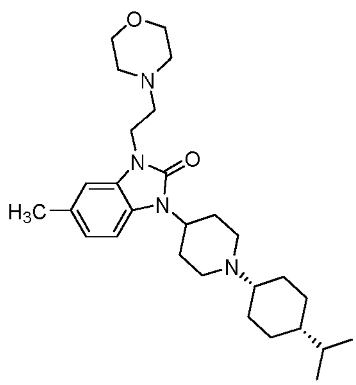

**EX69B**

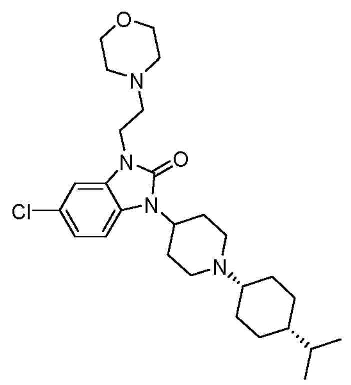

**EX70B**

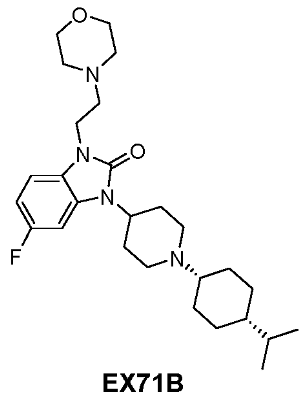

**EX71B**

.

In some embodiments, the present application further discloses a pharmaceutical composition, including: an effective dose of the piperidine derivative shown in General Formula (I) of the present application, and a stereoisomer, a pharmaceutically acceptable salt, a solvate, a deuterate, a metabolite, or a prodrug thereof, or further including one or more other therapeutic agents and a pharmaceutically acceptable carrier or excipient.

The pharmaceutically acceptable excipient(s) may, for example, be selected from carriers (e.g., solid, liquid, or semi-solid carriers), adjuvants, diluents (e.g., solid diluents such as fillers or filling agents; and liquid diluents such as solvents and co-solvents), granulants, binders, flow aids, coating agents, controlled release agents (e.g., lagging or delayed release polymers or waxes), disintegrants, buffers, lubricants, preservatives, antifungal agents and antibacterial agents, antioxidants, tension regulators, thickeners, flavor enhancers, sweeteners, pigments, plasticizers, taste masking agents, stabilizers, or any other excipients commonly used in pharmaceutical compositions.

As used herein, the term "pharmaceutically acceptable" means that the compounds, materials, compositions and/or dosage forms are suitable, within sound medical judgment, for exposure to the tissues of a subject (e.g., a human subject) without undue toxicity, irritation, allergy, or other problems or complications, commensurate with reasonable benefit/risk ratio. Each excipient must also be "acceptable" in the sense of compatibility with other components of the formulation.

Pharmaceutical compositions comprising compounds having Chemical Formula (I) may be formulated according to known techniques, referring to, for example, Remington's Pharmaceutical Sciences, Mack Publishing Company, Easton, PA, USA.

The pharmaceutical composition may be in any form suitable for oral, parenteral, topical, intranasal, endobronchial, sublingual, ophthalmic, otic, rectal, intravaginal, or transdermal administration.

Pharmaceutical dosage forms suitable for oral administration include tablets (coated or uncoated), capsules (hard shell or soft shell), caplets, pills, lozenges, syrups, solutions, powders, granules, elixirs and suspensions, sublingual tablets, or patches such as buccal patches.

In some embodiments, the pharmaceutical composition further includes one or more other therapeutic agents.

In some embodiments, the present application provides the piperidine derivative shown in General Formula (I) of the present application, and a stereoisomer, a pharmaceutically acceptable salt, a solvate, a deuterate, a metabolite, or a prodrug thereof, for use as a medicine.

In some embodiments of the present application, the present application provides the piperidine derivative shown in General Formula (I) of the present application, and a stereoisomer, a pharmaceutically acceptable salt, a solvate, a deuterate, a metabolite, or a prodrug thereof, for modulating a $\mu$-opioid peptide receptor (MOPR) and a $\kappa$-opioid peptide receptor (KOPR), or a $\mu$-opioid peptide receptor (MOPR) and a nociceptin opioid peptide receptor (NOPR)/opioid receptor like-1 (ORL-1) receptor.

In some embodiments of the present application, the present application provides the piperidine derivative shown in General Formula (I) of the present application, and a stereoisomer, a pharmaceutically acceptable salt, a solvate, a deuterate, a metabolite, or a prodrug thereof, for the treatment of pain, anxiety, depression, alcohol addiction, substance abuse/dependence.

In some embodiments, the present application further provides a method of using a piperidine derivative shown in General Formula (I), or a stereoisomer, a pharmaceutically acceptable salt, a solvate, a deuterate, a metabolite, or a prodrug thereof, or the composition of the present application in the treatment of pain, anxiety, depression, alcohol addiction, substance abuse/dependence. Pain may be selected from: acute pain, chronic pain, bone pain, joint pain, postoperative pain, muscle pain, toothache, headache, inflammatory pain, neuropathic pain, and Crohn's disease-related abdominal pain.

In some embodiments of the present application, the present application provides use of the piperidine derivative shown in General Formula (I) of the present application, or a stereoisomer, a pharmaceutically acceptable salt, a solvate, a

deuterate, a metabolite, or a prodrug thereof, or a pharmaceutical composition thereof in the preparation of drugs for the treatment of pain, anxiety, depression, alcohol addiction, substance abuse/dependence.

In some embodiments, the present application provides the above use, and the pain may be selected from: acute pain, chronic pain, bone pain, joint pain, postoperative pain, muscle pain, toothache, headache, inflammatory pain, neuropathic pain, and Crohn's disease-related abdominal pain.

In some embodiments, the present application provides a method for treating disorders in a patient co-mediated by a μ-opioid peptide receptor (MOPR) and a κ-opioid peptide receptor (KOPR), or co-mediated by a μ-opioid peptide receptor (MOPR) and a nociceptin opioid peptide receptor (NOPR)/opioid receptor like-1 (ORL-1) receptor, the method including administering the piperidine derivative shown in General Formula (I) of the present application, or a stereoisomer, a pharmaceutically acceptable salt, a solvate, a deuterate, a metabolite, or a prodrug thereof, or a pharmaceutical composition thereof to a patient in need thereof.

In some embodiments, the present application provides a method for treating or preventing pain, anxiety, depression, alcohol addiction, substance abuse/dependence in a patient, the method including administering the piperidine derivative shown in General Formula (I) of the present application, or a stereoisomer, a pharmaceutically acceptable salt, a solvate, a deuterate, a metabolite, or a prodrug thereof, or the pharmaceutical composition to a patient in need thereof. The pain may be selected from: acute pain, chronic pain, bone pain, joint pain, postoperative pain, muscle pain, toothache, headache, inflammatory pain, neuropathic pain, and Crohn's disease-related abdominal pain.

In some embodiments, the present application provides a method for modulating a μ-opioid peptide receptor (MOPR) and a κ-opioid peptide receptor (KOPR), or a μ-opioid peptide receptor (MOPR) and a nociceptin opioid peptide receptor (NOPR)/opioid receptor like-1 (ORL-1) receptor, the method including administering the piperidine derivative shown in General Formula (I) of the present application, or a stereoisomer, a pharmaceutically acceptable salt, a solvate, a deuterate, a metabolite, or a prodrug thereof, or the pharmaceutical composition.

In some embodiments, the present application provides a method for modulating a μ-opioid peptide receptor (MOPR) and a κ-opioid peptide receptor (KOPR), or a μ-opioid peptide receptor (MOPR) and a nociceptin opioid peptide receptor (NOPR)/opioid receptor like-1 (ORL-1) receptor to treat or prevent pain, anxiety, depression, alcohol addiction, substance abuse/dependence in a patient, the method including administering the piperidine derivative shown in General Formula (I) of the present application, or a stereoisomer, a pharmaceutically acceptable salt, a solvate, a deuterate, a metabolite, or a prodrug thereof, or the pharmaceutical composition to a patient in need thereof. The pain may be selected from: acute pain, chronic pain, bone pain, joint pain, postoperative pain, muscle pain, toothache, headache, inflammatory pain, neuropathic pain, and Crohn's disease-related abdominal pain.

In some embodiments, the present application provides a preparation method for the aforementioned piperidine derivative, or a stereoisomer, a pharmaceutically acceptable salt, a solvate, a deuterate, a metabolite, or a prodrug thereof, including the steps of:

(1) coupling a compound of Formula (I-1) with a compound of Formula (I-2) to obtain a compound of Formula (I-3);

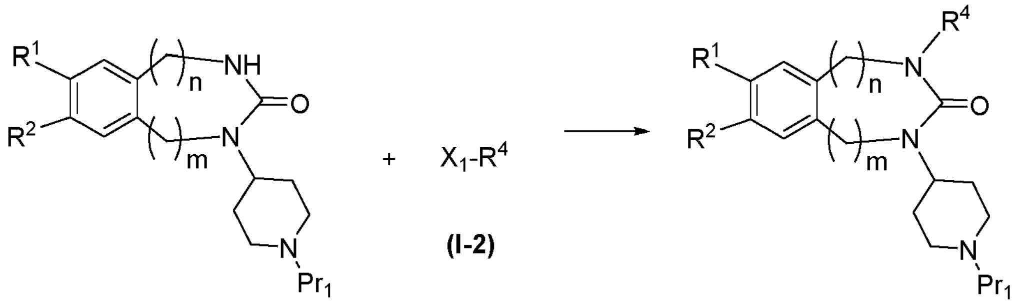

(I-1) (I-2) (I-3)

(2) subjecting the compound of Formula (I-3) to a deprotection reaction to obtain Formula (I-4), and then being coupled with a compound of Formula (I-5) to obtain a compound of Formula (I);

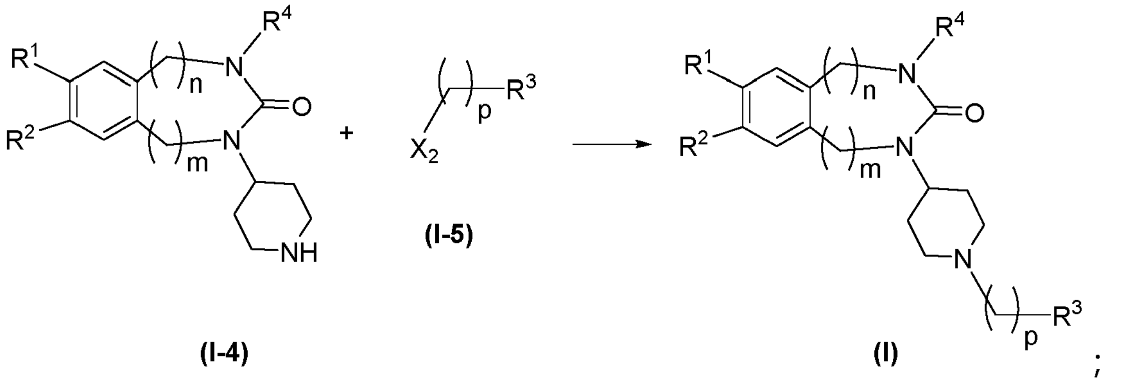

(I-4) (I-5) (I) ;

or,

(1') coupling a compound of Formula (I-6) with a compound of Formula (I-5) to obtain a compound of Formula (I-7);

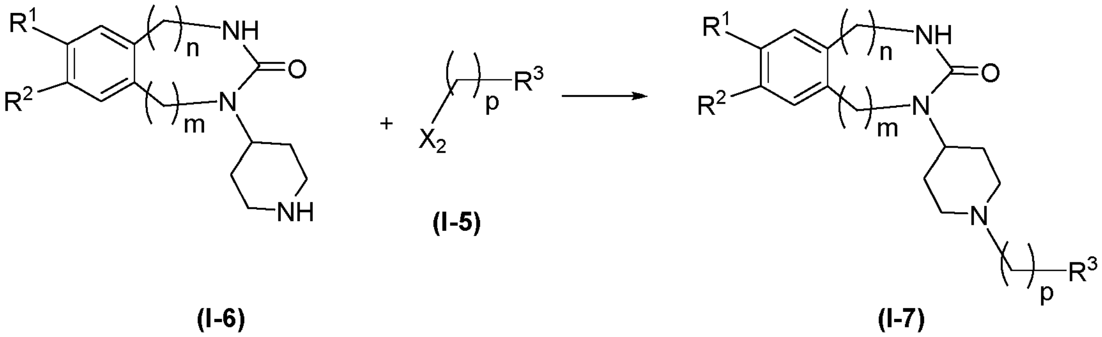

(I-6) (I-5) (I-7)

(2) coupling the compound of Formula (I-7) with the compound of Formula (I-2) to obtain a compound of Formula (I);

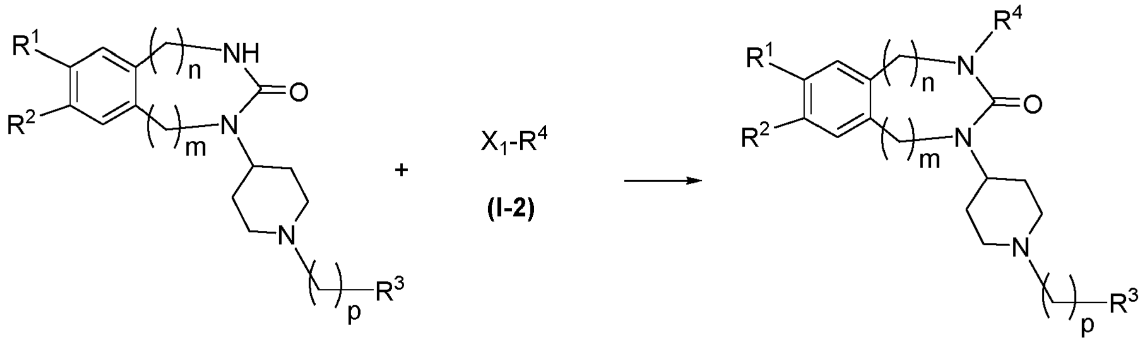

(I-7) (I-2) (I) ;

In the above preparation method, in Formulas (I-2) and (I-5), $X_1$ and $X_2$ each represents a leaving group, such as bromine, chlorine, or sulfonate group, or the compound of Formula (I-5) is aldehyde, ketone, or boric acid; in formulas (I-1) and (I-3), $Pr_1$ represents an amino protection group, such as tert-butyl oxycarbonyl, benzyloxycarbonyl CBz, or fluorenomethoxycarbonyl Fmoc, etc.; and the other groups in Formulas (I-1) to (I-7) and Formula (I) are defined as above.

Beneficial effect

The compound of the present application has good opioid receptor agonistic activity, is suitable for medicinal use, and has clinical application value. Moreover, the compound of the present application can be synthesized in simple steps, and thus has good economic utilization value.

Definition and description of terminology

Unless otherwise stated, the definitions of groups and terms set forth in the specification and claims of the present application include their definitions as examples, exemplary definitions, preferred definitions, definitions set forth in tables, definitions of specific compounds in examples, etc., and may be arbitrarily combined with one another. The so-combined group definitions and compound structures should fall within the scope described in the specification of the present application.

Carbon, hydrogen, oxygen, sulfur, nitrogen, or halogen involved in the groups and compounds of the present application all includes their isotopes, and carbon, hydrogen, oxygen, sulfur, nitrogen, or halogen involved in the groups and compounds of the present application are optionally further replaced by one or more of their corresponding isotopes, wherein isotopes of carbon include $^{12}C$, $^{13}C$, and $^{14}C$, isotopes of hydrogen include protium (H), deuterium (D, also known as heavy hydrogen), tritium (T, also known as superheavy hydrogen), isotopes of oxygen include $^{16}O$, $^{17}O$, and $^{18}O$, isotopes of sulfur include $^{32}S$, $^{33}S$, $^{34}S$, and $^{36}S$, isotopes of nitrogen include $^{14}N$ and $^{15}N$, isotopes of fluorine include $^{19}F$, isotopes of chlorine include $^{35}Cl$ and $^{37}Cl$, and isotopes of bromine include $^{79}Br$ and $^{81}Br$.

"Alkyl" refers to linear and branched monovalent saturated hydrocarbon groups, with the main chain including 1-10 carbon atoms. Examples of alkyl include, but are not limited to, methyl, ethyl, n-propyl, isopropyl, n-butyl, isobutyl, sec-butyl, *tert-butyl,* n-pentyl, 2-pentyl, 3-pentyl, 2-methyl-2-butyl, 3-methyl-2-butyl, n-hexyl, n-heptyl, n-octyl, n-nonyl, n-decyl, etc.; and alkyl may be further substituted with any substituent.

"Cycloalkyl" refers to monovalent saturated carbocyclic hydrocarbon groups, monocyclic, typically having 3-10 carbon atoms. Non-limiting examples include cyclopropyl, cyclobutyl, cyclopentyl, cyclohexyl, cycloheptyl, etc. Cycloalkyl may optionally be further substituted with any substituent.

"Heterocyclic alkane" refers to saturated cyclic hydrocarbon groups containing at least one heteroatom, monocyclic, heteroatom being N, O, S, P, and their oxidized forms. Non-limiting examples include aziridinyl, oxiranyl, thiiranyl, azetidinyl, oxetanyl, thietanyl, pyrrolidinyl, pyrazolidinyl, tetrahydrofuranyl, tetrahydrothienyl, etc. Heterocyclic alkane may optionally be further substituted with any substituent.

"Aryl" refers to 6 to 14-membered all-carbon monocyclic or fused polycyclic (i.e. a ring sharing an adjacent pair of carbon atoms) groups having a conjugated $\pi$-electron system, preferably 6 to 10-membered, e.g., phenyl and naphthyl. The aryl ring includes the aryl ring described above fused to a heteroaryl, heterocyclic or cycloalkyl ring, wherein the ring connected to the parent structure is an aryl ring. Aryl may be substituted or unsubstituted, when it is substituted, the substituent may be substituted at any usable connection site, and the substituent is preferably independently and optionally substituted with one or more substituents selected from hydrogen atom, halogen, alkyl, alkoxy, haloalkyl, hydroxyl, hydroxyalkyl, cyano, amino, nitro, cycloalkyl, heterocyclic, aryl, and heteroaryl.

"Heteroaryl" refers to a heteroaromatic system including 1 to 4 heteroatoms and 5 to 14 ring atoms, wherein the heteroatoms are selected from oxygen, sulfur, and nitrogen. Heteroaryl is preferably 5 to 10-membered (e.g., 5, 6, 7, 8, 9, or 10-membered), more preferably 5 or 6-membered, e.g., furyl, thienyl, pyridyl, pyrrolyl, N-alkylpyrrolyl, pyrimidinyl, pyrazinyl, pyridazinyl, imidazolyl, pyrazolyl, triazolyl, tetrazolyl, etc. The heteroaryl ring includes heteroaryl described above fused to an aryl, heterocyclic, or cycloalkyl ring, wherein the ring connected to the parent structure is a heteroaryl ring.

"Pharmaceutically acceptable salts" refer to those salts which maintain the biological effectiveness and properties of free acids or free bases, and where the free acids are obtained by reaction with non-toxic inorganic or organic bases, or where the free acids are obtained by reaction with non-toxic inorganic or organic acids.

"Carriers" refer to vehicles or diluents that do not cause significant irritation to an organism and do not eliminate bioactivity and properties of a given compound.

"Excipients" refer to inert substances added to pharmaceutical compositions to further depend on compounds for administration. Examples of excipients include, but are not limited to, calcium carbonate, calcium phosphate, various sugars and different types of starch, cellulose derivatives (including microcrystalline cellulose), gelatin, vegetable oils, polyethylene glycols, diluents, granulants, lubricants, binders, disintegrants, etc.

"Prodrugs" refer to compounds that can be converted to biologically active compounds of the present application under physiological conditions or under the action of a solvent. The prodrug of the present application is prepared by modifying the phenolic group in the compound, and the modification may be removed by conventional operations or in vivo to give the parent compound. When the prodrug of the present application is administered to a mammalian individual, the prodrug is fragmented to form free hydroxyl groups. Examples of prodrugs include, but are not limited to, phenolic hydroxyl and phosphate sodium salt derivatives of the compounds of the present application.

"Effective dose" refers to an amount of a compound that causes physiological or medical response in a tissue, system or subject, and this amount is a desirable amount, including an amount of the compound that, when administered to a subject, is sufficient to prevent or mitigate to some extent one or more symptoms of the disease or condition under treatment.

"Solvates" refer to the compounds of the present application or salts thereof, and they also include stoichiometric

or non-stoichiometric solvents that bind by intermolecular non-covalent forces. When the solvent is water, it is hydrate.

"Optional" or "optionally" means that the subsequently described event or environment may, but not necessarily occur, and the description includes occasions where the event or environment occurs or does not occur. For example, "alkyl optionally substituted with F" means that alkyl may, but does not have to, be substituted with F, and the description includes cases where alkyl is substituted with F and cases where alkyl is not substituted with F.

Additional features and advantages of the present application will be set forth in the description which follows, and in part will become apparent from the description, or may be learned by practice of the present application. The objects and other advantages of the present application may be realized and obtained by the structure particularly indicated in the description, the claims, and the accompanying drawings.

**Specific embodiments**

To further clarify the purpose, technical solutions, and advantages of the present application, examples of the present application will be described in detail below. It should be noted that the examples of the present application and the features of the examples may be arbitrarily combined with each other provided that there is no conflict.

The compound of the general formula of the present application, and the preparation method and use thereof will be described in further detail below with reference to specific examples. The following examples are intended to illustrate and explain the present application only and should not be construed as limiting the protection scope of the present application. All techniques implemented based on the foregoing contents of the present application are covered within the scope of the present application which is intended to be protected.

Unless otherwise stated, the raw materials and reagents used in the following examples are all commercially available, or may be prepared by known methods. The following abbreviations are used in the present application: equiv. represents equivalent ratio; sat. represents saturation; M represents mol/L; r.t represents room temperature; EtOH represents ethanol; MeOH represents methanol; DCM represents dichloromethane; DCE represents 1,2-dichloroethane; DMSO represents dimethyl sulfoxide; DMF represents *N,N*-dimethylformamide; EA represents ethyl acetate; PE represents petroleum ether; THF represents tetrahydrofuran; TEA represents triethylamine; TFA represents trifluoroacetic acid; AcOH represents acetic acid; CDI represents carbonyl diimidazole; Boc represents tert-butoxycarbonyl (an amino protection group); NaBH $(OAc)_3$ represents sodium triacetoxy borohydride; $Boc_2O$ represents di-tert-butyl dicarbonate; AIBN represents azobisisobutyronitrile; HMPA represents hexamethylphosphoryl triamine; MsCl represents methyl sulfonyl chloride; NBS represents N-bromosuccinimide; and TMS represents trimethylsilane.

Compounds are named according to conventional nomenclature in the art or using ChemDraw® software (PerkinElmer), and commercial reagents adopt the names in supplier catalogs.

$^1H$ NMR data is collected and recorded at 400 MHz by Bruker Ultrashield 400 NMR instrument, and chemical shift $\delta$ value (ppm) is reported using $CDCl_3$, $CD_3OD$, $D_2O$, or DMSO-$d_6$ as a solvent and TMS ($\delta$=0) as internal standard. Mass spectrum is collected and recorded by Shimadzu LCMS-2020 liquid chromatography-mass spectrometry system, and detected by Shimadzu shim-pack VP-ODS (150L*2.0, 4.6 $\mu$M) chromatographic column. Mobile phase A is an aqueous solution of 0.1% trifluoroacetic acid, and mobile phase B is acetonitrile. High Performance Liquid Chromatography (HPLC) is measured by Agilent Technologies 1260 Infinity liquid chromatograph, and detected using YMC Triart C18 EXRS (4.6*150 mm, 3 $\mu$m) chromatographic column. Conditions of gradient elution: at a flow rate of 1.0 mL/min, 30-10% solvent A1 and 70-90% solvent B1, and then 90% B1 and 10% A1, maintaining for 0.5 min, with the percentage being the volume percentage of a given solvent based on the total solvent volume. Solvent A1: an aqueous solution of 0.1% ammonia water; and solvent B1: acetonitrile. Percentage is the volume percentage of a solute in a solution; injection volume: determined by the concentration of reaction solution, for a general sample with the concentration of 0.2 mg/mL, 2 $\mu$L is injected; detection wavelength: 254/220 nm; and the temperature of chromatographic column: 30°C.

**Example 1:** Synthesis of 5,6-dichloro-1-(1-(3,4-dichlorobenzyl)piperidin-4-yl) -3-(2-morpholinoethyl)-1,3-dihydro-2H-benzo[d]imidazol-2-one (compound EX1)

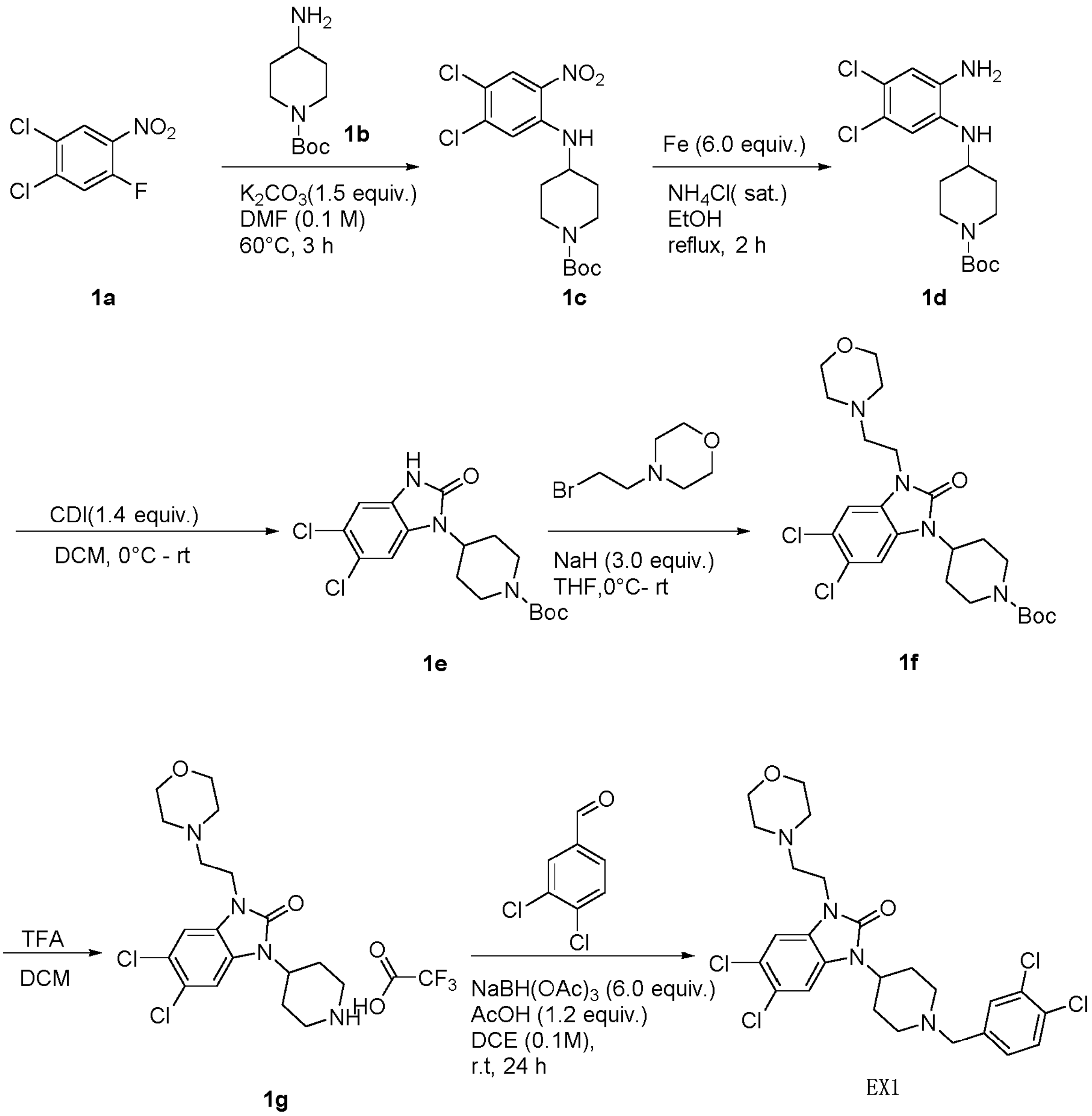


Synthesis of intermediate **1c**: 4,5-dichloro-2-fluoronitrobenzene (**1a**) (10.00 g, 47.62 mmol), N-Boc-4-aminopiperidine (9.54 g, 47.62 mmol) and solvent DMF (120 mL) were added into a clean dry single-neck flask (250 mL), and anhydrous $K_2CO_3$ (9.87 g, 71.43 mmol) was added to the flask. The mixture was heated to 60°C and stirred for reaction for 2 hours. The reaction of the raw materials was detected, by TLC plate, to be substantially complete. After the completion of the reaction, the reaction system was cooled to room temperature, and the reaction solution was slowly poured into an ice-water mixture (500 mL) under stirring condition. A large amount of yellow solid was precipitated and filtered, and the filter cake was washed with a small amount of water, and dried to obtain 17.58 g yellow solid product, with a yield of 94.59%. $^1H$ NMR (400 MHz, chloroform-d) δ 8.30 (s, 1H), 8.01 (d, *J* = 7.4 Hz, 1H), 6.98 (s, 1H), 4.03 (d, *J* = 13.3 Hz, 2H), 3.68 - 3.53 (m, 1H), 3.06 (t, *J* = 12.3 Hz, 2H), 2.05 (d, *J* = 13.0 Hz, 2H), 1.59 - 1.48 (m, 2H), 1.48 (s, 9H).

Synthesis of intermediate **1d**: *tert-butyl* 4-((4,5-dichloro-2-nitrophenyl) amino)piperidin-1-carboxylate (1c) (17.50 g, 44.84 mmol) was added into a single-neck flask (1 L), and anhydrous ethanol (450 mL) was added to the reaction flask. Iron powder (15.03 g, 269.05 mmol) and saturated $NH_4Cl$ aqueous solution (45 mL) were added to the reaction system under stirring at room temperature. The mixture was heated to 80°C, and stirring was continued for reaction for 1.5 hours. The reaction of the raw materials was detected, by TLC plate, to be substantially complete. Filtration was performed using diatomite, the filter cake was washed with a small amount of anhydrous ethanol, and the filtrate was evaporated under reduced pressure using a rotary evaporator to remove the solvent to obtain a white solid. The solid was dissolved in ethyl acetate (350 mL), and after washing with water and saturated NaCl aqueous solution, the organic phase was separated therefrom, which was dried with anhydrous $Na_2SO_4$ and then filtered. The filtrate was evaporated under reduced pressure using a rotary evaporator to remove the solvent to obtain a gray-white solid crude product. Petroleum

ether (200 mL) was added to the crude product, followed by dispersing, slurrying, filtering, and drying to obtain 14.23 g orange solid product, with a yield of 88.08%. $^1$H NMR (400 MHz, chloroform-d) δ 6.78 (s, 1H), 6.66 (s, 1H), 4.04 (s, 2H), 3.52 - 3.16 (m, 4H), 2.95 (t, *J* = 12.5 Hz, 2H), 2.08 - 1.96 (m, 2H), 1.47 (d, *J* = 1.5 Hz, 9H), 1.43 - 1.30 (m, 2H).

Synthesis of intermediate **1e**: *tert-butyl* 4-((2-amino-4,5-dichlorophenyl)amino) piperidin-1-carboxylate (**1d)** (14.20 g, 39.41 mmol) and anhydrous tetrahydrofuran (300 mL) were added into a clean dry three-necked flask (500 mL), under argon displacement protection, the flask was cooled to 0°C in an ice water bath, a dichloromethane solution (50 mL) of N,N'-carbonyldiimidazole (8.31 g, 51.24 mmol) was added dropwise, thereafter, the ice water bath was removed, the flask was stirred at room temperature for 1 hour, and heated to 35°C,stirring was performed for reaction for 18 hours, and the reaction of the raw materials was detected, by TLC plate, to be substantially complete. Saturated $NaHCO_3$ aqueous solution (150 mL) was slowly added to the reaction system under stirring condition, followed by extraction with ethyl acetate (250 mL×3). The organic phase was dried through anhydrous $Na_2SO_4$ and then filtered. The filtrate was evaporated under reduced pressure using a rotary evaporator to remove the solvent to obtain a gray-white solid crude product. Petroleum ether (250 mL) and ethyl acetate (2.5 mL) were added to the crude product, followed by dispersing, slurrying, filtering, and drying to obtain 14.50 g gray-white solid product, with a yield of 95.22%. $^1$H NMR (400 MHz, chloroform-d) δ 10.66 (s, 1H), 7.23 (s, 1H), 7.20 (s, 1H), 4.50 - 4.22 (m, 3H), 2.97 - 2.77 (m, 2H), 2.35 - 2.17 (m, 2H), 1.88 - 1.76 (m, 2H), 1.53 (s, 9H).

Synthesis of intermediate **1g**: *tert-butyl* 4-(4,5-dichloro-2-carbonyl-2,3-dihydro-1H-benzo[d]imidazol-1-yl)piperidin-1-carboxylate (**1e**) (5.00 g, 12.94 mmol) was added into a dry three-necked round-bottom flask (250 mL), anhydrous tetrahydrofuran (130 mL) was added thereto, under argon displacement protection, the flask was cooled to 0°C in an ice water bath, NaH (2.59 g, 64.72 mmol, 60% in mineral oil) was added in batches to the reaction mixture, thereafter, stirring was performed for reaction for 30 minutes, 4-(2-bromoethyl)morphinoline hydrobromide (4.81 g, 17.47 mmol) was slowly added to the reaction mixture, thereafter, stirring was continued for reaction for 1 hour, the ice water bath was removed, the flask was heated slowly to 35°C, and stirred for reaction for 18 hours, and the reaction of the raw materials was detected, by TLC plate, to be substantially complete. Under the conditions of cooling in an ice water bath and stirring, ice water was added dropwise to the reaction system for quenching the reaction, followed by extraction with ethyl acetate (150 mL×3). The organic phase was dried through anhydrous $Na_2SO_4$ and then filtered. The filtrate was evaporated under reduced pressure using a rotary evaporator to remove the solvent to obtain a gray-white solid crude product. Petroleum ether (150 mL) was added to the crude product, followed by dispersing, stirring, filtering, and drying to obtain 5.85 g white solid product, with a yield of 90.49%. $^1$H NMR (400 MHz, chloroform-*d*) δ 7.17 (s, 1H), 7.14 (s, 1H), 4.47 - 4.19 (m, 3H), 3.95 (t, *J* = 6.6 Hz, 2H), 3.74 - 3.60 (m, 4H), 2.85 (t, *J* = 12.5 Hz, 2H), 2.65 (t, *J* = 6.6 Hz, 2H), 2.59 - 2.44 (m, 4H), 2.24 (qd, *J*= 12.8, 4.5 Hz, 2H), 1.80 (d, *J*= 12.1 Hz, 2H), 1.51 (s, 9H).

*Tert-butyl* 4-(5,6-dichloro-3-(2-morpholinoethyl)-2-carbonyl-2,3-dihydro-1H-benzo[*d*] imidazol-1-yl)piperidin-1-carboxylate (1f) (5.85 g, 11.71 mmol) was added into a dry single neck flask (100 mL), anhydrous dichloromethane (60 mL) was added thereto, while the flask was cooled in an ice water bath, trifluoroacetic acid (15 mL) was added dropwise thereto, thereafter, the ice water bath was removed, stirring was performed at room temperature for reaction for 3 hours, and the reaction of the raw materials was detected, by TLC plate, to be substantially complete. The solvent was removed by evaporation under reduced pressure with a rotary evaporator to obtain a light brown oil crude product. Ether (150 mL) was added to the crude product, followed by dispersing, stirring, filtering, and drying to obtain 6.01 g white solid product, with a yield of 99.95%. The white solid did not need further purification, and could be directly used in the next experiment.

Synthesis of compound EX1: 5,6-dichloro-1-(2-morpholinoethyl)-3-(piperidin-4-yl) -1,3-dihydro-2H-benzo[d] imidazol-2-one trifluoroacetate (1g) (100 mg, 195 μM) and anhydrous 1,2-dichloroethane (2 mL) were added into a dry Schlenk tube, dry triethylamine (19.7 mg, 195 μmol, 27 μL) was added thereto, and stirred for 5 minutes, and 3,4-dichlorobenzaldehyde (51 mg, 292 μmol), acetic acid (14 mg, 234 μmol, 14 μL), $NaBH(OAc)_3$ (1.17 mmol, 248 mg), and 4Å molecular sieve (100 mg) were added to the reaction tube. Under argon protection, stirring was performed at room temperature for reaction for 24 hours. The reaction of the raw materials was detected, by TLC plate, to be substantially complete. Saturated sodium bicarbonate aqueous solution was added to the reaction flask for quenching the reaction, followed by extraction with dichloromethane for three times, combining the organic phases, washing with saturated saline, drying with anhydrous sodium sulfate, filtering, and evaporation under reduced pressure by a rotary evaporator to remove the solvent to obtain a crude product, which was separated and purified by silica gel chromatography column (mobile phase was dichloromethane and methanol, in a ratio of 150:1 to 70:1) to obtain 55 mg white solid product, with a yield of 50.6%. LC-MS(ESI) m/z: 557.10 $(M+H)^+$; $^1$H NMR (400 MHz, chloroform-*d*) δ 7.48 (s, 1H), 7.41 (d, *J* = 8.2 Hz, 1H), 7.30 (s, 1H), 7.22 (d, *J* = 8.0 Hz, 1H), 7.12 (s, 1H), 4.34 - 4.22 (m, 1H), 3.94 (t, *J* = 6.6 Hz, 2H), 3.67 (t, *J* = 4.6 Hz, 4H), 3.51 (s, 2H), 3.01 (d, *J* = 11.2 Hz, 2H), 2.64 (t, *J* = 6.6 Hz, 2H), 2.52 (t, *J* = 4.6 Hz, 4H), 2.39 (q, *J* = 11.1 Hz, 2H), 2.17 (t, *J* = 11.7 Hz, 2H), 1.78 (d, *J* = 11.7 Hz, 2H).

**Example** ***2:*** Synthesis of 5,6-dichloro-1-(1-(3,4-difluorobenzyl) piperidin-4-yl)-3-(2-morpholinoethyl)-1,3-dihydro-2*H*-benzo[*d*]imidazol-2-one (compound ***EX2)***

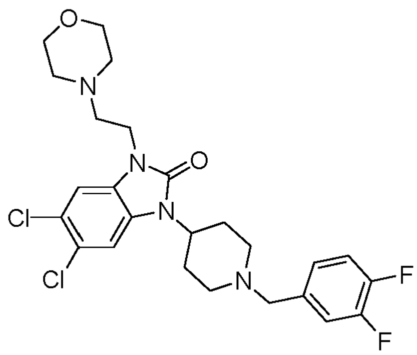

EX2

It was synthesized from the raw materials of intermediate **1g** and 3,4-difluorobenzaldehyde, according to the synthesis method in Example 1. LC-MS (ESI) m/z: 525.20 $(M+H)^+$; $^1H$ NMR (400 MHz, chloroform-d) δ 7.30 (s, 1H), 7.26 - 7.18 (m, 1H), 7.16 - 7.10 (m, 2H), 7.10 - 7.03 (m, 1H), 4.36 - 4.23 (m, 1H), 3.94 (t, *J* = 6.6 Hz, 2H), 3.67 (t, *J* = 4.6 Hz, 4H), 3.51 (s, 2H), 3.01 (d, *J* = 11.3 Hz, 2H), 2.64 (t, *J* = 6.6 Hz, 2H), 2.52 (t, *J* = 4.6 Hz, 4H), 2.38 (q, *J* = 11.0, 10.5 Hz, 2H), 2.16 (t, *J* = 11.5 Hz, 2H), 1.78 (d, *J* = 11.7 Hz, 2H).

***Example 3:*** Synthesis of 5,6-dichloro-1-(1-(2-chloro-4-fluorobenzyl) piperidin-4-yl)-3-(2-morpholinoethyl)-1,3-dihydro-2H-benzo[d]imidazol-2-one (compound ***EX3)***

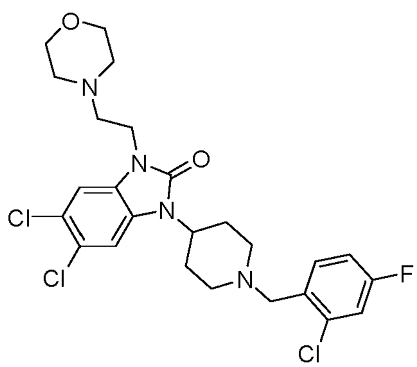

EX3

It was synthesized from the raw materials of intermediate **1g** and 2-chloro-4-fluorobenzaldehyde, according to the synthesis method in Example 1. LC-MS (ESI) m/z: 541.10 $(M+H)^+$; $^1H$ NMR (400 MHz, chloroform-*d*) δ 7.52 (t, *J* = 7.3 Hz, 1H), 7.30 (s, 1H), 7.15 - 7.09 (m, 2H), 7.04 - 6.98 (m, 1H), 4.37 - 4.23 (m, 1H), 3.95 (t, *J* = 6.6 Hz, 2H), 3.73 - 3.57 (m, 6H), 3.05 (d, *J* = 11.0 Hz, 2H), 2.65 (t, *J* = 6.6 Hz, 2H), 2.52 (t, *J* = 4.7 Hz, 4H), 2.47 - 2.33 (m, 2H), 2.28 (t, *J* = 11.6 Hz, 2H), 1.79 (d, *J* = 11.6 Hz, 2H).

**Example 4:** Synthesis of 5,6-dichloro-1-(2-morpholinoethyl)-3-(1-(4-(trifluoromethyl)benzyl) piperidin-4-yl)-1,3-dihydro-2*H*-benzo[*d*]imidazol-2-one (compound **EX4)**

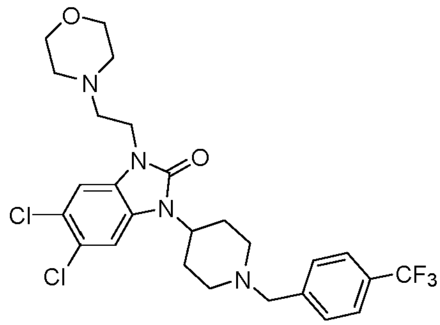

EX4

It was synthesized from the raw materials of intermediate **1g** and 4-trifluoromethyl benzaldehyde, according to the synthesis method in Example 1. LC-MS (ESI) m/z: 557.20 $(M+H)^+$; $^1H$ NMR (400 MHz, chloroform-d) δ 7.60 (d, *J* = 7.9 Hz, 2H), 7.50 (d, *J* = 7.9 Hz, 2H), 7.30 (s, 1H), 7.13 (s, 1H), 4.36 - 4.24 (m, 1H), 3.94 (t, *J* = 6.6 Hz, 2H), 3.67 (t, *J* = 4.7 Hz, 4H), 3.62 (s, 2H), 3.02 (d, *J* = 11.3 Hz, 2H), 2.64 (t, *J* = 6.6 Hz, 2H), 2.52 (t, *J* = 4.6 Hz, 4H), 2.40 (q, *J* = 11.5 Hz, 2H), 2.19 (t, *J* = 11.5 Hz, 2H), 1.78 (d, *J* = 11.8 Hz, 2H).

**Example 5:** Synthesis of 5,6-dichloro-1-(2-morpholinoethyl)-3-(1-(4-(trifluoromethoxy) benzyl)piperidin-4-yl)-1,3-dihydro-2H-benzo[d]imidazol-2-one (compound **EX5)**

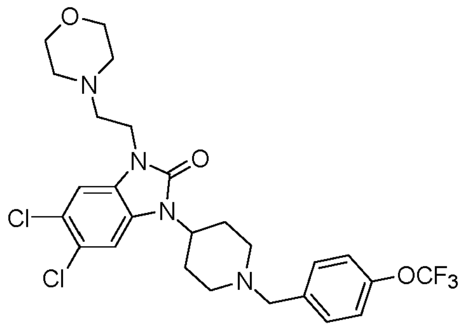

EX5

It was synthesized from the raw materials of intermediate **1g** and 4-trifluoromethoxybenzaldehyde, according to the synthesis method in Example 1. LC-MS (ESI) m/z: 573.20; $^1H$ NMR (400 MHz, chloroform-*d*) δ 7.40 (d, *J* = 8.1 Hz, 2H), 7.31 (s, 1H), 7.19 (d, *J* = 8.1 Hz, 2H), 7.12 (s, 1H), 4.36 - 4.23 (m, 1H), 3.94 (t, *J* = 6.6 Hz, 2H), 3.67 (t, *J* = 4.6 Hz, 4H), 3.56 (s, 2H), 3.03 (d, *J* = 11.2 Hz, 2H), 2.64 (t, *J* = 6.6 Hz, 2H), 2.52 (t, *J* = 4.6 Hz, 4H), 2.38 (q, *J* = 10.3 Hz, 2H), 2.17 (t, *J* = 11.3 Hz, 2H), 1.78 (d, *J* = 10.6 Hz, 2H).

**Example 6:** Synthesis of 5,6-dichloro-1-(1-(2,4-dichlorobenzyl) piperidin-4-yl)-3-(2-morpholinoethyl)-1,3-dihydro-2H-benzo[d]imidazol-2-one (compound **EX6)**

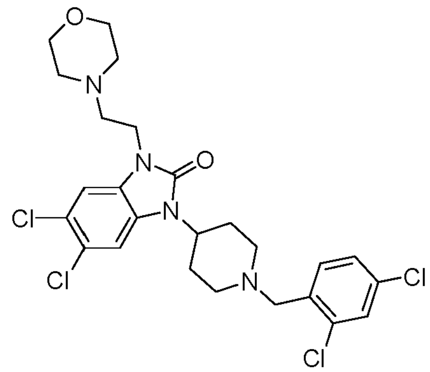

EX6

It was synthesized from the raw materials of intermediate **1g** and 2,4-dichlorobenzaldehyde, according to the synthesis method in Example 1. LC-MS (ESI) m/z: 557.10 $(M+H)^+$; $^1$H NMR (400 MHz, chloroform-*d*) δ 7.50 (d, *J* = 8.3 Hz, 1H), 7.39 (d, *J* = 2.1 Hz, 1H), 7.31 - 7.26 (m, 2H), 7.12 (s, 1H), 4.37 - 4.22 (m, 1H), 3.95 (t, *J* = 6.6 Hz, 2H), 3.67 (t, *J* = 4.6 Hz, 4H), 3.64 (s, 2H), 3.04 (d, *J* = 10.9 Hz, 2H), 2.65 (t, *J* = 6.6 Hz, 2H), 2.52 (t, *J* = 4.6 Hz, 4H), 2.40 (q, *J* = 11.9 Hz, 2H), 2.28 (t, *J* = 11.2 Hz, 2H), 1.78 (d, *J* = 10.9 Hz, 2H).

**Example 7:** Synthesis of 1-(1-(4-(tert-butyl)benzyl) piperidin-4-yl)-5,6-dichloro-3 -(2-morpholinoethyl)-1,3-dihydro-2*H*-benzo[*d*]imidazol-2-one (compound **EX7)**

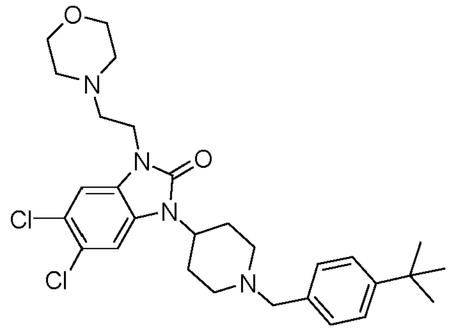

EX7

It was synthesized from the raw materials of intermediate **1g** and 4-*tert*-butylbenzaldehyde, according to the synthesis method in Example 1. LC-MS (ESI) m/z: 545.30 $(M+H)^+$, $^1$H NMR (400 MHz, chloroform-*d*) δ 7.39 - 7.32 (m, 3H), 7.29 (d, *J* = 8.1 Hz, 2H), 7.11 (s, 1H), 4.37 - 4.24 (m, 1H), 3.94 (t, *J* = 6.6 Hz, 2H), 3.66 (t, *J* = 4.6 Hz, 4H), 3.55 (s, 2H), 3.06 (d, *J* = 11.2 Hz, 2H), 2.64 (t, *J* = 6.6 Hz, 2H), 2.52 (t, *J* = 4.6 Hz, 4H), 2.45 - 2.28 (m, 2H), 2.15 (t, *J* = 11.1 Hz, 2H), 1.76 (d, *J* = 10.9 Hz, 2H), 1.33 (s, 9H).

**Example 8:** Synthesis of 1-(1-(4-(*tert*-butyl)cyclohexyl)piperidin-4-yl)-5, 6-dichloro-3-(2-morpholinoethyl)-1,3-dihydro-2*H*-benzo[d]imidazol-2-one (compound **EX8)**

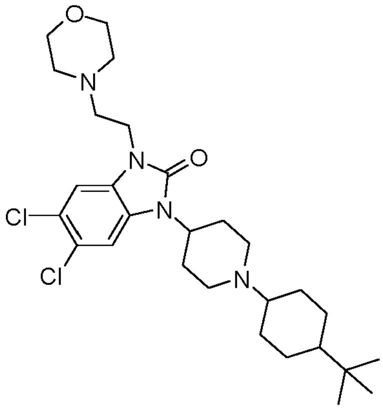

EX8

It was synthesized from the raw materials of intermediate **1g** and 4-*tert*-butyl cyclohexanone, according to the synthesis method in Example 1. LC-MS (ESI) m/z: 537.30 $(M+H)^+$; $^1H$ NMR (400 MHz, chloroform-d) δ 7.26 (s, 1H), 7.12 (s, 1H), 4.31 - 4.18 (m, 1H), 3.94 (t, *J* = 6.7 Hz, 2H), 3.67 (t, *J* = 4.6 Hz, 4H), 3.26 (d, *J* = 11.4 Hz, 2H), 2.65 (t, *J* = 6.7 Hz, 2H), 2.52 (t, *J* = 4.6 Hz, 4H), 2.40 - 2.21 (m, 3H), 2.08 - 1.89 (m, 4H), 1.78 (d, *J* = 10.7 Hz, 2H), 1.51 - 1.28 (m, 6H), 1.17 - 1.05 (m, 1H), 0.89 (s, 9H).

**Example 9:** Synthesis of 5,6-dichloro-1-(1-cyclohexylpiperidin-4-yl)-3-(2-morpholinoethyl) -1,3-dihydro-2H-benzo[d] imidazol-2-one (compound **EX9)**

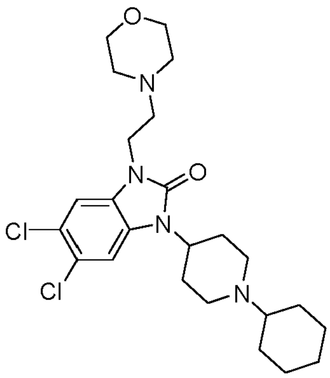

EX9

It was synthesized from the raw materials of intermediate **1g** and cyclohexanone, according to the synthesis method in Example 1. LC-MS (ESI) m/z: 481.20 $(M+H)^+$; $^1H$ NMR (400 MHz, chloroform-d) δ 7.39 (s, 1H), 7.11 (s, 1H), 4.38 - 4.21 (m, 1H), 3.94 (t, *J* = 6.6 Hz, 2H), 3.67 (t, *J* = 4.6 Hz, 4H), 3.06 (d, *J* = 10.9 Hz, 2H), 2.64 (t, *J* = 6.6 Hz, 2H), 2.52 (t, *J* = 4.6 Hz, 4H), 2.48 - 2.20 (m, 5H), 1.95 - 1.73 (m, 6H), 1.65 (d, *J* = 12.7 Hz, 1H), 1.34 - 1.18 (m, 4H), 1.19 - 1.03 (m, 1H).

**Example 10:** Synthesis of 5,6-dichloro-1-(1-cycloheptylpiperidin-4-yl)-3-(2-morpholinoethyl) -1,3-dihydro-2*H*-benzo[*d*] imidazol-2-one (compound EX10)

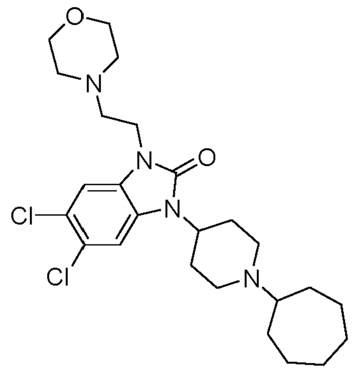

EX10

It was synthesized from the raw materials of intermediate **1g** and cycloheptanone, according to the synthesis method in Example 1. LC-MS (ESI) m/z: 495.20 $(M+H)^+$; $^1H$ NMR (400 MHz, chloroform-d) δ 7.38 (s, 1H), 7.11 (s, 1H), 4.35 - 4.19 (m, 1H), 3.94 (t, *J* = 6.6 Hz, 2H), 3.67 (t, *J* = 4.6 Hz, 4H), 2.94 (d, *J* = 11.0 Hz, 2H), 2.70 - 2.58 (m, 3H), 2.52 (t, *J* = 4.6 Hz, 4H), 2.44 (t, *J* = 11.3 Hz, 2H), 2.28 (q, *J* = 11.6, 10.5 Hz, 2H), 1.92 - 1.82 (m, 2H), 1.78 (d, *J* = 10.0 Hz, 2H), 1.75 - 1.65 (m, 2H), 1.62 - 1.37 (m, 8H).

Example 11: 5,6-dichloro-1-(1-(cyclohexylmethyl) piperidin-4-yl)-3-(2-morpholinoethyl) -1,3-dihydro-2H-benzo[d]imidazol-2-one (compound EX11)

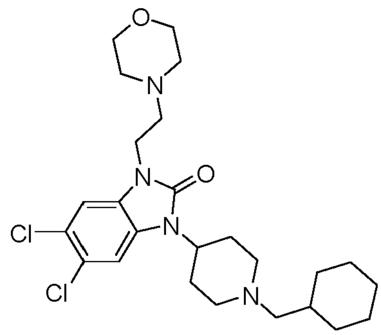

EX11

It was synthesized from the raw materials of intermediate **1g** and cyclohexyl formaldehyde, according to the synthesis method in Example 1. LC-MS (ESI) m/z: 495.20 $(M+H)^+$; $^1H$ NMR (400 MHz, chloroform-d) δ 7.32 (s, 1H), 7.11 (s, 1H), 4.35 - 4.20 (m, 1H), 3.94 (t, *J*= 6.6 Hz, 2H), 3.67 (t, *J* = 4.6 Hz, 4H), 3.02 (d, *J* = 11.3 Hz, 2H), 2.64 (t, *J* = 6.6 Hz, 2H), 2.52 (t, *J* = 4.6 Hz, 4H), 2.34 (q, *J* = 11.6, 10.0 Hz, 2H), 2.17 (d, *J* = 7.0 Hz, 2H), 2.06 (t, *J* = 11.2 Hz, 2H), 1.88 - 1.63 (m, 7H), 1.49 (bs, 1H), 1.32 - 1.11 (m, 3H), 0.90 (q, *J* = 10.9, 10.2 Hz, 2H).

**Example 12: Synthesis of 5,6-dichloro-1-(1-(4-isopropylcyclohexyl) piperidin-4-yl)-3-(2-morpholinoethyl)-1,3-dihydro-2*H*-benzo[d]imidazol-2-one (compound EX12)**

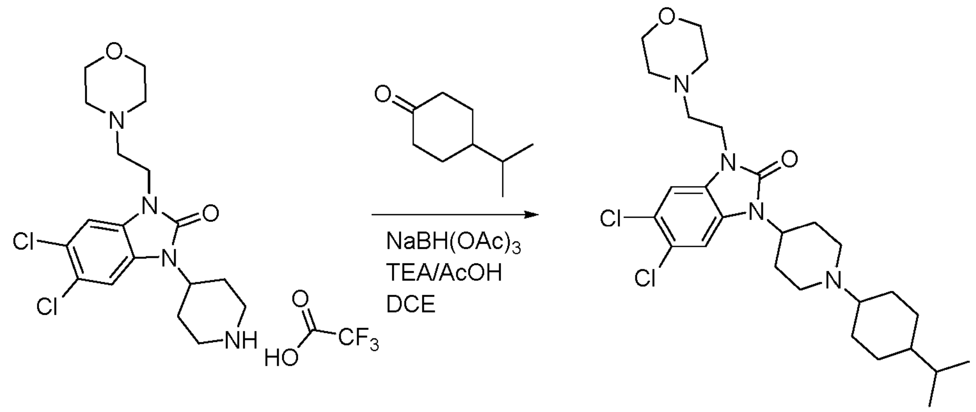


1g EX12

Synthesis of compound **EX12:** 5,6-dichloro-1-(2-morpholinoethyl)-3-(piperidin-4-yl) -1,3-dihydro-2H-benzo[*d*]imidazol-2-one trifluoroacetate (1g) (100 mg, 195 μM) and anhydrous 1,2-dichloroethane (2 mL) were added into a dry Schlenk tube, dry triethylamine (23 mg, 233 μmol, 32 μL) was added thereto, and stirred for 5 minutes, and 4-isopropylcyclohexanone (55 mg, 390 μmol), acetic acid (15 mg, 253 μmol, 14 μL), and $NaBH(OAc)_3$ (165 mg, 779 μmol) were added to the reaction tube. Under argon protection, the tube was heated to 40°C and stirred for reaction for 72 hours. The reaction of the raw materials was detected, by TLC plate, to be substantially complete. Saturated sodium bicarbonate aqueous solution was added to the reaction flask for quenching the reaction, followed by extraction with dichloromethane for three times, combining the organic phases, washing with saturated saline, drying with anhydrous sodium sulfate, filtering, and evaporation under reduced pressure by a rotary evaporator to remove the solvent to obtain a crude product, which was separated and purified by silica gel chromatography column (mobile phase was dichloromethane and methanol, in a ratio of 150:1 to 70:1) to obtain 86 mg white solid product, with a yield of 84.3%.

**Example 12A:** Synthesis of 5,6-dichloro-1-(1-(trans-4-isopropylcyclohexyl) piperidin-4-yl)-3-(2-morpholinoethyl)-1,3-dihydro-2*H*-benzo[d]imidazol-2-one (compound **EX12A)**

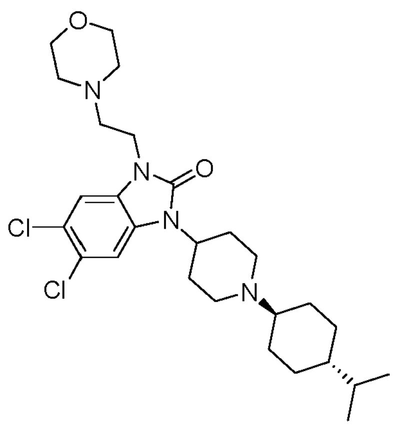

EX12A

Compound **EX12** in Example 12 was subjected to separation and purification by high performance liquid chromatography (Waters high performance liquid chromatography, chromatographic column: YMC-Triart-C18 EXRS 20 mm×100 mm×5 μm; mobile phase: A = water + 0.1 vol% ammonia (25%), B = acetonitrile; gradient 70%-90% B, 10 minutes) to obtain a trans product, 15 mg white solid, with a yield of 14.71%. LC-MS (ESI) m/z: 523.20 $(M+H)^+$; $^1H$ NMR (400 MHz, chloroform-d) δ 7.36 (s, 1H), 7.11 (s, 1H), 4.33 - 4.20 (m, 1H), 3.94 (t, *J* = 6.7 Hz, 2H), 3.67 (d, *J* = 4.7 Hz, 4H), 3.15 (d, *J* = 11.1 Hz, 2H), 2.64 (t, *J* = 6.7 Hz, 2H), 2.52 (d, *J* = 4.6 Hz, 4H), 2.38 - 2.17 (m, 5H), 1.85 - 1.68 (m, 6H), 1.65 - 1.59 (m, 2H), 1.57 - 1.49 (m, 2H), 1.44 - 1.33 (m, 2H), 1.19 - 1.09 (m, 1H), 0.91 (s, 3H), 0.89 (s, 3H).

**Example 12B:** Synthesis of 5,6-dichloro-1-(1-(cis-4-isopropylcyclohexyl)piperidin-4-yl) -3-(2-morpholinoethyl)-1,3-dihydro-2*H*-benzo[d]imidazol-2-one (compound **EX12B)**

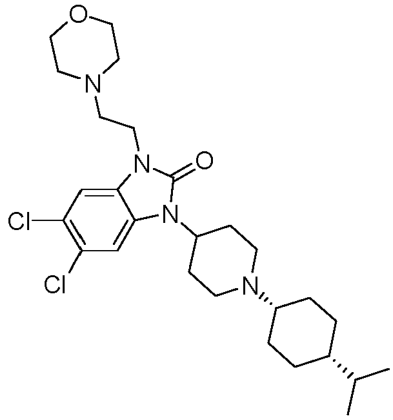

EX12B

Compound **EX12** in Example 12 was subjected to separation and purification by high performance liquid chromatography (Waters high performance liquid chromatography, chromatographic column: YMC-Triart-C18 EXRS 20 mm×100 mm×5 μm; mobile phase: A = water + 0.1 vol% ammonia (25%), B = acetonitrile; gradient 70%-90% B, 10 minutes) to obtain a cis product, 44 mg white solid, with a yield of 43.14%. LC-MS (ESI) m/z: 523.20 $(M+H)^+$; $^1H$ NMR (400 MHz, chloroform-d) δ 7.36 (s, 1H), 7.11 (s, 1H), 4.32 - 4.21 (m, 1H), 3.94 (t, *J* = 6.7 Hz, 2H), 3.67 (d, *J* = 4.7 Hz, 4H), 3.15 (d, *J* = 11.1 Hz, 2H), 2.64 (t, *J* = 6.7 Hz, 2H), 2.52 (d, *J*= 4.7 Hz, 4H), 2.36 - 2.17 (m, 5H), 1.86 - 1.67 (m, 6H), 1.65 - 1.52 (m, 4H), 1.43 - 1.33 (m, 2H), 1.18 - 1.08 (m, 1H), 0.90 (s, 3H), 0.88 (s, 3H).

**Example 13:** Synthesis of 5,6-dichloro-1-(1-(4-chlorophenyl)piperidin-4-yl)-3-(2-morpholinoethyl)-1,3-dihydro-2*H*-benzo[*d*]imidazol-2-one (compound **EX13)**

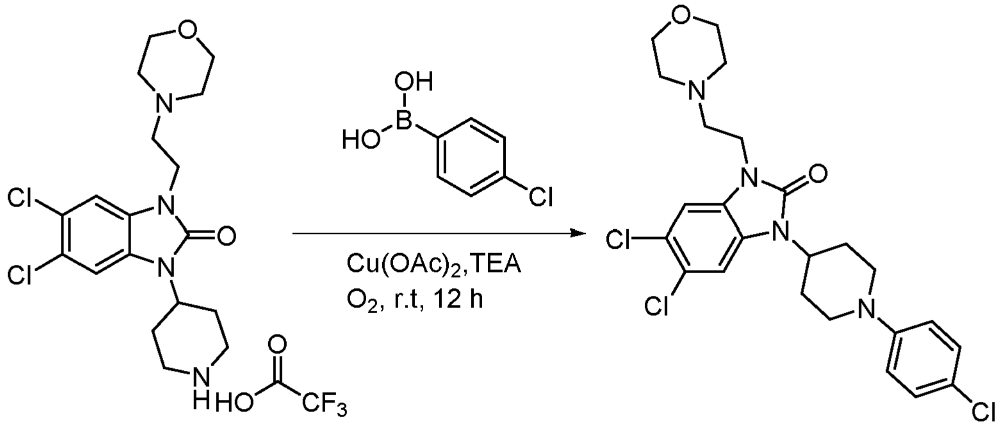


1g EX13

Intermediate **1g** (100 mg, 194 μmol) and anhydrous dichloromethane (2 mL) were added into a dry Schlenk tube, and triethylamine (39 mg, 389 μmol, 54 μL) was added thereto, and stirred for 5 minutes. 4-chlorophenylboronic acid (61 mg, 389 μmol), $Cu(OAc)_2$ (35 mg, 194 μmol) and 4Å molecular sieve (100 mg) were added to the reaction tube. The mixture was stirred at room temperature for reaction for 24 hours. The reaction of the raw materials was detected, by TLC plate, to be substantially complete. Dichloromethane (10 mL) was added into the reaction tube, followed by filtration with diatomite. The filtrate was evaporated under reduced pressure by a rotary evaporator to remove the solvent to obtain a crude product, which was separated and purified by silica gel chromatography column (mobile phase was dichloromethane/methanol, in a ratio of 110:1 to 100:1) to obtain 40 mg white solid product, with a yield of 40.3%. LC-MS (ESI) m/z:

509.00 $(M+H)^+$; $^1H$ NMR (400 MHz, chloroform-d) δ 7.26 - 7.20 (m, 3H), 7.15 (s, 1H), 6.91 (d, *J* = 9.0 Hz, 2H), 4.49 - 4.37 (m, 1H), 3.97 (s, 2H), 3.78 (d, *J* = 12.5 Hz, 2H), 3.68 (s, 4H), 2.88 (t, *J* = 11.7 Hz, 2H), 2.68 (bs, 2H), 2.62 - 2.37 (m, 6H), 1.92 (d, *J* = 10.7 Hz, 2H).

**Example 14:** Synthesis of 1-(1-(4-(*tert-butyl*) phenyl)piperidin-4-yl)-5,6-dichloro-3-(2-morpholinoethyl)-1,3-dihydro-2H-benzo[d]imidazol-2-one (compound **EX14)**

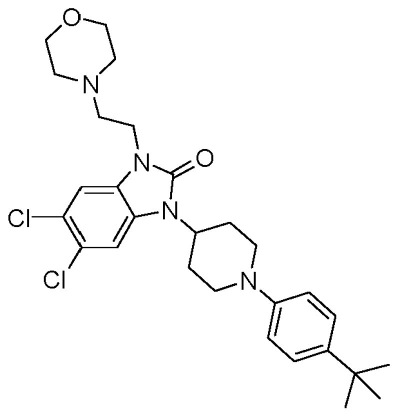

EX14

It was synthesized from the raw materials of intermediate **1g** and 4-tert-butylphenylboronic acid, according to the synthesis method in Example 13. LC-MS (ESI) m/z: 531.10 $(M+H)^+$; $^1H$ NMR (400 MHz, chloroform-d) δ 7.32 (d, *J* = 8.7 Hz, 2H), 7.27 (s, 1H), 7.14 (s, 1H), 6.94 (d, *J* = 8.7 Hz, 2H), 4.50 - 4.37 (m, 1H), 3.96 (t, *J* = 6.7 Hz, 2H), 3.81 (d, *J* = 11.9 Hz, 2H), 3.72 - 3.63 (m, 4H), 2.87 (t, *J* = 11.6 Hz, 2H), 2.66 (t, *J* = 6.6 Hz, 2H), 2.59 - 2.43 (m, 6H), 1.90 (d, *J* = 10.5 Hz, 2H), 1.31 (s, 9H).

**Example 15:** Synthesis of 5,6-dichloro-1-(2-morpholinoethyl)-3-(1-(5-(trifluoromethyl) pyridin-2-yl)piperidin-4-yl)-1,3-dihydro-2*H*-benzo[d]imidazol-2-one (compound EX15)

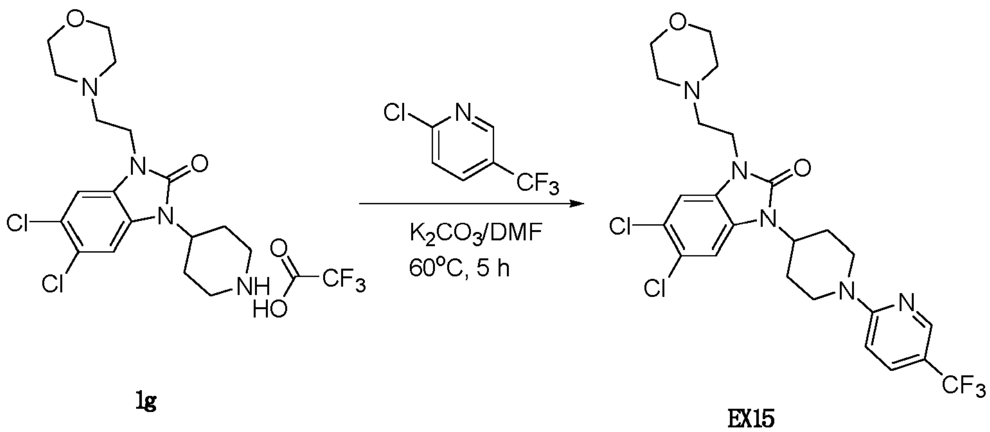


1g

EX15

Intermediate **1g** (100 mg, 194 μmol) and anhydrous DMF (2 mL) were added into a dry Schlenk tube, and 2-chloro-5-trifluoromethylpyridine (35 mg, 194 μmol) and $K_2CO_3$ (81 mg, 584 μmol) were added thereto. The mixture was heated to 60°C, and stirred for reaction for 5 hours. The reaction of the raw materials was detected, by TLC plate, to be substantially complete. After the reaction mixture was cooled to room temperature, a small amount of water was added, followed by extraction with ethyl acetate (10 mL×3). The organic phase was washed with water and saturated saline, dried with anhydrous sodium sulfate, and then filtered. The filtrate was evaporated under reduced pressure by a rotary evaporator to remove the solvent to obtain a crude product, which was separated and purified by a silica gel chromatography column (the mobile phase was petroleum ether/ethyl acetate, in a ratio of 5:1 to 1:1) to obtain 45 mg white solid

product, with a yield of 42.4%. LC-MS (ESI) m/z: 544.01 $(M+H)^+$; $^1H$ NMR (400 MHz, chloroform-d) δ 8.43 (s, 1H), 7.67 (dd, $J$ = 9.0, 2.5 Hz, 1H), 7.14 (s, 1H), 7.12 (s, 1H), 6.73 (d, $J$ = 9.0 Hz, 1H), 4.70 - 4.60 (m, 2H), 4.58 - 4.46 (m, 1H), 3.95 (t, $J$ = 6.5 Hz, 2H), 3.73 - 3.61 (m, 4H), 3.04 (td, $J$ = 13.1, 2.6 Hz, 2H), 2.65 (t, $J$ = 6.5 Hz, 2H), 2.59 - 2.45 (m, 4H), 2.37 (qd, $J$ = 12.7, 4.3 Hz, 2H), 1.99 - 1.89 (m, 2H).

**Example 16:** Synthesis of 5,6-dichloro-1-(1-(4-chloro-2-fluorobenzyl)piperidin-4-yl)-3-(2-morpholinoethyl)-1,3-dihydro-2H-benzo[d]imidazol-2-one (compound **EX16)**

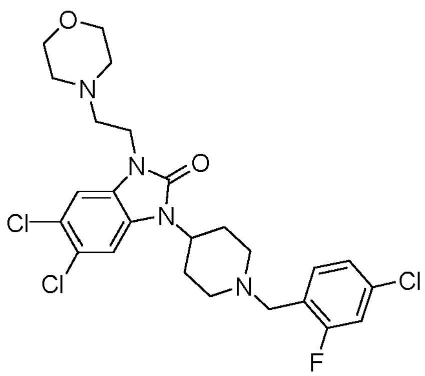

EX16

It was synthesized from the raw materials of intermediate **1g** and 2-fluoro-4-chlorobenzaldehyde, according to the synthesis method in Example 1. LC-MS (ESI) m/z: 541.20 $(M+H)^+$; $^1H$ NMR (400 MHz, chloroform-d) δ 7.40 (t, $J$ = 8.1 Hz, 1H), 7.30 (s, 1H), 7.15 (dd, $J$ = 8.3, 2.0 Hz, 1H), 7.12 (s, 1H), 7.09 (dd, $J$ = 9.6, 2.1 Hz, 1H), 4.33 - 4.21 (m, 1H), 3.94 (t, $J$ = 6.6 Hz, 2H), 3.66 (t, $J$ = 4.6 Hz, 4H), 3.61 (s, 2H), 3.04 (d, $J$ = 11.1 Hz, 2H), 2.64 (t, *J= 6.6* Hz, 2H), 2.52 (t, $J$ = 4.6 Hz, 4H), 2.46 - 2.29 (m, 2H), 2.23 (t, $J$ = 11.7 Hz, 2H), 1.78 (d, $J$ = 10.4 Hz, 2H).

**Example 17:** Synthesis of 5,6-dichloro-1-(1-(2,4-difluorobenzyl) piperidin-4-yl)-3-(2-morpholinoethyl)-1,3-dihydro-2H-benzo[d]imidazol-2-one (compound EX17)

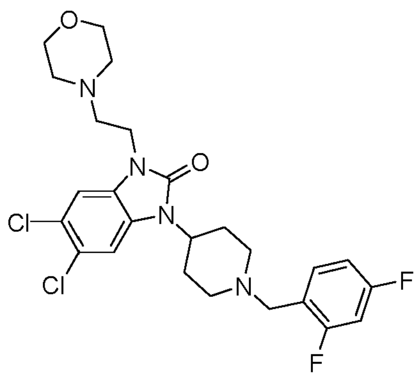

EX17

It was synthesized from the raw materials of intermediate **1g** and 2,4-difluorobenzaldehyde, according to the synthesis method in Example 1. LC-MS (ESI) m/z: 525.30 $(M+H)^+$; $^1H$ NMR (400 MHz, chloroform-d) δ 7.47 - 7.35 (m, 1H), 7.31 (s, 1H), 7.12 (s, 1H), 6.89 (td, $J$= 8.4, 2.5 Hz, 1H), 6.81 (td, $J$ = 9.4, 2.5 Hz, 1H), 4.34 - 4.21 (m, 1H), 3.94 (t, $J$ = 6.6 Hz, 2H), 3.66 (t, $J$ = 4.6 Hz, 4H), 3.61 (s, 2H), 3.04 (d, $J$ = 11.1 Hz, 2H), 2.64 (t, $J$ = 6.6 Hz, 2H), 2.52 (t, $J$ = 4.6 Hz, 4H), 2.45 - 2.30 (m, 2H), 2.22 (t, $J$ = 11.7 Hz, 2H), 1.78 (d, $J$ = 11.3 Hz, 2H).

Example 18: Synthesis of 5,6-dichloro-1-(1-(2-chloro-4-methylbenzyl) piperidin-4-yl)-3-(2-morpholinoethyl)-1,3-dihydro-2H-benzo[d]imidazol-2-one (compound EX18)

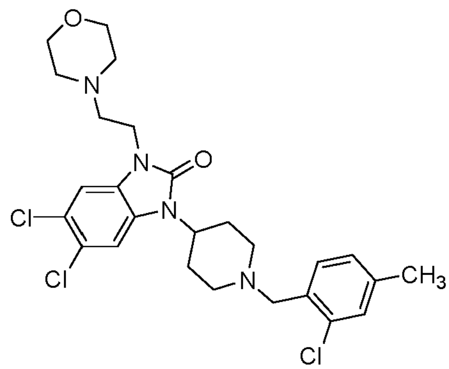

EX18

It was synthesized from the raw materials of intermediate **1g** and 2-chloro-4-methylbenzaldehyde, according to the synthesis method in Example 1. LC-MS (ESI) m/z: 538.30 $(M+H)^+$; $^1H$ NMR (400 MHz, chloroform-d) δ 7.40 (d, *J* = 7.6 Hz, 1H), 7.31 (s, 1H), 7.19 (s, 1H), 7.11 (s, 1H), 7.08 (d, *J* = 7.8 Hz, 1H), 4.37 - 4.23 (m, 1H), 3.94 (t, *J* = 6.6 Hz, 2H), 3.73 - 3.59 (m, 6H), 3.07 (d, *J* = 11.0 Hz, 2H), 2.64 (t, *J* = 6.6 Hz, 2H), 2.52 (t, *J* = 4.7 Hz, 4H), 2.46 - 2.34 (m, 2H), 2.33 (s, 3H), 2.31 - 2.20 (m, 2H), 1.77 (d, *J* = 11.2 Hz, 2H).

**Example 19:** Synthesis of 5,6-dichloro-1-(1-(4-fluoro-2-methylbenzyl) piperidin-4-yl)-3-(2-morpholinoethyl)-1,3-dihydro-2*H*-benzo[d]imidazol-2-one (compound EX19)

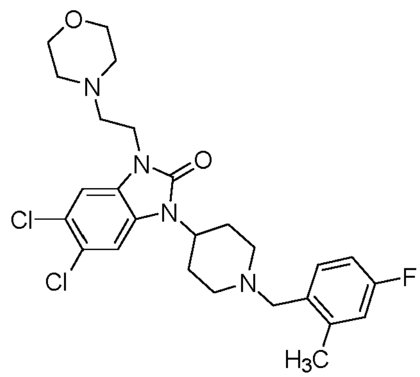

EX19

It was synthesized from the raw materials of intermediate **1g** and 4-fluoro-2-methylbenzaldehyde, according to the synthesis method in Example 1. LC-MS (ESI) m/z: 521.30 $(M+H)^+$; $^1H$ NMR (400 MHz, chloroform-d) δ 7.28 - 7.21 (m, 2H), 7.12 (s, 1H), 6.93 - 6.81 (m, 2H), 4.36 - 4.22 (m, 1H), 3.94 (t, *J* = 6.7 Hz, 2H), 3.73 - 3.61 (m, 4H), 3.46 (s, 2H), 3.01 (d, *J* = 11.1 Hz, 2H), 2.64 (t, *J* = 6.8 Hz, 2H), 2.58 - 2.44 (m, 4H), 2.40 (s, 3H), 2.37 - 2.25 (m, 2H), 2.16 (t, *J* = 11.7 Hz, 2H), 1.76 (d, *J* = 12.7 Hz, 2H).

**Example 20:** Synthesis of 5,6-dichloro-1-(1-(4-ethylcyclohexyl) piperidin-4-yl)-3-(2-morpholinoethyl)-1,3-dihydro-2H-benzo[d]imidazol-2-one (compound **EX20)**

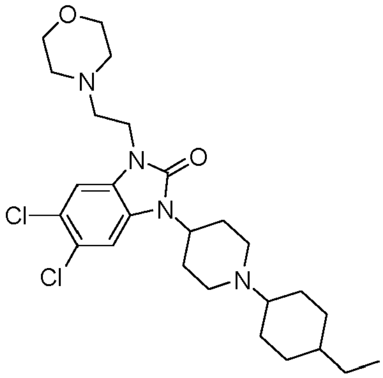

EX20

It was synthesized from the raw materials of intermediate **1g** and 4-ethylcyclohexanone, according to the synthesis method in Example 1. LC-MS (ESI) m/z: 509.30; (cis-trans isomer mixture) $^1$H NMR (400 MHz, chloroform-*d*) δ 7.38 (s, 1H), 7.11 (s, 1H), 4.39 - 4.20 (m, 1H), 3.95 (t, *J* = 6.7 Hz, 2H), 3.73 - 3.61 (m, 4H), 3.21 - 3.00 (m, 2H), 2.64 (t, *J* = 6.8 Hz, 2H), 2.59 - 2.47 (m, 4H), 2.47 - 2.15 (m, 5H), 2.00 - 1.72 (m, 4H), 1.68 - 1.53 (m, 4H), 1.51 - 1.41 (m, 2H), 1.40 - 1.29 (m, 2H), 1.28 - 1.15 (m, 1H), 0.88 (t, *J* = 7.4 Hz, 3H).

**Example 21:** Synthesis of 5,6-dichloro-1-(1-(4-methylcyclohexyl) piperidin-4-yl)-3-(2-morpholinoethyl)-1,3-dihydro-2H-benzo[d]imidazol-2-one (compound EX21)

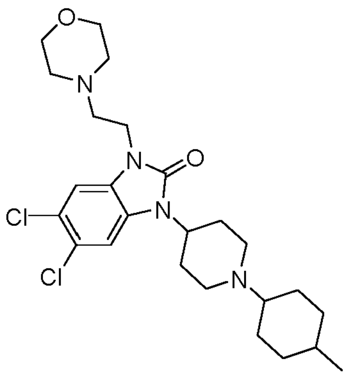

EX21

It was synthesized from the raw materials of intermediate **1g** and 4-methylcyclohexanone, according to the synthesis method in Example 1. LC-MS (ESI) m/z: 495.30 $(M+H)^+$; (cis-trans isomer mixture, main product: by-product = 2:1) main product: $^1$H NMR (400 MHz, chloroform-d) δ 7.39 (d, *J* = 9.8 Hz, 1H), 7.12 (s, 1H), 4.38 - 4.21 (m, 1H), 3.95 (t, *J* = 6.7 Hz, 2H), 3.73 - 3.62 (m, 4H), 3.13 (d, *J* = 7.7 Hz, 1H), 2.64 (t, *J* = 6.7 Hz, 2H), 2.60 - 2.46 (m, 4H), 2.45 - 2.21 (m, 5H), 1.91 - 1.73 (m, 4H), 1.67 - 1.43 (m, 6H), 1.30 (q, *J*= 12.1 Hz, 1H), 0.96 (d, *J* = 6.9 Hz, 3H).

**Example 22:** Synthesis of 5,6-dichloro-1-(2-morpholinoethyl)-3-(1-(4-(trifluoromethyl) cyclohexyl)piperidin-4-yl)-1,3-dihydro-2*H*-benzo[*d*]imidazol-2-one (compound EX22)

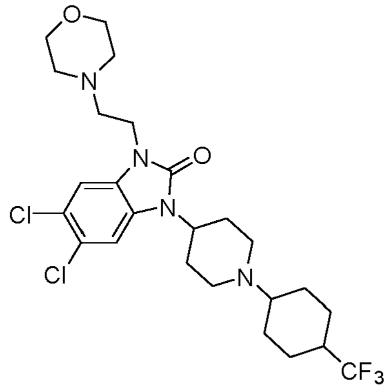

EX22

It was synthesized from the raw materials of intermediate **1g** and 4-trifluoromethyl cyclohexanone, according to the synthesis method in Example 1. LC-MS (ESI) m/z: 549.30 $(M+H)^+$; (cis-trans isomer mixture, main product: by-product = 2:1) main product: $^1H$ NMR (400 MHz, chloroform-d) δ 7.31 (s, 1H), 7.12 (s, 1H), 4.38 - 4.19 (m, 1H), 3.95 (t, *J* = 6.8 Hz, 2H), 3.75 - 3.59 (m, 4H), 3.17 (d, *J* = 11.2 Hz, 2H), 2.65 (t, *J* = 6.8 Hz, 2H), 2.59 - 2.48 (m, 4H), 2.44 - 2.28 (m, 3H), 2.25 - 2.12 (m, 2H), 2.11 - 1.99 (m, 2H), 1.98 - 1.86 (m, 3H), 1.81 (d, *J* = 12.1 Hz, 2H), 1.66 - 1.51 (m, 2H), 1.45 - 1.21 (m, 2H).

**Example 23:** Synthesis of 5,6-dichloro-1-(1'-isopropyl-[1,4'-bipiperidine]-4-yl)-3-(2-morpholinoethyl)-1,3-dihydro-2*H*-benzo[*d*]imidazol-2-one (compound **EX23**)

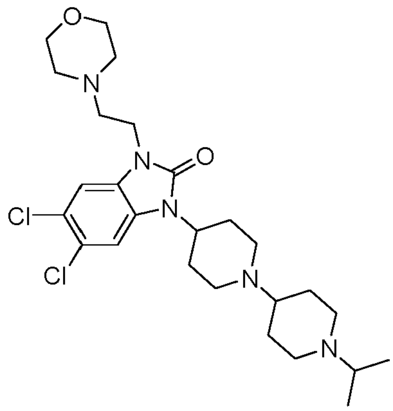

EX23

It was synthesized from the raw materials of intermediate **1g** and *N*-isopropyl-4-piperidinone, according to the synthesis method in Example 1. LC-MS (ESI) m/z: 524.40 $(M+H)^+$; $^1H$ NMR (400 MHz, chloroform-*d*) δ 7.37 (s, 1H), 7.11 (s, 1H), 4.29 (t, *J* = 12.7 Hz, 1H), 3.94 (t, *J* = 6.8 Hz, 2H), 3.79 - 3.58 (m, 5H), 3.12 - 2.93 (m, 4H), 2.76 (br s, 1H), 2.64 (t, *J* = 6.8 Hz, 2H), 2.57 - 2.48 (m, 4H), 2.48 - 2.23 (m, 6H), 2.17 (br s, 2H), 1.89 - 1.76 (m, 4H), 1.67 (br s, 2H), 1.07 (d, *J* = 6.3 Hz, 6H).

**Example 24:** Synthesis of 5,6-dichloro-1-(1-(4-fluorobenzyl)piperidin-4-yl)-3-(2-morpholinoethyl)-1,3-dihydro-2*H*-benzo[*d*]imidazol-2-one (compound **EX24)**

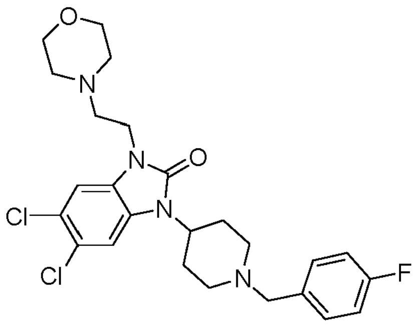

EX24

It was synthesized from the raw materials of intermediate **1g** and 4-fluorobenzaldehyde, according to the synthesis method in Example 1. LC-MS (ESI) m/z: 507.30 $(M+H)^+$; $^1H$ NMR (400 MHz, chloroform-d) δ 7.38 - 7.28 (m, 3H), 7.12 (s, 1H), 7.03 (t, *J* = 8.6 Hz, 2H), 4.38 - 4.23 (m, 1H), 3.94 (t, *J* = 6.7 Hz, 2H), 3.67 (t, *J* = 4.6 Hz, 4H), 3.53 (s, 2H), 3.02 (d, *J* = 11.2 Hz, 2H), 2.64 (t, *J* = 6.7 Hz, 2H), 2.52 (t, *J* = 4.7 Hz, 4H), 2.36 (q, *J* = 22.4, 10.0 Hz, 2H), 2.14 (t, *J* = 11.9 Hz, 2H), 1.77 (d, *J* = 11.0 Hz, 2H).

**Example 25:** Synthesis of 5,6-dichloro-1-(1-(4-chlorophenylmethyl) piperidin-4-yl)-3-(2-morpholinoethyl)-1,3-dihydro-2*H*-benzo[*d*]imidazol-2-one (compound **EX25)**

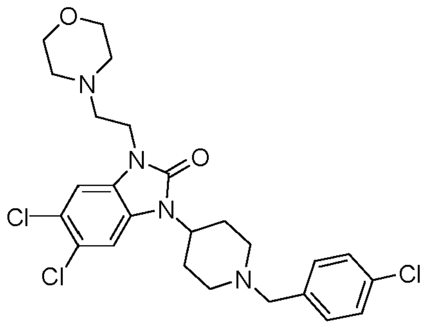

EX25

It was synthesized from the raw materials of intermediate **1g** and 4-chlorobenzaldehyde, according to the synthesis method in Example 1. LC-MS (ESI) m/z: 523.20 $(M+H)^+$; $^1H$ NMR (400 MHz, chloroform-d) δ 7.32 (s, 5H), 7.12 (s, 1H), 4.38 - 4.23 (m, 1H), 3.94 (t, *J* = 6.7 Hz, 2H), 3.73 - 3.62 (m, 4H), 3.55 (s, 2H), 3.13 - 2.92 (m, 2H), 2.64 (t, *J* = 6.6 Hz, 2H), 2.59 - 2.47 (m, 4H), 2.38 (br s, 2H), 2.26 - 2.07 (m, 2H), 1.78 (d, *J* = 12.3 Hz, 2H).

**Example 26:** Synthesis of 5,6-dichloro-1-(1-(2,3-dihydro-1*H*-inden-2-yl) piperidin-4-yl)-3-(2-morpholinoethyl)-1,3-dihydro-2H-benzo[d]imidazol-2-one (compound **EX26)**

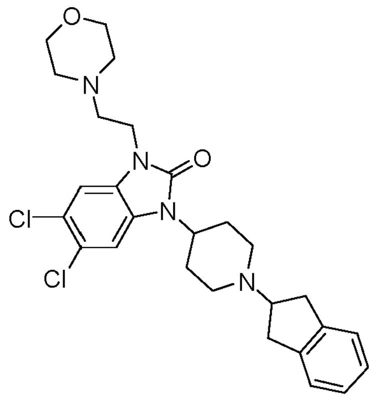

EX26

It was synthesized from the raw materials of intermediate **1g** and 2-indanone, according to the synthesis method in Example 1. LC-MS (ESI) m/z: 515.30 $(M+H)^+$; $^1H$ NMR (400 MHz, chloroform-d) δ 7.39 (s, 1H), 7.24 - 7.17 (m, 2H), 7.17 - 7.09 (m, 3H), 4.43 - 4.28 (m, 1H), 3.95 (t, *J* = 6.5 Hz, 2H), 3.73 - 3.61 (m, 4H), 3.35 - 3.23 (m, 1H), 3.23 - 3.04 (m, 4H), 2.95 (dd, *J* = 15.5, 8.5 Hz, 2H), 2.64 (t, *J* = 6.7 Hz, 2H), 2.58 - 2.47 (m, 4H), 2.39 (q, *J* = 12.5 Hz, 2H), 2.24 (t, *J* = 11.8 Hz, 2H), 1.84 (d, *J* = 11.9 Hz, 2H).

**Example 27:** Synthesis of 5,6-dichloro-1-(1-(2-chlorocyclohexyl) piperidin-4-yl)-3-(2-morpholinoethyl)-1,3-dihydro-2*H*-benzo[d]imidazol-2-one (compound **EX27)**

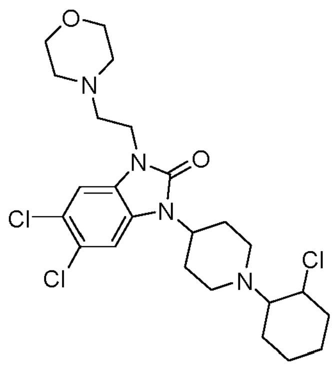

EX27

It was synthesized from the raw materials of intermediate **1g** and 2-chlorocyclohexanone, according to the synthesis method in Example 1. LC-MS (ESI) m/z: 515.30 $(M+H)^+$; $^1H$ NMR (400 MHz, chloroform-*d*) δ 7.37 (s, 1H), 7.11 (s, 1H), 4.62 (s, 1H), 4.39 - 4.25 (m, 1H), 3.95 (t, *J* = 6.7 Hz, 2H), 3.67 (t, *J* = 4.4 Hz, 4H), 3.28 (d, *J* = 9.9 Hz, 2H), 2.64 (t, *J* = 6.7 Hz, 2H), 2.57 - 2.43 (m, 6H), 2.41 - 2.21 (m, 3H), 2.07 (d, *J*= 10.7 Hz, 1H), 1.91 - 1.66 (m, 8H), 1.56 - 1.45 (m, 1H), 1.32 (br s, 1H).

**Example 28:** Synthesis of 5,6-dichloro-1-(1-(4-chloro-2-methylbenzyl) piperidin-4-yl)-3-(2-morpholinoethyl)-1,3-dihydro-2H-benzo[d]imidazol-2-one (compound **EX28)**

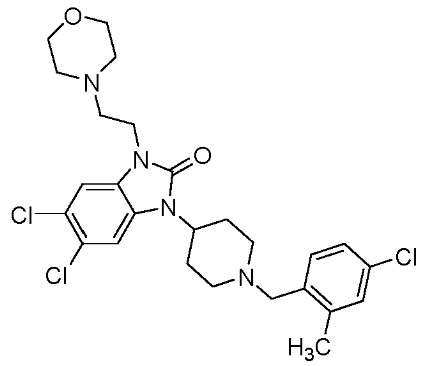

EX28

It was synthesized from the raw materials of intermediate **1g** and 4-chloro-2-methylbenzaldehyde, according to the synthesis method in Example 1. LC-MS (ESI) m/z: 537.20 $(M+H)^+$; $^1H$ NMR (400 MHz, chloroform-d) δ 7.24 (d, *J* = 8.8 Hz, 2H), 7.19 - 7.09 (m, 3H), 4.35 - 4.22 (m, 1H), 3.94 (t, *J* = 6.7 Hz, 2H), 3.67 (t, *J* = 4.6 Hz, 4H), 3.46 (s, 2H), 3.01 (d, *J* = 11.1 Hz, 2H), 2.64 (t, *J* = 6.6 Hz, 2H), 2.52 (t, *J* = 4.6 Hz, 4H), 2.42 - 2.26 (m, 5H), 2.17 (*t, J* = 11.7 Hz, 2H), 1.76 (d, *J* = 11.6 Hz, 2H).

**Example 29:** Synthesis of 5,6-dichloro-1-(1-(4-chloro-2-methoxybenzyl) piperidin-4-yl)-3-(2-morpholinoethyl)-1,3-dihydro-2H-benzo[d]imidazol-2-one (compound **EX29)**

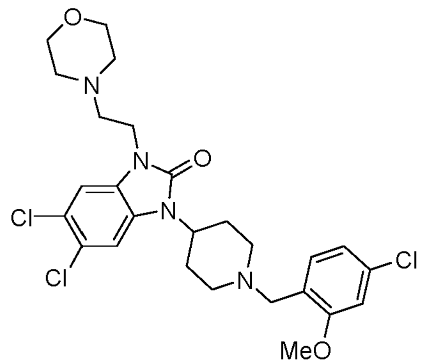

EX29

It was synthesized from the raw materials of intermediate **1g** and 4-chloro-2-methoxybenzaldehyde, according to the synthesis method in Example 1. LC-MS (ESI) m/z: 553.20 $(M+H)^+$; $^1H$ NMR (400 MHz, chloroform-d) δ 7.35 (d, *J* = 7.1 Hz, 2H), 7.12 (s, 1H), 6.96 (d, *J* = 8.1 Hz, 1H), 6.87 (s, 1H), 4.36 - 4.23 (m, 1H), 3.94 (t, *J* = 6.6 Hz, 2H), 3.83 (s, 3H), 3.66 (t, *J* = 4.6 Hz, 4H), 3.59 (s, 2H), 3.06 (d, *J* = 11.3 Hz, 2H), 2.64 (t, *J* = 6.6 Hz, 2H), 2.52 (t, *J* = 4.6 Hz, 4H), 2.47 - 2.30 (m, 2H), 2.23 (t, *J* = 11.9 Hz, 2H), 1.77 (d, *J* = 11.3 Hz, 2H).

**Example 30:** Synthesis of 5,6-dichloro-1-(1-(2, 4-dimethylbenzyl) piperidin-4-yl)-3-(2-morpholinoethyl)-1,3-dihydro-2*H*-benzo[*d*]imidazol-2-one (compound **EX30)**

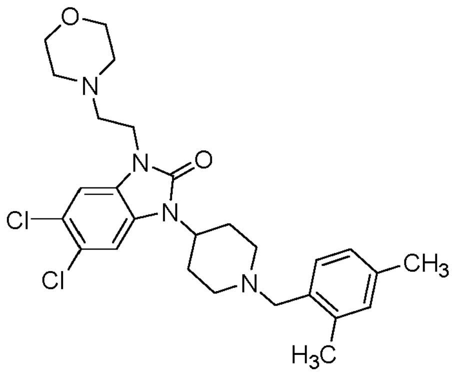

EX30

It was synthesized from the raw materials of intermediate **1g** and 2,4-dimethylbenzaldehyde, according to the synthesis method in Example 1. LC-MS (ESI) m/z: 517.20 $(M+H)^+$; $^1H$ NMR (400 MHz, chloroform-d) δ 7.33 - 7.26 (m, 1H), 7.22 - 7.14 (m, 1H), 7.11 (s, 1H), 7.04 - 6.94 (m, 2H), 4.38 - 4.22 (m, 1H), 3.94 (t, *J* = 6.6 Hz, 2H), 3.73 - 3.60 (m, 4H), 3.48 (s, 2H), 3.04 (d, *J* = 11.0 Hz, 2H), 2.64 (t, *J* = 6.6 Hz, 2H), 2.58 - 2.46 (m, 4H), 2.45 - 2.25 (m, 8H), 2.24 - 2.07 (m, 2H), 1.75 (d, *J* = 11.4 Hz, 2H).

**Example 31:** Synthesis of 5,6-dichloro-1-(2-morpholinoethyl)-3-(1-(2,4,6-trimethylbenzyl) piperidin-4-yl)-1,3-dihydro-2H-benzo[d]imidazol-2-one (compound **EX31)**

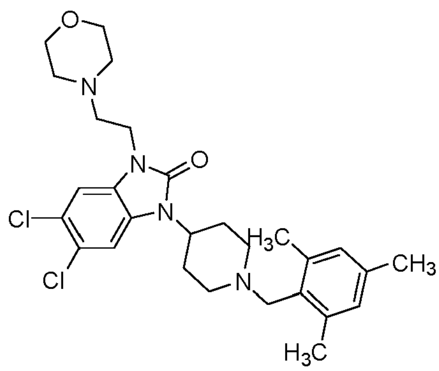

EX31

It was synthesized from the raw materials of intermediate **1g** and 2,4,6-trimethylbenzaldehyde, according to the synthesis method in Example 1. LC-MS (ESI) m/z: 531.30 $(M+H)^+$; $^1H$ NMR (400 MHz, chloroform-*d)* δ 7.20 (s, 1H), 7.10 (s, 1H), 6.86 (s, 2H), 4.36 - 4.20 (m, 1H), 3.93 (t, *J* = 6.7 Hz, 2H), 3.70 - 3.62 (m, 4H), 3.49 (s, 2H), 3.03 - 2.90 (m, 2H), 2.63 (t, *J* = 6.6 Hz, 2H), 2.56 - 2.48 (m, 4H), 2.39 (s, 6H), 2.32 - 2.18 (m, 7H), 1.78 - 1.66 (m, 2H).

**Example 32:** Synthesis of 5-chloro-1-(1-(2-chlorobenzyl)-4-yl)-3-(2-morpholinoethyl)-1,3-dihydro-2*H*-benzo[*d*]imidazol-2-one (compound **EX32)**

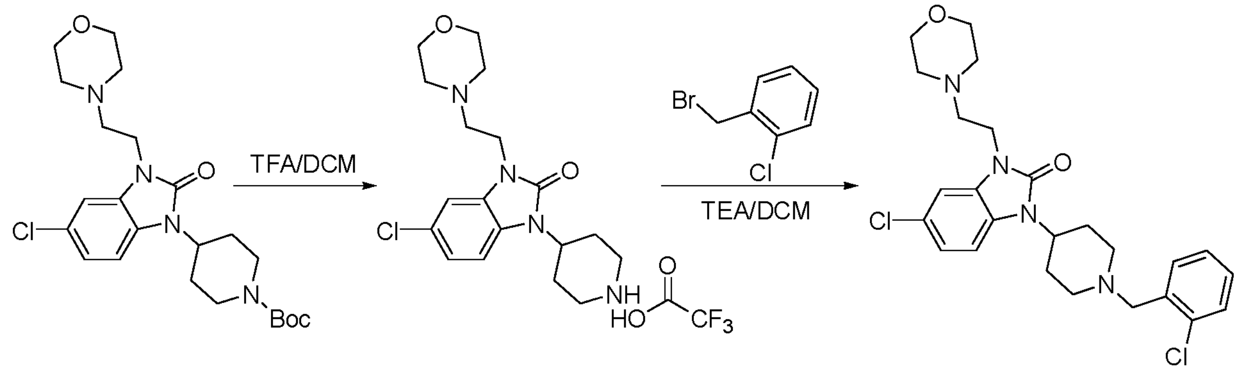


32a 32b EX32

Synthesis of intermediate **32a:** 5-chloro-2-fluoronitrobenzene and 1-N-Boc-4-aminopiperidine were used as raw materials for synthesis according to the synthesis method of intermediate **1f** to obtain 835 mg colorless waxy product, with a yield of 63.2%. $^1$H NMR (400 MHz, chloroform-d) δ 7.05 (s, 1H), 7.02 (s, 2H), 4.50 - 4.38 (m, 1H), 4.31 (br s, 2H), 3.97 (t, *J* = 6.4 Hz, 2H), 3.69 (t, *J* = 4.4 Hz, 4H), 2.85 (t, *J* = 13.4 Hz, 2H), 2.68 (d, *J* = 6.8 Hz, 2H), 2.55 (t, *J* = 4.5 Hz, 4H), 2.26 (qd, *J* = 12.5, 4.2 Hz, 2H), 1.80 (d, *J* = 12.8 Hz, 2H), 1.50 (s, 9H).

Synthesis of intermediate **32b:** intermediate **32a** was subjected to the action of trifluoroacetic acid at room temperature to remove Boc protection group to obtain 720 mg white trifluoroacetate solid, with a yield of 99.9%. The solid was directly used in the next reaction without purification.

Synthesis of compound **EX32:** intermediate **32b** and 2-chlorobenzyl bromide were used as raw materials in anhydrous dichloromethane, and triethylamine was added thereto for a substitution reaction to produce the target compound, which was separated and purified by column chromatography (mobile phase was dichloromethane/methanol, with the methanol volume ratio being 1%) to obtain a white solid product (250 mg, with a yield of 97.8%). LC-MS (ESI) m/z: 490.20 $(M+H)^+$; $^1$H NMR (400 MHz, chloroform-d) δ 7.53 (d, *J* = 7.5 Hz, 1H), 7.36 (d, *J* = 7.9 Hz, 1H), 7.30 - 7.24 (m, 1H), 7.21 (d, *J* = 7.6 Hz, 1H), 7.16 (d, *J* = 9.3 Hz, 1H), 7.06 - 6.99 (m, 2H), 4.42 - 4.29 (m, 1H), 3.96 (t, *J* = 6.9 Hz, 2H), 3.74 - 3.61 (m, 6H), 3.06 (d, *J* = 11.1 Hz, 2H), 2.66 (t, *J* = 6.9 Hz, 2H), 2.59 - 2.49 (m, 4H), 2.43 (q, *J* = 12.5 Hz, 2H), 2.28 (t, *J* = 11.8 Hz, 2H), 1.78 (d, *J* = 12.0 Hz, 2H).

**Example 33:** Synthesis of 5-chloro-1-(1-(2-chloro-4-fluorobenzyl) piperidin-4-yl)-6-fluoro-3-(2-morpholinoethyl)-1,3-dihydro-2*H*-benzo[d]imidazol-2-one (compound **EX33)**

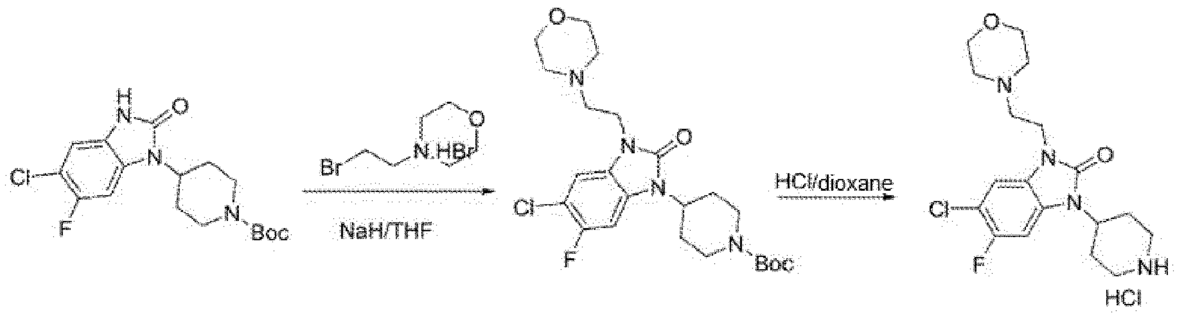


33a 33b 33c

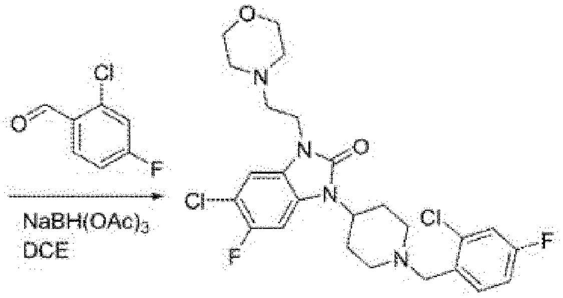


EX33

Synthesis of intermediate **33a:** 5-chloro-2,4-difluoronitrobenzene was used as the raw material for synthesis according to the synthesis method of intermediate **1e** in Example 1, to obtain 3.24 g white solid, with a yield of 72.80%. $^1H$ NMR (400 MHz, chloroform-*d*) δ 9.88 (s, 1H), 7.13 (d, *J* = 6.3 Hz, 1H), 6.96 (d, *J* = 9.2 Hz, 1H), 4.46 - 4.28 (m, 3H), 2.85 (t, *J* = 13.1 Hz, 2H), 2.25 (qd, *J* = 12.5, 4.6 Hz, 2H), 1.86 - 1.78 (m, 2H), 1.52 (s, 9H).

Synthesis of intermediate **33b: 33a** was used as the raw material for synthesis according to the synthesis method of intermediate **1f** in Example 1, to obtain intermediate **33b,** 1.29 g colorless oily product, with a yield of 98.78%. $^1H$ NMR (400 MHz, chloroform-*d*) δ 7.05 (d, *J*= 6.3 Hz, 1H), 6.94 (d, *J* = 9.1 Hz, 1H), 4.45 - 4.23 (m, 3H), 3.95 (t, *J* = 6.7 Hz, 2H), 3.73 - 3.63 (m, 4H), 2.90 - 2.77 (m, 2H), 2.65 (t, *J* = 6.7 Hz, 2H), 2.59 - 2.46 (m, 4H), 2.22 (qd, *J* = 12.7, 4.6 Hz, 2H), 1.86 - 1.74 (m, 2H), 1.50 (s, 9H).

Synthesis of intermediate **33c:** intermediate **33b** (1.30 g, 2.69 mmol) was added into a dry single neck flask (50 mL), anhydrous dioxane (5 mL) was added thereto, HCl/dioxane solution (4.0 M) was added dropwise thereto under cooling in an ice water bath, thereafter, the ice water bath was removed, and stirring was continued at room temperature for reaction for 3 hours. The reaction of the raw materials was detected, by TLC plate, to be substantially complete. The solvent was removed by evaporation under reduced pressure by a rotary evaporator to obtain a light brown oil crude product. Methyl *tert*-butyl ether (50 mL) was added to the crude product, followed by dispersing, stirring, filtering, and drying to obtain 1.13 g white solid product (intermediate **33c),** with a yield of 100%. The white solid did not need further purification, and could be directly used in the next experiment.

Synthesis of compound **EX33:** intermediate **33c** and 2-chloro-4-fluorobenzaldehyde were used as raw materials for synthesis according to the synthesis method in Example 1, to obtain a white solid product (58 mg, with a yield of 23.14%). LC-MS (ESI) m/z: 525.35 $(M+H)^+$; $^1H$ NMR (400 MHz, chloroform-*d*) δ 7.52 (t, *J* = 7.5 Hz, 1H), 7.16 - 7.06 (m, 2H), 7.06 - 6.97 (m, 2H), 4.39 - 4.25 (m, 1H), 3.95 (t, *J* = 6.7 Hz, 2H), 3.72 - 3.65 (m, 4H), 3.63 (s, 2H), 3.04 (d, *J* = 10.9 Hz, 2H), 2.65 (t, *J* = 6.7 Hz, 2H), 2.60 - 2.47 (m, 4H), 2.46 - 2.19 (m, 4H), 1.79 (d, *J*= 11.6 Hz, 2H).

**Example 34:** Synthesis of 5-chloro-6-fluoro-1-(1-(4-isopropylcyclohexyl) piperidin-4-yl)-3-(2-morpholinoethyl)-1,3-dihydro-2*H*-benzo[d]imidazol-2-one (compound **EX34)**

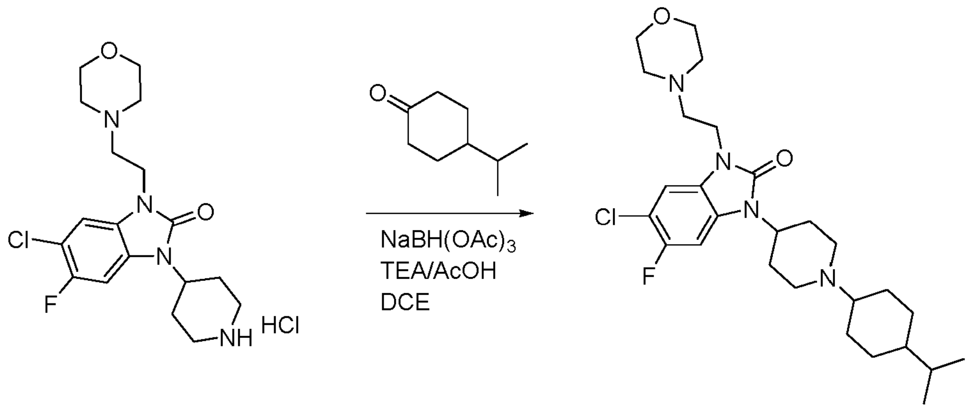


33c EX34

Synthesis of compound **EX34:** intermediate **33c** and 4-isopropylcyclohexanone were used as raw materials for synthesis according to the synthesis method of compound **EX12** to obtain a white solid product (156 mg, with a yield of 64.4%).

**Example 34A:** Synthesis of 5-chloro-6-fluoro-1-(1-(trans-4-isopropylcyclohexyl)piperidin-4-yl)-3-(2-morpholinoethyl)-1,3-dihydro-2*H*-benzo[d]imidazol-2-one (compound **EX34A)**

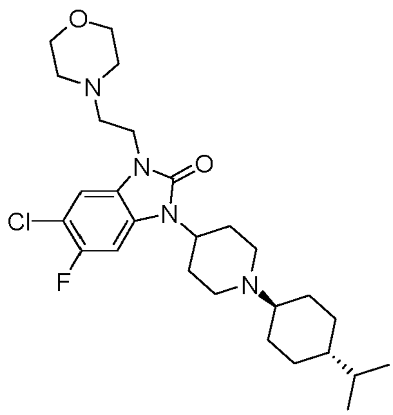

**EX34A**

Synthesis of compound **EX34A:** intermediate **33c** and 4-isopropylcyclohexanone were used as raw materials for synthesis according to the synthesis method of compound **EX12** to obtain compound EX34, which was subjected to preparation and separation by high performance liquid chromatography (Waters high performance liquid chromatography, chromatographic column: YMC-Triart-C18 EXRS 20 mm×100 mm×5 μm; mobile phase: A = water + 0.1 vol% ammonia (25%), B = acetonitrile; gradient 70%-90% B, 10 min) to obtain a trans product, a white solid product (16 mg, with a yield of 6.61%). LC-MS (ESI) m/z: 507.38 $(M+H)^+$; $^1H$ NMR (400 MHz, chloroform-d) δ 7.16 (d, *J* = 9.4 Hz, 1H), 7.02 (d, *J* = 6.2 Hz, 1H), 4.36 - 4.23 (m, 1H), 3.94 (t, *J* = 6.7 Hz, 2H), 3.67 (t, *J* = 4.7 Hz, 4H), 3.05 (d, *J* = 11.4 Hz, 2H), 2.64 (t, *J* = 6.7 Hz, 2H), 2.53 (t, *J* = 4.6 Hz, 4H), 2.44 - 2.20 (m, 5H), 1.91 (d, *J* = 11.7 Hz, 2H), 1.84 - 1.74 (m, 4H), 1.48 - 1.35 (m, 1H), 1.26 (q, *J*= 12.3 Hz, 2H), 1.08 - 0.95 (m, 3H), 0.87 (s, 3H), 0.85 (s, 3H).

**Example 34B:** Synthesis of 5-chloro-6-fluoro-1-(1-(cis-4-isopropylcyclohexyl) piperidin-4-yl)-3-(2-morpholinoethyl)-1,3-dihydro-2*H*-benzo[*d*]imidazol-2-one (compound **EX34B)**

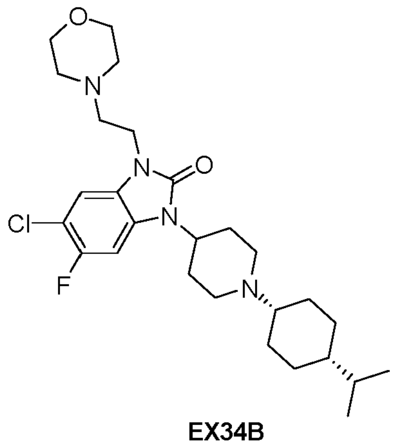

EX34B

Synthesis of compound **EX34B:** intermediate **33c** and 4-isopropylcyclohexanone were used as raw materials for synthesis according to the synthesis method of compound **EX12** to obtain compound **EX34,** which was subjected to preparation and separation by high performance liquid chromatography (Waters high performance liquid chromatography, chromatographic column: YMC-Triart-C18 EXRS 20 mm×100 mm×5 μm; mobile phase: A = water + 0.1 vol% ammonia (25%), B = acetonitrile; gradient 70%-90% B, 10 min) to obtain a cis product, a white solid product (48 mg, with a yield of 19.84%). LC-MS (ESI) m/z: 507.38 $(M+H)^+$; $^1H$ NMR (400 MHz, chloroform-d) δ 7.11 (d, *J* = 9.3 Hz, 1H), 7.03 (d, *J* = 6.2 Hz, 1H), 4.34 - 4.22 (m, 1H), 3.94 (t, *J* = 6.7 Hz, 2H), 3.68 (t, *J* = 4.7 Hz, 4H), 3.15 (d, *J* = 10.7 Hz, 2H), 2.64 (t, *J* = 6.7 Hz, 2H), 2.53 (t, *J* = 4.7 Hz, 4H), 2.36 - 2.13 (m, 5H), 1.83 - 1.75 (m, 2H), 1.73 - 1.59 (m, 5H), 1.57 - 1.45 (m, 2H), 1.44 - 1.32 (m, 2H), 1.18 - 1.08 (m, 1H), 0.90 (s, 3H), 0.89 (s, 3H).

**Example 35:** Synthesis of 5-chloro-3-(1-(2-chloro-4-fluorobenzyl) piperidin-4-yl)-6-fluoro-1-(2-morpholinoethyl)-1,3-dihydro-2*H*-benzo[*d*]imidazol-2-one (compound **EX35)**

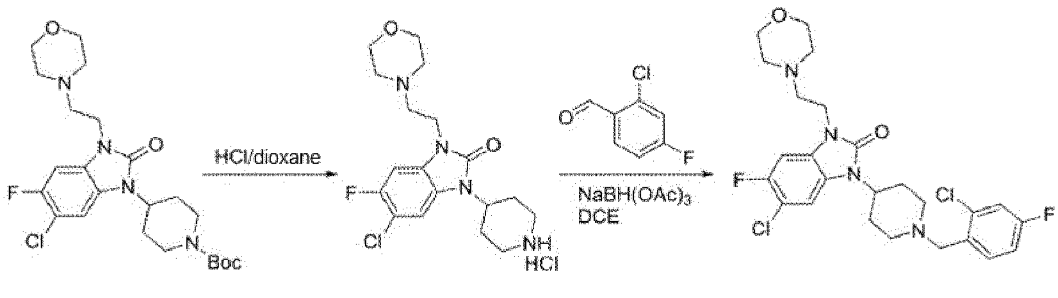


35a 35b EX35

Synthesis of intermediate **35a:** 4-chloro-2,5-difluoronitrobenzene was used as raw material for synthesis according to the synthesis method of intermediate **1f** in Example 1 to obtain a product (1.12 g, with a yield of 85.8%). $^1H$ NMR (400 MHz, chloroform-d) δ 7.09 (d, *J* = 5.5 Hz, 1H), 6.89 (d, *J* = 8.4 Hz, 1H), 4.48 - 4.17 (m, 3H), 3.95 (t, *J* = 6.8 Hz, 2H), 3.67 (t, *J* = 4.5 Hz, 4H), 2.88 - 2.76 (m, 2H), 2.65 (t, *J* = 6.8 Hz, 2H), 2.53 (t, *J* = 4.5 Hz, 4H), 2.25 (q, *J* = 12.3, 11.0 Hz, 2H), 1.80 (d, *J*= 12.1 Hz, 2H), 1.51 (s, 9H).

Synthesis of intermediate **35b: 35a** was used as raw material to obtain a white solid product (1.13 g, with a yield of 100%) according to the synthesis of intermediate **33c** in Example 33. $^1H$ NMR (400 MHz, Methanol-$d_4$) δ 7.47 (d, *J* = 6.2 Hz, 1H), 7.30 (d, *J* = 9.0 Hz, 1H), 4.59 - 4.48 (m, 1H), 4.30 (t, *J* = 6.0 Hz, 2H), 3.90 (br s, 4H), 3.66 - 3.50 (m, 5H), 3.45 (br s, 3H), 3.20 (td, *J* = 13.2, 3.0 Hz, 2H), 2.69 (qd, *J* = 13.5, 4.3 Hz, 2H), 2.16 - 2.03 (m, 2H).

Synthesis of compound **EX35:** intermediate **35b** and 2-chloro-4-fluorobenzaldehyde were used as raw materials for synthesis according to the synthesis method in Example 1 to obtain a white solid product (110 mg, with a yield of 43.89%). LC-MS (ESI) m/z: 525.36 $(M+H)^+$; $^1H$ NMR (400 MHz, chloroform-d) δ 7.53 (t, *J* = 7.5 Hz, 1H), 7.23 (d, *J*= 6.1 Hz, 1H), 7.15 - 7.08 (m, 1H), 7.05 - 6.96 (m, 1H), 6.88 (d, *J* = 8.6 Hz, 1H), 4.39 - 4.24 (m, 1H), 3.95 (t, *J* = 6.7 Hz, 2H), 3.72 - 3.58 (m, 6H), 3.05 (d, *J* = 11.0 Hz, 2H), 2.65 (t, *J* = 6.7 Hz, 2H), 2.60 - 2.47 (m, 4H), 2.40 (qd, *J* = 12.3, 3.4 Hz, 2H), 2.28 (t, *J*= 11.7 Hz, 2H), 1.79 (d, *J* = 11.6 Hz, 2H).

**Example 36:** Synthesis of 5-chloro-6-fluoro-3(1-((4-isopropylcyclohexyl) piperidin-4-yl)-1-(2-morpholinoethyl)-1,3-dihydro-2*H*-benzo[d]imidazol-2-one (compound **EX36)**

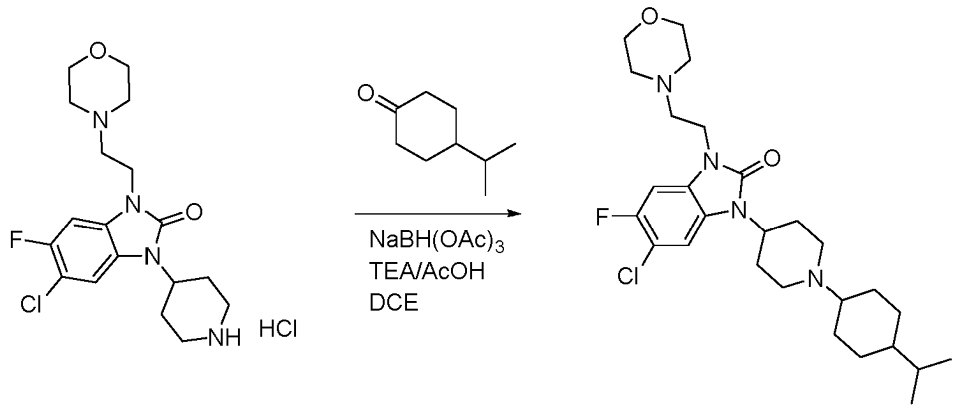


**35b** **EX36**

Synthesis of compound **EX36:** intermediate **35b** and 4-isopropylcyclohexanone were used as raw materials for synthesis according to the synthesis method of compound **EX12** to obtain a white solid product (126 mg, with a yield of 52.1%).

**Example 36A:** Synthesis of 5-chloro-6-fluoro-3(1-((trans-4-isopropylcyclohexyl)piperidin-4-yl)-1-(2-morpholinoethyl)-1,3-dihydro-2*H*-benzo[*d*]imidazol-2-one (compound **EX36A)**

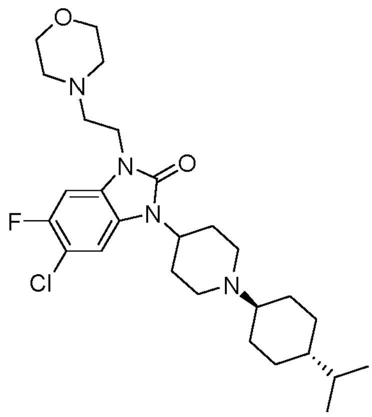

**EX36A**

Synthesis of compound **EX36A:** intermediate **35b** and 4-isopropylcyclohexanone were used as raw materials for synthesis according to the synthesis method of compound EX12 to obtain compound **EX36,** which was subjected to preparation and separation by high performance liquid chromatography (Waters high performance liquid chromatography, chromatographic column: YMC-Triart-C18 EXRS 20 mm×100 mm×5 μm; mobile phase: A = water + 0.1 vol% ammonia (25%), B = acetonitrile; gradient 70%-90% B, 10 min) to obtain a trans product, a white solid product (16 mg, with a yield of 6.61%). LC-MS (ESI) m/z: 507.54 $(M+H)^+$; $^1$H NMR (400 MHz, chloroform-d) δ 7.31 (d, *J* = 6.2 Hz, 1H), 6.85 (d, *J* = 8.7 Hz, 1H), 4.36 - 4.24 (m, 1H), 3.94 (t, *J* = 6.7 Hz, 2H), 3.66 (t, *J* = 4.6 Hz, 4H), 3.06 (d, *J* = 11.1 Hz, 2H), 2.64 (t, *J* = 6.7 Hz, 2H), 2.52 (t, *J* = 4.6 Hz, 4H), 2.45 - 2.22 (m, 5H), 1.92 (d, *J* = 12.1 Hz, 2H), 1.86 - 1.73 (m, 4H), 1.49 - 1.35 (m, 1H), 1.27 (q, *J* = 12.4, 11.7 Hz, 2H), 1.09 - 0.94 (m, 3H), 0.87 (s, 3H), 0.86 (s, 3H).

**Example 36B:** Synthesis of 5-chloro-6-fluoro-3(1-((cis-4-isopropylcyclohexyl) piperidin-4-yl) -1-(2-morpholinoethyl)-1,3-dihydro-2H-benzo[d]imidazol-2-one (compound **EX36B)**

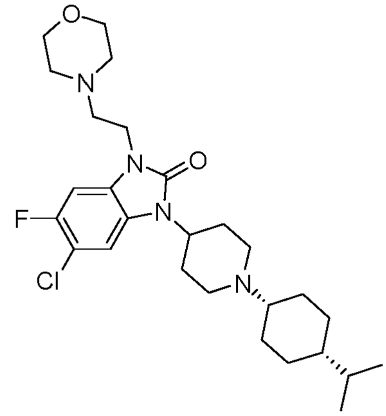

**EX36B**

Synthesis of compound **EX36B:** intermediate **35b** and 4-isopropylcyclohexanone were used as raw materials for synthesis according to the synthesis method of compound **EX12** to obtain compound **EX36,** which was subjected to preparation and separation by high performance liquid chromatography (Waters high performance liquid chromatography, chromatographic column: YMC-Triart-C18 EXRS 20 mm×100 mm×5 μm; mobile phase: A = water + 0.1 vol% ammonia (25%), B = acetonitrile; gradient 70%-90% B, 10 min) to obtain a cis product, a white solid product (46 mg, with a yield of 19.02%). LC-MS (ESI) m/z: 507.52 $(M+H)^+$; $^1$H NMR (400 MHz, chloroform-d) δ 7.27 (d, *J* = 6.1 Hz, 2H), 6.86 (d, *J* = 8.7 Hz, 1H), 4.34 - 4.21 (m, 1H), 3.94 (t, *J* = 6.7 Hz, 2H), 3.67 (t, *J* = 4.6 Hz, 4H), 3.15 (d, *J* = 11.5 Hz, 2H), 2.64 (t, *J* = 6.7 Hz, 2H), 2.52 (t, *J* = 4.5 Hz, 4H), 2.38 - 2.16 (m, 5H), 1.84 - 1.76 (m, 2H), 1.76 - 1.68 (m, 2H), 1.68 - 1.57 (m, 3H), 1.57 - 1.48 (m, 2H), 1.44 - 1.32 (m, 2H), 1.19 - 1.09 (m, 1H), 0.91 (s, 3H), 0.89 (s, 3H).

**Example 37:** Synthesis of 1-(1-(2-chloro-4-fluorobenzyl)piperidin-4-yl)-5,6-difluoro-3-(2-morpholinoethyl)-1,3-dihydro-2*H*-benzo[d]imidazol-2-one (compound **EX37)**

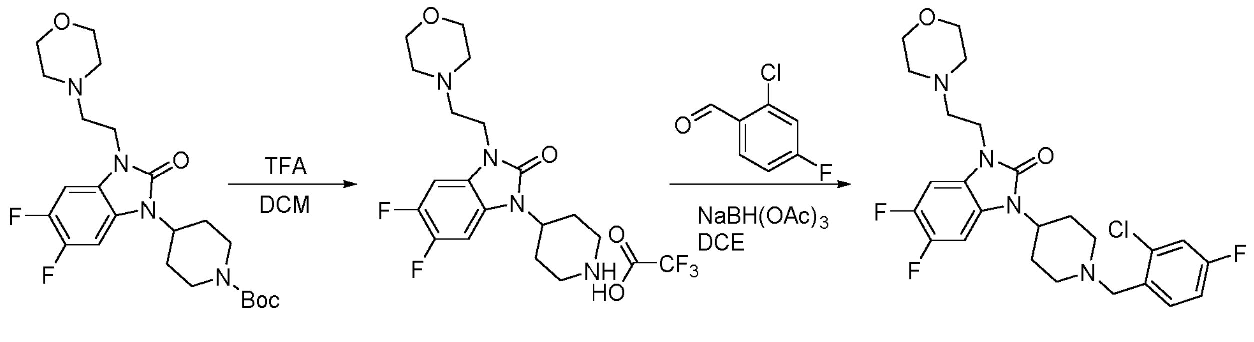


**37a** **37b** **EX37**

Synthesis of intermediate **37a:** 2-chloro-4,5-difluoronitrobenzene was used as raw material for synthesis according to the synthesis method of intermediate **1f** in Example 1 to obtain a product (1.30 g, with a yield of 98.5%). $^1$H NMR (400 MHz, chloroform-d) δ 6.95 (dd, *J*= 10.2, 6.7 Hz, 1H), 6.89 (dd, *J* = 9.7, 6.9 Hz, 1H), 4.46 - 4.21 (m, 3H), 3.94 (t, *J* = 6.7 Hz, 2H), 3.67 (t, *J= 4.6* Hz, 4H), 2.93 - 2.75 (m, 2H), 2.65 (t, *J* = 6.7 Hz, 2H), 2.53 (t, *J* = 4.6 Hz, 4H), 2.22 (qd, *J*= 12.6, 4.6 Hz, 2H), 1.86 - 1.74 (m, 2H), 1.50 (s, 9H).

Synthesis of intermediate **37b:** intermediate **37a** was used as raw material for synthesis according to the synthesis method of intermediate **1g** in Example 1 to obtain a white product (1.00 g, with a yield of 100%). $^1$H NMR (400 MHz, Deuterium Oxide) δ 7.22 (dd, *J* = 10.6, 6.8 Hz, 1H), 7.14 (dd, *J* = 10.1, 6.8 Hz, 1H), 4.47 - 4.32 (m, 1H), 4.22 (t, *J*= 5.9 Hz, 2H), 4.14 - 3.63 (m, 5H), 3.57 - 3.40 (m, 5H), 3.39 - 3.16 (m, 2H), 3.10 (td, *J* = 13.3, 3.0 Hz, 2H), 2.48 (qd, *J*= 13.5, 4.3 Hz,

2H), 2.03 (d, *J* = 13.7 Hz, 2H).

Synthesis of compound **EX37:** intermediate **37b** and 2-chloro-4-fluorobenzaldehyde were used as raw materials for synthesis according to the synthesis method in Example 1 to obtain a white solid product (106 mg, with a yield of 48.26%). LC-MS (ESI) m/z: 509.38 $(M+H)^+$; $^1H$ NMR (400 MHz, chloroform-*d*) δ 7.52 (t, *J* = 7.6 Hz, 1H), 7.16 - 7.05 (m, 2H), 7.05 - 6.96 (m, 1H), 6.93 - 6.83 (m, 1H), 4.40 - 4.24 (m, 1H), 3.95 (t, *J* = 6.7 Hz, 2H), 3.72 - 3.64 (m, 4H), 3.63 (s, 2H), 3.04 (d, *J* = 10.8 Hz, 2H), 2.65 (t, *J* = 6.8 Hz, 2H), 2.59 - 2.46 (m, 4H), 2.46 - 2.15 (m, 4H), 1.79 (d, *J* = 11.6 Hz, 2H).

**Example 38:** Synthesis of 5,6-difluoro-3(1-((4-isopropylcyclohexyl) piperidin-4-yl)-1-(2-morpholinoethyl)-1,3-dihydro-2*H*-benzo[*d*]imidazol-2-one (compound **EX38)**

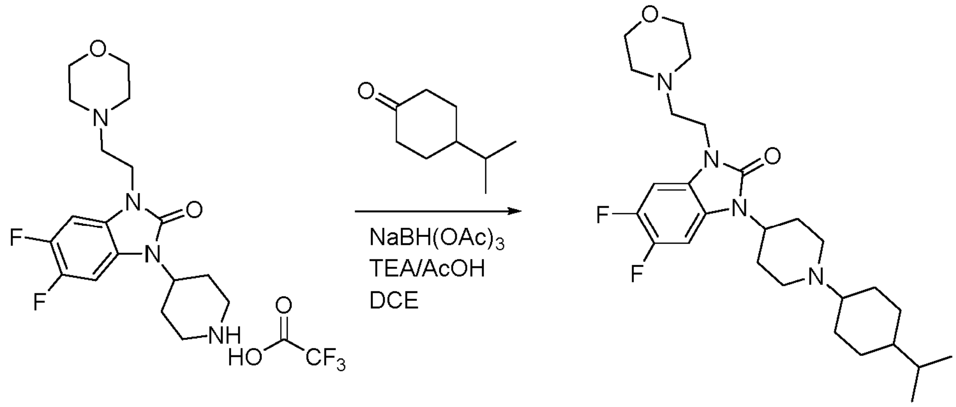


**37b** **EX38**

Synthesis of compound **EX38:** intermediate **37b** and 4-isopropylcyclohexanone were used as raw materials for synthesis according to the synthesis method of compound **EX12** to obtain a white solid product (132 mg, with a yield of 54.5%).

**Example 38A:** Synthesis of 5,6-difluoro-3(1-((trans-4-isopropylcyclohexyl) piperidin-4-yl)-1-(2-morpholinoethyl)-1,3-dihydro-2*H*-benzo[*d*]imidazol-2-one (compound **EX38A)**

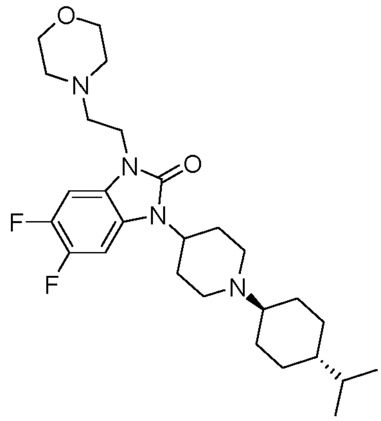

**EX38A**

Synthesis of compound **EX38A:** intermediate **37b** and 4-isopropylcyclohexanone were used as raw materials for synthesis according to the synthesis method of compound **EX12** to obtain compound **EX38,** which was subjected to preparation and separation by high performance liquid chromatography (Waters high performance liquid chromatography, chromatographic column: YMC-Triart-C18 EXRS 20 mm×100 mm×5 μm; mobile phase: A = water + 0.1 vol% ammonia (25%), B = acetonitrile; gradient 70%-90% B, 10 min) to obtain a trans product, a white solid product (15 mg, with a yield of 6.16%). LC-MS (ESI) m/z: 491.52 $(M+H)^+$; $^1H$ NMR (400 MHz, chloroform-*d*) δ 7.18 (dd, *J* = 10.5, 6.8 Hz, 1H), 6.86 (dd, *J* =

9.9, 6.8 Hz, 1H), 4.35 - 4.24 (m, 1H), 3.94 (t, *J* = 6.8 Hz, 2H), 3.67 (t, *J* = 4.6 Hz, 4H), 3.05 (d, *J* = 11.0 Hz, 2H), 2.64 (t, *J* = 6.8 Hz, 2H), 2.52 (t, *J* = 4.6 Hz, 4H), 2.45 - 2.16 (m, 6H), 1.91 (d, *J* = 11.2 Hz, 2H), 1.86 - 1.72 (m, 4H), 1.45 - 1.38 (m, 1H), 1.33 - 1.19 (m, 2H), 1.09 - 1.00 (m, 2H), 0.87 (s, 3H), 0.86 (s, 3H).

**Example 38B:** Synthesis of 5,6-difluoro-3(1-((cis-4-isopropylcyclohexyl) piperidin-4-yl)-1-(2-morpholinoethyl)-1,3-dihydro-2*H*-benzo[*d*]imidazol-2-one (compound **EX38B)**

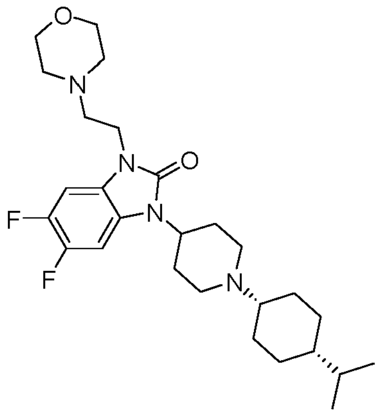

**EX38B**

Synthesis of compound **EX38B:** intermediate **37b** and 4-isopropylcyclohexanone were used as raw materials for synthesis according to the synthesis method of compound **EX12** to obtain compound **EX38,** which was subjected to preparation and separation by high performance liquid chromatography (Waters high performance liquid chromatography, chromatographic column: YMC-Triart-C18 EXRS 20 mm×100 mm×5 μm; mobile phase: A = water + 0.1 vol% ammonia (25%), B = acetonitrile; gradient 70%-90% B, 10 min) to obtain a cis product, a white solid product (45 mg, with a yield of 18.47%). LC-MS (ESI) m/z: 491.52 $(M+H)^+$; $^1H$ NMR (400 MHz, chloroform-d) δ 7.14 (dd, *J* = 10.4, 6.8 Hz, 1H), 6.86 (dd, *J* = 9.9, 6.8 Hz, 1H), 4.33 - 4.23 (m, 1H), 3.94 (t, *J* = 6.8 Hz, 2H), 3.67 (t, *J* = 4.6 Hz, 4H), 3.15 (d, *J* = 11.4 Hz, 2H), 2.64 (t, *J* = 6.8 Hz, 2H), 2.52 (t, *J* = 4.5 Hz, 4H), 2.37 - 2.12 (m, 5H), 1.84 - 1.75 (m, 3H), 1.75 - 1.57 (m, 5H), 1.57 - 1.47 (m, 2H), 1.44 - 1.33 (m, 2H), 1.19 - 1.07 (m, 1H), 0.90 (s, 3H), 0.89 (s, 3H).

**Example 39:** Synthesis of 5,6-dichloro-1-(1-(2-chloro-4-fluorobenzyl) piperidin-4-yl)-3-(2-(1,1-dioxothiomorpholine) ethyl)-1,3-dihydro-2*H*-benzo[*d*]imidazol-2-one (compound **EX39)**

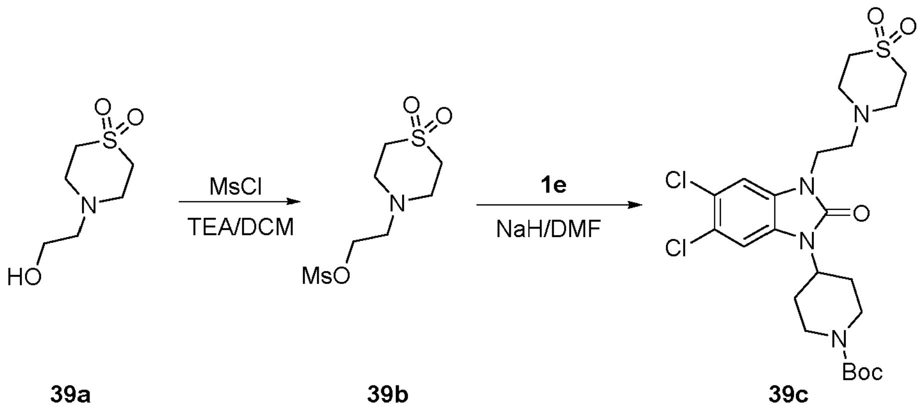


39a 39b 39c

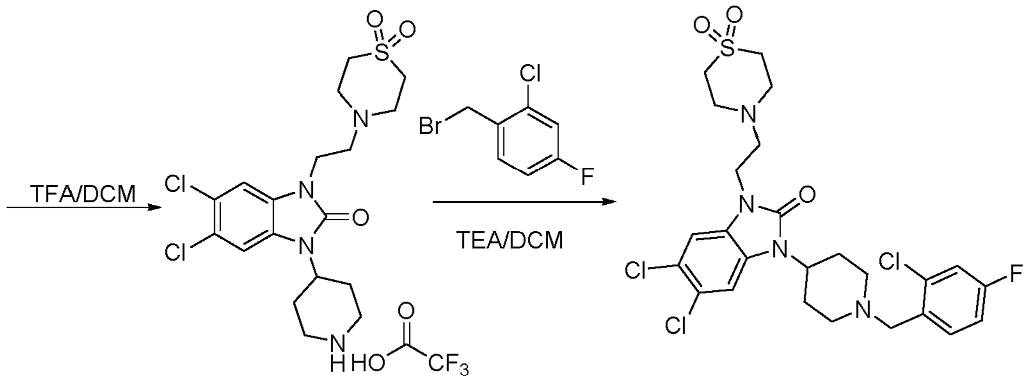


39d EX39

Synthesis of intermediate **39c:** 4-(2-hydroxyethyl)thiomorpholine-1,1-dioxide (2.00 g, 11.16 mmol) and anhydrous dichloromethane (120 mL) were added into a dry single neck flask (200 mL). Triethylamine (1.69 g, 2.33 mL, 16.74 mmol) was added to the reaction flask, under argon displacement protection, the flask was cooled to 0 °C in an ice water bath, methylsulfonyl chloride (1.53 g, 1.04 mL, 13.39 mmol) was added to the reaction system, and thereafter, stirring was continued at room temperature for reaction for 2 hours. The reaction of the raw materials was detected, by TLC, to be substantially complete. Saturated $NaHCO_3$ aqueous solution was added for quenching the reaction, followed by liquid separation. The organic phase was washed by saturated NaCl aqueous solution, followed by drying with anhydrous MgSO4, filtering, and rotary-drying to obtain light brown oily crude product **39b** (2.15 g, with a yield of 74.88%). The crude product was directly used in the next reaction without further purification.

*Tert*-butyl 4-(5,6-dichloro-2-carbonyl-2,3-dihydro-1*H*benzo[*d*]imidazol-1-yl) piperidin-1-carboxylate **(1e)** (500 mg, 1.29 mmol) and anhydrous DMF (8 mL) were added into a dry double-necked flask (25 mL), under argon displacement protection, the flask was cooled to 0°C in an ice water bath. NaH (207 mg, 5.18 mmol, 60% in mineral oil) was added to the reaction flask. Stirring was continued in an ice water bath for reaction for 30 minutes, and 2-(1,1-dioxothiomorpholine)ethyl mesylate **(39b)** (833 mg, 3.24 mmol) anhydrous DMF (5 mL) solution was added dropwise to the reaction mixture, with the temperature being held at 0°C, stirring was performed for reaction for 1 hour, and the temperature was gradually raised to 45°C for reaction for 12 hours. The reaction of the raw materials was detected, by TLC, to be substantially complete. After the completion of the reaction, the reaction mixture solution was cooled to room temperature, and slowly poured into ice water, and white solid was precipitated. After the solid was filtered out, washing with water, and drying were performed, and the crude product was dispersed in petroleum ether, followed by slurrying, filtering, and drying to obtain a white solid product (632 mg, with a yield of 89.2%). $^1$H NMR (400 MHz, chloroform-d) δ 7.19 (s, 1H), 7.03 (s, 1H), 4.45 - 4.24 (m, 3H), 3.95 (t, *J* = 6.2 Hz, 2H), 3.11 - 3.03 (m, 4H), 3.03 - 2.96 (m, 4H), 2.88 - 2.74 (m, 4H), 2.24 (qd, *J* = 12.6, 4.6 Hz, 2H), 1.85 - 1.73 (m, 2H), 1.51 (s, 9H).

Synthesis of intermediate **39d:** Synthesis was performed according to the synthesis method of intermediate **1f** in Example 1 to obtain a white solid product (205 mg, with a yield of 99.9%). The solid was directly used in the next reaction without purification.

Synthesis of compound **EX39:** intermediate **39d** (200 mg, 356 μmol) and anhydrous dichloromethane (3.5 mL) were added into a dry round-bottom flask (50 mL), triethylamine (216 mg, 2.14 mmol) was added to the reaction solution, which was stirred for 5 minutes and cooled to 0°C in an ice water bath, under argon protection, 2-chloro-4-fluorobenzyl

bromide (87.6 mg, 392 μmol) dichloromethane (1.5 mL) solution was added dropwise to the reaction mixture solution. Thereafter, the temperature was gradually raised to room temperature and stirring was continued for reaction for 12 hours. The reaction was detected, by TLC plate, to be completed. Saturated sodium bicarbonate aqueous solution was added to the reaction mixture solution for quenching the reaction, followed by extraction with dichloromethane (25 mL×3), washing with saturated saline, drying with anhydrous magnesium sulfate, and filtering. The filtrate was evaporated by a rotary evaporator to remove the solvent to obtain a light brown crude product, which was subjected to purification and separation with silica gel column chromatography (the mobile phase was DCM/MeOH, in a ratio of 150:1 to 100:1) to obtain a white solid product (165 mg, with a yield of 78.51%). LC-MS (ESI) m/z: 589.20 $(M+H)^+$; $^1$H NMR (400 MHz, chloroform-d) δ 7.51 (t, *J* = 7.4 Hz, 1H), 7.32 (s, 1H), 7.13 (dd, *J* = 8.6, 2.6 Hz, 1H), 7.07 - 6.95 (m, 2H), 4.35 - 4.19 (m, 1H), 3.95 (t, *J* = 6.2 Hz, 2H), 3.64 (s, 2H), 3.18 - 3.02 (m, 6H), 3.02 - 2.91 (m, 4H), 2.82 (t, *J* = 6.2 Hz, 2H), 2.48 - 2.33 (m, 2H), 2.33 - 2.19 (m, 2H), 1.86 - 1.62 (m, 3H).

**Example 40:** Synthesis of 5,6-dichloro-1-(1-(2-chloro-4-fluorobenzyl)piperidin-4-yl)-3-(2-(4-methylpiperazin-1-yl) ethyl-1,3-dihydro-2*H*-benzo[*d*]imidazol-2-one (compound **EX40)**

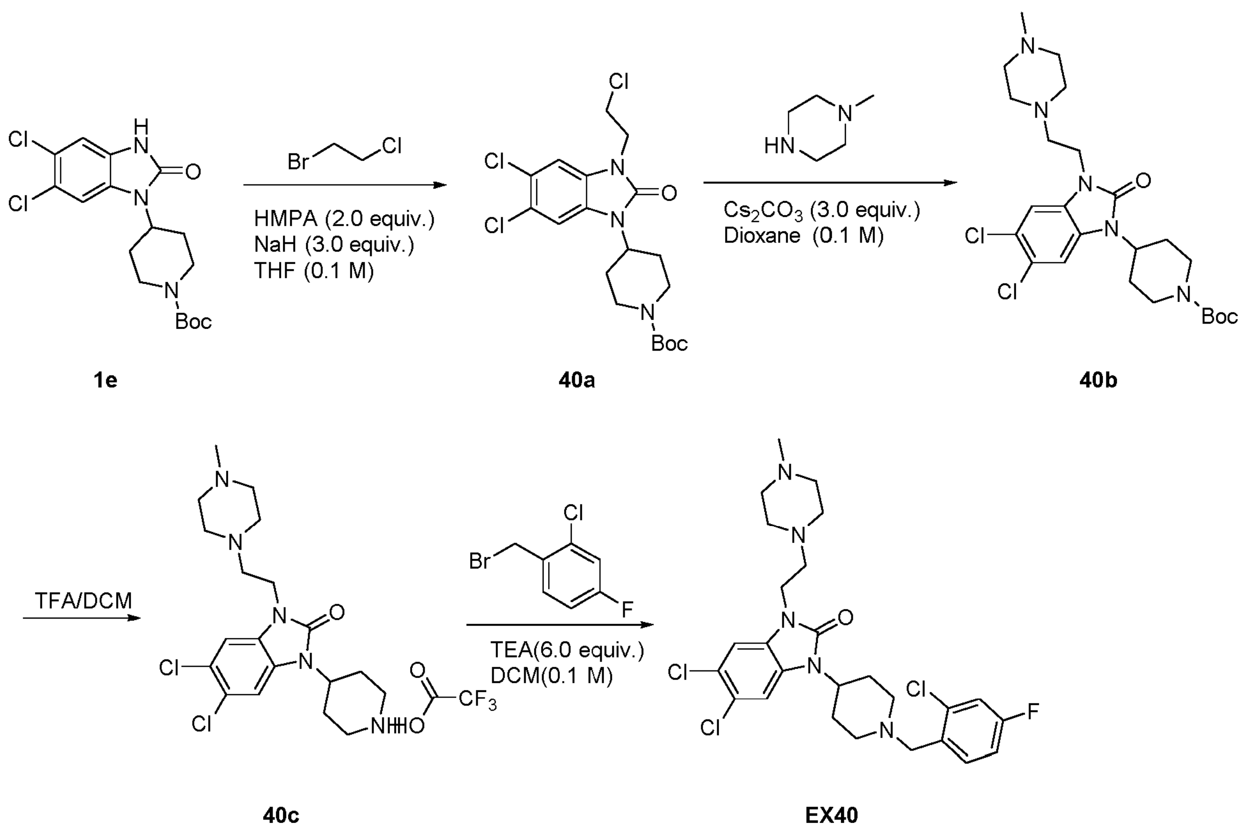


Synthesis of intermediate **40a:** *tert*-butyl 4-(5,6-dichloro-2-carbonyl-2, 3-dihydro-1*H*-benzo[*d*]imidazol-1-yl]piperidin-1-carboxylate (3.00 g, 7.77 mmol), hexamethylphosphoryl triamine (HMPA) (2.78 g, 15.53 mmol), and anhydrous tetrahydrofuran (80 mL) were added into a clean dry double-mouth flask (200 mL). Under argon displacement protection, the flask was cooled to 0°C in an ice water bath. NaH (932 mg, 23.30 mmol, 60% in mineral oil) was added to the reaction mixture in batches. After stirring for half an hour, 1-chloro-2bromoethane (4.46 g, 31.07 mmol, 2.68 mL) was slowly added to the reaction system. Thereafter, the ice water bath was removed, stirring was continued at room temperature for one hour, and the temperature was gradually raised to 80°C for reflux reaction for 8 hours. The reaction of the raw materials was detected, by TLC plate, to be substantially complete. After the completion of the reaction, the reaction system was cooled to room temperature, and under stirring condition, ice water was added dropwise to the reaction system for quenching the reaction, followed by extraction with ethyl acetate (150 mL×3). The organic phase was washed with saturated $NH_4Cl$ aqueous solution and saturated NaCl aqueous solution respectively, dried with anhydrous $Na_2SO_4$, and then filtered and evaporated with a rotary evaporator to remove the solvent, to obtain a light brown solid crude product. The crude product was dispersed in a petroleum ether/ethyl acetate (1% ethyl acetate) mixed solvent, stirred for 30 minutes, and then filtered to obtain a white solid product (2.91 g, with a yield of 83.5%). $^1$H NMR (400 MHz, chloroform-*d*) δ 7.19 (s, 1H), 7.17 (s, 1H),

4.47 - 4.22 (m, 3H), 4.16 (t, *J* = 6.1 Hz, 2H), 3.82 (t, *J* = 6.1 Hz, 2H), 2.96 - 2.73 (m, 2H), 2.24 (qd, *J* = 12.7, 4.6 Hz, 2H), 1.88 - 1.72 (m, 2H), 1.51 (s, 9H).

Synthesis of intermediate **40b:** *tert*-butyl 4-(5,6-dichloro-3-(2-chloroethyl)-2-carbonyl-2,3-dihydro-1*H*-benzo[*d*] imidazol-1-yl) piperidin-1-carboxylate **(40a)** (325 mg, 724 μmol), and $Cs_2CO_3$ (708 mg, 2.17 mmol) were added into a clean dry Schlenk tube (25 mL), anhydrous dioxane (7.2 mL) was added thereto, under stirring at room temperature, *N*-methylpiperazine (290 mg, 321 μL, 2.90 mmol) was added, and under argon displacement protection, the tube was heated to 105°C for reflux reaction for 12 hours. The reaction of the raw materials was detected, by TLC plate, to be substantially complete. After the completion of the reaction, the reaction flask was cooled to room temperature, an appropriate amount of water was added thereto, followed by extraction with ethyl acetate (15 mL×3). The organic phase was washed with saturated NaCl aqueous solution, and dried with anhydrous $Na_2SO_4$, and then filtered and evaporated with a rotary evaporator to remove the solvent, to obtain a light brown oily crude product, which was subjected to separation by silica gel column chromatography (the mobile phase was DCM/MeOH, with 0.5% ~ 2% MeOH) to obtain a light yellow waxy product (301 mg, with a yield of 81.10%). $^1$H NMR (400 MHz, chloroform-*d*) δ 7.16 (d, *J* = 0.8 Hz, 2H), 4.48 - 4.19 (m, 3H), 3.94 (t, *J* = 6.7 Hz, 2H), 2.93 - 2.76 (m, 2H), 2.73 - 2.50 (m, 6H), 2.50 - 2.30 (m, 4H), 2.28 (s, 4H), 2.28 - 2.16 (m, 3H), 1.85 - 1.75 (m, 2H), 1.51 (s, 9H).

Synthesis of intermediate **40c: 40b** was used as raw material for synthesis according to the synthesis method of intermediate **1g** in Example 1 to obtain a white solid product (273 mg, with a yield of 100%). The solid was directly used in the next reaction without purification.

Synthesis of compound **EX40: 40c** and 2-chloro-4-fluorobromobenzyl were used as raw materials for synthesis according to the synthesis method in Example 39 to obtain a white solid product (198 mg, with a yield of 69.05%). LC-MS (ESI) m/z: 554.20 (M+H)$^+$; $^1$H NMR (400 MHz, chloroform-*d*) δ 7.52 (t, *J* = 7.4 Hz, 1H), 7.30 (s, 1H), 7.17 - 7.09 (m, 2H), 7.01 (t, *J* = 8.3 Hz, 1H), 4.37 - 4.22 (m, 1H), 3.94 (t, *J* = 6.6 Hz, 2H), 3.63 (s, 2H), 3.05 (d, *J* = 11.1 Hz, 2H), 2.72 - 2.54 (m, 6H), 2.55 - 2.32 (m, 6H), 2.33 - 2.20 (m, 5H), 1.78 (d, *J* = 10.8 Hz, 2H).

**Example 41:** Synthesis of 1-(2-(4-acetylpiperazin-1-yl)ethyl)-5,6-3-(1-(2-chloro-4-fluorobenzyl)piperidin-4-yl)-1,3-dihydro-2*H*-benzo[*d*]imidazol-2-one (compound **EX41)**

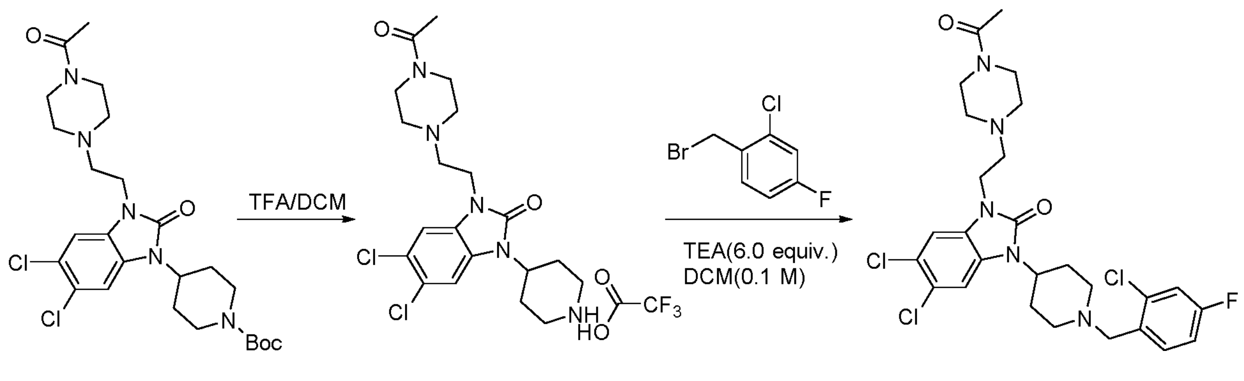


**41a** **41b** **EX41**

Synthesis of intermediate **41a:** 1-acetylpiperazine and **40a** were used as raw materials for synthesis according to the synthesis method of intermediate **40b** in Example 40 to obtain a light yellow waxy product (266 mg, with a yield of 73.62%). $^1$H NMR (400 MHz, chloroform-d) δ 7.16 (d, *J* = 0.8 Hz, 2H), 4.48 - 4.19 (m, 3H), 3.94 (t, *J* = 6.7 Hz, 2H), 2.93 - 2.76 (m, 2H), 2.73 - 2.50 (m, 6H), 2.50 - 2.30 (m, 4H), 2.28 (s, 4H), 2.28 - 2.16 (m, 3H), 1.85 - 1.75 (m, 2H), 1.51 (s, 9H).

Synthesis of compound **EX41: 41a** was used as raw material for synthesis according to the synthesis method of intermediate **40c** in Example 40 to obtain intermediate **41b** (235 mg, with a yield of 99.6%). Intermediate **41b** and 2-chloro-4-fluorobromobenzyl were used as raw materials for synthesis according to the synthesis method in Example 39 to obtain a white solid product (116 mg, with a yield of 46.75%). LC-MS (ESI) m/z: 584.20 (M+3H)$^+$; $^1$H NMR (400 MHz, chloroform-d) δ 7.52 (t, *J* = 7.5 Hz, 1H), 7.30 (s, 1H), 7.15 - 7.07 (m, 2H), 7.05 - 6.96 (m, 1H), 4.35 - 4.23 (m, 1H), 3.96 (t, *J* = 6.4 Hz, 2H), 3.64 (s, 2H), 3.57 (t, *J* = 5.0 Hz, 2H), 3.42 (t, *J* = 5.0 Hz, 2H), 3.05 (d, *J* = 11.0 Hz, 2H), 2.67 (t, *J* = 6.4 Hz, 2H), 2.53 (t, *J* = 5.0 Hz, 2H), 2.48 (t, *J* = 5.0 Hz, 2H), 2.39 (qd, *J* = 12.4, 3.4 Hz, 2H), 2.27 (t, *J* = 11.7 Hz, 2H), 2.07 (s, 3H), 1.78 (d, *J* = 11.2 Hz, 2H).

**Example 42:** Synthesis of 5,6-1-(1-(2-chloro-4-fluorobenzyl)piperidin-4-yl)-3-(2-(3-oxopiperazin-1-yl)ethyl)-1,3-dihydro-2*H*-benzo[*d*]imidazol-2-one (compound **EX42)**

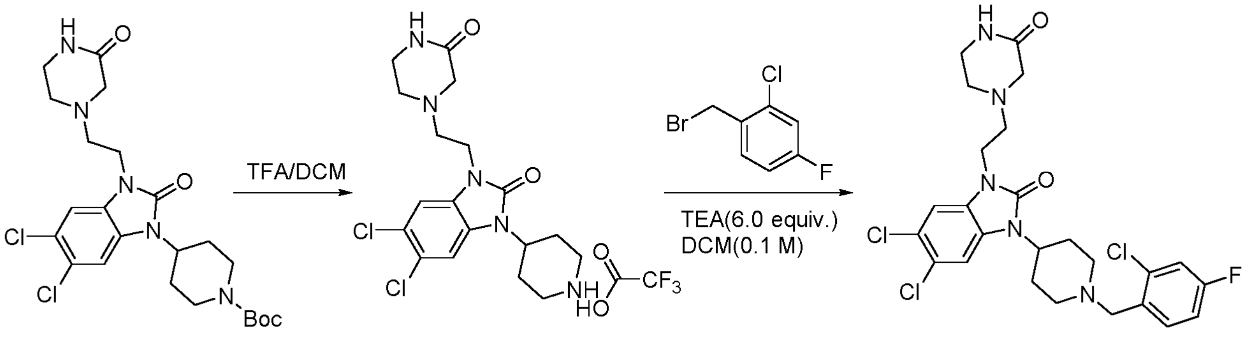


**42a** **42b** **EX42**

Synthesis of intermediate **42a:** 2-piperazinone and **40a** were used as raw materials for synthesis according to the synthesis method of intermediate **40b** in Example 40. to obtain a colorless waxy product (167 mg, with a yield of 48.8%). $^1$H NMR (400 MHz, chloroform-d) δ 7.18 (s, 1H), 7.07 (s, 1H), 6.43 (s, 1H), 4.47 - 4.19 (m, 3H), 3.97 (t, *J* = 6.4 Hz, 2H), 3.38 - 3.29 (m, 2H), 3.19 (s, 2H), 2.95 - 2.80 (m, 2H), 2.79 - 2.68 (m, 4H), 2.24 (qd, *J* = 12.6, 4.5 Hz, 2H), 1.87 - 1.73 (m, 2H), 1.51 (s, 9H).

Synthesis of compound **EX42: 42a** was used as raw material for synthesis according to the synthesis method of intermediate **40c** in Example 40 to obtain intermediate **42b** (150 mg, with a yield of 100%). Intermediate **42b** and 2-chloro-4-fluorobromobenzyl were used as raw materials for synthesis according to the synthesis method in Example 39 to obtain a white solid product (88 mg, with a yield of 56.02%). LC-MS (ESI) m/z: 554.20 $(M+H)^+$; $^1$H NMR (400 MHz, chloroform-d) δ 7.51 (t, *J* = 7.3 Hz, 1H), 7.30 (s, 1H), 7.12 (d, *J* = 8.5 Hz, 1H), 7.06 (s, 1H), 7.01 (t, *J* = 8.4 Hz, 1H), 6.42 (s, 1H), 4.34 - 4.22 (m, 1H), 3.96 (t, *J* = 6.5 Hz, 2H), 3.63 (s, 2H), 3.38 - 3.27 (m, 2H), 3.19 (s, 2H), 3.04 (d, *J* = 11.1 Hz, 2H), 2.81 - 2.66 (m, 4H), 2.38 (qd, *J* = 12.1, 3.5 Hz, 2H), 2.27 (t, *J* = 11.6 Hz, 2H), 1.78 (d, *J* = 11.4 Hz, 2H).

**Example 43:** Synthesis of 5,6-dichloro-1-(1-(2-chloro-4-fluorobenzyl) piperidin-4-yl)-3-(2-(pyrrolidin-1-yl)ethyl)-1,3-dihydro-2*H*-benzo[*d*]imidazol-2-one (compound **EX43)**

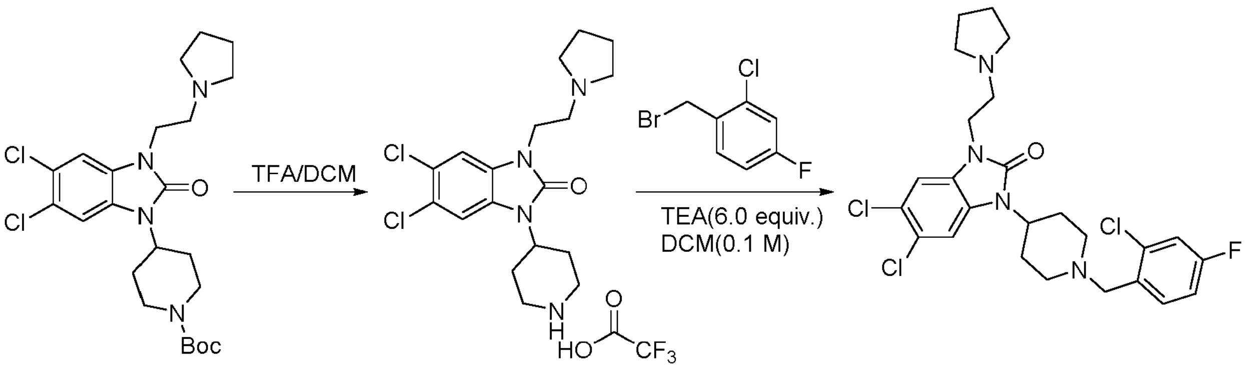


**43a** **43b** **EX43**

Synthesis of intermediate **43a:** pyrrole and **40a** were used as raw materials for synthesis according to the synthesis method of intermediate **40b** in Example 40 to obtain a colorless oily product (485 mg, with a yield of 90.1%). $^1$H NMR (400 MHz, chloroform-*d*) δ 7.16 (s, 1H), 7.13 (s, 1H), 4.41 (tt, *J* = 12.5, 4.2 Hz, 1H), 4.31 (s, 2H), 3.97 (t, *J* = 7.2 Hz, 2H), 2.86 (d, *J* = 13.8 Hz, 2H), 2.78 (t, *J* = 7.2 Hz, 2H), 2.65 - 2.55 (m, 4H), 2.23 (qd, *J* = 12.7, 4.6 Hz, 2H), 1.82 - 1.76 (m, 6H), 1.51 (s, 9H).

Synthesis of compound **EX43: 43a** was used as raw material for synthesis according to the synthesis method of intermediate **40c** in Example 40 to obtain intermediate **43b** (333 mg, with a yield of 90.5%). Intermediate **43b** and 2-chloro-4-fluorobromobenzyl were used as raw materials for synthesis according to the synthesis method in Example 39 to obtain a white solid product (182 mg, with a yield of 51.40%). LC-MS (ESI) m/z: 525.20 $(M+H)^+$; $^1H$ NMR (400 MHz, chloroform-d) δ 7.52 (t, *J* = 7.5 Hz, 1H), 7.29 (s, 1H), 7.16 - 7.07 (m, 2H), 7.05 - 6.96 (m, 1H), 4.39 - 4.23 (m, 1H), 3.97 (t, *J* = 7.2 Hz, 2H), 3.63 (s, 2H), 3.04 (d, *J* = 10.9 Hz, 2H), 2.79 (t, *J* = 7.3 Hz, 2H), 2.69 - 2.51 (m, 4H), 2.38 (qd, *J* = 12.3, 3.3 Hz, 2H), 2.26 (t, *J* = 11.6 Hz, 2H), 1.86 - 1.72 (m, 6H).

**Example 44:** Synthesis of 5,6-dichloro-1-(1-(2-chloro-4-fluorobenzyl) piperidin-4-yl)-3-(2-(piperidin-1-yl)ethyl)-1 ,3-dihydro-2*H*-benzo[*d*]imidazol-2-one (compound **EX44)**

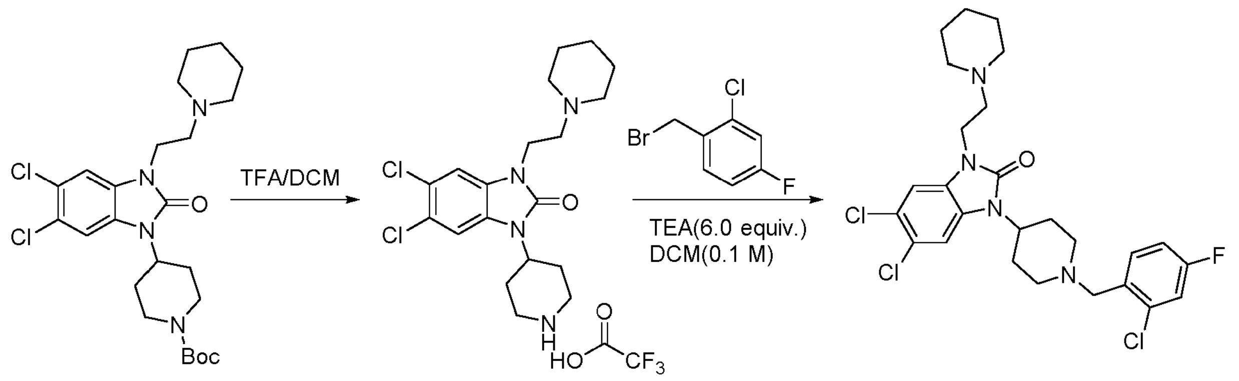


**44a** **44b** **EX44**

Synthesis of intermediate **44a:** piperidine and **40a** were used as raw materials for synthesis according to the synthesis method of intermediate **40b** in Example 40 to obtain a colorless oily product (461 mg, with a yield of 83.2%). $^1H$ NMR (400 MHz, chloroform-d) δ 7.19 (s, 1H), 7.15 (s, 1H), 4.40 (tt, *J* = 12.5, 4.1 Hz, 1H), 4.31 (s, 2H), 3.93 (t, *J* = 6.9 Hz, 2H), 2.84 (t, *J* = 13.4 Hz, 2H), 2.59 (t, *J* = 6.9 Hz, 2H), 2.53 - 2.37 (m, 4H), 2.23 (qd, *J* = 12.7, 4.6 Hz, 2H), 1.79 (d, *J* = 11.8 Hz, 2H), 1.60 - 1.53 (m, 4H), 1.51 (s, 9H), 1.47 - 1.39 (m, 2H).

Synthesis of compound **EX44:** 44a was used as raw material for synthesis according to the synthesis method of intermediate **40c** in Example 40 to obtain intermediate **44b** (303 mg, with a yield of 96.2%). Intermediate **44b** and 2-chloro-4-fluorobromobenzyl were used as raw materials for synthesis according to the synthesis method in Example 39 to obtain a white solid product (110 mg, with a yield of 27.1%). LC-MS (ESI) m/z: 539.20 $(M+H)^+$; $^1H$ NMR (400 MHz, chloroform-d) δ 7.52 (t, *J* = 7.5 Hz, 1H), 7.28 (d, *J* = 1.9 Hz, 1H), 7.19 (s, 1H), 7.15 - 7.09 (m, 1H), 7.05 - 6.96 (m, 1H), 4.37 - 4.25 (m, 1H), 3.95 (t, *J* = 6.9 Hz, 2H), 3.63 (s, 2H), 3.04 (d, *J* = 11.0 Hz, 2H), 2.60 (t, *J* = 7.0 Hz, 2H), 2.54 - 2.43 (m, 4H), 2.43 - 2.31 (m, 2H), 2.27 (t, *J* = 11.6 Hz, 2H), 1.78 (d, *J* = 11.6 Hz, 2H), 1.64 - 1.51 (m, 4H), 1.50 - 1.38 (m, 2H).

**Example 45:** Synthesis of 5,6-dichloro-1-(1-(cyclopropylmethyl) piperidin-4-yl)-3-(2-morpholinoethyl)-1,3-dihydro-2*H*-benzo[*d*]imidazol-2-one (compound **EX45)**

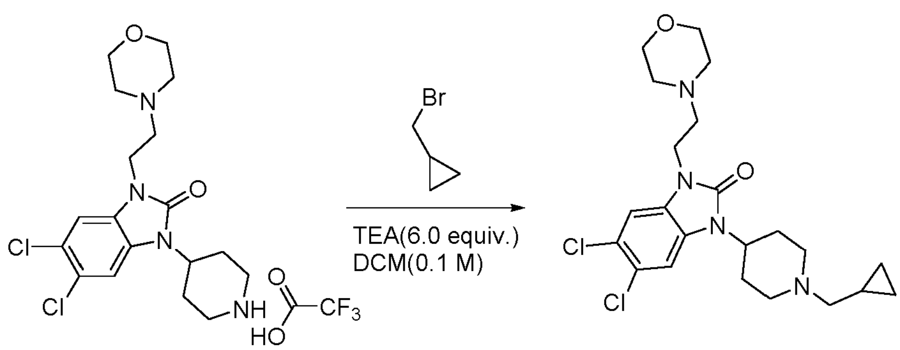


**1e** **EX45**

Intermediate **1e** and bromomethyl cyclopropane were used as raw materials for synthesis according to the synthesis method in Example 39 to obtain a white solid product (88 mg, with a yield of 50.0%). LC-MS (ESI) m/z: 453.20 $(M+H)^+$; $^1H$ NMR (400 MHz, chloroform-*d*) δ 7.42 (s, 1H), 7.12 (s, 1H), 4.42 - 4.27 (m, 1H), 3.95 (t, *J* = 6.6 Hz, 2H), 3.73 - 3.60 (m, 4H), 3.34 - 3.18 (m, 2H), 2.65 (t, *J* = 6.7 Hz, 2H), 2.59 - 2.46 (m, 4H), 2.47 - 2.26 (m, 4H), 2.24 - 2.10 (m, 2H), 1.87 - 1.75 (m, 2H), 0.99 - 0.84 (m, 1H), 0.62 - 0.49 (m, 2H), 0.21 - 0.06 (m, 2H).

**Example 46:** Synthesis of 5,6-dichloro-1-(1-(2-chloro-4-fluorobenzyl) piperidin-4-yl)-3-(2-(dimethylamino)ethyl)-1,3-dihydro-2*H*-benzo[*d*]imidazol-2-one (compound **EX46)**

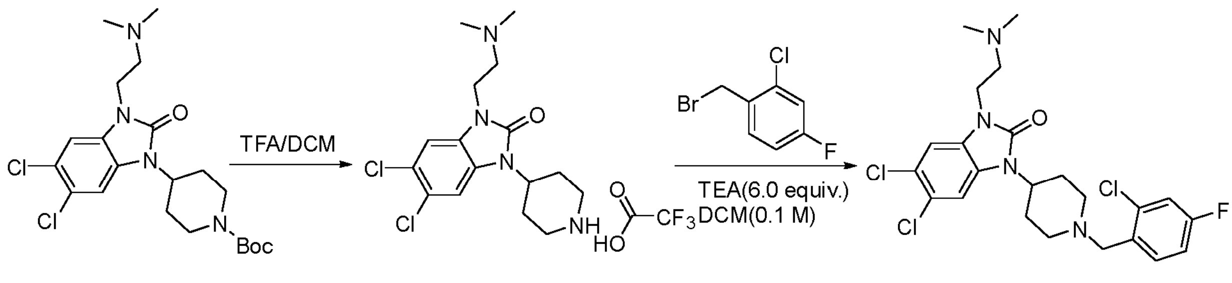


**46a** **46b** **EX46**

Synthesis of intermediate **46a:** *N*,*N*-dimethyl bromide ethyl hydrobromide and intermediate **1e** were used as raw materials for synthesis according to the synthesis method of intermediate **1f** to obtain a pink solid product (1.1 g, with a yield of 93.2%). $^1H$ NMR (400 MHz, chloroform-d) δ 7.16 (s, 1H), 7.09 (s, 1H), 4.44-4.31 (m, 3H), 3.93-3.90 (t, *J* = 6.8 Hz, 2H), 2.87-2.81 (t, *J* = 12.4 Hz, 2H), 2.62-2.58 (t, *J* = 6.8 Hz, 2H), 2.31 (s, 6H), 2.28-2.17 (m, 2H), 1.81-1.78 (d, *J* = 10.4 Hz, 2H), 1.50 (s, 9H).

Synthesis of compound **EX46: 46a** was used as raw material for synthesis according to the synthesis method of intermediate **40c** in Example 40 to obtain intermediate **46b** (1.1 g, with a yield of 98.9%). Intermediate **46b** and 2-chloro-4-fluorobromobenzyl were used as raw materials for synthesis according to the synthesis method in Example 39 to obtain a white solid product (300 mg, with a yield of 56.6%). LC-MS (ESI) m/z: 499.20 $(M+H)^+$; $^1H$ NMR (400 MHz, chloroform-d) δ 7.53 (t, *J* = 7.5 Hz, 1H), 7.31 (s, 1H), 7.15 - 7.08 (m, 2H), 7.04 - 6.97 (m, 1H), 4.38 - 4.25 (m, 1H), 3.96 (t, *J* = 7.0 Hz, 2H), 3.64 (s, 2H), 3.05 (d, *J* = 10.9 Hz, 2H), 2.66 (t, *J* = 6.9 Hz, 2H), 2.46 - 2.32 (m, 8H), 2.28 (t, *J* = 11.7 Hz, 2H), 1.79 (d, *J* = 11.7 Hz, 2H).

**Example 47:** Synthesis of 6,7-dichloro-3-(1-(2-chloro-4-fluorobenzyl) piperidin-4-yl)-1-(2-morpholinoethyl)-3,4-dihydroquinazolin-2(1*H*)-one (compound **EX47)**

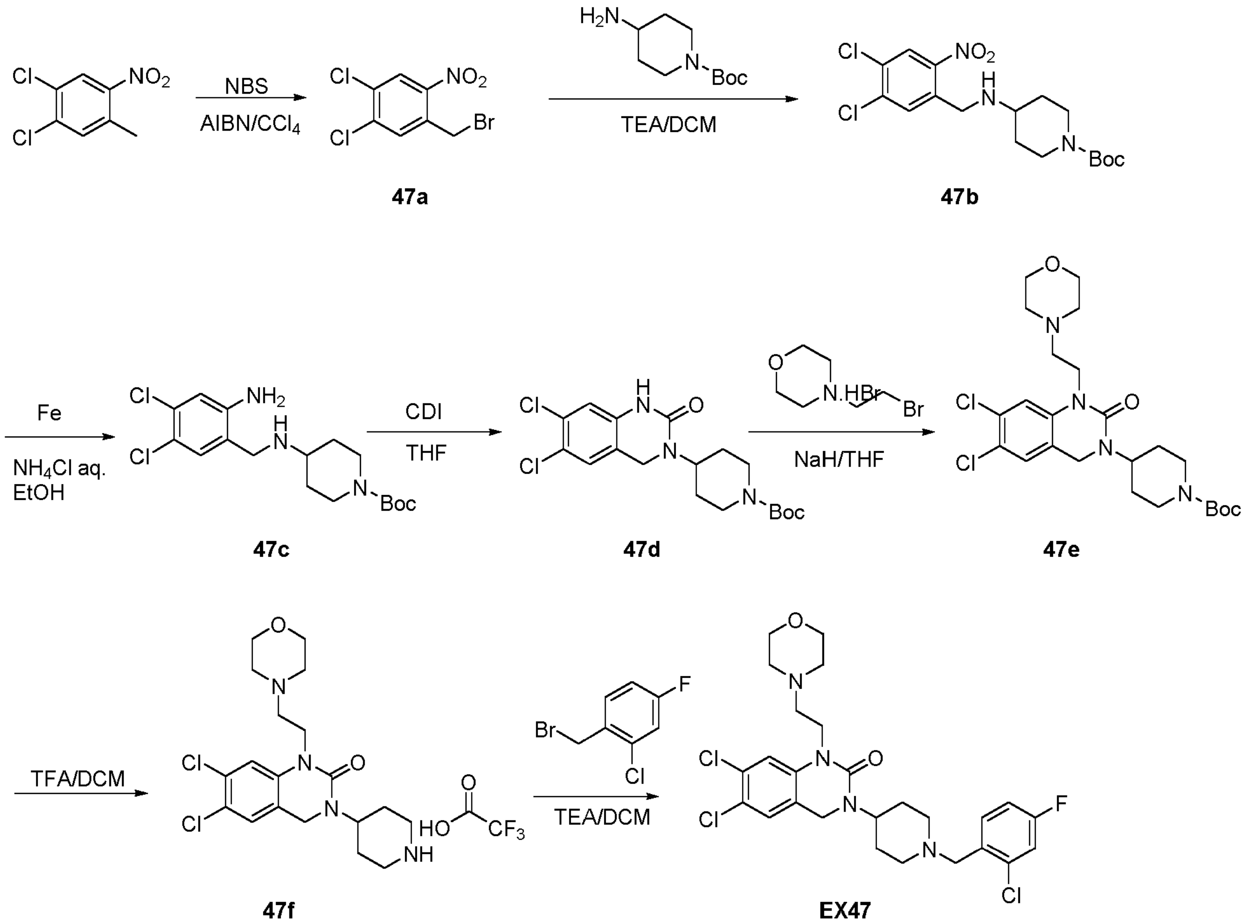


47a 47b 47c 47d 47e 47f EX47

Synthesis of intermediate **47a:** 4,5-dichloro-2-nitrotoluene (5 g, 23.06 mmol) was added into a clean dry single neck flask (500 mL), and solvents carbon tetrachloride (230 mL), *N*-bromosuccinimide (NBS) (4.51 g, 25.36 mmol), and azobisisobutyronitrile (AIBN) (379 mg, 2.31 mmol) were added to the reaction flask. The flask was heated to 80°C for reflux reaction for 18 hours. The reaction of the raw materials was detected, by TLC, to be substantially complete. The flask was cooled to room temperature, and saturated $NaHCO_3$ aqueous solution was added to the reaction system for quenching the reaction, followed by extraction with dichloromethane (100 mL×3), washing with saturated NaCl aqueous solution, drying with anhydrous MgSO4, filtering, and evaporation under reduced pressure by a rotary evaporator to remove the solvent to obtain a crude product, which was separated and purified by silica gel column chromatography (the mobile phase was petroleum ether) to obtain 3.02 g light yellow solid product, with a yield of 45.97%. The product was directly used in the next reaction.

Synthesis of intermediate **47b:** *N*-BOC-4-aminopiperidine (2.32 g, 11.58 mmol) and anhydrous dichloromethane (60 mL) were added into a clean dry round-bottom flask (250 mL), and triethylamine (1.60 g, 2.20 mL, 15.79 mmol) was added thereto. Under stirring at room temperature, 1-(bromomethyl)-4,5-dichloro-2-nitrobenzene **(47a)** (3.00 g, 10.53 mmol) dichloromethane (40 mL) solution was added dropwise to the reaction system. Thereafter, stirring was continued at room temperature for reaction for 12 hours. The reaction of the raw materials was detected, by TLC, to be completed. After the completion of the reaction, the solvent was removed by evaporation under reduced pressure with a rotary evaporator, and the residue was separated and purified by silica gel column chromatography (the mobile phase was petroleum ether and ethyl acetate, with the ratio gradient being 20:1-8:1) to obtain 2.77 g light yellow oil product, with a yield of 65.07%. $^1H$ NMR (400 MHz, chloroform-*d*) δ 8.08 (s, 1H), 7.87 (s, 1H), 4.07 (s, 2H), 4.01 (s, 2H), 2.90 - 2.74 (m, 2H), 2.70 - 2.57 (m, 1H), 1.93 - 1.78 (m, 2H), 1.46 (s, 9H), 1.34 - 1.25 (m, 2H).

Synthesis of intermediate **47c:** *tert*-butyl 4-((4,5-dichloro-2-nitrophenylmethyl) amino)piperidin-1-carboxylate (47b) (2.50 g, 6.18 mmol) and anhydrous ethanol (60 mL) were added into a single-neck flask (250 mL). Under stirring at room temperature, iron powder (2.07 g, 37.10 mmol) and saturated $NH_4Cl$ aqueous solution (10 mL) were slowly added to the reaction system. The flask was heated to 80°C, and stirring was continued for reaction for 2 hours. The reaction of the raw materials was detected, by TLC plate, to be complete. After the completion of the reaction, the reaction mixture solution was cooled to room temperature, and filtered with diatomite. The filter cake was washed with anhydrous ethanol, and the filtrate was collected. The solution was removed by evaporation under reduced pressure with a rotary evaporator to obtain a white solid. The solid was dissolved in ethyl acetate (100 mL), and added with a small amount of water (25 mL) to

precipitate a large amount of white solid. After filtration and drying, 2.00 g white solid product was obtained, with a yield of 86.41%. The product was directly used in the next reaction.

Synthesis of intermediate **47d:** intermediate **47c** was used as raw material for synthesis according to the synthesis method of intermediate **1e** in Example 1 to obtain a white solid product (590 mg, with a yield of 64.52%). $^1$H NMR (400 MHz, chloroform-d) δ 7.78 (s, 1H), 7.13 (s, 1H), 6.84 (s, 1H), 4.59 - 4.45 (m, 1H), 4.26 (s, 4H), 2.84 (s, 2H), 1.76 - 1.62 (m, 4H), 1.48 (s, 9H).

Synthesis of intermediate **47e:** intermediate **47d** was used as raw material for synthesis according to the synthesis method of intermediate **1f** in Example 1 to obtain a crude product, which was separated and purified by alkaline alumina column chromatography (the mobile phase was petroleum ether and ethyl acetate, with the ratio gradient being 5:1-0:1), to obtain a white solid product (627 mg, with a yield of 85.76%). $^1$H NMR (400 MHz, chloroform-d) δ 7.13 (s, 2H), 4.51 - 4.38 (m, 1H), 4.24 (s, 2H), 4.16 (s, 2H), 3.96 (t, *J* = 7.1 Hz, 2H), 3.80 - 3.63 (m, 4H), 2.90 - 2.71 (m, 2H), 2.69 - 2.42 (m, 6H), 1.73 - 1.60 (m, 4H), 1.47 (s, 9H).

Synthesis of compound **EX47: 47e** was used as raw material for synthesis according to the synthesis method of intermediate **1g** in Example 1 to obtain intermediate **47f,** a white solid product (616 mg, with a yield of 99.99%). The product was directly used in the next reaction. $^1$H NMR (400 MHz, Methanol-$d_4$) δ 7.42 (s, 1H), 7.25 (s, 1H), 4.54 - 4.42 (m, 1H), 4.40 (s, 2H), 4.30 (t, *J* = 5.8 Hz, 2H), 4.23 - 3.63 (m, 6H), 3.61 - 3.43 (m, 5H), 3.30 - 3.05 (m, 4H), 2.17 (qd, *J* = 13.2, 4.1 Hz, 2H), 2.05 - 1.91 (m, 2H).

Intermediate **47f** and 2-chloro-4-fluorobromobenzyl were used as raw materials for synthesis according to the synthesis method in Example 39 to obtain a white solid product (123 mg, with a yield of 46.67%). LC-MS (ESI) m/z: 555.20 $(M+H)^+$; $^1$H NMR (400 MHz, chloroform-d) δ 7.45 (t, *J* = 7.6 Hz, 1H), 7.17 - 7.05 (m, 3H), 6.97 (t, *J* = 8.2 Hz, 2H), 4.40 - 4.27 (m, 1H), 4.20 (s, 2H), 3.95 (t, *J* = 7.1 Hz, 2H), 3.77 - 3.67 (m, 4H), 3.58 (s, 2H), 2.98 (d, *J* = 11.0 Hz, 2H), 2.61 (t, *J* = 7.1 Hz, 2H), 2.59 - 2.50 (m, 4H), 2.24 (t, *J* = 11.7 Hz, 2H), 1.91 - 1.75 (m, 2H), 1.71 - 1.62 (m, 2H), 1.24 (t, *J* = 7.0 Hz, 1H).

**Example 48:** Synthesis of 5,6-dichloro-1-(2-(dimethylamino)ethyl)-3-(1-(4-isopropylcyclohexyl)piperidin-4-yl)-1,3-dihydro-2*H*-benzo[*d*]imidazol-2-one (compound **EX48)**

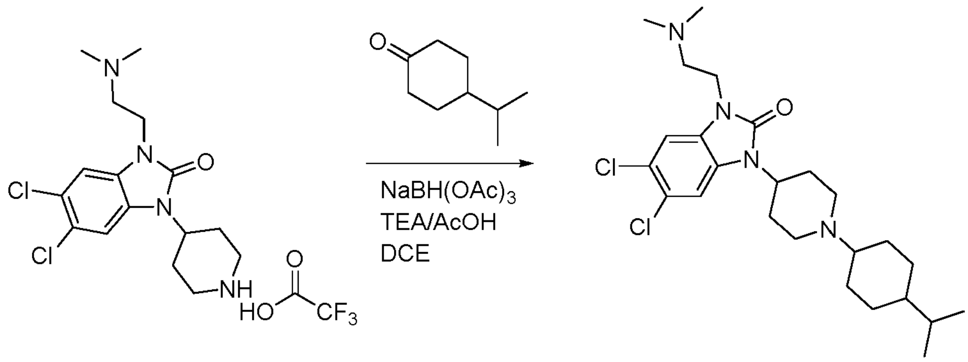


**46b** **EX48**

Synthesis of compound **EX48:** intermediate **46b** and 4-isopropylcyclohexanone were used as raw materials for synthesis according to the synthesis method of compound EX12 to obtain a white solid product (1.53 g, with a yield of 62.5%).

**Example 48A:** Synthesis of 5,6-dichloro-1-(2-(dimethylamino)ethyl)-3-(1-(trans-4-isopropylcyclohexyl)piperidin-4-yl)-1,3-dihydro-2*H*-benzo[*d*]imidazol-2-one (compound **EX48A)**

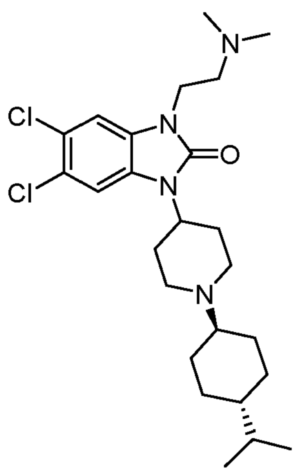

**EX48A**

Synthesis of compound **EX48A:** intermediate **46b** and 4-isopropylcyclohexanone were used as raw materials for synthesis according to the synthesis method of compound **EX12** to obtain compound **EX48,** which was subjected to preparation and separation by high performance liquid chromatography (Waters high performance liquid chromatography, chromatographic column: YMC-Triart-C18 EXRS 20 mm×100 mm×5 μm; mobile phase: A = water + 0.1 vol% ammonia (25%), B = acetonitrile; gradient 70%-90% B, 10 min) to obtain a white solid product (169 mg, with a yield of 6.91%). LC-MS (ESI) m/z: 481.30 $(M+H)^+$; $^1H$ NMR (400 MHz, chloroform-d) δ 7.41 (s, 1H), 7.08 (s, 1H), 4.36 - 4.26 (m, 1H), 3.92 (t, *J* = 7.0 Hz, 2H), 3.06 (d, *J* = 11.3 Hz, 2H), 2.60 (t, *J* = 6.9 Hz, 2H), 2.44 - 2.35 (m, 2H), 2.35 - 2.23 (m, 9H), 1.92 (d, *J*= 11.3 Hz, 2H), 1.84 - 1.76 (m, 4H), 1.74 - 1.69 (m, 2H), 1.47 - 1.37 (m, 1H), 1.26 (q, *J*= 11.9 Hz, 2H), 1.08 - 1.02 (m, 1H), 0.87 (s, 3H), 0.86 (s, 3H).

**Example 48B:** Synthesis of 5,6-dichloro-1-(2-(dimethylamino)ethyl)-3-(1-(cis-4-isopropylcyclohexyl)piperidin-4-yl)-1,3-dihydro-2*H*-benzo[*d*]imidazol-2-one (compound **EX48B)**

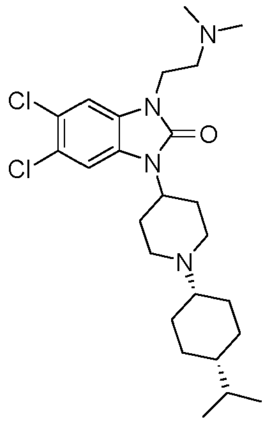

**EX48B**

Synthesis of compound **EX48B:** intermediate **46b** and 4-isopropylcyclohexanone were used as raw materials for synthesis according to the synthesis method of compound **EX12** to obtain compound **EX48,** which was subjected to preparation and separation by high performance liquid chromatography (Waters high performance liquid chromatography, chromatographic column: YMC-Triart-C18 EXRS 20 mm×100 mm×5 μm; mobile phase: A = water + 0.1 vol% ammonia (25%), B = acetonitrile; gradient 70%-90% B, 10 min) to obtain a white solid product (411 mg, with a yield of 16.80%). LC-MS (ESI) m/z: 481.30 $(M+H)^+$; $^1H$ NMR (400 MHz, chloroform-d) δ 7.35 (s, 1H), 7.08 (s, 1H), 4.35 - 4.22 (m, 1H), 3.92 (t, *J* = 7.0 Hz, 2H), 3.20 - 3.09 (m, 2H), 2.60 (t, *J* = 7.0 Hz, 2H), 2.36 - 2.26 (m, 8H), 2.26 - 2.16 (m, 2H), 1.85 - 1.67 (m, 5H), 1.67 - 1.57 (m, 3H), 1.57 - 1.48 (m, 2H), 1.43 - 1.33 (m, 2H), 1.18 - 1.10 (m, 1H), 0.91 (s, 3H), 0.89 (s, 3H).

**Example 49:** Synthesis of 1-(2-(2-oxa-6-azaspiro [3.3]heptan-6-yl) ethyl)-5,6-dichloro-3-(1-(2-chloro-4-fluorobenzyl) piperidin-4-yl)-1,3-dihydro-2*H*-benzo[*d*]imidazol-2-one (compound **EX49)**

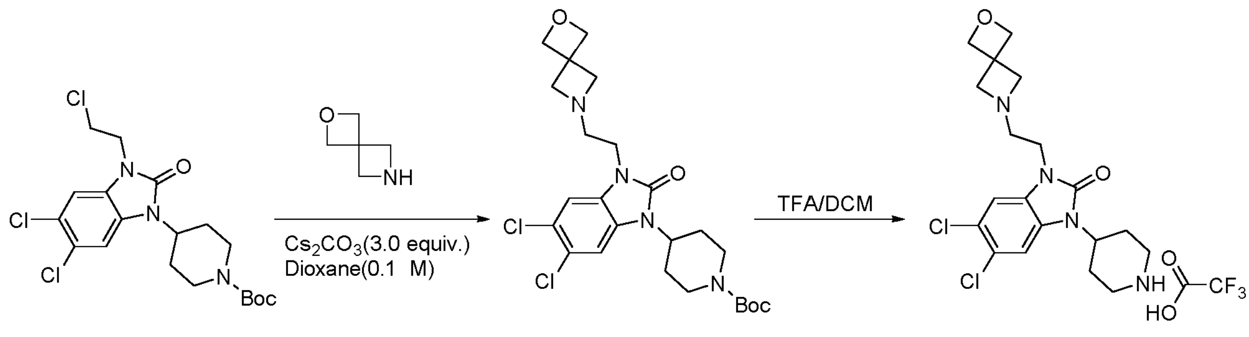


**40a** **49a** **49b**

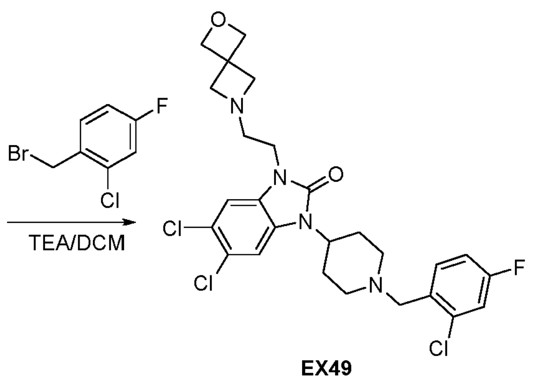


**EX49**

Synthesis of intermediate **49a:** *tert*-butyl 4-(5,6-dichloro-3-(2-chloroethyl)-2-carbonyl-2,3-dihydro-1*H*-benzo[d] imidazol-1-yl) piperidin-1-carboxylate **(40a)** (250 mg, 557 μmol) and $Cs_2CO_3$ (544 mg, 1.67 mmol) were added into a clean dry Schlenk tube (25 mL). Anhydrous dioxane (5.5 mL) was added to the tube. Under stirring at room temperature, 2-oxa-6-aza-spiro[3,3]heptane (138 mg, 1.39 mmol) was added. Under argon displacement protection, the tube was heated to 105°C for reflux reaction for 12 hours. The reaction of the raw materials was detected, by TLC plate, to be substantially complete. After the completion of the reaction, the reaction flask was cooled to room temperature, an appropriate amount of water was added thereto, followed by extraction with ethyl acetate (15 mL×3). The organic phase was washed with saturated NaCl aqueous solution, and dried with anhydrous $Na_2SO_4$, and then filtered and evaporated with a rotary evaporator to remove the solvent, to obtain a light brown oily crude product, which was subjected to separation by silica gel column chromatography (the mobile phase was DCM/MeOH, with 0.2% ~ 1% MeOH) to obtain 186 mg white solid product, with a yield of 65.28%. LC-MS (ESI) m/z: 454.10 $(M-56+H)^+$; $^1$H NMR (400 MHz, chloroform-*d*) δ 7.16 (s, 1H), 7.09 (s, 1H), 4.71 (s, 4H), 4.47 - 4.20 (m, 3H), 3.78 (t, *J* = 6.6 Hz, 2H), 3.38 (s, 4H), 2.83 (t, *J* = 13.5 Hz, 2H), 2.70 (t, *J* = 6.5 Hz, 2H), 2.23 (qd, *J* = 12.7, 4.4 Hz, 2H), 1.79 (d, *J* = 12.1 Hz, 2H), 1.51 (s, 9H).

Synthesis of intermediate **49b: 49a** (150 mg, 293 μmol) was used as raw material for synthesis according to the synthesis method of intermediate **1g** to obtain a white solid product (154 mg, with a yield of 99.95%). The solid was directly used in the next reaction without purification.

Synthesis of compound **EX49: 49b** and 2-chloro-4fluorobromobenzyl were used as raw materials for synthesis according to the synthesis method in Example 39, followed by separation by silica gel column chromatography (the mobile phase was DCM/MeOH, with 0.2% ~ 1% MeOH) to obtain a white solid product (86 mg, with a yield of 54.38%). LC-MS (ESI) m/z: 554.12 $(M+H)^+$; $^1$H NMR (400 MHz, Methanol-$d_4$) δ 7.66 (s, 1H), 7.59 (dd, *J* = 8.6, 6.3 Hz, 1H), 7.40 (s, 1H), 7.26 (dd, *J* = 8.7, 2.6 Hz, 1H), 7.13 (td, *J* = 8.4, 2.5 Hz, 1H), 4.33 (tt, *J* = 12.5, 4.4 Hz, 1H), 3.92 (t, *J* = 5.9 Hz, 2H), 3.71 (s, 2H), 3.63 (s, 4H), 3.22 (s, 4H), 3.12 (d, *J* = 11.1 Hz, 2H), 2.91 (t, *J* = 5.8 Hz, 2H), 2.48 (qd, *J* = 12.5, 3.6 Hz, 2H), 2.34 (t, *J* = 11.8 Hz, 2H), 1.79 (d, *J* = 11.4 Hz, 2H).

**Example 50:** Synthesis of 5,6-dichloro-1-(1-((5-chlorothiophen-2-yl)methyl) piperidin-4-yl)-3-(2-morpholinoethyl)-1,3-dihydro-2*H*-benzo[*d*]imidazol-2-one (compound **EX50)**

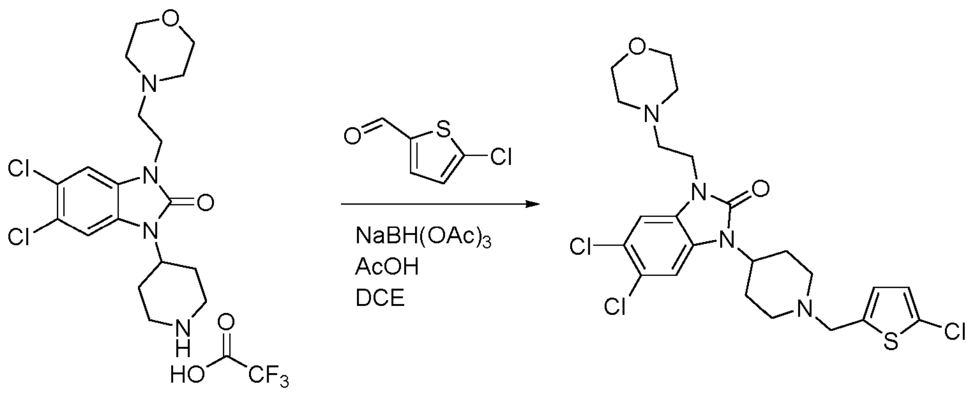


**1g** **EX50**

Intermediate **1g** in Example 1 and 5-chlorothiophene-2-formaldehyde were used as raw materials for synthesis according to the synthesis method in Example **1** to obtain a light yellow solid product (289 mg, with a yield of 93.3%). LC-MS (ESI) m/z: 530.30 $(M+H)^+$; $^1H$ NMR (400 MHz, chloroform-*d*) δ 7.31 (s, 1H), 7.13 (s, 1H), 6.75 (d, *J* = 3.1 Hz, 1H), 6.70 (d, *J* = 3.8 Hz, 1H), 4.36 - 4.24 (m, 1H), 3.95 (t, *J* = 6.6 Hz, 2H), 3.73 - 3.61 (m, 6H), 3.10 (d, *J* = 11.0 Hz, 2H), 2.64 (t, *J* = 6.6 Hz, 2H), 2.59 - 2.46 (m, 4H), 2.37 (qd, *J* = 12.5, 3.8 Hz, 2H), 2.20 (t, *J* = 11.7 Hz, 2H), 1.78 (d, *J*= 11.7 Hz, 2H).

**Example 51:** Synthesis of 6,7-dichloro-1-(1-(2-chloro-4-fluorobenzyl) piperidin-4-yl)-3-(2-morpholinoethyl)-3,4-dihydroquinazolin-2(1H)-one (compound **EX51)**

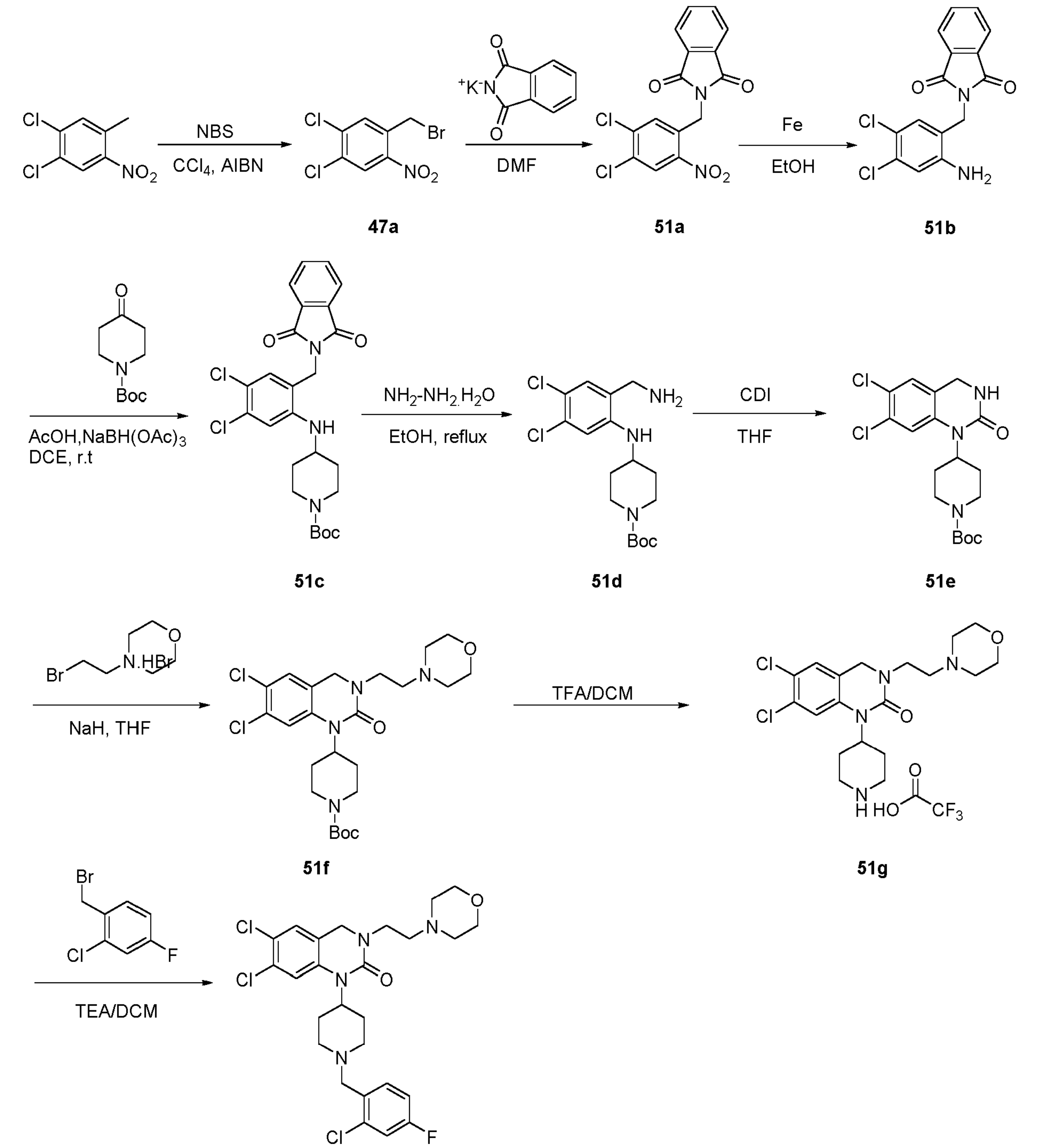


EX51

Synthesis of intermediate **51a:** 1-(bromomethyl)-4,5-dichloro-2-nitrobenzene **(47a)** (5.33 g, 18.71 mmol) and phthalimide potassium salt (3.30 g, 17.82 mmol) were added into a clean dry single neck flask (100 mL), and dry DMF (30 mL) was added thereto. The flask was heated to 125°C, and stirred for reaction for 12 hours. The reaction of the raw materials was detected, by TLC, to be complete. After the completion of the reaction, the reaction mixture solution was cooled to room temperature. Under stirring condition, the reaction mixture solution was slowly poured into an ice-water mixture (120 mL). A large amount of yellow solid was precipitated. After stirring evenly, filtration was performed, the filter cake was washed with a small amount of water. After drying, a yellow solid product (5.93 g, with a yield of 94.8%) was obtained. $^1$H NMR (400 MHz, chloroform-*d*) δ 8.25 (s, 1H), 7.97 - 7.89 (m, 2H), 7.84 - 7.77 (m, 2H), 7.32 (s, 1H), 5.25 (s, 2H).

Synthesis of intermediate **51b:** 2-(4,5-dichloro-2-nitrophenylmethyl) isodihydroindole-1,3-dione **(51a)** (2.5 g, 7.12 mmol) and anhydrous ethanol (71 mL) were added into a single-neck flask (250 mL). Under stirring at room temperature, iron powder (2.78 g, 49.84 mmol) and saturated $NH_4Cl$ aqueous solution (10 mL) were slowly added to the reaction system. The flask was heated to 55°C, and stirring was continued for reaction for 1 hour. The reaction of the raw

materials was detected, by TLC plate, to be complete. After the completion of the reaction, the reaction mixture solution was cooled to room temperature, and filtered with diatomite. The filter cake was washed with anhydrous ethanol, and the filtrate was collected. The solution was removed by evaporation under reduced pressure with a rotary evaporator to obtain a yellow solid. The solid was dissolved in ethyl acetate (100 mL), and added with a small amount of water (25 mL). After filtering out insoluble matter, liquid separation was performed to collect the organic phase, and anhydrous $Na_2SO_4$ was added for drying, followed by filtering, and evaporation under reduced pressure by a rotary evaporator to remove the solvent to obtain a crude product, which was separated and purified by silica gel column chromatography (the mobile phase was petroleum ether and ethyl acetate, with the ratio gradient being 10:1-3:1) to obtain a light yellow solid product (1.86 g, with a yield of 81.4%). $^1$H NMR (400 MHz, chloroform-*d*) δ 7.88 - 7.81 (m, 2H), 7.77 - 7.70 (m, 2H), 7.51 (s, 1H), 6.74 (s, 1H), 4.68 (s, 2H), 4.56 (s, 2H).

Synthesis of intermediate 51c: 2-(2-amino-4,5-dichlorophenylmethyl) isodihydroindole-1,3-dione **(51b)** (1.86 g, 5.79 mmol), 4-tert-butylpiperidone (1.38 g, 6.95 mmol), and anhydrous 1,2-dichloroethane (60 mL) were added into a clean dry round-bottom flask (100 mL). Under stirring at room temperature, acetic acid (348 mg, 5.79 mmol, 331 μL) and $NaBH(OAc)_3$ (3.07 g, 14.48 mmol) were added to the reaction system. Under argon displacement protection, stirring was performed at room temperature for reaction for 48 hours. The reaction was detected, by TLC plate, to be complete. After the completion of the reaction, saturated $NaHCO_3$ aqueous solution was added to the reaction system for quenching the reaction, followed by extraction with dichloromethane (50 mL×3), washing with saturated NaCl aqueous solution, drying with anhydrous MgSO4, filtering, and evaporation under reduced pressure by a rotary evaporator to remove the solvent to obtain a crude product, which was separated and purified by silica gel column chromatography (the mobile phase was petroleum ether and ethyl acetate, with the ratio gradient being 15:1-4:1) to obtain a light yellow solid product (925 mg, with a yield of 31.7%). $^1$H NMR (400 MHz, chloroform-d) δ 7.89 - 7.80 (m, 2H), 7.78 - 7.69 (m, 2H), 7.54 (s, 1H), 6.63 (s, 1H), 5.38 (d, *J* = 6.1 Hz, 1H), 4.64 (s, 2H), 4.14 - 3.97 (m, 2H), 3.43 - 3.29 (m, 1H), 3.09 - 2.90 (m, 2H), 2.07 - 1.94 (m, 2H), 1.57 - 1.40 (m, 12H).

Synthesis of intermediate **51e:** *tert*-butyl 4-((4,5-dichloro-2-((1,3-dicarbonyl isodihydroindole-2-yl)methyl)phenyl)amino)piperidin-1-carboxylate **(51c)** (800 mg, 1.59 mmol) and solvent ethanol (16 mL) were added into a clean dry single-neck flask (50 mL). Under stirring, hydrazine hydrate (467 mg, 7.93 mmol, 453 μL, 85%) was added to the reaction system. The flask was heated to 80°C for reflux reaction for 2 hours to precipitate a large amount of solid. The reaction was detected, by TLC, to be complete. After the completion of the reaction, the reaction system was cooled to room temperature, and filtered. The filtrate was collected, and evaporated under reduced pressure by a rotary evaporator to remove the solvent to obtain a light yellow oily crude product *tert*-butyl 4-((2-(aminomethyl)-4,5-dichlorophenyl) amino) piperidin-1-carboxylate **(51d)** (590 mg, with a yield of 99.38%). The crude product (590 mg, 1.58 mmol) was dissolved in dry tetrahydrofuran (15 mL), under argon displacement protection, N,N'-carbonyldiimidazole (358 mg, 2.21 mmol) dichloromethane (6 mL) solution was added dropwise to the reaction system, and stirring was performed at room temperature for reaction for 18 hours. The reaction was detected, by TLC plate, to be substantially complete. After the completion of the reaction, saturated $NaHCO_3$ solution was added dropwise to the reaction system for quenching the reaction, followed by extraction with ethyl acetate three times (25 mL×3). The organic phase was washed with saturated NaCl solution, added with anhydrous $Na_2SO_4$ for drying, filtered, and evaporated under reduced pressure by a rotary evaporator to remove the solvent to obtain a crude product, which was separated and purified by alkaline alumina column chromatography (the mobile phase was petroleum ether and ethyl acetate, with the ratio gradient being 5:1-1:1) to obtain a white solid product (528 mg, with a yield of 83.7%). $^1$H NMR (400 MHz, chloroform-d) δ 7.14 (s, 1H), 7.07 (s, 1H), 5.62 (s, 1H), 4.41 - 4.17 (m, 4H), 4.09 - 3.95 (m, 1H), 2.91 - 2.68 (m, 2H), 2.64 - 2.45 (m, 2H), 1.83 - 1.70 (m, 2H), 1.49 (s, 9H).

Synthesis of intermediate **51f:** *tert*-butyl 4-(6,7-dichloro-2-carbonyl-3,4-dihydroquinazolin-1(2*H*)-yl) piperidin-1-carboxylate **(51e)** (220 mg, 549 μmol) and the solvent, dry tetrahydrofuran (5.5 mL), were added into a dry clean three-necked flask (50 mL), and under argon displacement protection, the flask was cooled in an ice water bath to 0°C. NaH (40 mg, 1.65 mmol, 60% in mineral oil) was added to the reaction system, and stirred for reaction for 30 minutes. 4-(2-bromoethyl) morpholine hydrobromide (212 mg, 769 μmol) was added to the reaction system, stirring was continued for 30 minutes, then the ice water bath was removed, the flask was slowly heated to 35°C, and stirring was continued for reaction for 18 hours. The reaction was detected, by TLC plate, to be substantially complete. After the completion of the reaction, under cooling in an ice water bath, ice water was added dropwise to the reaction system for quenching the reaction, followed by extraction with ethyl acetate three times (25 mL×3). The organic phase was washed with saturated NaCl solution, added with anhydrous $Na_2SO_4$ for drying, filtered, and evaporated under reduced pressure by a rotary evaporator to remove the solvent to obtain a crude product, which was separated and purified by alkaline alumina column chromatography (the mobile phase was petroleum ether and ethyl acetate, with the ratio gradient being 5:1-1:1) to obtain a white solid product (192 mg, with a yield of 68.0%). $^1$H NMR (400 MHz, chloroform-*d*) δ 7.14 (s, 1H), 7.03 (s, 1H), 4.41 - 4.15 (m, 4H), 4.04 - 3.92 (m, 1H), 3.73 - 3.62 (m, 4H), 3.50 (t, *J* = 6.6 Hz, 2H), 2.90 - 2.69 (m, 2H), 2.61 - 2.43 (m, 8H), 1.83 - 1.72 (m, 2H), 1.48 (s, 9H).

Synthesis of intermediate **51g:** *tert*-butyl 4-(6,7-dichloro-3-(2-morpholinoethyl)-2-carbonyl-3,4-dihydroquinazoline-1(2*H*)-yl) piperidin-1-carboxylate **(51f)** (185 mg, 360 μmol) and the solvent dichloromethane (5 mL) were added into

a dry clean round-bottom flask (25 mL). Under stirring at room temperature, trifluoroacetic acid (1 mL) was added dropwise for reaction for 2 hours. The reaction was detected, by TLC plate, to be substantially complete. The reaction mixture solution was evaporated under reduced pressure by a rotary evaporator to remove the solvent. Ether (10 mL) was added to the residue, followed by dispersing, crushing, and filtering to obtain a white solid product (190 mg, with a yield of 99.99%). The product was directly used in the next reaction.

Synthesis of compound EX51: 6,7-dichloro-3-(2-morpholinoethyl)-1-(piperidin-4-yl)-3,4-dihydroquinazolin-2(1*H*)-ketotrifluoroacetate **(51g)** (190 mg, 360 μmol) and dry dichloromethane (3.5 mL) were added into a clean dry Schlenk reaction flask (10 mL). Triethylamine (146 mg, 1.44 mmol, 200 μL) was added thereto, and stirred at room temperature for 5 minutes. 2-chloro-4fluorobenzyl bromide (97 mg, 433 μmol) was added to the reaction system, and stirred at room temperature for reaction for 12 hours. The reaction was detected, by TLC plate, to be substantially complete. After the completion of the reaction, the reaction mixture solution was evaporated under reduced pressure by a rotary evaporator to remove the solvent to obtain a crude product, which was separated and purified by alkaline alumina column chromatography (the mobile phase was petroleum ether and ethyl acetate, with the ratio gradient being 5:1-1:1) to obtain a white solid product (158 mg, with a yield of 78.9%). LC-MS (ESI) m/z: 556.20 $(M+H)^+$; $^1H$ NMR (400 MHz, chloroform-d) δ 7.56 (t, *J* = 7.5 Hz, 1H), 7.17 - 7.05 (m, 3H), 6.98 (t, *J* = 8.6 Hz, 1H), 4.25 (s, 2H), 3.94 - 3.81 (m, 1H), 3.74 - 3.64 (m, 4H), 3.61 (s, 2H), 3.52 (t, *J* = 6.7 Hz, 2H), 3.01 (d, *J* = 11.2 Hz, 2H), 2.70 (q, *J* = 11.6, 10.9 Hz, 2H), 2.56 (t, *J* = 5.9 Hz, 2H), 2.53 - 2.44 (m, 4H), 2.24 (t, *J* = 11.8 Hz, 2H), 1.75 (d, *J* = 12.2 Hz, 2H).

**Example 52:** Synthesis of 6,7-dichloro-1-(1-(2-chloro-4-fluorophenyl) piperidin-4-yl)-3,4-dihydroquinazolin-2(1*H*)-one (compound **EX52)**

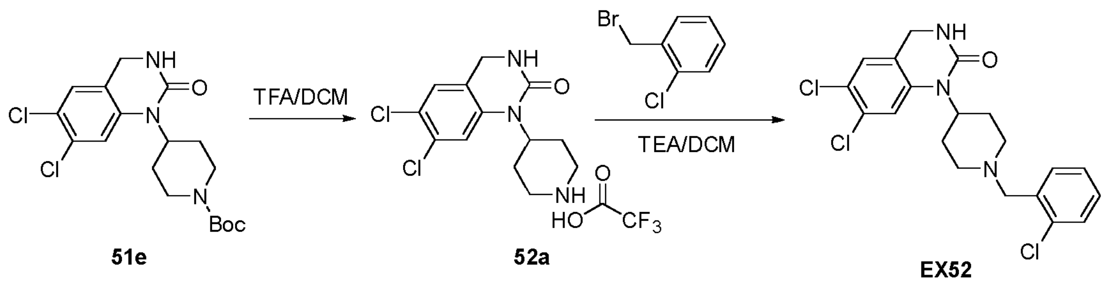


**51e** **52a** **EX52**

Synthesis of compound **EX52:** intermediate **51e** was used as raw material and dichloromethane was used as solvent to obtain 218 mg white solid **52a,** with a yield of 100%, under the action of trifluoroacetic acid. The solid was directly used in the next reaction without further purification.

**52a** and 2-chlorobromobenzyl were used as raw materials for synthesis according to the synthesis method in Example 39 to obtain a white solid product (74 mg, with a yield of 72.2%). LC-MS (ESI) m/z: 425.30 $(M+H)^+$; $^1H$ NMR (400 MHz, chloroform-*d*) δ 7.57 (d, *J* = 6.8 Hz, 1H), 7.35 (dd, *J* = 7.9, 1.4 Hz, 1H), 7.27 (t, *J* = 7.0 Hz, 2H), 7.23 - 7.15 (m, 2H), 7.13 (s, 1H), 5.31 (s, 1H), 4.23 (s, 2H), 4.05 - 3.87 (m, 1H), 3.68 (s, 2H), 3.05 (d, *J* = 10.5 Hz, 2H), 2.71 (q, *J* = 10.7 Hz, 2H), 2.25 (t, *J* = 11.1 Hz, 2H), 1.75 (d, *J* = 11.2 Hz, 2H).

**Example 53:** Synthesis of 5,6-dichloro-1-(1-(2-chlorobenzyl)piperidin-4-yl)-3-(2-(2-oxopyrrolidin-1-yl)ethyl)-1,3-dihydro-2*H*-benzo[d]imidazol-2-one (compound **EX53)**

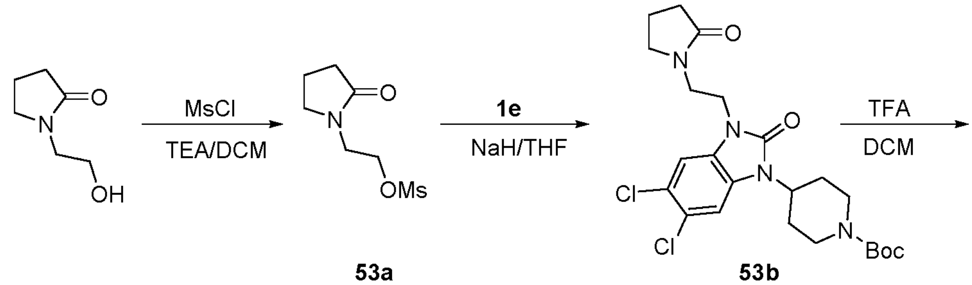


53a 53b

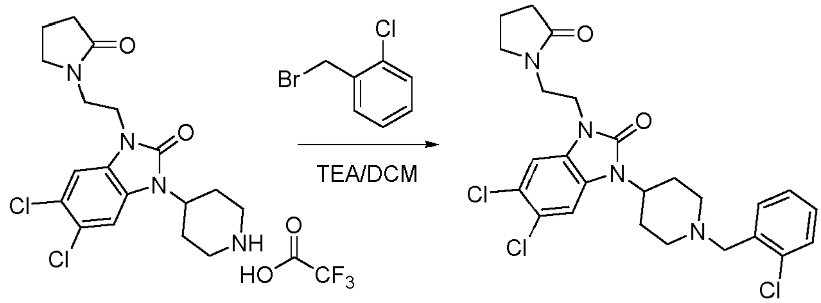


53c EX53

Synthesis of intermediate **53b:** 1-(2-hydroxypyrrole)-2-one was used as raw material for synthesis of **53a** according to the synthesis method of **39b** to obtain 1.8 g light yellow oily crude product, with a yield of 82.4%. The product was directly used in the next reaction without purification.

**53a** and **1e** were used as raw materials for synthesis of **53b** according to the synthesis method of **39c** to obtain 231 mg white solid product, with a yield of 35.88%. $^1$H NMR (400 MHz, chloroform-d) δ 7.16 (s, 1H), 7.15 (s, 1H), 4.40 - 4.27 (m, 3H), 4.00 (t, *J* = 6.0 Hz, 2H), 3.58 (t, *J* = 6.0 Hz, 2H), 3.36 (t, *J* = 7.0 Hz, 2H), 2.84 (t, *J* = 12.7 Hz, 2H), 2.32 - 2.18 (m, 4H), 1.99 - 1.89 (m, 2H), 1.83 - 1.74 (m, 2H), 1.51 (s, 9H).

Synthesis of intermediate **53c: 53b** was used as raw material for synthesis according to the synthesis method of intermediate **1f** in Example 1 to obtain 215 mg white solid product, with a yield of 90.5%. The product was directly used in the next reaction without purification.

Synthesis of compound **EX53: 53c** and 2-chlorobromobenzyl were used as raw materials for synthesis according to the synthesis method in Example 39 to obtain a white solid product (68 mg, with a yield of 78.4%). LC-MS (ESI) m/z: 522.30 $(M+H)^+$; $^1$H NMR (400 MHz, chloroform-d) δ 7.55 (d, *J* = 7.7 Hz, 1H), 7.37 (d, *J* = 7.8 Hz, 1H), 7.31 (s, 1H), 7.28 (d, *J* = 6.5 Hz, 1H), 7.21 (t, *J* = 7.6 Hz, 1H), 7.16 (s, 1H), 4.34 - 4.22 (m, 1H), 4.00 (t, *J* = 6.0 Hz, 2H), 3.69 (s, 2H), 3.58 (t, *J* = 6.0 Hz, 2H), 3.35 (t, *J* = 7.0 Hz, 2H), 3.08 (d, *J* = 11.1 Hz, 2H), 2.40 (qd, *J* = 12.0, 3.6 Hz, 2H), 2.33 - 2.20 (m, 4H), 1.99 - 1.87 (m, 2H), 1.77 (d, *J* = 10.0 Hz, 2H).

**Example 54:** Synthesis of 2-(5,6-dichloro-3-(1-(2-chloro-4-fluorobenzyl) piperidin-4-yl)-2-oxo-2,3-dihydro-1*H*-benzo[d] imidazol-1-yl)-N,N-dimethyl acetamide (compound **EX54)**

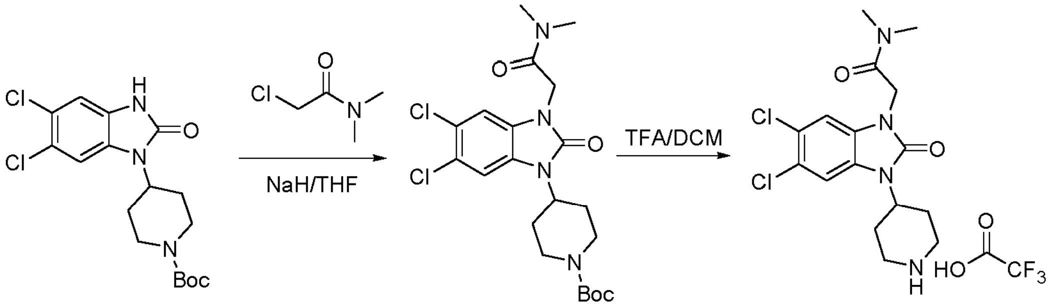


**1e** **54a** **54b**

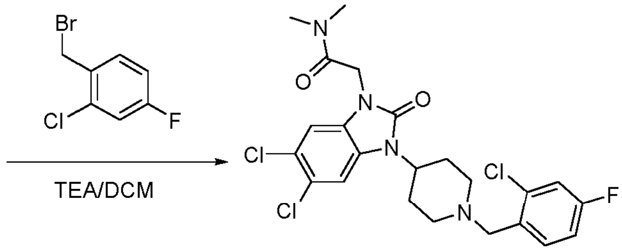


**EX54**

Synthesis of intermediate **54a:** intermediate **1e** and 2-chloro-*N*,*N*-dimethyl acetamide were used as raw materials for synthesis according to the synthesis method of intermediate **1f** in Example 1 to obtain a colorless oily product (610 mg, with a yield of 99.9%). $^1$H NMR (400 MHz, chloroform-*d*) δ 7.17 (s, 1H), 7.05 (s, 1H), 4.62 (s, 2H), 4.44 - 4.34 (m, 1H), 4.31 (d, *J* = 13.6 Hz, 2H), 3.14 (s, 3H), 2.99 (s, 3H), 2.84 (t, *J* = 12.2 Hz, 2H), 2.25 (qd, *J* = 12.7, 4.6 Hz, 2H), 1.82 (d, *J*= 10.4 Hz, 2H), 1.51 (s, 9H).

Synthesis of intermediate **54b:** intermediate **54a** was used as raw material for synthesis according to the synthesis method of intermediate 1g in Example 1 to obtain a white solid product (628 mg, with a yield of 100%). The product was directly used in the next experiment.

Synthesis of compound **EX54: 54b** and 2-chloro-4-fluorobromobenzyl were used as raw materials for synthesis according to the synthesis method in Example 39 to obtain a white solid product (299 mg, with a yield of 94.1%). LC-MS (ESI) m/z: 514.20 $(M+H)^+$; $^1$H NMR (400 MHz, chloroform-d) δ 7.52 (t, *J* = 7.5 Hz, 1H), 7.30 (s, 1H), 7.12 (d, *J* = 8.2 Hz, 1H), 7.05 (s, 1H), 7.01 (t, *J* = 8.7 Hz, 1H), 4.62 (s, 2H), 4.38 - 4.24 (m, 1H), 3.63 (s, 2H), 3.14 (s, 3H), 3.04 (d, *J* = 10.5 Hz, 2H), 2.99 (s, 3H), 2.39 (q, *J* = 12.6, 11.8 Hz, 2H), 2.27 (t, *J* = 11.7 Hz, 2H), 1.81 (d, *J*= 11.5 Hz, 2H).

**Example** 55: Synthesis of 2-(5,6-dichloro-3-(1-(4-fluorobenzyl)piperidin-4-yl)-2-oxo-2, 3-dihydro-1*H*-benzo[*d*]imidazol-1-yl)-*N*,*N*-dimethyl acetamide (compound **EX55**)

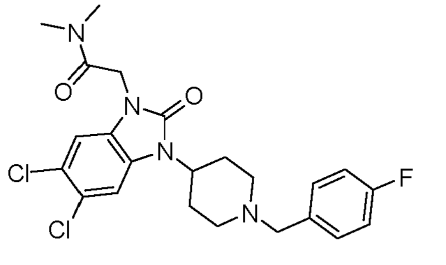

**EX55**

**54b** and 4-fluorobromobenzyl were used as raw materials for synthesis according to the synthesis method in Example 39 to obtain a white solid product (238 mg, with a yield of 60.2%). LC-MS (ESI) m/z: 480.20 $(M+H)^+$; $^1$H NMR (400 MHz, chloroform-d) δ 7.37 - 7.28 (m, 3H), 7.07 - 6.98 (m, 3H), 4.61 (s, 2H), 4.35 - 4.23 (m, 1H), 3.52 (s, 2H), 3.13 (s, 3H), 3.01 (d, *J* = 12.0 Hz, 2H), 2.98 (s, 3H), 2.37 (qd, *J* = 12.4, 3.8 Hz, 2H), 2.14 (t, *J* = 11.7 Hz, 2H), 1.79 (d, *J*= 11.4 Hz, 2H).

**Example 56:** Synthesis of 5,6-dichloro-1-(2-(dimethylamino)ethyl)-3-(1-(4-fluorobenzyl) piperidin-4-yl)-1,3-dihydro-2*H*-benzo[*d*]imidazol-2-one (compound **EX56)**

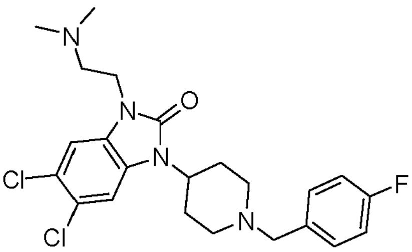

**EX56**

**46b** and 4-fluorobromobenzyl were used as raw materials for synthesis according to the synthesis method in Example 39 to obtain a white solid product (140 mg, with a yield of 41.8%). LC-MS (ESI) m/z: 466.20 $(M+H)^+$; $^1H$ NMR (400 MHz, chloroform-d) δ 7.36 - 7.28 (m, 3H), 7.09 (s, 1H), 7.02 (t, *J* = 8.7 Hz, 2H), 4.37 - 4.25 (m, 1H), 3.92 (t, *J* = 7.0 Hz, 2H), 3.52 (s, 2H), 3.02 (d, *J* = 11.6 Hz, 2H), 2.60 (t, *J* = 7.0 Hz, 2H), 2.42 - 2.32 (m, 2H), 2.31 (s, 6H), 2.14 (t, *J* = 11.2 Hz, 2H), 1.77 (d, *J* = 13.7 Hz, 2H).

**Example 57:** Synthesis of 5,6-dichloro-1-(1-(2-methylbenzyl)piperidin-4-yl)-3-(2-morpholinoethyl)-1,3-dihydro-2*H*-benzo[*d*]imidazol-2-one (compound **EX57)**

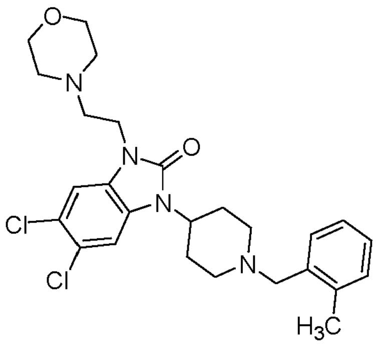

**EX57**

Intermediate **1g** and 2-methylbenzaldehyde were used as raw materials for synthesis according to the synthesis method in Example 1 to obtain a white solid product (120 mg, with a yield of 61.2%). LC-MS (ESI) m/z: 504.20 $(M+H)^+$; $^1H$ NMR (400 MHz, chloroform-d) δ 7.33 - 7.28 (m, 1H), 7.28 - 7.25 (m, 1H), 7.20 - 7.14 (m, 3H), 7.11 (s, 1H), 4.36 - 4.24 (m, 1H), 3.93 (t, *J* = 6.6 Hz, 2H), 3.66 (t, *J* = 4.6 Hz, 4H), 3.51 (s, 2H), 3.04 (d, *J* = 11.1 Hz, 2H), 2.64 (t, *J* = 6.6 Hz, 2H), 2.51 (t, *J* = 4.6 Hz, 4H), 2.41 (s, 3H), 2.39 - 2.27 (m, 2H), 2.18 (t, *J* = 11.4 Hz, 2H), 1.76 (d, *J* = 10.4 Hz, 2H).

**Example 58:** Synthesis of 5,6-dichloro-1-(1-(2-chlorobenzyl)piperidin-4-yl)-3-(2-morpholinoethyl)-1,3-dihydro-2*H*-benzo[*d*]imidazol-2-one (compound **EX58**)

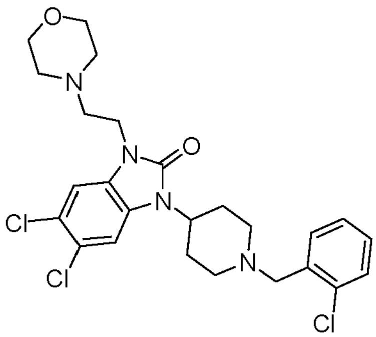

**EX58**

Intermediate **1g** and 2-chlorobromobenzyl were used as raw materials for synthesis according to the synthesis method in Example 39 to obtain a white solid product (78 mg, with a yield of 50.9%). LC-MS (ESI) m/z: 524.20 $(M+H)^+$; $^1H$ NMR (400 MHz, chloroform-d) δ 7.54 (d, *J* = 7.6 Hz, 1H), 7.36 (d, *J* = 7.9 Hz, 1H), 7.31 (s, 1H), 7.27 (t, *J* = 7.4 Hz, 1H), 7.20 (t, *J* = 7.5 Hz, 1H), 7.12 (s, 1H), 4.36 - 4.24 (m, 1H), 3.94 (t, *J* = 6.6 Hz, 2H), 3.69 (s, 2H), 3.66 (t, *J* = 4.6 Hz, 4H), 3.08 (d, *J* = 11.0 Hz, 2H), 2.64 (t, *J* = 6.6 Hz, 2H), 2.52 (t, *J* = 4.6 Hz, 4H), 2.41 (qd, *J* = 12.3, 3.5 Hz, 2H), 2.29 (t, *J* = 11.7 Hz, 2H), 1.78 (d, *J* = 11.5 Hz, 2H).

**Example 59:** Synthesis of 5,6-dichloro-1-(1-(2-fluorobenzyl)piperidin-4-yl)-3-(2-morpholinoethyl)-1,3-dihydro-2*H*-benzo[*d*]imidazol-2-one (compound **EX59)**

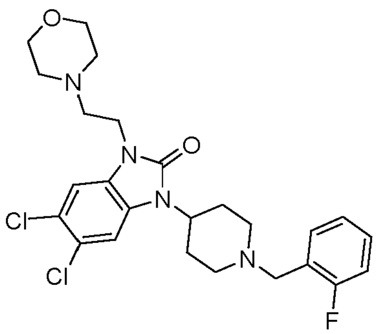

**EX59**

**1g** and 2-fluorobromobenzyl were used as raw materials for synthesis according to the synthesis method in Example 39 to obtain a white solid product (106 mg, with a yield of 71.5%). LC-MS (ESI) m/z: 508.20 $(M+H)^+$; $^1H$ NMR (400 MHz, chloroform-*d*) δ 7.43 (td, *J* = 7.5, 1.9 Hz, 1H), 7.33 (s, 1H), 7.30 - 7.22 (m, 1H), 7.15 (t, *J* = 7.5 Hz, 1H), 7.12 (s, 1H), 7.05 (t, *J* = 9.1 Hz, 1H), 4.36 - 4.22 (m, 1H), 3.94 (t, *J* = 6.7 Hz, 2H), 3.73 - 3.56 (m, 6H), 3.07 (d, *J* = 11.4 Hz, 2H), 2.64 (t, *J* = 6.6 Hz, 2H), 2.53 (t, *J* = 4.6 Hz, 4H), 2.37 (qd, *J* = 12.4, 3.8 Hz, 2H), 2.23 (t, *J* = 11.3 Hz, 2H), 1.77 (d, *J* = 11.6 Hz, 2H).

**Example 60:** Synthesis of 5,6-dichloro-1-(1-(2-fluorobenzyl)piperidin-4-yl)-3-(oxiran-2-ylmethyl)-1,3-dihydro-2*H*-benzo[*d*]imidazol-2-one (compound **EX60)**

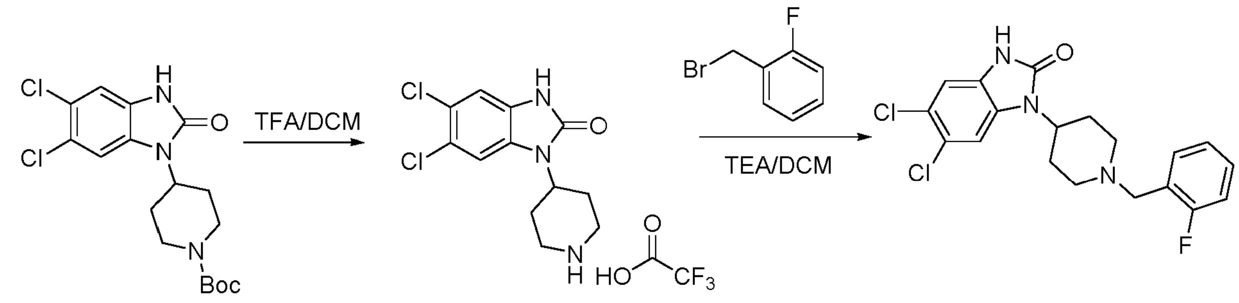


1e 60a 60b

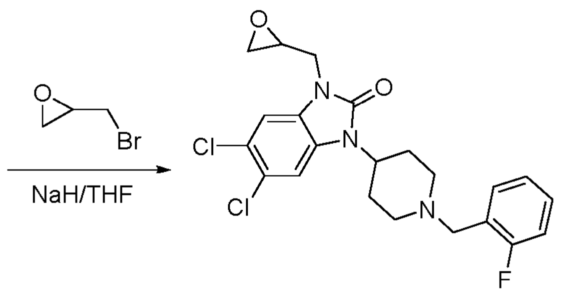


EX60

Synthesis of intermediate **60a:** intermediate **1e** was used as raw material and was subjected to the action of trifluoroacetic acid at room temperature to remove Boc protection group to obtain 520 mg white trifluoroacetate solid, with a yield of 99.8%. The solid was directly used in the next reaction without purification.

Synthesis of intermediate **60b:** intermediate **60a** and 2-fluorobromobenzyl were used as raw materials for synthesis according to the synthesis method in Example 39 to obtain 197 mg white solid intermediate **60b,** with a yield of 99.9%. $^1$H NMR (400 MHz, chloroform-d) δ 10.67 (s, 1H), 7.43 (t, *J* = 7.7 Hz, 1H), 7.35 (s, 1H), 7.31 - 7.25 (m, 1H), 7.20 (s, 1H), 7.15 (t, *J* = 7.3 Hz, 1H), 7.06 (t, *J* = 9.0 Hz, 1H), 4.35 - 4.20 (m, 1H), 3.70 (s, 2H), 3.11 (d, *J* = 11.1 Hz, 2H), 2.41 (q, *J* = 12.6 Hz, 2H), 2.26 (t, *J* = 11.9 Hz, 2H), 1.80 (d, *J* = 11.9 Hz, 2H).

Synthesis of compound **EX60:** intermediate **60b** and bromomethyl ethylene oxide were used raw materials for synthesis according to the synthesis method of intermediate **1f** to obtain a light yellow solid product (48 mg, with a yield of 42.0%). LC-MS (ESI) m/z: 451.10 (M+H)$^+$; $^1$H NMR (400 MHz, chloroform-d) δ 7.43 (t, *J* = 7.1 Hz, 1H), 7.34 (s, 1H), 7.30 - 7.23 (m, 2H), 7.15 (t, *J* = 7.3 Hz, 1H), 7.06 (t, *J* = 9.1 Hz, 1H), 4.41 (dd, *J* = 15.2, 2.6 Hz, 1H), 4.36 - 4.24 (m, 1H), 3.67 (s, 2H), 3.62 (dd, *J* = 15.3, 6.4 Hz, 1H), 3.27 - 3.17 (m, 1H), 3.08 (d, *J* = 10.7 Hz, 2H), 2.87 (t, *J* = 4.3 Hz, 1H), 2.65 (dd, *J* = 4.6, 2.6 Hz, 1H), 2.38 (q, *J* = 11.3, 10.8 Hz, 2H), 2.24 (t, *J* = 11.4 Hz, 2H), 1.85 - 1.71 (m, 2H).

**Example 61:** Synthesis of 3-(1-(2-chlorobenzyl)piperidin-4-yl)-5-fluoro-1-(2-morpholinoethyl) -1,3-dihydro-2*H*-benzo [*d*]imidazol-2-one (compound **EX61)**

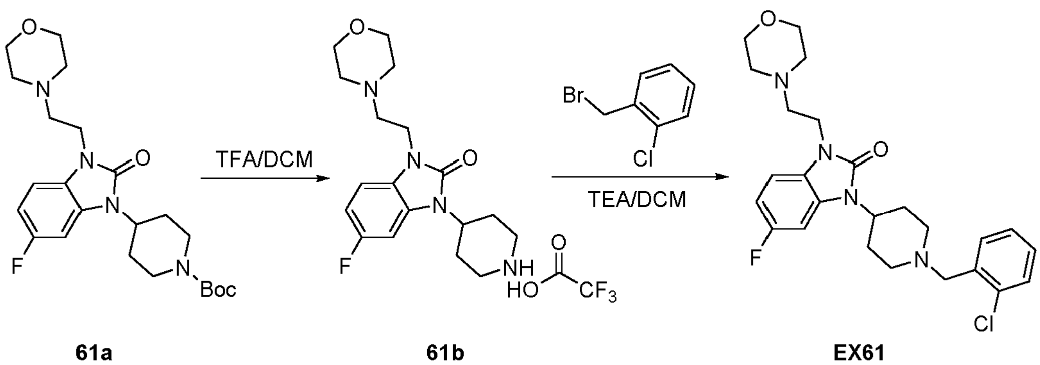


61a 61b EX61

Synthesis of intermediate **61a:** 2,4-difluoronitrobenzene and 1-*N*-Boc-4-aminopiperidine were used as raw materials for synthesis according to the synthesis method of intermediate **1f** to obtain 1.07 g yellow waxy product, with a yield of 80.0%. $^1$H NMR (400 MHz, chloroform-*d*) δ 6.97 - 6.90 (m, 1H), 6.88 (d, *J* = 9.1 Hz, 1H), 6.80 (t, *J* = 8.9 Hz, 1H), 4.50 - 4.17 (m, 3H), 3.99 (t, *J* = 6.9 Hz, 2H), 3.68 (t, *J* = 4.4 Hz, 4H), 2.85 (s, 2H), 2.67 (t, *J* = 7.2 Hz, 2H), 2.54 (t, *J* = 4.5 Hz, 4H), 2.26 (qd, *J* = 12.9, 4.3 Hz, 2H), 1.80 (d, *J* = 12.6 Hz, 2H), 1.50 (s, 9H).

Synthesis of intermediate **61b:** intermediate **61a** was subjected to the action of trifluoroacetic acid at room temperature to remove Boc protection group to obtain 1.03 g white trifluoroacetate solid, with a yield of 99.9%. The solid was directly used in the next reaction without purification.

Synthesis of compound **EX61:** intermediate **61b** and 2-chlorobromobenzyl were used as raw materials for synthesis according to the synthesis method in Example 39 to obtain 226 mg light yellow solid product, with a yield of 88.4%. LC-MS (ESI) m/z: 474.20 (M+H)$^+$; $^1$H NMR (400 MHz, chloroform-d) δ 7.56 (d, *J* = 7.6 Hz, 1H), 7.35 (d, *J* = 7.9 Hz, 1H), 7.31 - 7.23 (m, 1H), 7.19 (t, *J* = 7.7 Hz, 1H), 7.04 (d, *J* = 9.1 Hz, 1H), 6.95 - 6.88 (m, 1H), 6.79 (t, *J* = 8.9 Hz, 1H), 4.42 - 4.29 (m, 1H), 3.98 (t, *J* = 6.8 Hz, 2H), 3.74 - 3.59 (m, 6H), 3.07 (d, *J* = 11.0 Hz, 2H), 2.66 (t, *J* = 7.0 Hz, 2H), 2.59 - 2.50 (m, 4H), 2.44 (q, *J* = 12.8 Hz, 2H), 2.29 (t, *J* = 11.6 Hz, 2H), 1.79 (d, *J*= 11.7 Hz, 2H).

**Example 62:** Synthesis of 1-(1-(2-chlorobenzyl)piperidin-4-yl)-5-methyl-3-(2-morpholinoethyl)-1,3-dihydro-2*H*-benzo [*d*]imidazol-2-one (compound **EX62)**

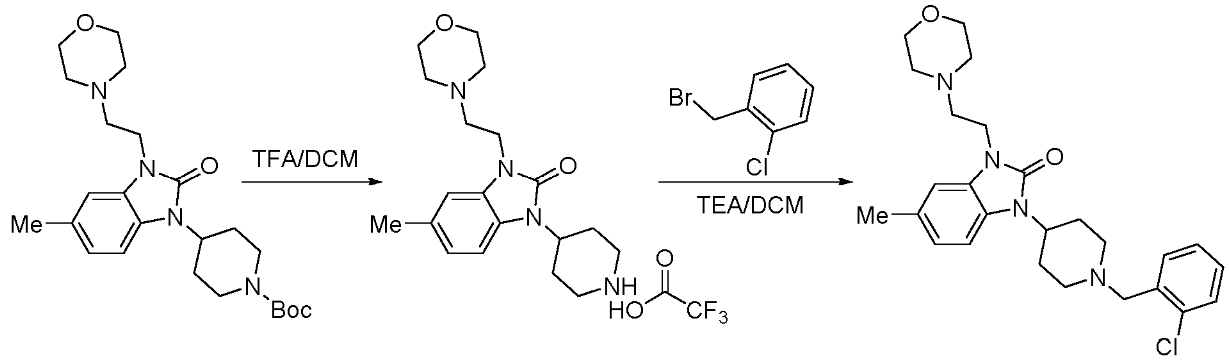


62a 62b EX62

Synthesis of intermediate **62a:** 4-fluoro-3-nitrotoluene and 1-*N*-Boc-4-aminopiperidine were used as raw materials for synthesis according to the synthesis method of intermediate **1f** to obtain 526 mg colorless waxy product, with a yield of 78.4%. $^1$H NMR (400 MHz, chloroform-d) δ 7.01 (d, *J* = 8.0 Hz, 1H), 6.90 - 6.82 (m, 2H), 4.51 - 4.39 (m, 1H), 4.30 (br s, 2H), 3.99 (t, *J* = 7.0 Hz, 2H), 3.69 (t, *J* = 4.5 Hz, 4H), 2.95 - 2.76 (m, 2H), 2.67 (t, *J* = 7.3 Hz, 2H), 2.55 (t, *J* = 4.4 Hz, 4H), 2.40 (s, 3H), 2.29 (qd, *J* = 12.4, 3.7 Hz, 2H), 1.80 (d, *J* = 13.1 Hz, 2H), 1.50 (s, 9H).

Synthesis of intermediate **62b:** intermediate **62a** was subjected to the action of trifluoroacetic acid at room temperature to remove Boc protection group to obtain 459 mg white trifluoroacetate solid, with a yield of 99.8%. The solid was directly used in the next reaction without purification.

Synthesis of compound **EX62:** intermediate **62b** and 2-chlorobromobenzyl were used as raw materials for synthesis according to the synthesis method in Example 39 to obtain a white solid product (186 mg, with a yield of 90.9%). LC-MS (ESI) m/z: 470.20 (M+H)$^+$; $^1$H NMR (400 MHz, chloroform-*d*) δ 7.54 (d, *J* = 7.6 Hz, 1H), 7.35 (d, *J* = 7.8 Hz, 1H), 7.30 - 7.23 (m, 1H), 7.20 (d, *J*= 7.8 Hz, 1H), 7.15 (d, *J*= 8.6 Hz, 1H), 6.91 - 6.81 (m, 2H), 4.42 - 4.29 (m, 1H), 3.98 (t, *J* = 7.1 Hz, 2H), 3.76 - 3.60 (m, 6H), 3.05 (d, *J* = 11.1 Hz, 2H), 2.67 (t, *J* = 7.2 Hz, 2H), 2.55 (t, *J* = 4.6 Hz, 4H), 2.51 - 2.43 (m, 2H), 2.40 (s, 3H), 2.28 (t, *J* = 11.7 Hz, 2H), 1.78 (d, *J* = 11.8 Hz, 2H).

**Example 63:** Synthesis of 6,7-dichloro-1-(1-(2-fluorobenzyl)piperidin-4-yl)-3, 4-dihydroquinazolin-2(1*H*)-one (compound **EX63)**

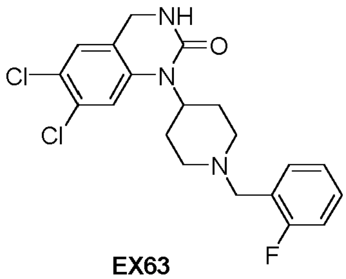

**EX63**

**52a** and 2-fluorobromobenzyl were used as raw materials for synthesis according to the synthesis method in Example 39 to obtain a white solid product (56 mg, with a yield of 56.8%). LC-MS (ESI) m/z: 425.10 $(M+H)^+$; $^1H$ NMR (400 MHz, chloroform-d) δ 7.45 (t, *J* = 7.5 Hz, 1H), 7.29 - 7.22 (m, 1H), 7.19 - 7.08 (m, 3H), 7.04 (t, *J* = 9.1 Hz, 1H), 5.48 (s, 1H), 4.22 (s, 2H), 3.99 - 3.85 (m, 1H), 3.67 (s, 2H), 3.05 (d, *J* = 10.9 Hz, 2H), 2.69 (q, *J* = 11.7, 10.9 Hz, 2H), 2.20 (t, *J* = 11.3 Hz, 2H), 1.74 (d, *J* = 11.7 Hz, 2H).

**Example 64:** Synthesis of 5,6-dichloro-1-(2-morpholinoethyl)-3-(1-phenylethylpiperidin-4-yl) -1,3-dihydro-2H-benzo[*d*] imidazol-2-one (compound **EX64)**

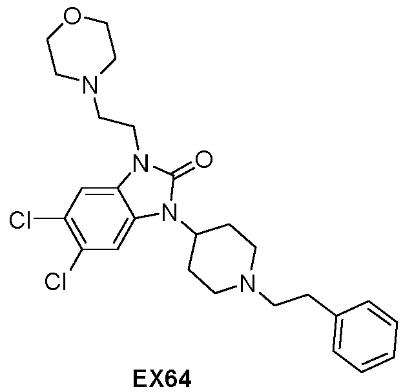

**EX64**

Intermediate **1g** and phenylacetaldehyde were used as raw materials for synthesis according to the synthesis method in Example 1 to obtain a white solid product (115 mg, with a yield of 59%). HRMS (ESI): *m/z* calcd for $C_{26}H_{33}Cl_2N_4O_2$ ($[M+H]^+$) 503.1975, found 503.1968; HPLC purity: 98.90%, $t_R$= 5.10 min. $^1H$ NMR (400 MHz, chloroform-d) δ 7.38 (s, 1H), 7.33 - 7.28 (m, 2H), 7.25 - 7.18 (m, 3H), 7.12 (s, 1H), 4.34 (tt, *J* = 12.5, 4.3 Hz, 1H), 3.94 (t, *J* = 6.6 Hz, 2H), 3.66 (t, *J* = 4.6 Hz, 4H), 3.17 (d, *J* = 11.2 Hz, 2H), 2.84 (dd, *J* = 10.4, 6.0 Hz, 2H), 2.71 - 2.61 (m, 4H), 2.52 (t, *J* = 4.6 Hz, 4H), 2.39 (qd, *J* = 12.4, 3.8 Hz, 2H), 2.22 (t, *J* = 11.5 Hz, 2H), 1.82 (d, *J* = 10.9 Hz, 2H).

**Example 65:** Synthesis of 5,6-dichloro-1-(1-(2,6-dichlorobenzyl) piperidin-4-yl)-3-(2-morpholinoethyl)-1,3-dihydro-2H-benzo[*d*]imidazol-2-one (compound **EX65)**

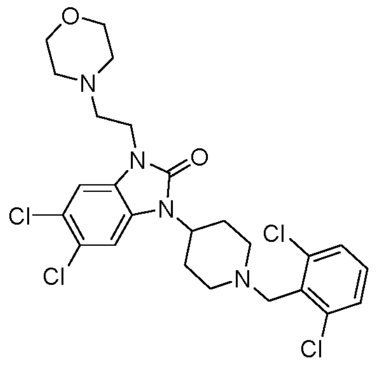

**EX65**

Intermediate **1g** and 2,6-dichlorobenzaldehyde were used as raw materials for synthesis according to the synthesis method in Example 1 to obtain a white solid product (163 mg, with a yield of 75%). HRMS (ESI): *m*/*z* calcd for $C_{25}H_{29}Cl_4N_4O_2$ ([M+H]$^+$) 557.1039, found 557.1034; HPLC purity: 99.46%, $t_R$= 5.89 min. $^1$H NMR (400 MHz, chloroform-d) δ 7.32 (d, *J* = 8.0 Hz, 2H), 7.26 (s, 1H), 7.15 (t, *J* = 8.0 Hz, 1H), 7.10 (s, 1H), 4.30 (tt, *J* = 12.3, 4.2 Hz, 1H), 3.94 (t, *J* = 6.6 Hz, 2H), 3.80 (s, 2H), 3.66 (t, *J* = 4.5 Hz, 4H), 3.07 (d, *J* = 11.1 Hz, 2H), 2.64 (t, *J* = 6.6 Hz, 2H), 2.51 (t, *J= 4.6* Hz, 4H), 2.41 (t, *J*= 11.4 Hz, 2H), 2.31 (qd, *J*= 12.1, 3.5 Hz, 2H), 1.74 (d, *J* = 11.0 Hz, 2H).

**Example 66:** Synthesis of 5,6-dichloro-1-(1-(2,6-dimethylbenzyl) piperidin-4-yl)-3-(2-morpholinoethyl)-1,3-dihydro-2H-benzo[*d*]imidazol-2-one (compound **EX66)**

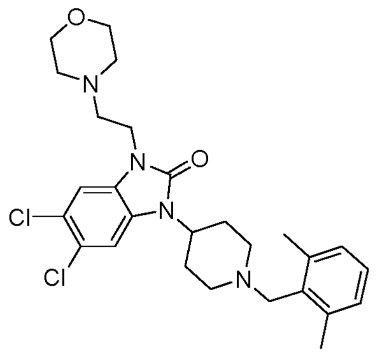

**EX66**

Intermediate **1g** and 2,6-dimethylbenzaldehyde were used as raw materials for synthesis according to the synthesis method in Example 1 to obtain a white solid product (163 mg, with a yield of 75%). HRMS (ESI): *m*/*z* calcd for $C_{27}H_{35}Cl_2N_4O_2$ ([M+H]$^+$) 517.2132, found 517.2118; HPLC purity: 100%, $t_R$= 6.60 min. $^1$H NMR (400 MHz, chloroform-d) δ 7.20 (s, 1H), 7.12 - 7.04 (m, 2H), 7.04 - 6.99 (m, 2H), 4.35 - 4.22 (m, 1H), 3.92 (t, *J* = 6.6 Hz, 2H), 3.65 (t, *J* = 4.6 Hz, 4H), 3.53 (s, 2H), 2.97 (d, *J* = 8.2 Hz, 2H), 2.63 (t, *J* = 6.6 Hz, 2H), 2.51 (t, *J* = 4.6 Hz, 4H), 2.42 (s, 6H), 2.35 - 2.19 (m, 4H), 1.73 (d, *J* = 9.2 Hz, 2H).

**Example 67:** 6,7-dichloro-1-(1-(4-isopropylcyclohexyl)piperidin-4-yl)-3-(2-morpholinoethyl) -3,4-dihydroquinazolin-2(1*H*)-one

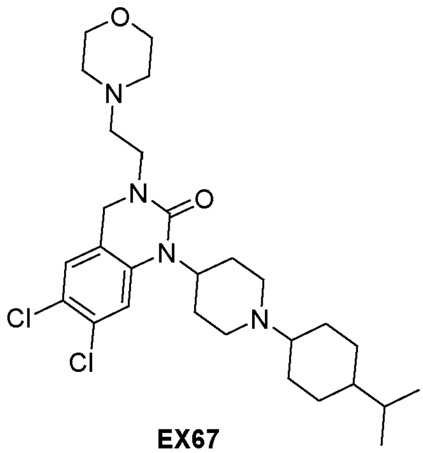

**EX67**

Intermediate **51g** and 4-isopropylcyclohexanone were used as raw materials for synthesis of compound **EX67** (345 mg white solid, with a yield of 46%) according to the synthesis method of compound **EX12.** The compound was a mixture of cis-trans isomers, in a ratio of about 5:2. HRMS (ESI): *m/z* calcd for $C_{28}H_{43}Cl_2N_4O_2$ ([M+H]$^+$) 537.2758, found 537.2741; HPLC purity (mixture): 28.09%, $t_{R1}$= 7.48 min; 71.91%, $t_{R2}$= 8.61 min. Main products: $^1$H NMR (400 MHz, chloroform-d) δ 7.26 (s, 1H), 7.12 (s, 1H), 4.24 (s, 2H), 4.05 (m, 1H), 3.68 (t, *J* = 4.6 Hz, 4H), 3.51 (t, *J* = 6.7 Hz, 2H), 3.25 (d, *J* = 11.4 Hz, 2H), 2.77 - 2.61 (m, 2H), 2.60 - 2.44 (m, 7H), 2.34 (t, *J* = 11.3 Hz, 2H), 1.86 - 1.69 (m, 4H), 1.69 - 1.52 (m, 4H), 1.45 - 1.31 (m, 3H), 1.19 - 1.08 (m, 1H), 0.89 (d, *J* = 6.6 Hz, 6H). Secondary products: $^1$H NMR (400 MHz, chloroform-d) δ 7.35 (s, 1H), 7.12 (s, 1H), 4.24 (s, 2H), 4.21 - 4.12 (m, 1H), 3.68 (t, *J* = 4.6 Hz, 4H), 3.51 (t, *J* = 6.7 Hz, 2H), 3.25 (d, *J* = 11.4 Hz, 2H), 2.77 - 2.61 (m, 2H), 2.60 - 2.44 (m, 7H), 2.01 (d, *J* = 11.9 Hz, 2H), 1.86 - 1.69 (m, 4H), 1.69 - 1.52 (m, 4H), 1.19 - 1.08 (m, 1H), 1.08 - 0.95 (m, 3H), 0.86 (d, *J*= 7.0 Hz, 6H).

**Example 68:** 6,7-dichloro-3-(1-(4-isopropylcyclohexyl)piperidin-4-yl)-1-(2-morpholinoethyl) -3,4-dihydroquinazolin-2(1*H*)-one

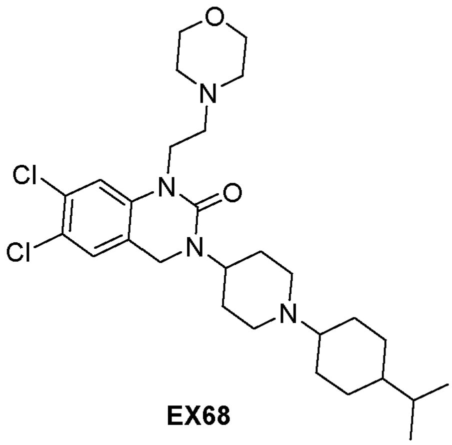

**EX68**

Intermediate **47f** and 4-isopropylcyclohexyl were used as raw materials for synthesis according to the synthesis method of compound **EX12** to obtain compound **EX68,** 78 mg white solid, with a yield of 38%. The compound was a mixture of cis-trans isomers, in a ratio of about 3:1. HRMS (ESI): *m/z* calcd for $C_{28}H_{43}Cl_2N_4O_2$ ([M+H]$^+$) 537.2758, found 537.2741; HPLC purity (220 nm): 23.07%, $t_{R1}$= 9.12 min; 74.25%, $t_{R1}$= 10.33 min. Main products: $^1$H NMR (400 MHz, chloroform-d) δ 7.13 (s, 1H), 7.11 (s, 1H), 4.30 (tt, *J* = 12.1, 4.1 Hz, 1H), 4.22 (s, 2H), 3.96 (t, *J* = 7.1 Hz, 2H), 3.73 (t, *J*=4.6 Hz, 4H), 3.11 (d, *J*= 11.3 Hz, 2H), 2.62 (t, *J* = 7.1 Hz, 2H), 2.56 (t, *J* = 4.7 Hz, 4H), 2.33 - 2.25 (m, 1H), 2.25 - 2.15 (m, 2H), 1.97 - 1.77 (m, 3H), 1.77 - 1.65 (m, 4H), 1.65 - 1.49 (m, 4H), 1.46 - 1.32 (m, 2H), 1.18 - 1.08 (m, 1H), 0.89 (d, *J* = 6.6 Hz, 6H). Secondary products: $^1$H NMR (400 MHz, chloroform-d) δ 7.11 (s, 1H), 7.10 (s, 1H), 4.30 (tt, *J* = 12.1, 4.1 Hz, 1H), 4.22 (s, 2H), 3.96 (t, *J* = 7.1 Hz, 2H), 3.73 (t, *J* = 4.6 Hz, 4H), 3.04 (d, *J* = 11.7 Hz, 2H), 2.62 (t, *J* = 7.1 Hz, 2H), 2.56 (t, *J* = 4.7 Hz, 4H), 2.42 - 2.33 (m, 2H), 2.33 - 2.25 (m, 1H), 1.97 - 1.77 (m, 3H), 1.77 - 1.65 (m, 4H), 1.65 - 1.49 (m, 4H), 1.46 - 1.32 (m, 2H), 1.18 - 1.08 (m, 1H), 0.86 (d, *J* = 6.8 Hz, 6H).

**Example 69B:** 1-(1-((1S, 4S)-4-isopropylcyclohexyl) piperidin-4-yl)-5-methyl-3-(2-morpholinoethyl)-1,3-dihydro-2*H*-benzo[*d*]imidazol-2-one

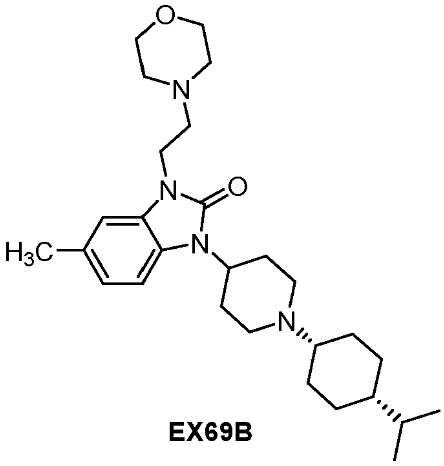

**EX69B**

Intermediate **62b** and 4-isopropylcyclohexyl were used as raw materials for synthesis according to the synthesis method of compound **EX12** to obtain compound **EX69,** which was subjected to separation by high performance liquid chromatography to obtain cis-product EX69B, 96 mg white solid, with a separation yield of 19%. HRMS (ESI): *m*/*z* calcd for $C_{28}H_{45}N_4O_2$ ($[M+H]^+$) 469.3537, found 469.3542; HPLC purity (220 nM): 96.12%, $t_R$= 4.87 min. $^1H$ NMR (400 MHz, chloroform-d) δ 7.19 (d, *J* = 7.5 Hz, 1H), 6.87 (d, *J* = 8.4 Hz, 1H), 6.84 (s, 1H), 4.39 - 4.25 (m, 1H), 3.98 (t, *J* = 7.2 Hz, 2H), 3.70 (t, *J* = 4.6 Hz, 4H), 3.15 (d, *J* = 9.2 Hz, 2H), 2.67 (t, *J* = 7.2 Hz, 2H), 2.56 (t, *J* = 4.6 Hz, 4H), 2.48 - 2.29 (m, 6H), 2.29 - 2.15 (m, 2H), 1.85 - 1.76 (m, 2H), 1.76 - 1.68 (m, 2H), 1.68 - 1.58 (m, 3H), 1.58 - 1.47 (m, 2H), 1.44 - 1.32 (m, 2H), 1.18 - 1.09 (m, 1H), 0.89 (d, *J* = 6.6 Hz, 6H).

**Example 70B:** 5-chloro-1-(1-((1S, 4S)-4-isopropylcyclohexyl) piperidin-4-yl)-3-(2-morpholinoethyl)-1,3-dihydro-2*H*-benzo[*d*]imidazol-2-one

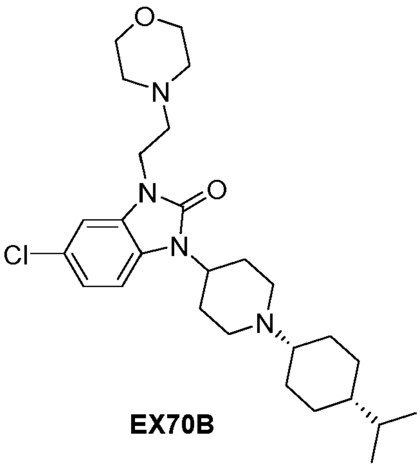

**EX70B**

Intermediate **32b** and 4-isopropylcyclohexyl were used as raw materials for synthesis according to the synthesis method of compound **EX12** to obtain compound **EX70,** which was subjected to separation by high performance liquid chromatography to obtain cis-product EX70B, 106 mg white solid, with a separation yield of 17%. HRMS (ESI): *m*/*z* calcd for $C_{27}H_{42}ClN_4O_2$ ($[M+H]^+$) 489.2991, found 489.2996; HPLC purity: 96.24%, $t_R$= 5.53 min. $^1H$ NMR (400 MHz, chloroform-d) δ 7.21 (d, *J* = 7.8 Hz, 1H), 7.09 - 6.96 (m, 2H), 4.32 (tt, *J* = 13.0, 4.3 Hz, 1H), 3.97 (t, *J* = 6.9 Hz, 2H), 3.69 (t, *J* = 4.6 Hz, 4H), 3.16 (d, *J* = 11.2 Hz, 2H), 2.66 (t, *J* = 6.8 Hz, 2H), 2.54 (t, *J* = 4.6 Hz, 4H), 2.47 - 2.30 (m, 3H), 2.30 - 2.17 (m, 2H), 1.87 - 1.76 (m, 2H), 1.76 - 1.66 (m, 2H), 1.66 - 1.58 (m, 3H), 1.58 - 1.48 (m, 2H), 1.45 - 1.33 (m, 2H), 1.19 - 1.09 (m, 1H), 0.90 (d, *J* =

6.6 Hz, 6H).

**Example 71B:** 5-fluoro-3-(1-((1S, 4S)-4-isopropylcyclohexyl) piperidin-4-yl)-1-(2-morpholinoethyl)-1,3-dihydro-2*H*-benzo[*d*]imidazol-2-one

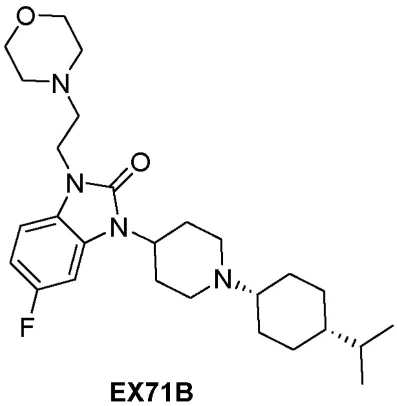

**EX71B**

Intermediate **61b** and 4-isopropylcyclohexyl were used as raw materials for synthesis according to the synthesis method of compound **EX12** to obtain compound **EX71,** which was subjected to separation by high performance liquid chromatography to obtain cis-product **EX71B,** 76 mg light yellow solid, with a separation yield of 12%. HRMS (ESI): *m*/*z* calcd for $C_{27}H_{42}FN_4O_2$ ($[M+H]^+$) 473.3286, found 473.3297; HPLC purity: >99%, $t_R$= 4.18 min. $^1$H NMR (400 MHz, chloroform-d) δ 7.12 - 7.02 (m, 1H), 6.91 (dd, *J* = 8.6, 4.5 Hz, 1H), 6.78 (td, *J* = 9.1, 2.4 Hz, 1H), 4.31 (tt, *J* = 12.4, 4.3 Hz, 1H), 3.98 (t, *J* = 7.0 Hz, 2H), 3.68 (t, *J* = 4.6 Hz, 4H), 3.16 (d, *J* = 11.0 Hz, 2H), 2.66 (t, *J* = 7.0 Hz, 2H), 2.53 (t, *J* = 4.7 Hz, 4H), 2.44 - 2.27 (m, 3H), 2.22 (t, *J* = 11.7 Hz, 2H), 1.85 - 1.76 (m, 2H), 1.76 - 1.58 (m, 5H), 1.58 - 1.46 (m, 2H), 1.44 - 1.33 (m, 2H), 1.18 - 1.08 (m, 1H), 0.90 (d, *J* = 6.6 Hz, 6H).

**Test for bioactivity**

Test of affinity of compounds to MOP receptor, NOP receptor (ORL-1 receptor), KOP receptor, and DOP receptor:

The compounds were dissolved in 100% DMSO solution (10 mM) as a stock solution for later use. The compounds were diluted in a 384-well round bottom plate. The initial screening concentration of the compounds was: 1 μM; 0.1 μm, repeated twice. The starting concentration for compound rescreening was 1 μM (MOP receptor) or 10 μM (NOP receptor) or 100 μM (KOP receptor and DOP receptor), 3-fold dilution, 10 concentration gradients, repeated twice; positive controls were: DAMGO (MEC) (starting concentration was 1 μM, 3-fold dilution, 10 concentration gradients, repeated three times, MOP receptor); Nociceptin (N/O FQ) (Sigma) (starting concentration was 1 μM, 3-fold dilution, 10 concentration gradients, repeated three times, NOP receptor); Naltrexone hydrochloride (Sigma) (starting concentration was 0.1 μM, 3-fold dilution, 10 concentration gradients, repeated three times, KOP receptor); Naltrexone hydrochloride (starting concentration was 10 μM, 3-fold dilution, 10 concentration gradients, repeated three times, DOP receptor). The working solution was 50 mM Tris pH 7.4, 5 mM $MgCl_2$, the washing buffer was 50 mM Tris pH 7.4, stored at 4°C, 0.5% BSA solution (0.5 mL BSA added in 100 mL double distilled water, stored at 4°C).

The reaction system was 500 μL, and 100 μL of working solution and 5 μL of solution of the compounds (1% DMSO, 99% water) were added to a 96-well deep-well plate, and shaken well (5 min, 500 rpm). 299 μL of reaction solution containing 1 μL CHO-K1 MOP receptor cell membrane (PerkinElmer) (or HEK293-NOP receptor cell membrane, or CHO-K1 DOP receptor cell membrane, PerkinElmer) was added to each well (298 μL of reaction solution containing 2 μL CHO-K1 KOP receptor cell membrane, PerkinElmer, was added in KOP receptor test) and shaken well (5 min, 500 rpm). 100 μL of deuterated ligand was added to each well: [$^3$H]-DAMGO (PerkinElmer) (Fianl conc.1 nM) (MOP receptor), [$^3$H]-Nociceptin (PerkinElmer) (Fianl conc.0.5 nM) (NOP receptor), [$^3$H]-U69593 (PerkinElmer) (Fianl conc.1.5 nM) (KOP receptor), [$^3$H]-DADLE (PerkinElmer) (Fianl conc.1 nM) (DOP receptor), and shaken well (5 min, 500 rpm). The plate was subjected to incubation at 27°C for 1 hour. UNIFILTER-96 GF/C filter plate (0.5% PEI) was pre-incubated at 4°C for 1 hour. The UNIFILTER-96 GF/C filter plate (0.5% PEI) was washed twice (per well) with 1 mL of washing buffer. The mixture solution containing cell membrane was transferred to the UNIFILTER-96 GF/C filter plate (0.5% PEI), which was washed four times with washing buffer, 50 mL each time, followed by drying (55°C, 10 minutes). Then, 40 μL of scintillation cocktail was added to each well, and scintillation counts were read using TopCount to determine the radioactivity bound to the

membrane.

Each test compound was tested for binding rate (% Inhibition) at 10 concentrations, its $IC_{50}$ (the concentration at which 50% of binding is inhibited) was determined by the graph in which the X axis is logarithm of concentration and the Y axis is response count, and the data was processed using Xl-fit 5.3.1 software. Nonlinear regression equation:

Y=minimum value + (maximum value - minimum value)/(1+10^(log (IC50-X) ×Hill coefficient)

X= logarithm of the concentration of the compound;

*Y*= percentage of inhibition (% Inhibition);

Maximum and minimum values: complex numbers having the same unit as *Y;*

*LogIC50:* with the same unit as logarithm of the *X* axis;

*Hill* coefficient: slope factor or Hill coefficient.

For the binding ability of representative compounds of the present application to receptor cell membranes, radiolabeled [$^3$H]-DAMGO (PerkinElmer), [$^3$H]-Nociceptin (PerkinElmer), [$^3$H]-U69,593 (PerkinElmer), [$^3$H]-DADLE (PerkinElmer) were used as interchangeable ligands, $K_i$ value is determined by the equation $K_i = IC_{50}/(1 + L/K_d)$, wherein $K_d$ is binding affinity of [$^3$H]- radioligand, and L is the concentration of [$^3$H]-radioligand used.

The determined affinity $K_i$ values of the compounds of the present application are shown in the following table, wherein "/" means no test data:

| Compound | Opioid receptor affinity $K_i$ (nM) | | | |
|---|---|---|---|---|
| | *h*MOPr | *h*NOPr | *h*KOPr | *h*DOPr |
| DAMGO | 0.50 | / | / | / |
| Morphine | 1.20 | / | 40.08 | 821.59 |
| Nociceptin | / | 4.15 | / | / |
| Naltrexone HCl salt | / | / | 0.29 | 29.62 |
| EX1 | 22.78 | / | 50.07 | >$10^4$ |
| EX2 | 8.24 | / | 6.53 | 1932.56 |
| EX3 | 0.33 | 24.51 | 0.73 | 138.16 |
| EX4 | 7.10 | / | 396.67 | 1562.73 |
| EX5 | 4.61 | / | / | / |
| EX6 | 0.60 | 53.44 | 6.37 | 315.31 |
| EX8 | 9.07 | 118.46 | 275.45 | 1496.00 |
| EX16 | 1.18 | 211.48 | 12.60 | 1065.67 |
| EX17 | 1.87 | 98.14 | 2.19 | 1767.11 |
| EX18 | 0.21 | 39.65 | 2.96 | 382.67 |
| EX19 | 0.81 | 75.17 | 8.78 | 870.52 |
| EX20 | 11.36 | 29.42 | 61.92 | 2881.93 |
| EX24 | 2.36 | / | 15.89 | 1079.77 |
| EX25 | 0.97 | 398.25 | 17.89 | 1606.32 |
| EX26 | 2.38 | 17.54 | 38.40 | 1908.19 |
| EX28 | 0.77 | 135.63 | 22.49 | 366.25 |
| EX29 | 0.15 | 52.54 | 12.68 | 118.77 |

(continued)

| Compound | Opioid receptor affinity $K_i$ (nM) | | | |
|---|---|---|---|---|
| | *h*MOPr | *h*NOPr | *h*KOPr | *h*DOPr |
| EX30 | 0.52 | 62.37 | 5.57 | 427.79 |
| EX31 | 0.56 | 559.19 | 44.48 | 5016.87 |
| EX33 | 0.72 | 18.08 | 13.47 | 717.90 |
| EX35 | 1.86 | 46.63 | 12.36 | 1918.30 |
| EX37 | 5.07 | 61.23 | 43.72 | 2361.60 |
| EX39 | 0.59 | 14.47 | 3.27 | / |
| EX40 | 1.74 | 20.19 | 0.39 | / |
| EX41 | 2.30 | 19.77 | 1.82 | / |
| EX42 | 1.06 | 13.62 | 2.17 | / |
| EX43 | 1.31 | 8.63 | 2.54 | / |
| EX44 | 3.20 | 15.12 | 1.40 | / |
| EX46 | 1.60 | 5.70 | 2.58 | / |
| EX47 | 188.31 | 2188.27 | 27.07 | 98.82 |
| EX49 | 2.38 | 7.10 | 4.80 | 277.41 |
| EX50 | 13.29 | 518.49 | 7.84 | 877.58 |
| EX51 | 0.84 | 10.40 | 4.19 | 168.40 |
| EX52 | 2.45 | 13.64 | 0.75 | 1048.78 |
| EX53 | 1.72 | 3.63 | 3.95 | 153.84 |
| EX54 | 1.77 | 67.87 | 2.72 | 3292.40 |
| EX55 | 16.60 | 78.47 | 9.86 | 3687.60 |
| EX56 | 4.50 | 42.47 | 7.72 | 3222.03 |
| EX57 | 1.26 | 20.85 | 0.38 | 135.08 |
| EX58 | 2.30 | 38.15 | 0.90 | 697.21 |
| EX59 | 5.95 | 32.69 | 0.13 | 1082.91 |
| EX60 | 2.30 | 9.33 | 2.24 | 416.64 |
| EX61 | 9.19 | 73.43 | 5.70 | 1770.73 |
| EX62 | 16.80 | 128.11 | 3.83 | 3040.01 |
| EX63 | 8.67 | 26.34 | 0.75 | 1223.72 |
| EX64 | 43.10 | 486.59 | 12.24 | 1488.76 |
| EX65 | 10.47 | / | 6.40 | 1830.45 |
| EX66 | 9.66 | / | 8.68 | 2858.12 |
| EX67 | 0.59 | 31.86 | 7.34 | $>10^4$ |
| EX68 | 192.14 | 581.25 | / | / |
| EX12 | 3.40 | 58.53 | 69.30 | 3812.90 |
| EX12B | 10.21 | 38.11 | 66.32 | $>10^4$ |
| EX34 | 9.58 | 82.82 | 202.48 | 17621.40 |
| EX36 | 14.78 | 67.91 | 261.84 | 29007.20 |
| EX38 | 29.84 | 38.86 | 340.73 | 35120.80 |

(continued)

| Compound | Opioid receptor affinity $K_i$ (nM) | | | |
|---|---|---|---|---|
| | *h*MOPr | *h*NOPr | *h*KOPr | *h*DOPr |
| EX48B | 2.62 | 4.12 | 10.69 | >$10^4$ |
| EX69B | 76.30 | 328.83 | / | / |
| EX70B | 29.29 | 167.04 | / | / |
| EX71B | 31.60 | 16.53 | 144.06 | >$10^4$ |

In the present application, the compounds represented by EX3, EX6, EX16, EX17, EX54, EX58, and EX67 show high selectivity and high affinity for MOP receptor and KOP receptor, and such bifunctional agonists, which act on both MOP receptor and KOP receptor, have been reported in existing literatures, to have the potential to reduce the side effects of single-selective MOP receptor agonists, such as addiction, and balance the side effects such as irritability and aversion produced by single-selective KOP receptor agonists through the euphoriant effect produced by agonizing MOP receptor, making it possible to achieve good analgesic effect with fewer side effects; and the compounds represented by EX12B and EX48B show high selectivity and high affinity for MOP receptor and NOP receptor, and such MOPr/NOPr bifunctional agonists can enhance the analgesic effect caused by activating MOP receptor and inhibit dopamine release to reduce addiction, by agonizing NOP receptor, making it possible to develop novel analgesic drugs without addiction.

Test of agonistic function of compounds for MOP receptor, NOP receptor (ORL-1 receptor), KOP receptor, and DOP receptor (cAMP assay):

Opioid receptor is a G protein-coupled receptor, which is mainly coupled with $G_i$ protein. When it is bound to a ligand to be activated, it can inhibit adenylate cyclase activity via Gi protein, thereby reducing intracellular cAMP level.

A cAMP kit (LANCE Ultra cAMP kit, PE) was used to test the agonistic or inhibitory effects of the compounds on four opioid receptors (MOPr, KOPr, DOPr, and NOPr (ORL-1)). cAMP assay is a competitive immunoassay to detect intracellular cAMP accumulation, and the measured signal value is negatively correlated with the concentration of cAMP.

A standard was prepared according to the instructions of the kit. A 384-well cell culture plate was used, and each well was added with 5 μL standard, 5 μL experimental buffer, 5 μL 4×Eu-cAMP tracer working solution, and 5 μL 4×ULight-anti-cAMP working solution. The plate was incubated at room temperature for 1 h. Readings were taken with microplate reader. The excitation wavelength was 330 nm, and the emission wavelengths were 620 nm and 665 nm. The signal ratio of 665 nm to 620 nm was calculated, and a standard curve was made by the ratio and cAMP concentration.

Cell lines (CHO-hMOPr, CHO-hNOPr, HEK293-hKOPr, HEK293-hDOPr) of opioid receptors expressed in a stable transfected manner, cells were digested, centrifuged, re-suspended in an experimental buffer containing 0.5 mM IBMX, counted, and inoculated into a 384-well cell culture plate at a density of 5 μl, about 3000 cells, per well. The compound was diluted with experimental buffer to prepare 4× solution, and 4× compound was added at 2.5 μl per well, and incubated at 37°C for 10 min. 2.5 μl 4×Forskolin was further added, followed by incubation at 37°C for 30 min. Then 5 μl 4×Eu-cAMP tracer working solution and 5 μl 4×ULight-anti-cAMP working solution were added sequentially, and incubated at room temperature for 1 h. Readings were taken with microplate reader. The excitation wavelength was 330 nm, and the emission wavelengths were 620 nm and 665 nm. The signal ratio of 665 nm to 620 nm, i.e., Ratio (665/620), was calculated.

Endomorphin 1 was selected for MOPr positive compound, Nociceptin for NOPr (ORL-1) positive compound, Dynorphin A 1-10 for KOPr positive compound, and DADLE for DOPr positive compound. The test compound was detected starting from the concentration of 10 μM, in 3-fold dilution, with 10 concentrations, and in a manner of double double-well detection.

The graph was made by Ratio (665/620) and the concentration of compounds, and curve fitting and $EC_{50}$ calculation were performed using the GraphPad Prism 8.0 (GraphPad Software, San Diego, California USA) software nonlinear regression method.

$$Y=Bottom + (Top-Bottom)/(1+10^{\wedge}((LogEC50-X)*HillSlope))$$

*X*: logarithm of compound concentration;
Y: %activity.

According to the above method, the compounds of the present application were tested, and "NA" means no test

data, as listed in the table below.

| Example | *h*MOP receptor | | *h*NOP receptor (ORL-1 receptor) | | *h*KOP receptor | | *h*DOP receptor | |
|---|---|---|---|---|---|---|---|---|
| | $EC_{50}$ (nM) | $E_{max}$(%) | $EC_{50}$ (nM) | $E_{max}$(%) | $EC_{50}$ (nM) | $E_{max}$(%) | $EC_{50}$ (nM) | $E_{max}$(%) |
| Endomorphin 1 | 10.0 | 98.23 | NA | NA | NA | NA | NA | NA |
| DMGO | 11.0 | 95.0 | NA | NA | NA | NA | NA | NA |
| Nociceptin | NA | NA | 0.33 | 101.40 | NA | NA | NA | NA |
| Dynorphin A1-10 | NA | NA | NA | NA | 2.20 | 95.27 | NA | NA |
| DADLE | NA | NA | NA | NA | NA | NA | 0.16 | 108.40 |
| EX3 | 8.8 | 94.5 | 358.8 | 76.7 | 37.0 | 92.1 | 566.5 | 117.0 |
| EX6 | 22.4 | 85.4 | 684.0 | 91.0 | 157.5 | 97.2 | 1569.0 | 124.1 |
| EX16 | 51.4 | 105.2 | 4025.0 | 92.75 | 823.0 | 91.0 | 2624.0 | 138.6 |
| EX17 | 94.2 | 114.7 | 1092.0 | 71.9 | 190.4 | 108.1 | 2239.0 | 125.2 |
| EX18 | 24.6 | 91.7 | 615.0 | 86.4 | 67.1 | 110.8 | 1380.0 | 118.8 |
| EX19 | 59.0 | 91.6 | 593.0 | 80.2 | 243.7 | 110.5 | 2077.0 | 119.4 |
| EX38B | 530.0 | 76.14 | NA | NA | NA | NA | NA | NA |
| EX39 | 3.8 | 104.00 | 279.1 | 73.26 | 79.08 | 74.93 | NA | NA |
| EX40 | 73.2 | 89.1 | 451.0 | 73.2 | 21.4 | 95.8 | 2736.0 | 114.6 |
| EX44 | 86.2 | 97.06 | 998.5 | 81.78 | 353.0 | 94.00 | NA | NA |
| EX46 | 35.5 | 103.10 | 493.8 | 86.99 | 267.6 | 100.20 | NA | NA |
| EX51 | 51.5 | 99.17 | 207.0 | 90.10 | 269.3 | 70.67 | NA | NA |
| EX52 | 43.7 | 98.87 | 261.9 | 77.59 | 85.5 | 91.40 | NA | NA |
| EX57 | 20.2 | 101.70 | 375.7 | 67.22 | 11.76 | 100.40 | NA | NA |
| EX58 | 47.2 | 101.60 | 612.9 | 65.25 | 28.94 | 121.20 | NA | NA |
| EX59 | 60.6 | 101.50 | 420.6 | 74.28 | 1.04 | 102.20 | NA | NA |
| EX12 | 326.1 | 80.7 | 436.0 | 33.6 | 3482.0 | 108.4 | 3978.0 | 71.8 |
| EX12B | 560.1 | 66.77 | 400 | 52.88 | 633 | 80.58 | 16040 | 115.1 |
| EX34B | 1164 | 55.85 | 688.1 | 54.76 | / | / | / | / |
| EX36B | 1024 | 62.12 | 616.7 | 82.83 | / | / | / | / |
| EX38B | 530.0 | 76.14 | 282.9 | 79.98 | / | / | / | / |
| EX48B | 529.7 | 84.75 | 213.40 | 89.29 | / | / | / | / |

The results of the testing of cAMP agonistic function of preferred compounds showed that the compounds that were bi-selective for MOP receptor and KOP receptor also showed good agonistic bioactivity for MOP receptor and KOP receptor, and were bifunctional agonists for MOP receptor/KOP receptor. Similarly, the compounds that were bi-selective for MOP receptor and NOP receptor also have good agonistic bioactivity for the two opioid receptors.

Pharmacodynamic test in animals-formalin-induced inflammatory pain models

The animal models of formalin-induced inflammatory pain were established by Dubuisson and Dennis in 1977. It has been experimentally verified over time that injecting formalin diluted by a certain multiple subcutaneously into the hindfoot paw of rats using a microsyringe can create a sustained injurious stimulus that results in a spontaneous behavioral response of pain in the animals.

Experimental animals: SPF grade male SD rats, 6-7 weeks old, body weight ranged from 160 g to 180 g; using as

few animals as possible on the premise of meeting research purposes and scientific standards.

Experimental method: 1. before the start of the experiment, all animals were reared adaptively in the animal feeding room for one week to adapt to the environment. The room temperature was kept at 22±2°C, the room was illuminated with light, and strong sound and light stimulation were avoided. The rats were randomly grouped according to the needs of the experiment, 6-8 rats in each group, and numbered. All the test experiments were conducted between 8 am and 12 am. In order to adapt the rats to the formalin test environment, separate groups of rats were placed in experimental test boxes (transparent and visible) for 60 min per day for 3 days.

2. Reagent preparation. The analytically pure formaldehyde aqueous solution was prepared into a 2% formalin solution in a sealed vial for later use (after use, it was placed in 4°C refrigerator).

3. Preparation of test compound solution. The solvent system was 5% DMSO+10% Solutol+85% saline, and the concentration actually needed was prepared in situ according to the test experimental requirements.

4. Administration mode and modeling. The administration mode in this experiment was subcutaneous injection. Animals were reared in the experimental environment until day 6 when the experiment was carried out, and the body weight of the rats, room temperature, humidity, and routine control parameters were recorded. The rats were administered with drugs according to their body weight. 15 min after administration, 50 μl of 2% formalin solution was subcutaneously injected into the dorsum of the foot of rats for modeling.

5. Evaluation of the models. After formalin injection, the rats were placed in a test box, and the number of movements (the number of foot lifts and licks of the injected foot) of the rats within 60 min after formalin injection, including the number of foot lifts in the early acute phase (Phase I) and the late stressful phase (Phase II) (Phase I (0-9 min), Phase II (10-60 min)), and the average cumulative number of foot lifts, were counted by computer.

MPE% (Maximum Possible Effect, in percentage) was calculated.

%MPE = (1-the number of movements in the administration group/the number of movements in the negative control group) × 100%

Data statistics and analysis:

The measurement data was expressed by mean±standard deviation, all data statistics were carried out by SPSS13.0 statistical software, all data were subjected to homogeneity test for variance, data with homogeneity of variance ($p>0.05$) were subjected to one-way analysis of variance, and data with differences ($p\leq0.05$) were subjected to LSD multiple comparative analysis, with $p\leq0.05$ meaning having statistically significant differences; data with heterogeneity of variance ($p\leq0.05$) were subjected to Kruskal-Wallis non-parametric test, data differences ($p\leq0.05$) were subjected to Mann-Whitney pairwise analysis and comparison, with $p\leq0.05$ meaning having statistically significant differences. Graph was made using GraphPad Prism 8.0 (GraphPad Software, San Diego, California USA) software.

As can be seen from the time-effect curve (FIG. 1A), the time-effect curve of EX3, at a dose of 10 mg/kg, within the 0-40 min time interval substantially coincides with the effect curve of the morphine group (mor), and then the time-effect curve gradually approaches that of the model group (model), which suggests that compound EX3 (10 mg/kg) has good analgesic effect within the 0-40 min time interval, comparable to the analgesic effect of morphine (3 mg/kg). As can be seen from the maximum possible analgesic effect curve (FIG. 1B) of statistical analysis, compared with the control group (sham) (100±3.3%), the model group is significantly lower than its mean value, the maximum possible effect is 0±18.33%, and the maximum possible analgesic effect value of the 10 mg/kg dose group of compound EX3 is 94.04±5.81%, significantly higher than that of the model control group ($p<0.05$). The above experimental results show that compound EX3 has good inhibitory effect on formalin-induced inflammatory pain.

## Pharmacodynamic test in animals-spared nerve injury models

Spared Nerve Injury (SNI) animal models are developed to study the pharmacological mechanism of neuropathic pain. The model is established by artificial surgery, which can produce sustained and regenerative pain hypersensitivity on the surgical side to simulate typical symptoms of clinical neuropathic pain disorders. Creation of SNI models: on Day 0 of the experiment, the rat was anesthetized with an anesthesia machine fed with isoflurane, and fixed prone on the operating table, with the lateral gluteal region fully exposed, the left hind limb was shaved and sterilized with alcohol wipe, the skin was cut using surgical scissors in the femur in a direction parallel to the sciatic nerve, the gluteal muscles and biceps femoris muscle were bluntly separated to expose the sciatic nerve trunk, and the surrounding adhesion tissues and fascia were carefully separated. This proceeded until three branches were exposed, i.e., tibial nerve, common peroneal nerve, and sural nerve. For the rats in the surgery group, the tibial nerve and common peroneal nerve were ligated with silk thread, the tibial nerve and common peroneal nerve were cut off respectively, the sural nerve was ensured to be intact, and the muscles and epidermis were sutured layer by layer. After surgery, 60,000 units of penicillin were injected subcutaneously into each rat. The rats were reared in cages, and the administration experiment was carried out on Day 10 after operation.

Main test indexes and method: body weight, all animals were weighed once a week and the weights were recorded in detail; measurement of mechanical pain threshold, the animals were placed in a customized multi-unit metal mesh cage for pain testing, the animals were allowed to acclimatize for 10 min, after the animals' grooming and exploratory activities were completed and they acclimatized to the testing environment, electronic Von Frey Hairs (produced by Bioseb, France) were used to stimulate the paw on the pinkie side of the hind limb on the SNI surgical side of the rats, pressure was continuously increased until the rats showed an obvious foot contraction response, the value of Von Frey Hairs at this time was recorded, which was the threshold of mechanical pain response, taking "grams" as the test unit, the test was repeated twice, and then the mean value was taken as the final test index; foot weight-bearing measurement, the weights borne by the two hind limb feet of the rats in the SNI model group were unbalanced, a foot support force measuring instrument (Dynamic Weight Bearing Test, BIO-DWB-DUAL, BIOSEB) was used to measure the weight bearing difference (left foot-right foot) between the two hind limb feet of the animals, measurement was repeated twice, and the mean value was taken as the balance value of the weight bearing difference between the two hind limb feet of the animals. The time points for the mechanical pain threshold and foot weight-bearing testing were before modeling, before administration, and 1, 2, and 4 hours after administration, a total of 5 test points. In the experiment of the present application, rats were randomly divided into 5 groups (8 rats per group) according to the body weight of the animals, and the mechanical pain threshold and base value of foot weight bearing before administration, which were solvent control group (5% DMSO+10% Solutol HS15+85% normal saline), positive control group (Gabapentin, 100 mg/kg, the dose was calculated through conversion based on clinical dose of Gabapentin), and test compound high, medium, and low dose groups (10, 3, and 1 mg/kg). The positive control group was administered orally, which was the same as the administration in clinical practice, the other experimental groups were all administered by subcutaneous injection into the abdomen, and all experimental groups were administered in a single dose. The experimental results were reported by mechanical pain threshold Von Frey Hairs and foot weight bearing difference, and the corresponding analgesic rates were calculated. Relative analgesic rate (%) of Von Frey Hairs = (test value - mean value of control group)/(SNI preoperative base value - mean value of control group) $\times$ 100; and relative analgesic rate (%) of foot weight bearing = (mean value of control group - test value)/(mean value of control group - SNI preoperative base value) $\times$ 100. Data were analyzed using GraphPad Prism 8.0 (GraphPad Software, San Diego, California USA), and graph was made.

As could be seen from the experimental results (A and C in FIG. 2), the results of Von Frey Hairs test 1 h after administration showed that all three doses of compound EX3 could improve the mechanical pain threshold of the animals to a certain extent, especially the medium dose and the high dose, which were significantly different from the model group ($p<0.01$), the measured mechanical pain thresholds of the rats in the medium dose group and the high dose group were 7.0 $\pm$0.1 g and 9.5$\pm$0.2 g, respectively, the mean values of relative analgesic rates of Von Frey Hairs were 12.93% and 49.02%, respectively, and the analgesic effect of the high dose group was better than that of the positive control group (Gabapentin, 100 mg/kg, the relative analgesic mean value of Von Frey Hairs was 29.71%). As time progressed, the mechanical pain thresholds of the rats in the test drug groups all decreased in the second hour, but all of them were highly significantly different from the model group ($p<0.01$), the mechanical pain threshold of the rats in the compound EX3 high dose (10 mg/kg) group was 8.3$\pm$0.1 g, and the mean value of relative analgesic rate of Von Frey Hairs was 33.99%, which was close to that of the positive control group (the mean value of relative analgesic rate of Von Frey Hairs in the positive control group was 36.26%).

The weights borne by the two hind feet of SNI model rats were unbalanced. In order to further verify the analgesic effect of compound EX3 for SNI neuropathic pain in rats, the weight bearing difference (left foot-right foot) of two hind feet of the animals was measured in this experiment, and the mean value was taken as the balance value of the weight bearing difference of two hind feet of the animals. The test time points were the same as those in Von Frey Hairs test. As could be seen from the experimental results (B and D in FIG. 2), the balance value of foot weight bearing difference in the animals in the model group was increased significantly after modeling, which was 59.87$\pm$1.60 g (3.88$\pm$0.56 g before operation). Under the action of drugs, the balance value of weight bearing difference between the hind feet of the rats was decreased significantly, which was most obvious at the time point of the first hour, there were highly significant differences between each experimental group and model group ($p<0.01$), in the compound EX3 high dose group (10 mg/kg), the balance value of weight bearing difference between the hind feet of the rats was 46.82$\pm$1.68 g, and the mean value of relative analgesic rate of foot weight bearing was 22.11%, which was similar to that of the positive control Gabapentin group (the mean value of relative analgesic rate of foot weight bearing in the rats in the positive control group was 20.85%). As time progressed, the analgesic effect of drugs gradually weakened, but was still significantly different from that in the model group ($p<0.01$), in the second hour, the relative analgesic rate of foot weight bearing in the compound EX3 medium and low dose groups was still maintained at about 10%, the balance value of the weight bearing difference between the hind feet of the rats in the high dose group (10 mg/kg) was 46.54$\pm$0.80 g, the mean value of relative analgesic rate of foot weight bearing was 20.03%, which was still maintained at a high level, slightly better than that in the positive control group (the mean value of relative analgesic rate of foot weight bearing was 17.55%), and in the fourth hour, the balance value of the weight bearing difference between the hind feet of the rats in the compound EX3 high dose group was still significantly different from that in the model group ($p<0.01$), and the mean value of relative analgesic rate of foot weight bearing was 15.38%, which was

slightly better than that in the positive control group (the mean value of relative analgesic rate of foot weight bearing was 14.07%).

From the evaluation of SNI neuropathic pain models, it could be seen that compound EX3 could effectively inhibit neuropathic pain in rats, and had good dose dependence, and the analgesic effect was consistent with the metabolic cycle of the drug, and the drug exerted its maximum efficacy in the first hour. Compared with the positive control Gabapentin (100 mg/kg), the high dose group (10 mg/kg) had certain analgesic advantages, had a longer duration of action, and still had good analgesic effect in the fourth hour. Compound EX3 exhibited superior analgesic effect to the positive control Gabapentin at a low dose.

The above results show that the compounds shown in the present application are bi-selective for MOP receptor and KOP receptor, and exert good analgesic effect through this bifunctional action on MOP receptor and KOP receptor, and the inhibition for neuropathic pain is especially far superior to that of the clinical drug Gabapentin. According to the reports in numerous existing literatures, this effect cannot be achieved by single selective MOP receptor agonists.

The compounds disclosed in the present application, preferably some of them, have been evaluated for pharmacodynamics in vivo using the above methods, and the results show that the compounds disclosed in the present application have good efficacy in treating inflammatory pain and neuropathic pain. By continuous observation of the phenomena during the experiment and analysis of data, the compounds disclosed in the present application have fewer side effects, such as constipation and itching, than the positive control.

Although the embodiments disclosed in the present application are as above, the contents described are only the embodiments adopted for the convenience of understanding the present application, and are not used to limit the present application. Any person skilled in the field to which the present application belongs may make any modifications and changes in the form and details of implementation without departing from the spirit and scope disclosed in the present application, but the scope of protection of the present application shall still be subject to the scope defined in the appended claims.

## Claims

**1.** A compound shown in Formula (I), a stereoisomer, a pharmaceutically acceptable salt, a solvate, or a deuterate thereof,

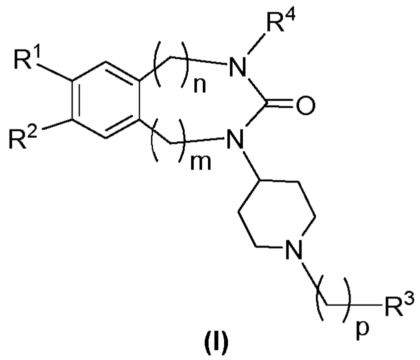

**(I)**

wherein

n is 0 or 1;
m is 0 or 1;
p is 0, 1, or 2;
$R^1$ and $R^2$ are each independently selected from: hydrogen, halogen, $C_{1-3}$ alkyl, and $C_{1-3}$ alkoxy; provided that $R^1$ and $R^2$ are not hydrogen at the same time;
$R^3$ is selected from unsubstituted cyclopropyl, cyclobutyl, cyclopentyl, cyclohexyl, cycloheptyl, or cyclooctyl; substituted cyclopropyl, cyclobutyl, cyclopentyl, cyclohexyl, cycloheptyl, or cyclooctyl substituted by 1-3 groups independently selected from the following: fluorine, chlorine, bromine, iodine, $C_{1-4}$ alkyl, halogenated $C_{1-4}$ alkyl, $C_{1-4}$ alkoxy, halogenated $C_{1-4}$ alkoxy, and phenyl; unsubstituted phenyl; or substituted phenyl substituted by 1-3 groups independently selected from the following: fluorine, chlorine, bromine, iodine, $C_{1-4}$ alkyl, halogenated $C_{1-4}$ alkyl, $C_{1-4}$ alkoxy, and halogenated $C_{1-4}$ alkoxy; or unsubstituted furyl, thienyl, pyridyl, pyrrolyl, N-alkylpyrrolyl, pyrimidinyl, pyrazinyl, pyridazinyl, imidazolyl, pyrazolyl, triazolyl, or tetrazolyl, or substituted furyl, thienyl, pyridyl, pyrrolyl, N-alkylpyrrolyl, pyrimidinyl, pyrazinyl, pyridazinyl, imidazolyl, pyrazolyl, triazolyl, or tetrazolyl substituted

by 1-2 groups independently selected from the following: halogen, $C_{1-4}$ alkyl, halogenated $C_{1-4}$ alkyl, $C_{1-4}$ alkoxy, halogenated $C_{1-4}$ alkoxy, aryl, and heteroaryl; or unsubstituted tetrahydrofuranyl, tetrahydropyrrolyl, tetrahydrothienyl, piperidinyl, morpholinyl, or piperazinyl; substituted tetrahydrofuranyl, tetrahydropyrrolyl, tetrahydrothienyl, piperidinyl, morpholinyl, or piperazinyl substituted by 1-3 groups independently selected from the following: fluorine, chlorine, bromine, iodine, $C_{1-4}$ alkyl, halogenated $C_{1-4}$ alkyl, $C_{1-4}$ alkoxy, halogenated $C_{1-4}$ alkoxy, and phenyl;

$R^4$ is selected from hydrogen, 2-(morpholinyl)ethyl, 2-(1,1-dioxothiomorpholine)ethyl, 2-(4-methylpiperazin-1-yl) ethyl, 2-(4-acetylpiperazin-1-yl)ethyl, 2-(3-oxopiperazin-1-yl)ethyl, 2-(pyrrolidin-1-yl)ethyl, 2-(piperidin-1-yl) ethyl, 2-(N,N'-dimethylamino)ethyl, 2-(2-oxopyrrolidin-1-yl)ethyl, N,N'-dimethylacetamide, oxiran-2-ylmethyl, or 2-(2-oxa-6-azaspiro[3.3]heptan-6-yl)ethyl; provided that when $R^4$ is hydrogen, m is 0, and n is 1.

**2.** The compound according to claim 1, or a stereoisomer, a pharmaceutically acceptable salt, a solvate, or a deuterate thereof, wherein

n is 0;
m is 0;
p is 0, 1, or 2.

**3.** The compound according to claim 1, or a stereoisomer, a pharmaceutically acceptable salt, a solvate, or a deuterate thereof, wherein

n is 1;
m is 0;
p is 0, 1, or 2.

**4.** The compound according to claim 1, or a stereoisomer, a pharmaceutically acceptable salt, a solvate, or a deuterate thereof, wherein

n is 0;
m is 1;
p is 0, 1, or 2.

**5.** The compound according to claim 1, or a stereoisomer, a pharmaceutically acceptable salt, a solvate, or a deuteratethereof, wherein $R^1$ and $R^2$ are each independently selected from: hydrogen, chlorine, fluorine, methyl; provided that $R^1$ and $R^2$ are not hydrogen at the same time; optionally, $R^1$ and $R^2$ are both chlorine, or $R^1$ is chlorine and $R^2$ is fluorine, or $R^1$ is fluorine and $R^2$ is chlorine, or $R^1$ and $R^2$ are both fluorine, or $R^1$ is fluorine, methyl, or chlorine and $R^2$ is hydrogen, or $R^1$ is hydrogen and $R^2$ is chlorine or fluorine.

**6.** The compound according to claim 1, or a stereoisomer, a pharmaceutically acceptable salt, a solvate, or a deuterate thereof, wherein $R^3$ is phenyl, 3,4-dichlorophenyl, 3,4-difluorophenyl, 2-chloro-4-fluorophenyl, 2-fluoro-4-chlorophenyl, 4-trifluoromethylphenyl, 4-trifluoromethoxyphenyl, 2,4-dichlorophenyl, 2,6-dichlorophenyl, 2,4-difluorophenyl, 2-chloro-4-methylphenyl, 2-methyl-4-fluorophenyl, 2-methyl-4-chlorophenyl, 2-methoxy-4-chlorophenyl, 2,4-dimethylphenyl, 2,6-dimethylphenyl, 2,4,6-trimethylphenyl, 4-tert-butylphenyl, 2-chlorophenyl, 2-methylphenyl, 2-fluorophenyl, 2-methoxyphenyl, 4-chlorophenyl, 4-fluorophenyl, or 4-methoxyphenyl.

**7.** The compound according to claim 1, or a stereoisomer, a pharmaceutically acceptable salt, a solvate, or a deuterate thereof, wherein $R^3$ is cyclopropyl, cyclopentyl, cyclohexyl, cycloheptyl, 4-tert-butylcyclohexyl, 4-isopropylcyclohexyl, 4-ethylcyclohexyl, 4-methylcyclohexyl, 4-trifluoromethylcyclohexyl, 2,3-dihydro-1H-inden-2-yl, or 2-chlorocyclohexyl.

**8.** The compound according to claim 1, or a stereoisomer, a pharmaceutically acceptable salt, a solvate, or a deuterate thereof, wherein $R^3$ is N-isopropylpiperidin-4-yl.

**9.** The compound according to claim 1, or a stereoisomer, a pharmaceutically acceptable salt, a solvate, or a deuterate thereof, selected from one of the following compounds:

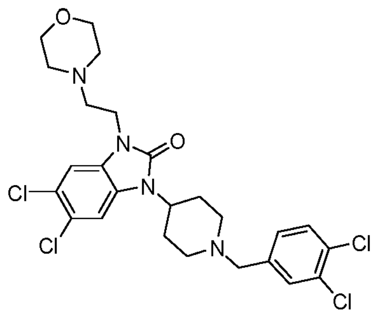

**EX1**

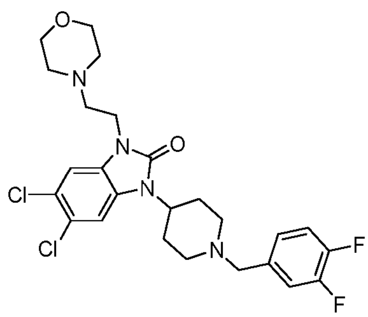

**EX2**

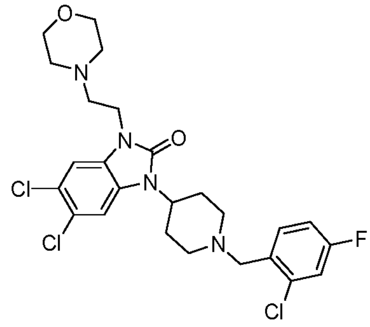

**EX3**

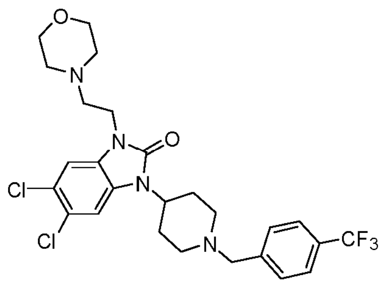

**EX4**

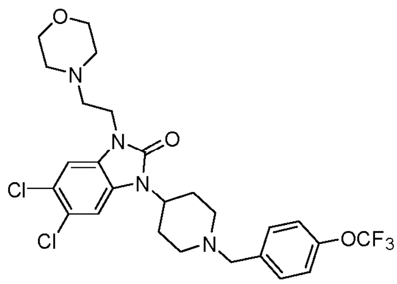

**EX5**

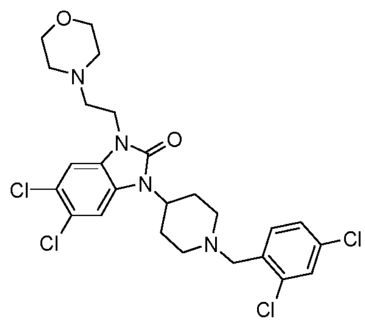

**EX6**

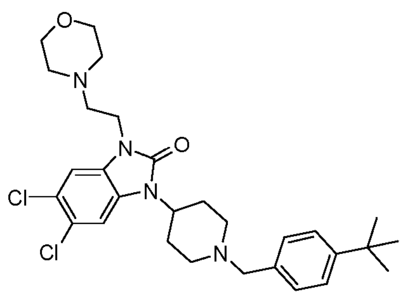

**EX7**

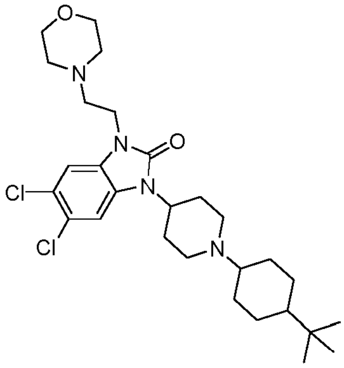

**EX8**

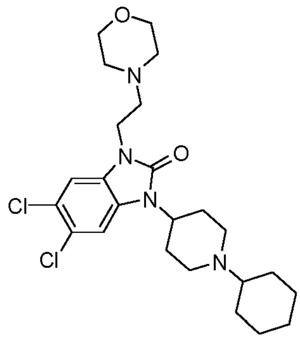

**EX9**

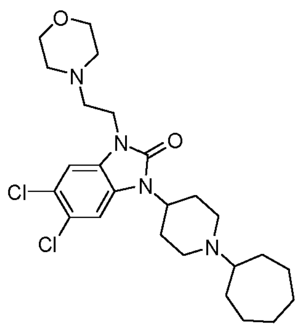

**EX10**

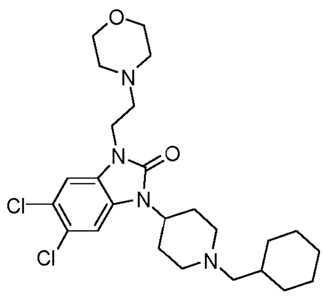

**EX11**

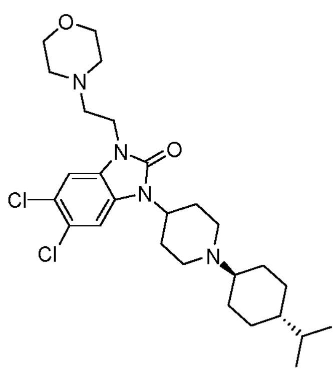

**EX12A**

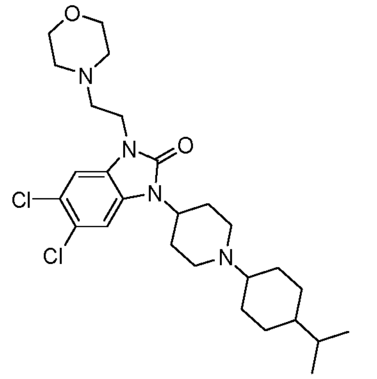

**EX12**

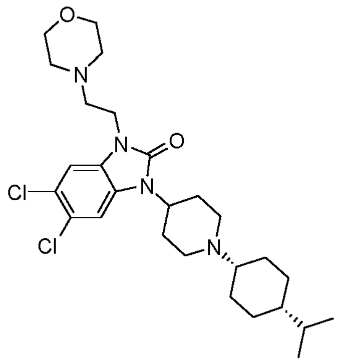

**EX12B**

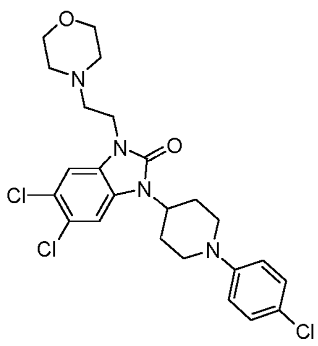

**EX13**

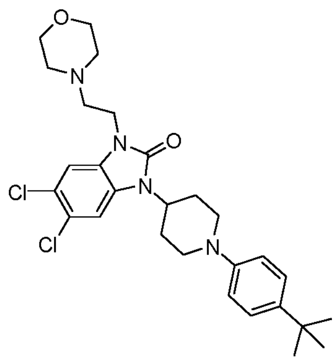

**EX14**

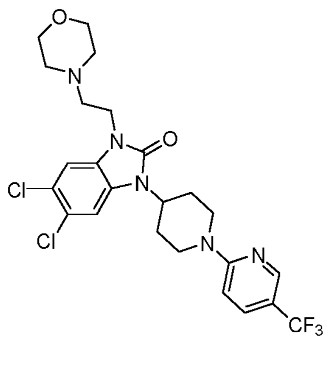

**EX15**

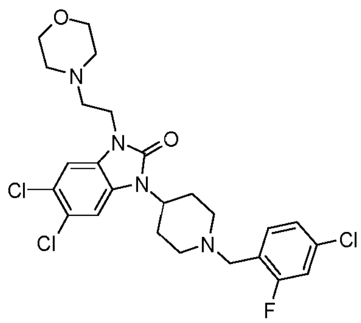

**EX16**

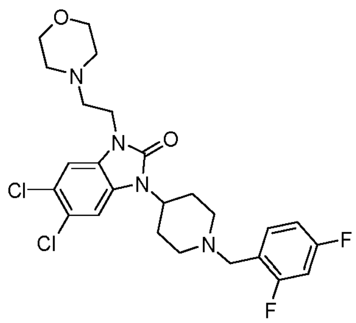

**EX17**

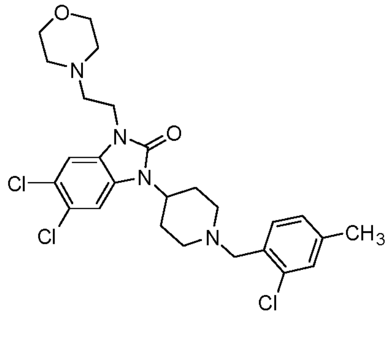

**EX18**

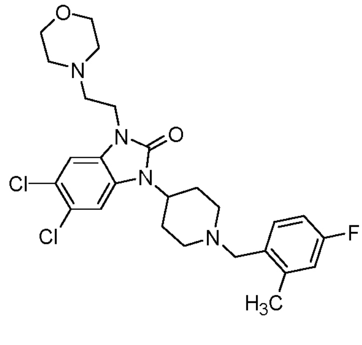

**EX19**

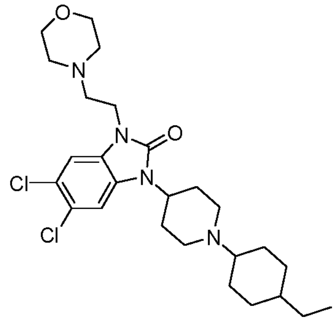

**EX20**

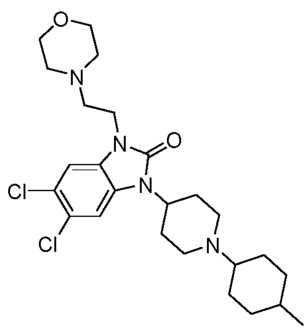

EX21

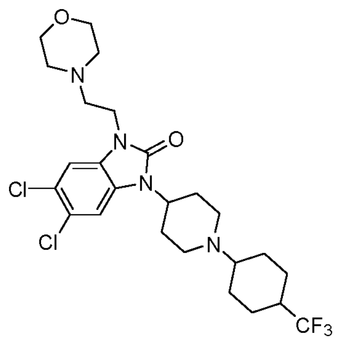

EX22

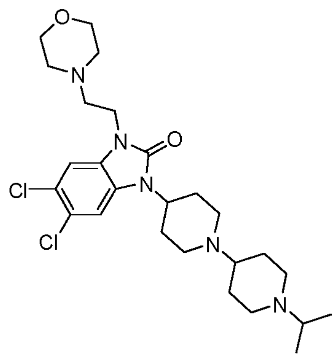

EX23

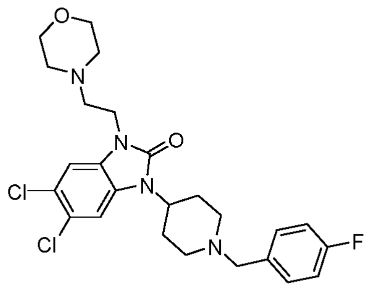

EX24

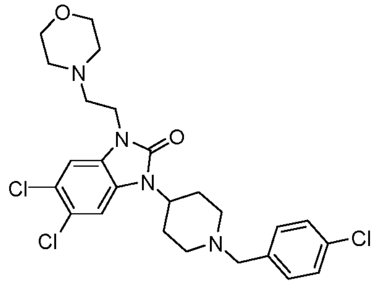

EX25

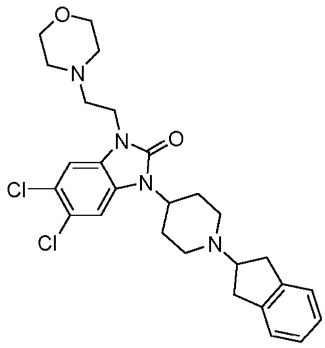

EX26

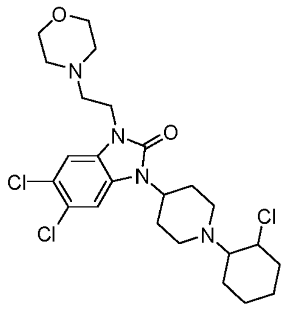

EX27

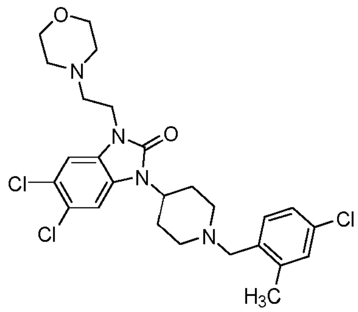

EX28

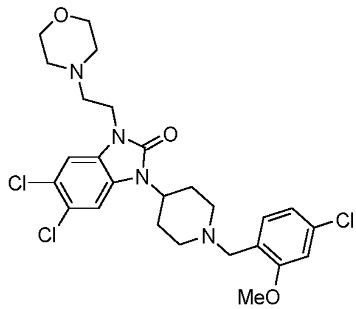

EX29

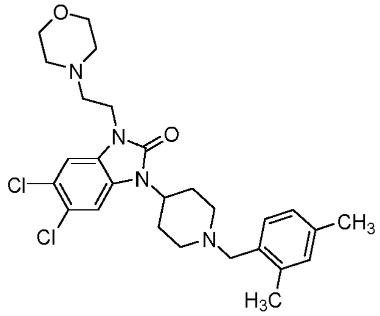

EX30

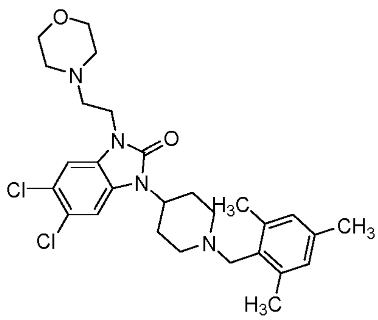

EX31

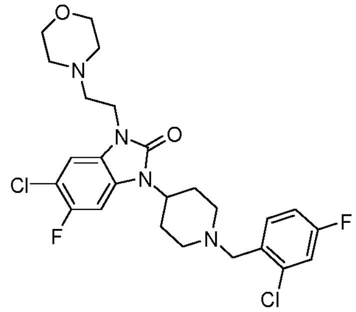

EX33

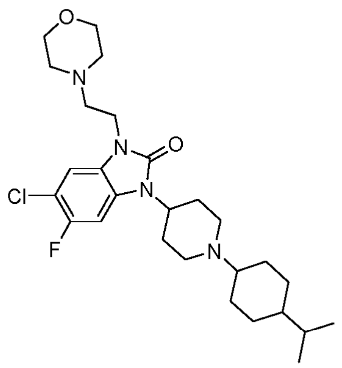

**EX34**

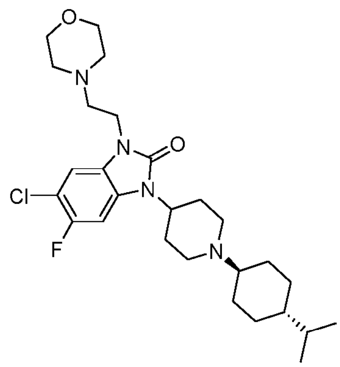

**EX34A**

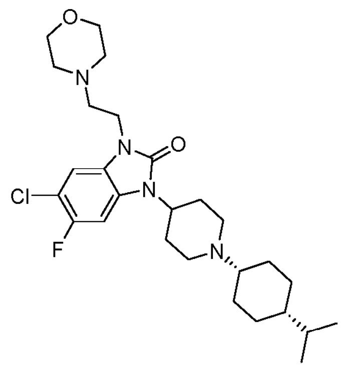

**EX34B**

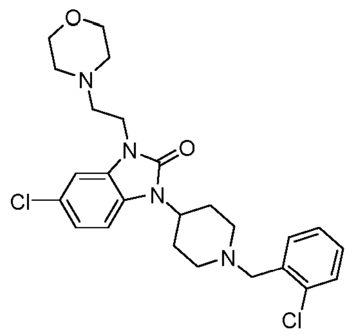

**EX32**

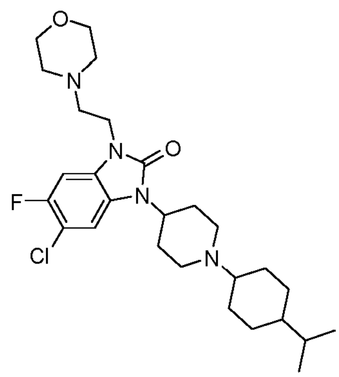

**EX36**

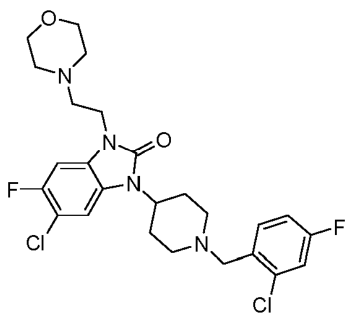

**EX35**

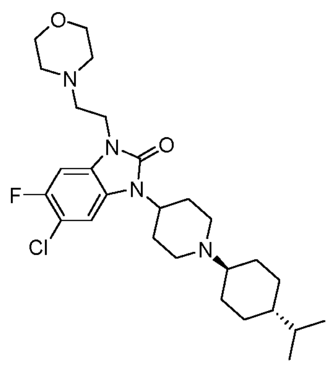

**EX36A**

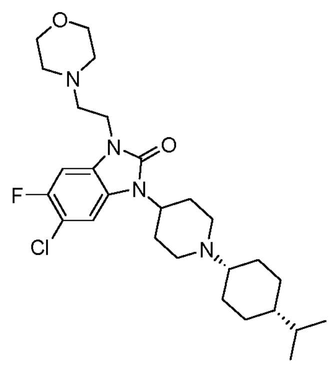

**EX36B**

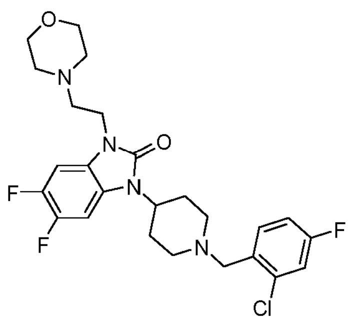

**EX37**

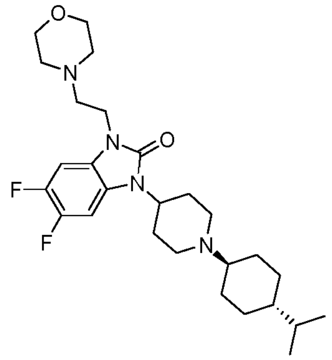

**EX38A**

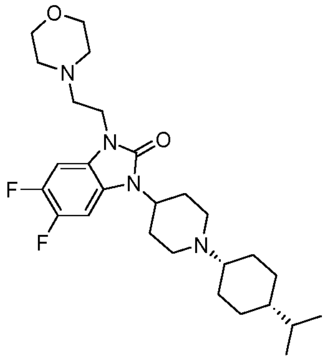

**EX38B**

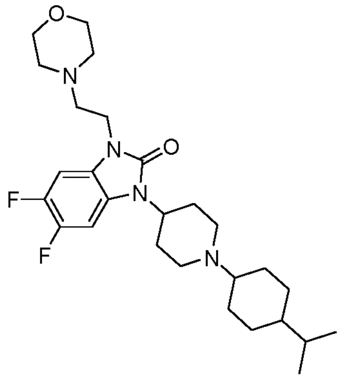

**EX38**

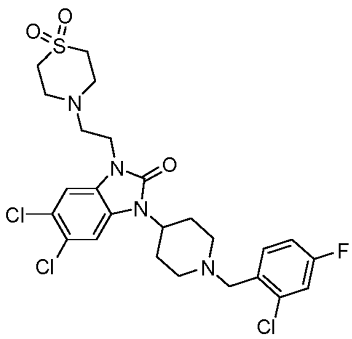

**EX39**

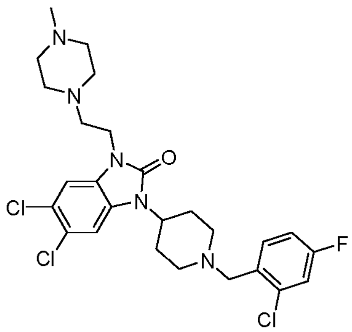

**EX40**

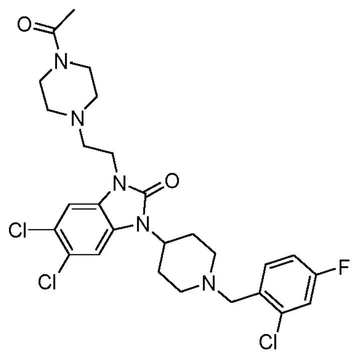

**EX41**

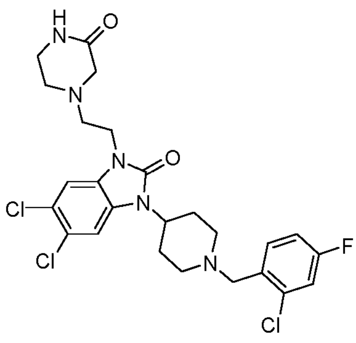

**EX42**

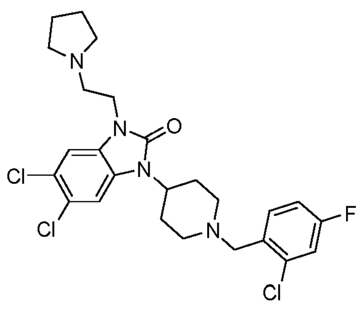

**EX43**

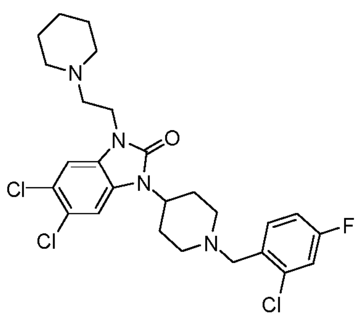

**EX44**

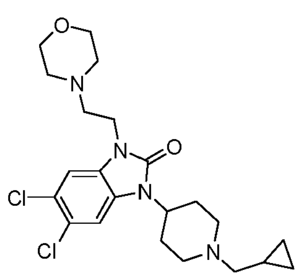

EX45

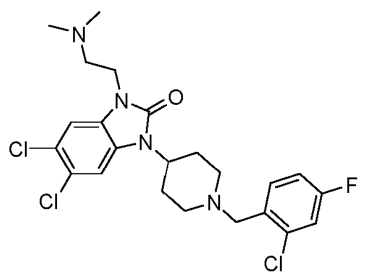

EX46

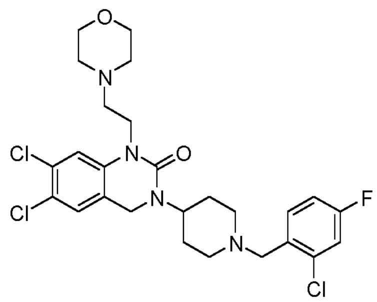

EX47

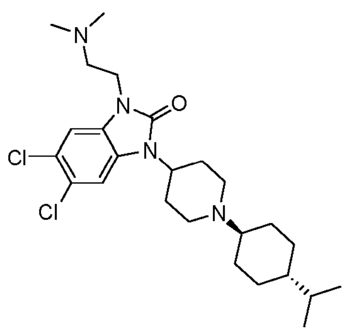

EX48A

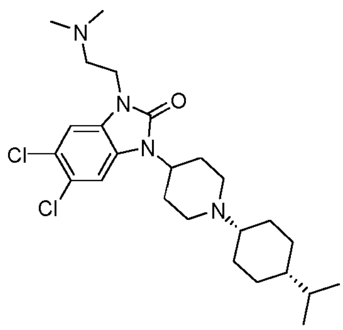

EX48B

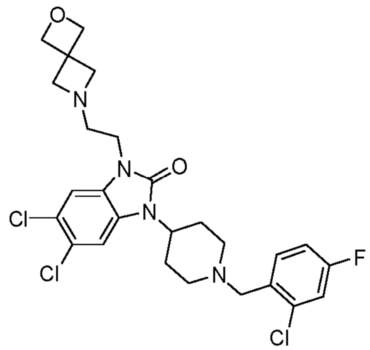

EX49

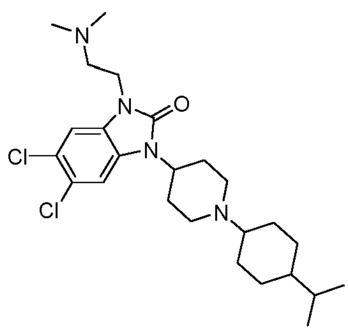

EX48

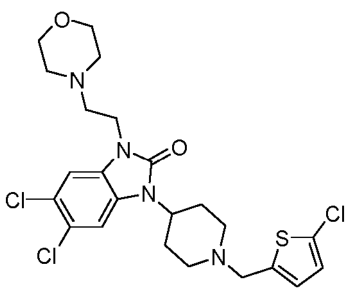

EX50

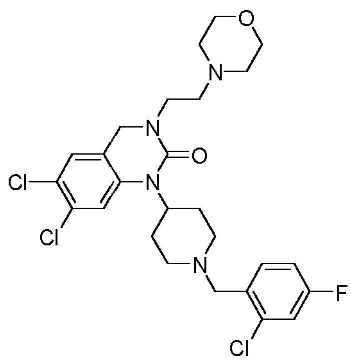

EX51

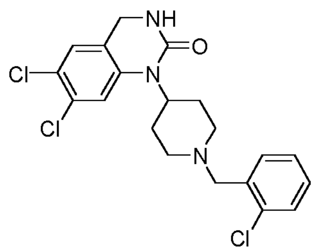

EX52

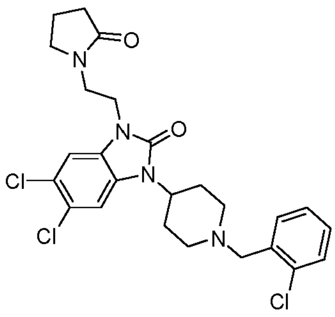

EX53

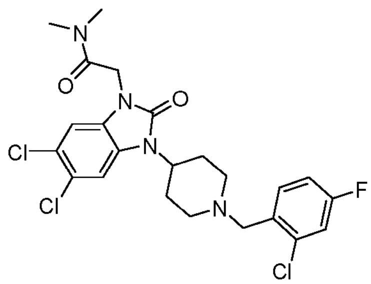

EX54

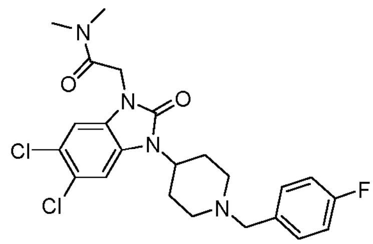

EX55

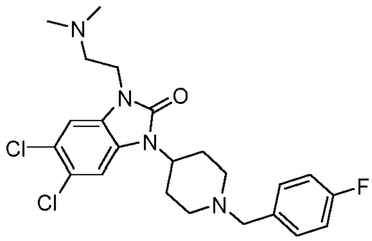

EX56

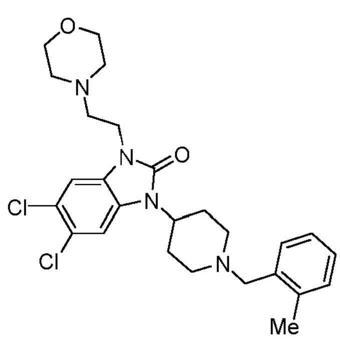

EX57

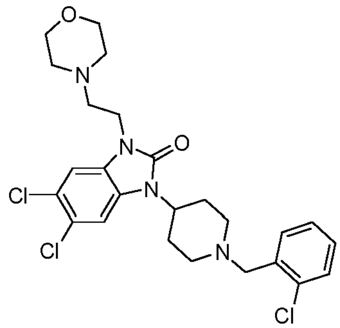

EX58

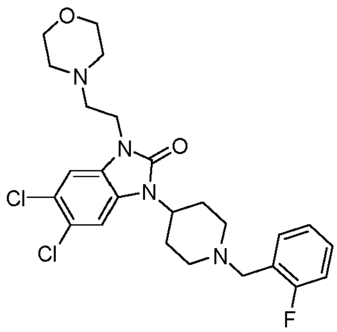

EX59

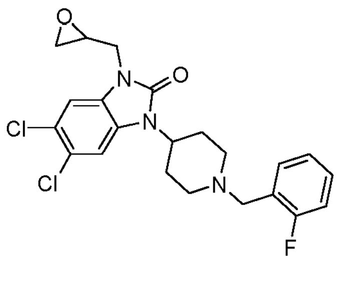

EX60

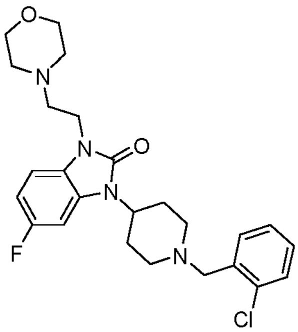

EX61

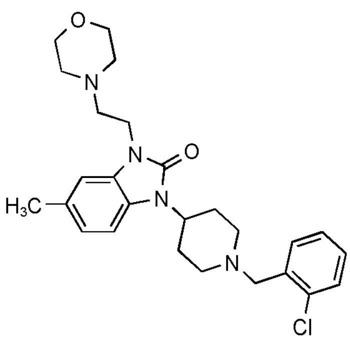

EX62

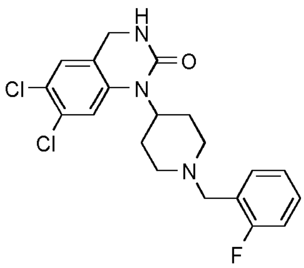

EX63

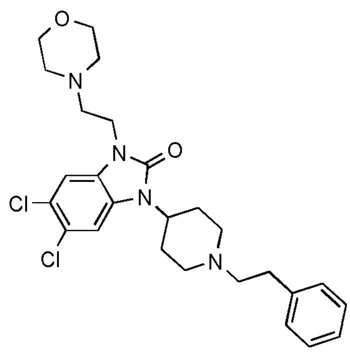

EX64

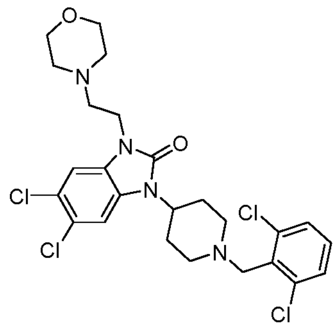

**EX65**

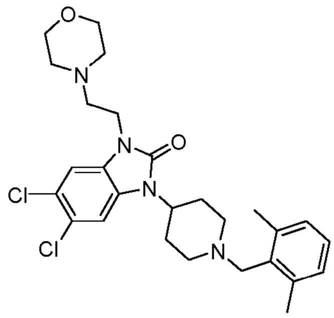

**EX66**

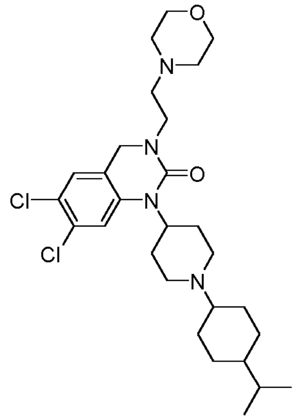

**EX67**

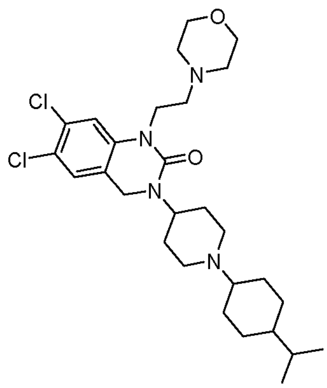

**EX68**

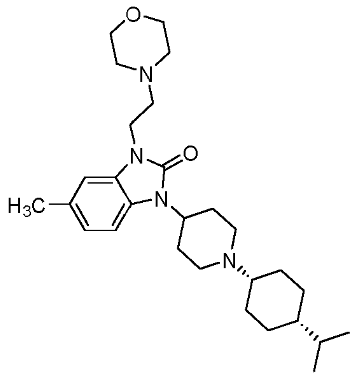

**EX69B**

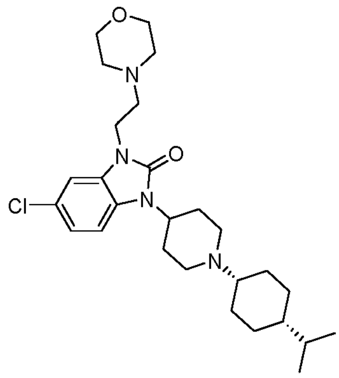

**EX70B**

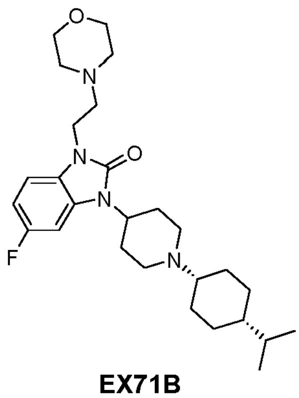

**EX71B**

**10.** A pharmaceutical composition, comprising the compound of any one of claims 1 to 9, or a stereoisomer, a pharmaceutically acceptable salt, a solvate, or a deuterate thereof, and a pharmaceutically acceptable carrier.

**11.** The compound of any one of claims 1 to 9, or a stereoisomer, a pharmaceutically acceptable salt, a solvate, or a deuterate thereof, for use in treating pain, anxiety, depression, alcohol addiction, substance abuse/dependence.

**Patentansprüche**

**1.** Verbindung, die in Formel (I) gezeigt ist, ein Stereoisomer, ein pharmazeutisch unbedenkliches Salz, ein Solvat oder ein Deuterat davon,

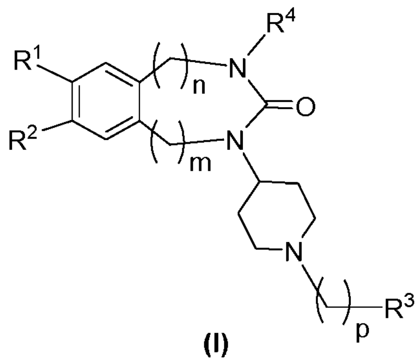

(I)

wobei

n 0 oder 1 ist;
m 0 oder 1 ist;
p 0, 1 oder 2 ist;
$R^1$ und $R^2$ jeweils unabhängig ausgewählt sind aus: Wasserstoff, Halogen, $C_{1-3}$-Alkyl und $C_{1-3}$-Alkoxy; vorausgesetzt, dass $R^1$ und $R^2$ nicht gleichzeitig Wasserstoff sind;
$R^3$ ausgewählt ist aus unsubstituiertem Cyclopropyl, Cyclobutyl, Cyclopentyl, Cyclohexyl, Cycloheptyl oder Cyclooctyl; substituiertem Cyclopropyl, Cyclobutyl, Cyclopentyl, Cyclohexyl, Cycloheptyl oder Cyclooctyl substituiert durch 1-3 Gruppen unabhängig ausgewählt aus den folgenden: Fluor, Chlor, Brom, Iod, $C_{1-4}$-Alkyl, halogeniertem $C_{1-4}$-Alkoxy, halogeniertem $C_{1-4}$-Alkoxy und Phenyl; unsubstituiertem Phenyl; oder substituiertem Phenyl substituiert durch 1-3 Gruppen unabhängig ausgewählt aus den folgenden: Fluor, Chlor, Brom, Iod, $C_{1-4}$-Alkyl, halogeniertem $C_{1-4}$-Alkyl, $C_{1-4}$-Alkoxy und halogeniertem $C_{1-4}$-Alkoxy; oder unsubstituiertem Furyl, Thienyl, Pyridyl, Pyrrolyl, N-Alkylpyrrolyl, Pyrimidinyl, Pyrazinyl, Pyridazinyl, Imidazolyl, Pyrazolyl, Triazolyl oder Tetrazolyl, oder substituiertem Furyl, Thienyl, Pyridyl, Pyrrolyl, N-Alkylpyrrolyl, Pyrimidinyl, Pyrazinyl, Pyridazinyl, Imidazolyl, Pyrazolyl, Triazolyl oder Tetrazolyl, substituiert mit 1-2 Gruppen, unabhängig ausgewählt aus den folgenden: Halogen, $C_{1-4}$-Alkyl, halogeniertem $C_{1-4}$-Alkyl, $C_{1-4}$-Alkoxy, halogeniertem $C_{1-4}$-Alkoxy, Aryl und Heteroaryl; oder unsubstituiertem Tetrahydrofuranyl, Tetrahydropyrrolyl, Tetrahydrothienyl, Piperidinyl, Morpholinyl, oder Piperazinyl; substituiertem Tetrahydrofuranyl, Tetrahydropyrrolyl, Tetrahydrothienyl, Piperidinyl, Morpholinyl oder Piperazinyl, substituiert durch 1-3 Gruppen, die unabhängig ausgewählt sind aus den folgenden: Fluor, Chlor, Brom, Iod, $C_{1-4}$-Alkyl, halogeniertem $C_{1-4}$-Alkyl, $C_{1-4}$-Alkoxy, halogeniertem $C_{1-4}$-Alkoxy und Phenyl;
$R^4$ ausgewählt ist aus Wasserstoff, 2-(Morpholinyl)ethyl, 2-(1,1-Dioxothiomorpholin)ethyl, 2-(4-Methylpiperazin-1-yl)ethyl, 2-(4-Acetylpiperazin-1-yl)ethyl, 2-(3-Oxopiperazin-1-yl)ethyl, 2-(Pyrrolidin-1-yl)ethyl, 2-(Piperidin-1-yl)ethyl, 2-(N,N'-Dimethylamino)ethyl, 2-(2-Oxopyrrolidin-1-yl)ethyl, N,N'-Dimethylacetamid, Oxiran-2-ylmethyl oder 2-(2-Oxa-6-azaspiro [3.3]heptan-6-yl)ethyl; vorausgesetzt, dass, wenn $R^4$ Wasserstoff ist, m 0 ist und n 1 ist.

**2.** Verbindung gemäß Anspruch 1 oder ein Stereoisomer, ein pharmazeutisch unbedenkliches Salz, ein Solvat oder ein Deuterat davon, wobei

n 0 ist;
m 0 ist;
p 0, 1 oder 2 ist.

**3.** Verbindung gemäß Anspruch 1 oder ein Stereoisomer, ein pharmazeutisch unbedenkliches Salz, ein Solvat oder ein Deuterat davon, wobei

n 1 ist;
m 0 ist;
p 0, 1 oder 2 ist.

**4.** Verbindung gemäß Anspruch 1 oder ein Stereoisomer, ein pharmazeutisch unbedenkliches Salz, ein Solvat oder ein Deuterat davon, wobei

n 0 ist;
m 1 ist;
p 0, 1 oder 2 ist.

5. Verbindung gemäß Anspruch 1 oder ein Stereoisomer, ein pharmazeutisch unbedenkliches Salz, ein Solvat oder ein Deuterat davon, wobei $R^1$ und $R^2$ jeweils unabhängig ausgewählt sind aus:
Wasserstoff, Chlor, Fluor, Methyl; vorausgesetzt, dass $R^1$ und $R^2$ nicht gleichzeitig Wasserstoff sind; optional $R^1$ und $R^2$ beide Chlor sind oder $R^1$ Chlor ist und $R^2$ Fluor ist oder $R^1$ Fluor ist und $R^2$ Chlor ist oder $R^1$ und $R^2$ beide Fluor sind oder $R^1$ Fluor, Methyl oder Chlor ist und $R^2$ Wasserstoff ist oder $R^1$ Wasserstoff ist und $R^2$ Chlor oder Fluor ist.

6. Verbindung gemäß Anspruch 1 oder ein Stereoisomer, ein pharmazeutisch unbedenkliches Salz, ein Solvat oder ein Deuterat davon, wobei $R^3$ Phenyl, 3,4-Dichlorphenyl, 3,4-Difluorphenyl, 2-Chlor-4-fluorphenyl, 2-Fluor-4-chlorphenyl, 4-Trifluormethylphenyl, 4-Trifluormethoxyphenyl, 2,4-Dichlorphenyl, 2,6-Dichlorphenyl, 2,4-Difluorphenyl, 2-Chlor-4-methylphenyl, 2-Methyl-4-fluorphenyl, 2-Methyl-4-chlorphenyl, 2-Methoxy-4-chlorphenyl, 2,4-Dimethylphenyl, 2,6-Dimethylphenyl, 2,4,6-Trimethylphenyl, 4-tert-Butylphenyl, 2-Chlorphenyl, 2-Methylphenyl, 2-Fluorphenyl, 2-Methoxyphenyl, 4-Chlorphenyl, 4-Fluorphenyl oder 4-Methoxyphenyl ist.

7. Verbindung gemäß Anspruch 1 oder ein Stereoisomer, ein pharmazeutisch unbedenkliches Salz, ein Solvat oder ein Deuterat davon, wobei $R^3$ Cyclopropyl, Cyclopentyl, Cyclohexyl, Cycloheptyl, 4-tert-Butylcyclohexyl, 4-Isopropylcyclohexyl, 4-Ethylcyclohexyl, 4-Methylcyclohexyl, 4-Trifluormethylcyclohexyl, 2,3-Dihydro-1H-inden-2-yl oder 2-Chlorcyclohexyl ist.

8. Verbindung gemäß Anspruch 1 oder ein Stereoisomer, ein pharmazeutisch unbedenkliches Salz, ein Solvat oder ein Deuterat davon, wobei $R^3$ N-Isopropylpiperidin-4-yl ist.

9. Verbindung gemäß Anspruch 1 oder ein Stereoisomer, ein pharmazeutisch unbedenkliches Salz, ein Solvat oder ein Deuterat davon, ausgewählt aus einer der folgenden Verbindungen:

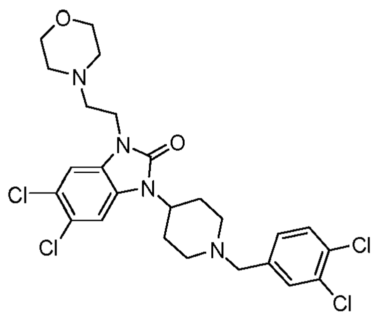
EX1

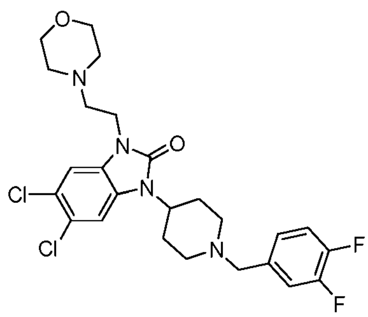
EX2

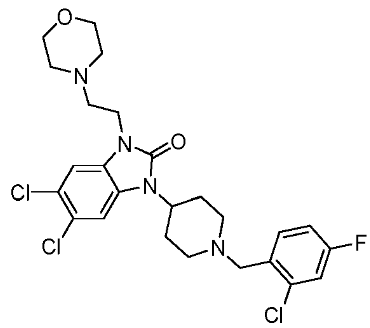
EX3

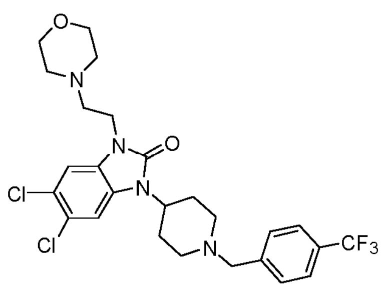
EX4

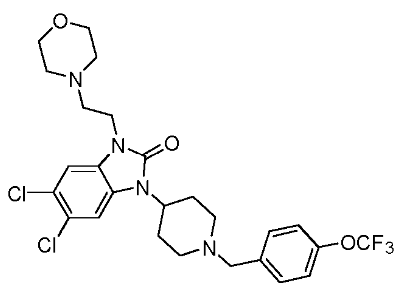
EX5

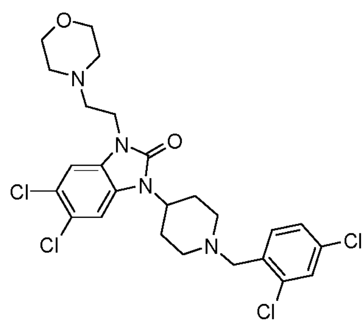
EX6

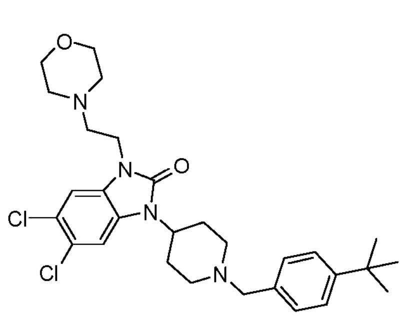

**EX7**

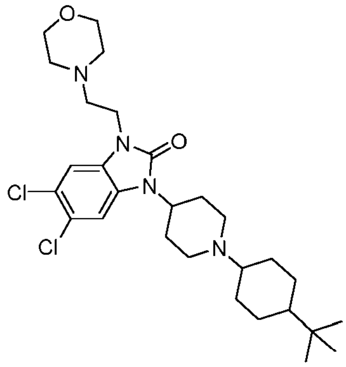

**EX8**

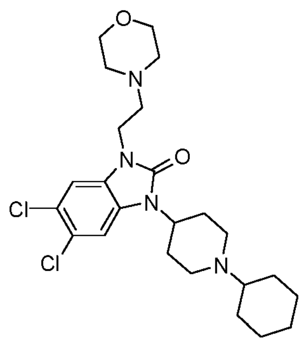

**EX9**

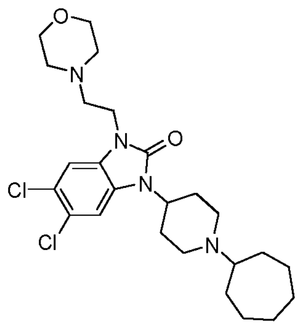

**EX10**

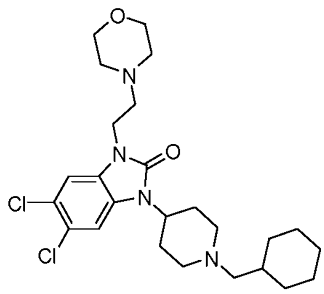

**EX11**

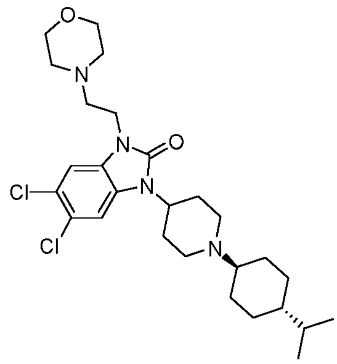

**EX12A**

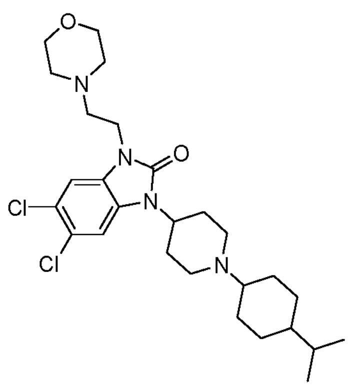

**EX12**

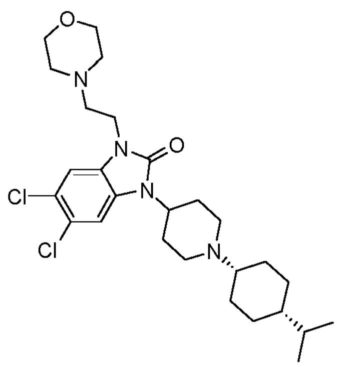

**EX12B**

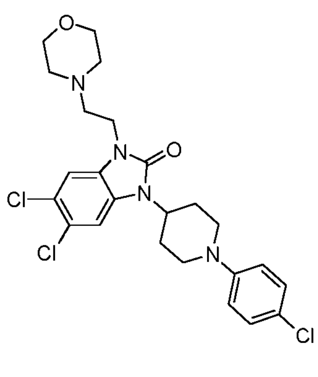

**EX13**

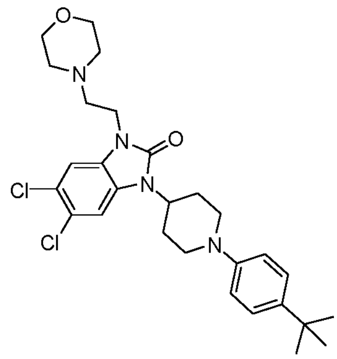

**EX14**

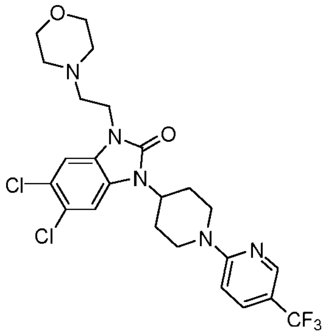

**EX15**

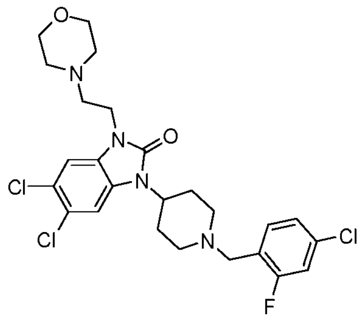

**EX16**

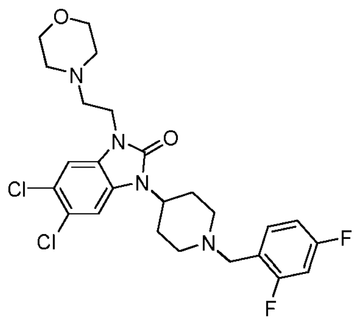

**EX17**

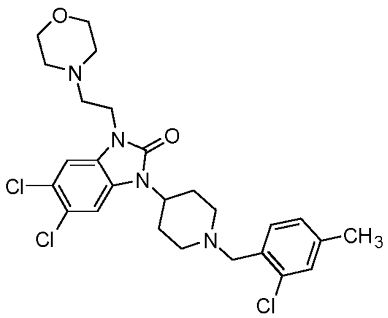

**EX18**

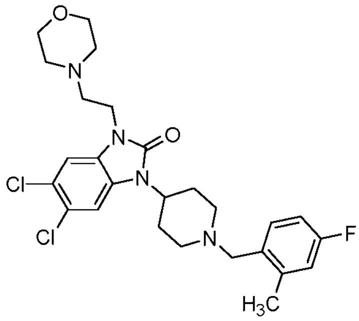

**EX19**

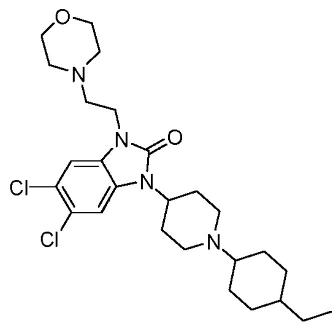

**EX20**

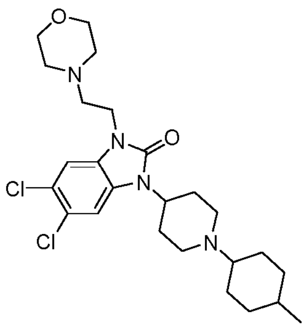

**EX21**

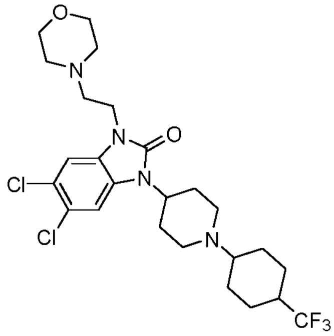

**EX22**

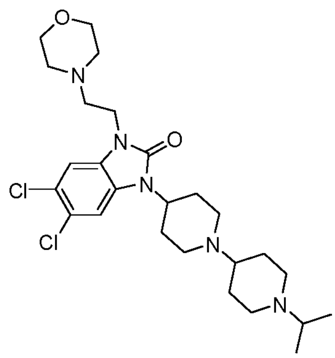

**EX23**

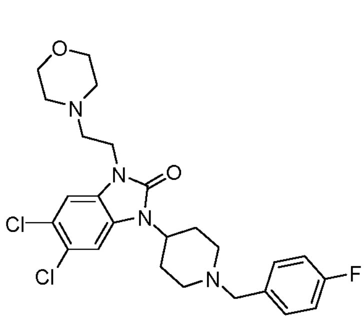

**EX24**

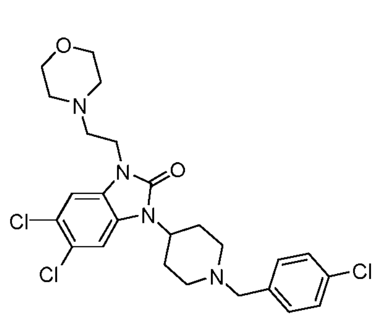

**EX25**

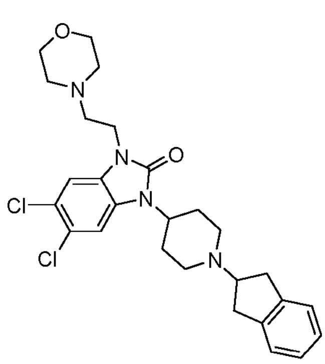

**EX26**

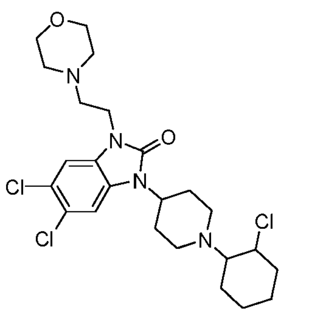

**EX27**

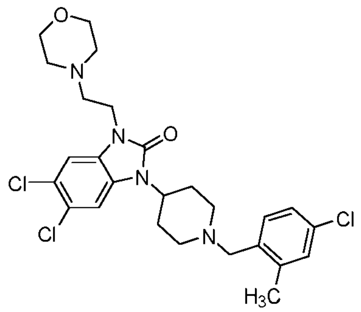

**EX28**

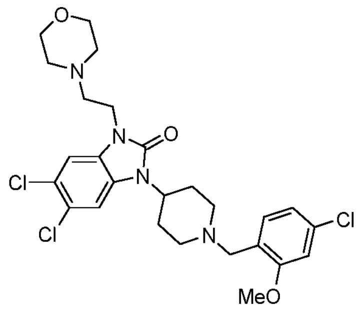

**EX29**

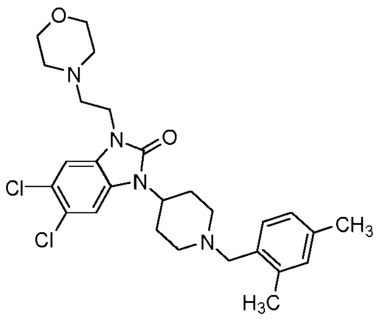

**EX30**

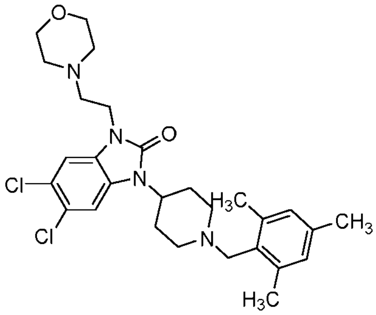

**EX31**

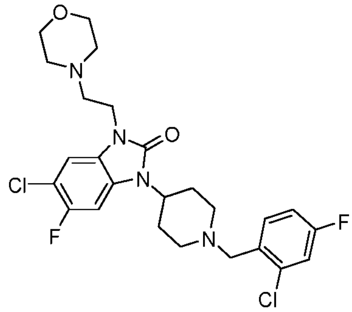

**EX33**

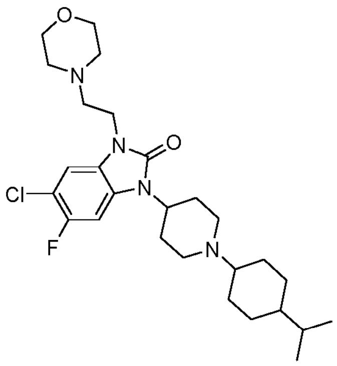

**EX34**

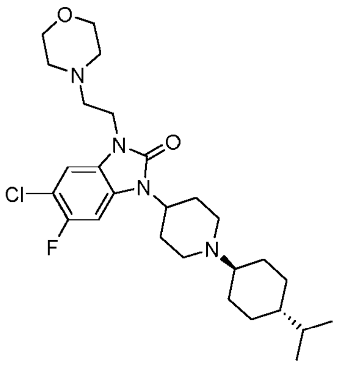

**EX34A**

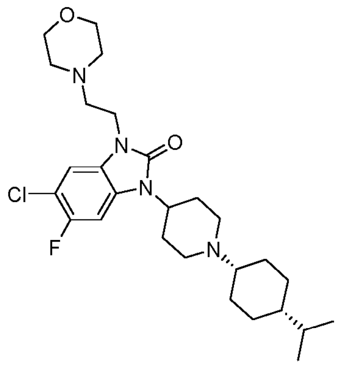

**EX34B**

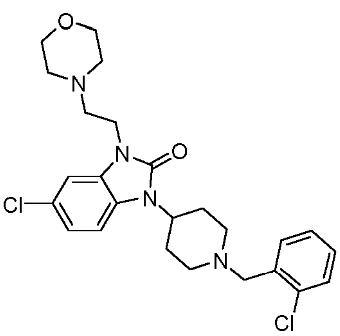

**EX32**

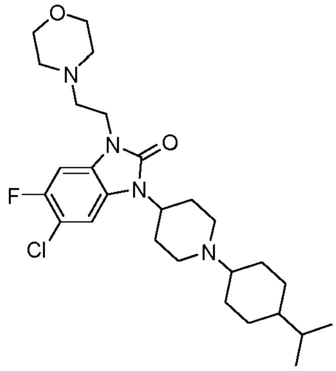

**EX36**

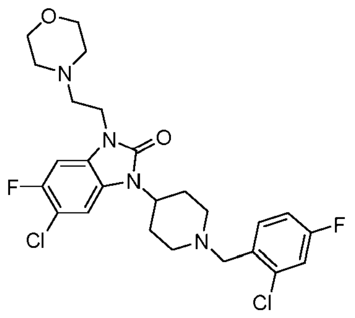

**EX35**

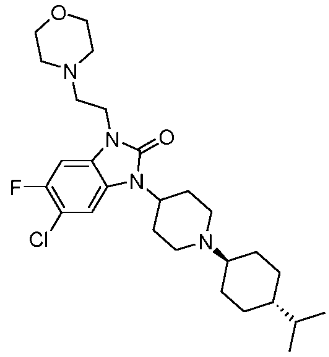

**EX36A**

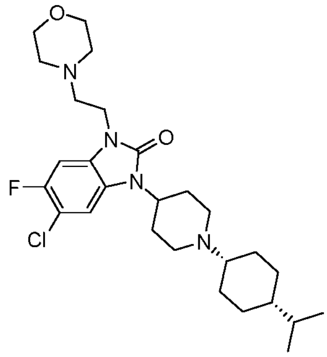

**EX36B**

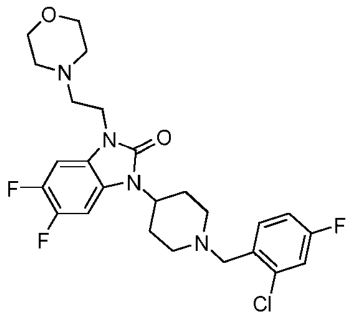

**EX37**

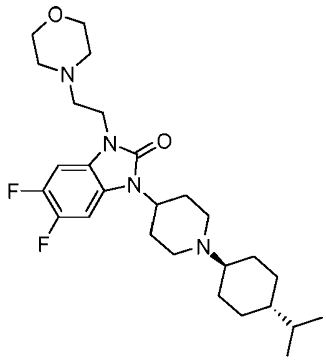

**EX38A**

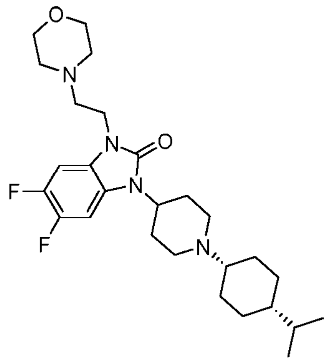

**EX38B**

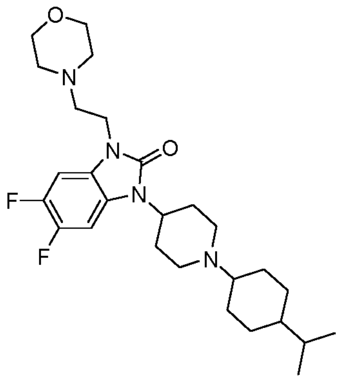

**EX38**

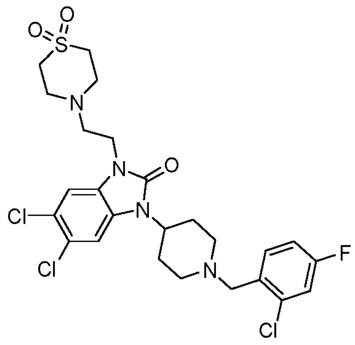

EX39

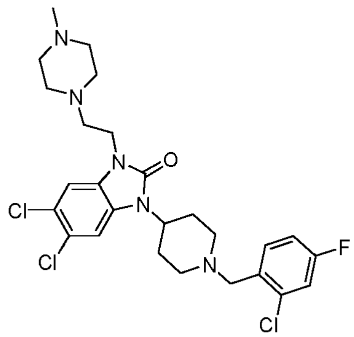

EX40

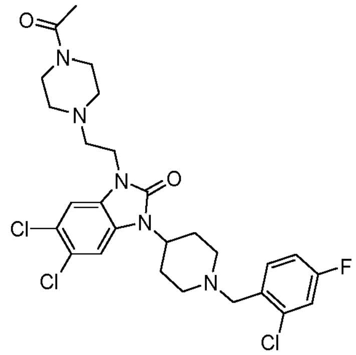

EX41

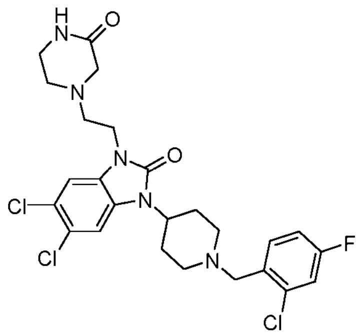

EX42

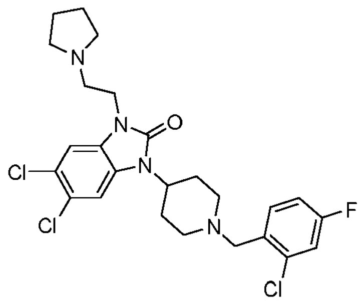

EX43

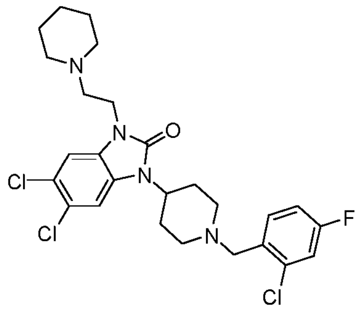

EX44

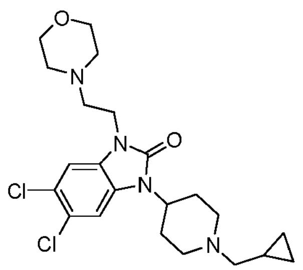

EX45

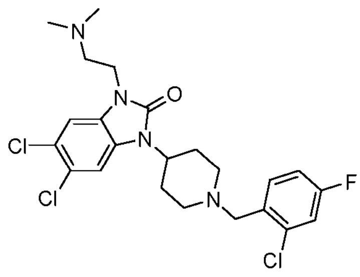

EX46

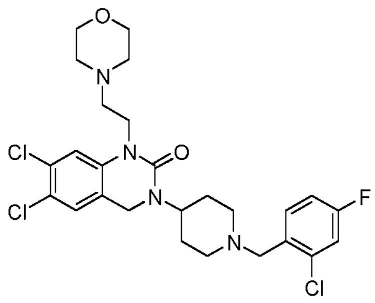

EX47

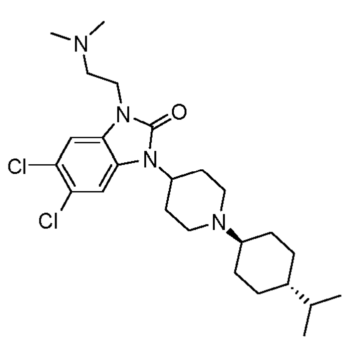

EX48A

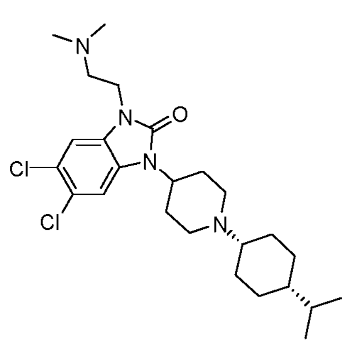

EX48B

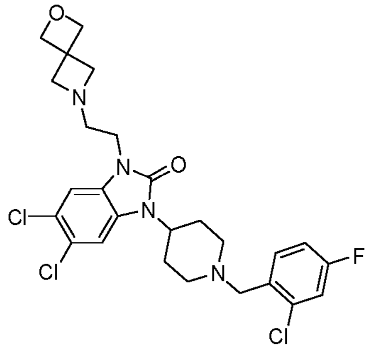

EX49

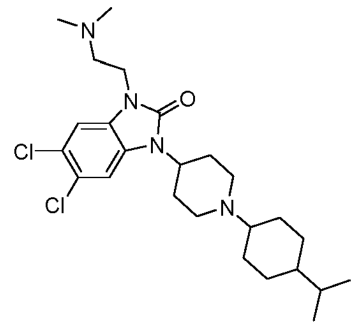

**EX48**

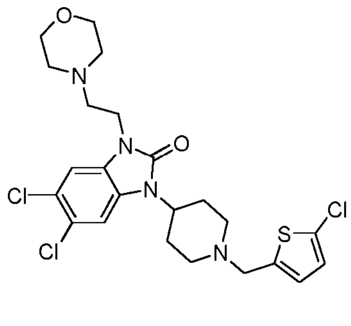

**EX50**

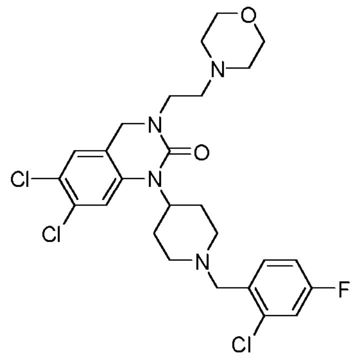

**EX51**

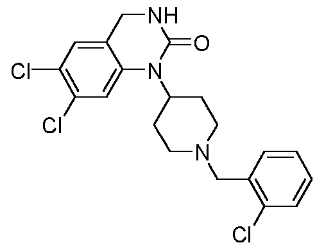

**EX52**

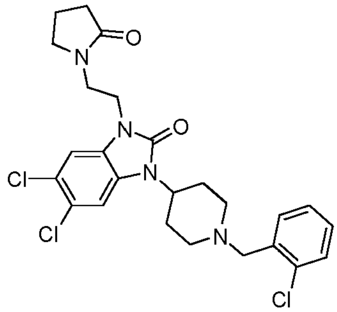

**EX53**

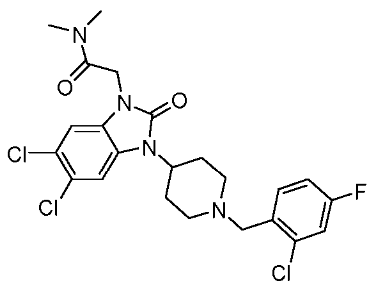

**EX54**

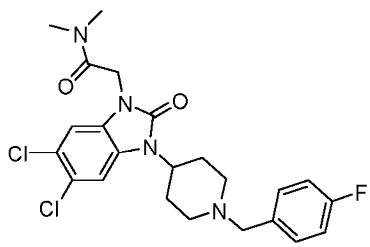

**EX55**

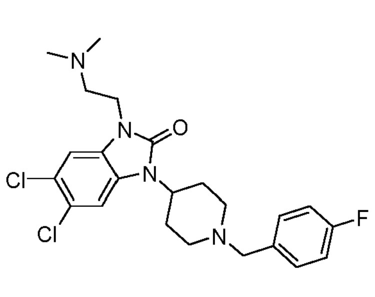

**EX56**

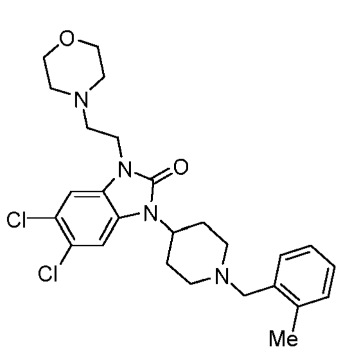

**EX57**

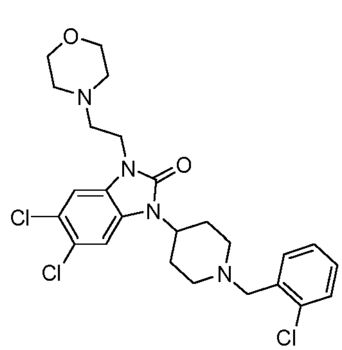

**EX58**

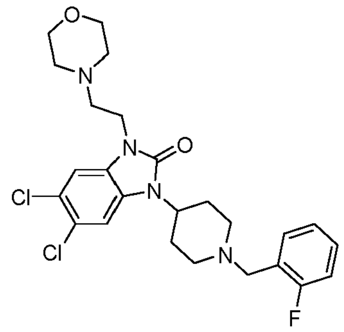

**EX59**

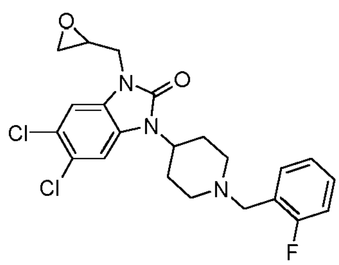

**EX60**

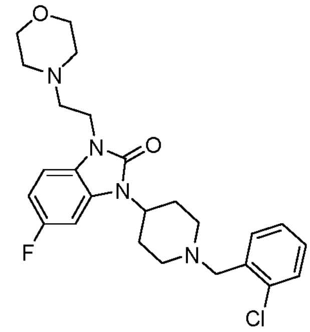

**EX61**

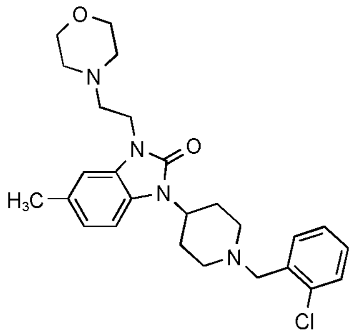

**EX62**

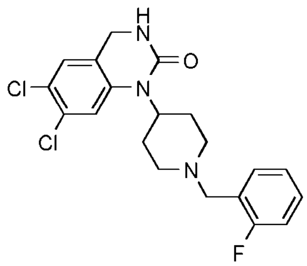

**EX63**

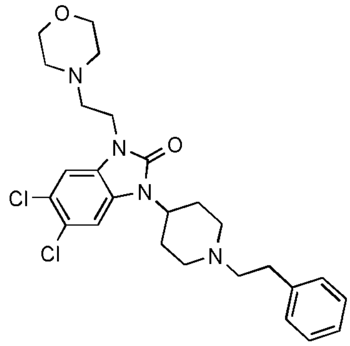

**EX64**

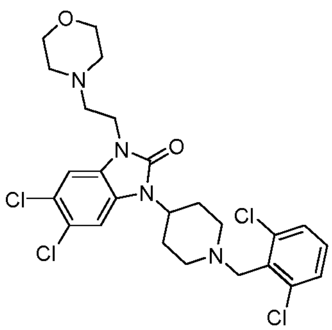

**EX65**

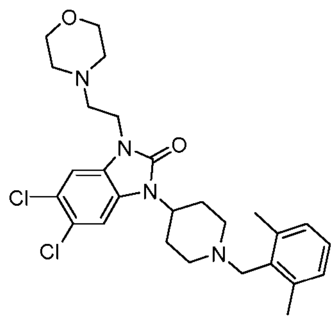

**EX66**

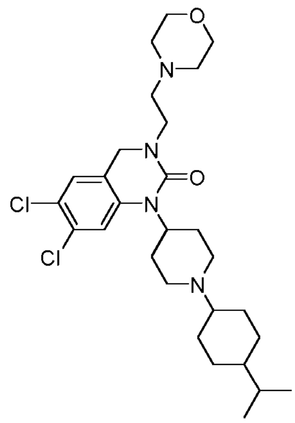

**EX67**

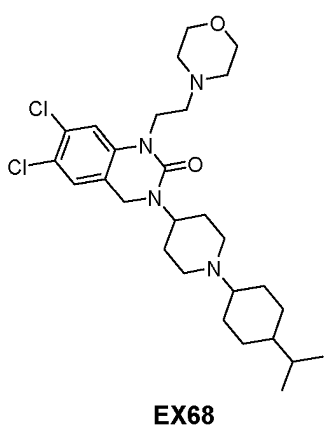

**EX68**

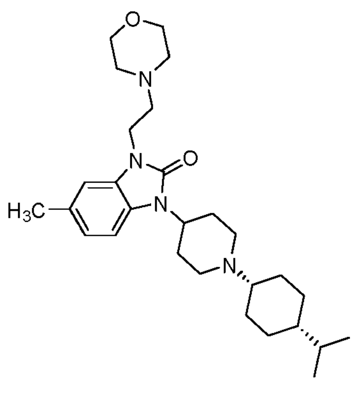

**EX69B**

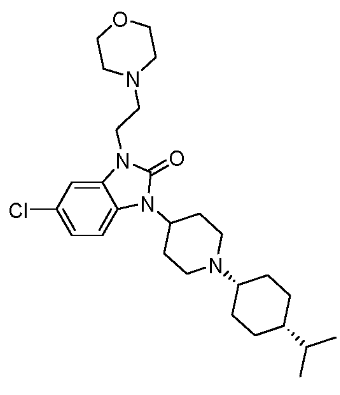

**EX70B**

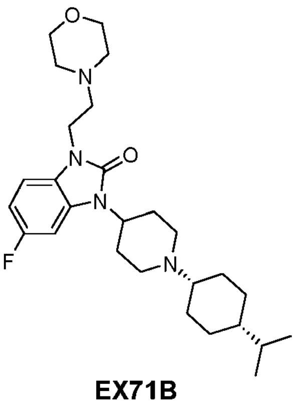

**EX71B**

**10.** Pharmazeutische Zusammensetzung, umfassend die Verbindung nach einem der Ansprüche 1 bis 9 oder ein Stereoisomer, ein pharmazeutisch unbedenkliches Salz, ein Solvat oder ein Deuterat davon und einen pharmazeutisch unbedenklichen Träger.

**11.** Verbindung gemäß einem der Ansprüche 1 bis 9 oder ein Stereoisomer, ein pharmazeutisch unbedenkliches Salz, ein Solvat oder ein Deuterat davon zur Verwendung bei der Behandlung von Schmerz, Angst, Depression, Alkoholabhängigkeit, Drogenmissbrauch/-abhängigkeit.

## Revendications

**1.** Composé représenté par la formule (I), stéréoisomère, sel pharmaceutiquement acceptable, solvate ou deutérate de celui-ci,

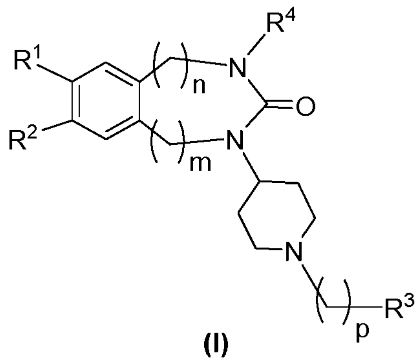

**(I)**

dans laquelle

vaut 0 ou 1 ;
m vaut 0 ou 1 ;
p vaut 0, 1 ou 2 ;
$R^1$ et $R^2$ sont chacun indépendamment choisis parmi : un atome d'hydrogène, un atome d'halogène, les groupes $C_{1-3}$ alkyle et $C_{1-3}$ alcoxy ; à condition que $R^1$ et $R^2$ ne représentent pas un atome d'hydrogène en même temps ;
$R^3$ est choisi parmi un groupe cyclopropyle, cyclobutyle, cyclopentyle, cyclohexyle, cycloheptyle ou cyclooctyle non substitué ; cyclopropyle, cyclobutyle, cyclopentyle, cyclohexyle, cycloheptyle ou cyclooctyle substitué, substitué par 1 à 3 groupes indépendamment choisis parmi les groupes suivants : les atomes de fluor, de chlore, de brome, d'iode, les groupes $C_{1-4}$ alkyle, $C_{1-4}$ alkyle halogéné, $C_{1-4}$ alcoxy, $C_{1-4}$ alcoxy halogéné et phényle ; un groupe phényle non substitué ; ou un groupe phényle substitué, substitué par 1 à 3 groupes indépendamment choisis parmi les groupes suivants : les atomes de fluor, de chlore, de brome, d'iode, les groupes $C_{1-4}$ alkyle, $C_{1-a}$ alkyle halogéné, $C_{1-4}$ alcoxy, et $C_{1-4}$ alcoxy halogéné ; ou un groupe furyle, thiényle, pyridyle, pyrrolyle, N-alkylpyrrolyle, pyrimidinyle, pyrazinyle, pyridazinyle, imidazolyle, pyrazolyle, triazolyle ou tétrazolyle non substitué, ou un groupe furyle, thiényle, pyridyle, pyrrolyle, N-alkylpyrrolyle, pyrimidinyle, pyrazinyle, pyridazinyle, imidazolyle, pyrazolyle, triazolyle ou tétrazolyle substitué, substitué par 1 à 2 groupes indépendamment choisis

parmi les groupes suivants : un atome d'halogène, les groupes $C_{1-4}$ alkyle, $C_{1-4}$ alkyle halogéné, $C_{1-4}$ alcoxy, $C_{1-4}$ alcoxy halogéné, aryle et hétéroaryle ; ou un groupe tétrahydrofuranyle, tétrahydropyrrolyle, tétrahydrothiényle, pipéridinyle, morpholinyle ou pipérazinyle non substitué ; un groupe tétrahydrofuranyle, tétrahydropyrrolyle, tétrahydrothiényle, pipéridinyle, morpholinyle ou pipérazinyle substitué, substitué par 1 à 3 groupes indépendamment choisis parmi les groupe suivants : les atomes de fluor, de chlore, de brome, d'iode, les groupes $C_{1-4}$ alkyle, $C_{1-4}$ alkyle halogéné, $C_{1-4}$ alcoxy, $C_{1-4}$ alcoxy halogéné et phényle ;
$R^4$ est choisi parmi un atome d'hydrogène, un groupe 2-(morpholinyl)éthyle, 2-(1,1-dioxothiomorpholine)éthyle, 2-(4-méthylpipérazin-1-yl)éthyle, 2-(4-acétylpipérazin-1-yl)éthyle, 2-(3-oxopipérazin-1-yl)éthyle, 2-(pyrrolidin-1-yl)éthyle, 2-(pipéridin-1-yl)éthyle, 2-(N,N'-diméthylamino)éthyle, 2-(2-oxopyrrolidin-1-yl)éthyle, N,N'-diméthylacétamide, oxiran-2-ylméthyle ou 2-(2-oxa-6-azaspiro[3.3]heptan-6-yl)éthyle ; à condition que lorsque $R^4$ représente un atome d'hydrogène, m vaille 0 et n vaille 1.

**2.** Composé selon la revendication 1, ou stéréoisomère, sel pharmaceutiquement acceptable, solvate ou deutérate de celui-ci,

n valant 0 ;
m valant 0 ;
p valant 0, 1 ou 2.

**3.** Composé selon la revendication 1, ou stéréoisomère, sel pharmaceutiquement acceptable, solvate ou deutérate de celui-ci,

n valant 1 ;
m valant 0 ;
p valant 0, 1 ou 2.

**4.** Composé selon la revendication 1, ou stéréoisomère, sel pharmaceutiquement acceptable, solvate ou deutérate de celui-ci,

n valant 0 ;
m valant 1 ;
p valant 0, 1 ou 2.

**5.** Composé selon la revendication 1, ou stéréoisomère, sel pharmaceutiquement acceptable, solvate ou deutérate de celui-ci, $R^1$ et $R^2$ étant chacun indépendamment choisi parmi : un atome d'hydrogène, de chlore, de fluor, un groupe méthyle ; à condition que $R^1$ et $R^2$ ne représentent pas un atome d'hydrogène en même temps ; éventuellement, $R^1$ et $R^2$ représentent tous deux un atome de chlore, ou $R^1$ représente un atome de chlore et $R^2$ représente un atome de fluor, ou $R^1$ représente un atome de fluor et $R^2$ représente un atome de chlore, ou $R^1$ et $R^2$ représentent tous deux un atome de fluor, ou $R^1$ représente un atome de fluor, un groupe méthyle ou un atome de chlore et $R^2$ représente un atome d'hydrogène, ou $R^1$ représente un atome d'hydrogène et $R^2$ représente un atome de chlore ou de fluor.

**6.** Composé selon la revendication 1, ou stéréoisomère, sel pharmaceutiquement acceptable, solvate ou deutérate de celui-ci, $R^3$ représentant un groupe phényle, 3,4-dichlorophényle, 3,4-difluorophényle, 2-chloro-4-fluorophényle, 2-fluoro-4-chlorophényle, 4-trifluorométhylphényle, 4-trifluorométhoxyphényle, 2,4-dichlorophényle, 2,6-dichlorophényle, 2,4-difluorophényle, 2-chloro-4-méthylphényle, 2-méthyl-4-fluorophényle, 2-méthyl-4-chlorophényle, 2-méthoxy-4-chlorophényle, 2,4-diméthylphényle, 2,6-diméthylphényle, 2,4,6-triméthylphényle, *4-tert*-butylphényle, 2-chlorophényle, 2-méthylphényle, 2-fluorophényle, 2-méthoxyphényle, 4-chlorophényle, 4-fluorophényle ou 4-méthoxyphényle.

**7.** Composé selon la revendication 1, ou stéréoisomère, sel pharmaceutiquement acceptable, solvate ou deutérate de celui-ci, $R^3$ représentant un groupe cyclopropyle, cyclopentyle, cyclohexyle, cycloheptyle, *4-tert*-butylcyclohexyle, 4-isopropylcyclohexyle, 4-éthylcyclohexyle, 4-méthylcyclohexyle, 4-trifluorométhylcyclohexyle, 2,3-dihydro-1*H*-indén-2-yle ou 2-chlorocyclohexyle.

**8.** Composé selon la revendication 1, ou stéréoisomère, sel pharmaceutiquement acceptable, solvate ou deutérate de celui-ci, $R^3$ représentant un groupe N-isopropylpipéridin-4-yle.

**9.** Composé selon la revendication 1, ou stéréoisomère, sel pharmaceutiquement acceptable, solvate ou deutérate de

celui-ci, choisi parmi l'un des composés suivants :

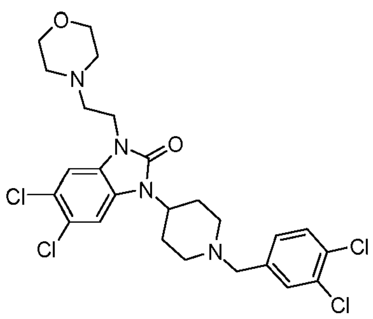

**EX1**

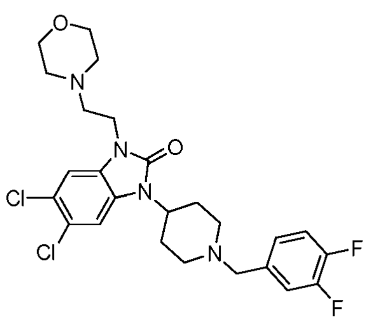

**EX2**

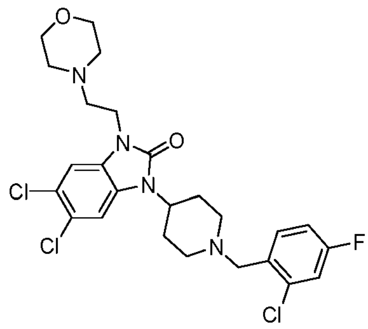

**EX3**

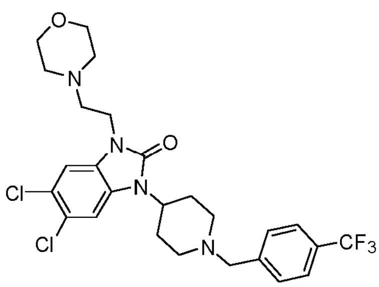

**EX4**

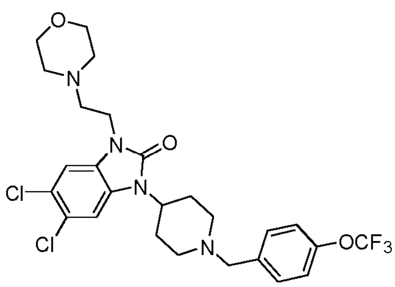

**EX5**

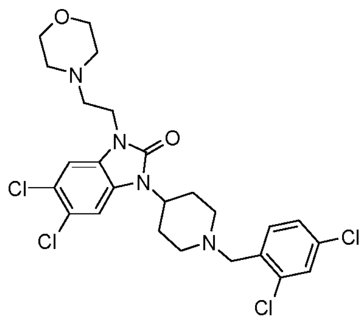

**EX6**

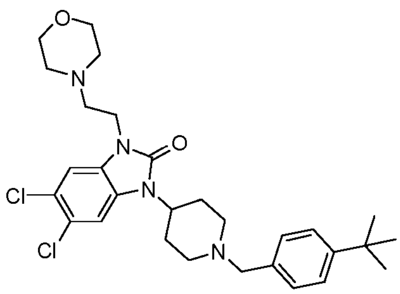

**EX7**

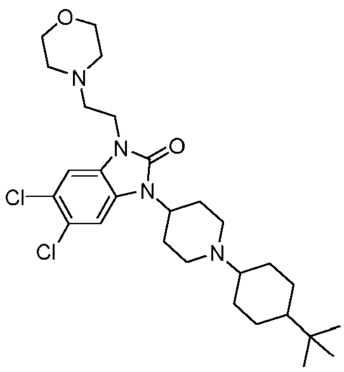

**EX8**

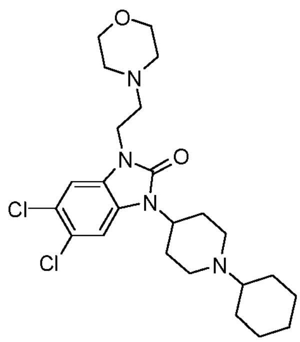

**EX9**

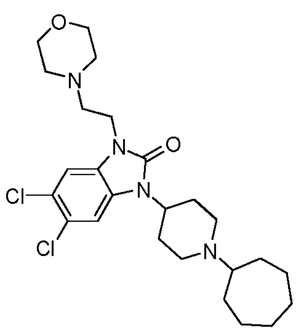

**EX10**

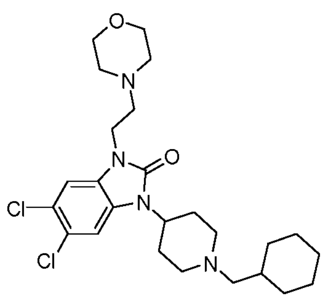

**EX11**

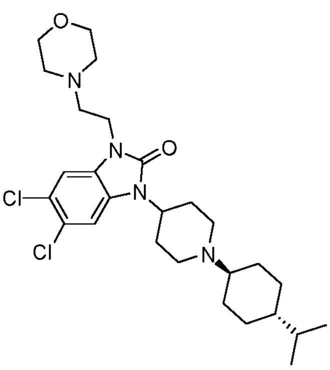

**EX12A**

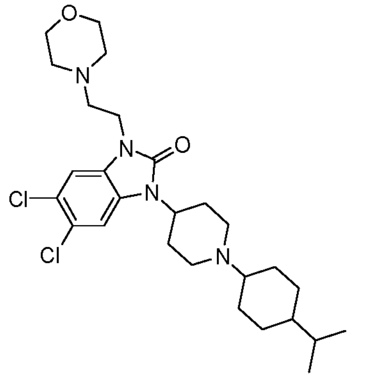

**EX12**

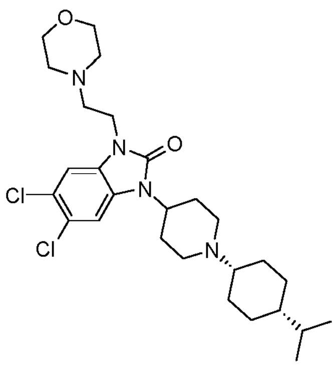

**EX12B**

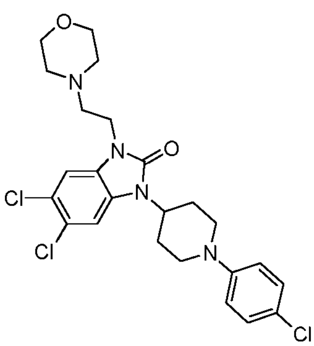

**EX13**

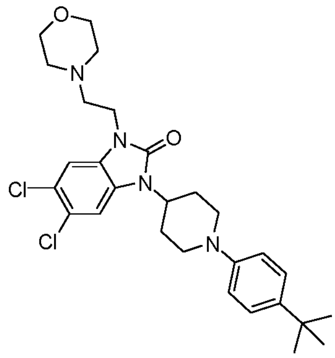

**EX14**

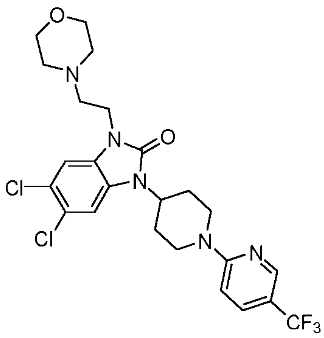

**EX15**

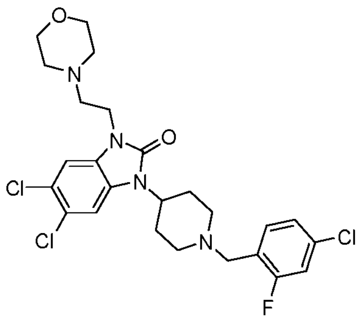

**EX16**

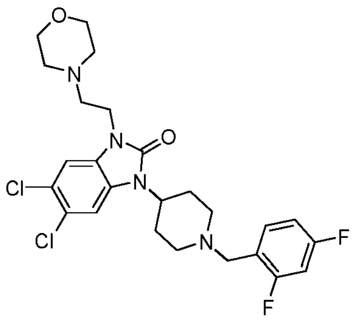

**EX17**

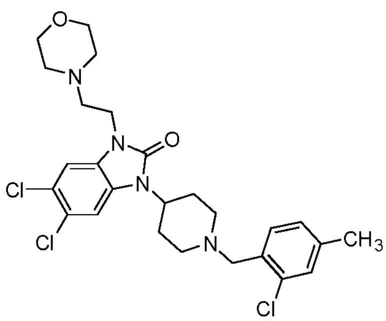

**EX18**

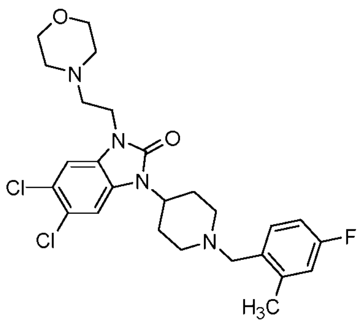

**EX19**

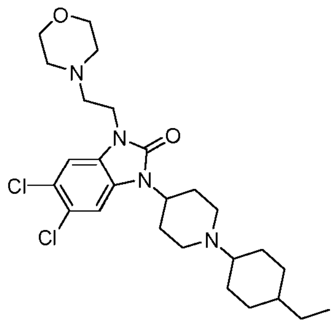

**EX20**

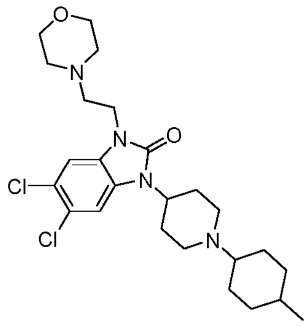

**EX21**

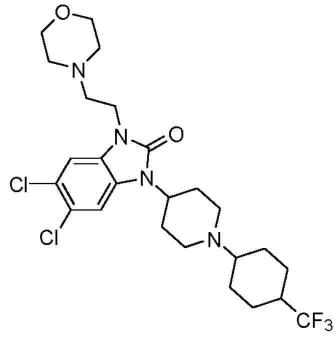

**EX22**

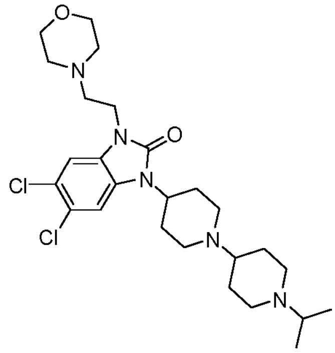

**EX23**

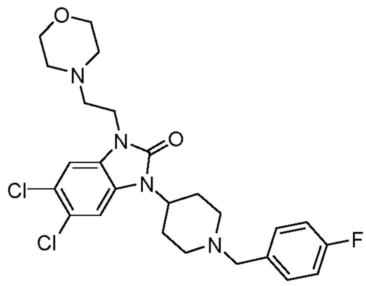

**EX24**

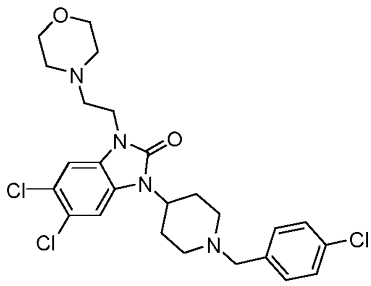

**EX25**

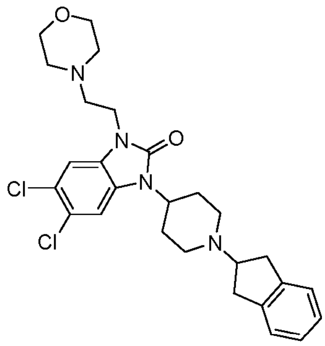

**EX26**

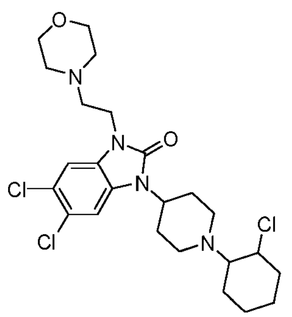

**EX27**

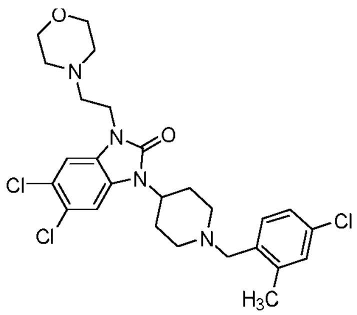

**EX28**

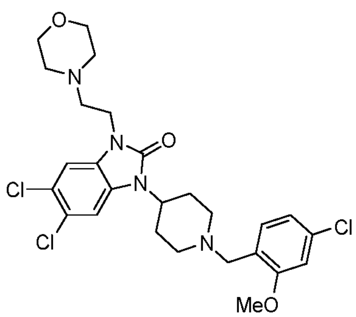

**EX29**

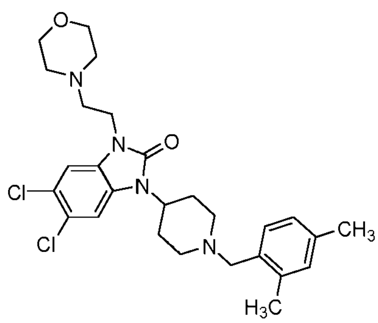

**EX30**

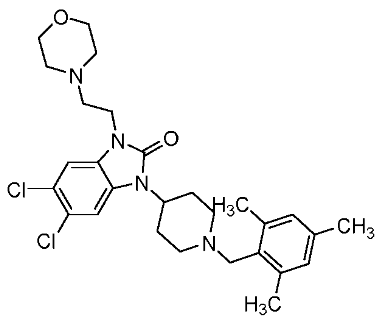

**EX31**

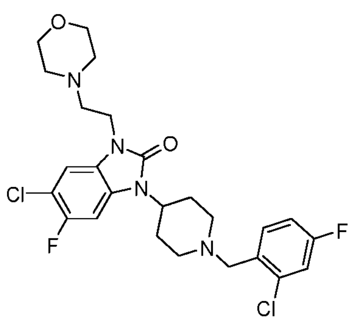

**EX33**

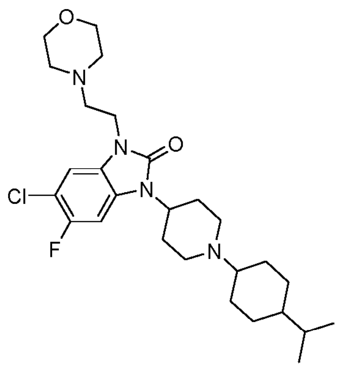

EX34

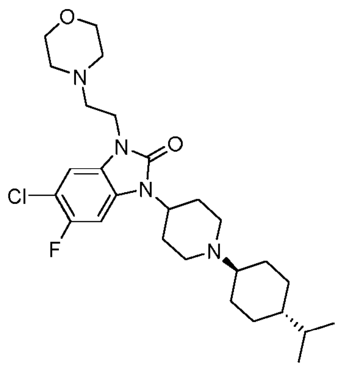

EX34A

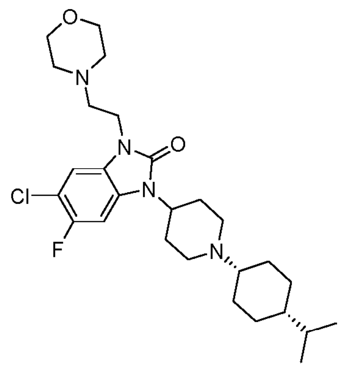

EX34B

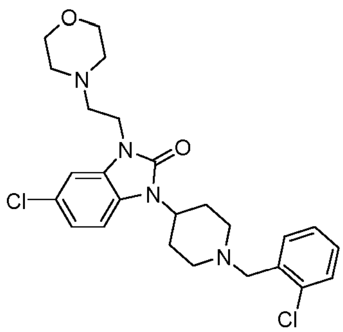

EX32

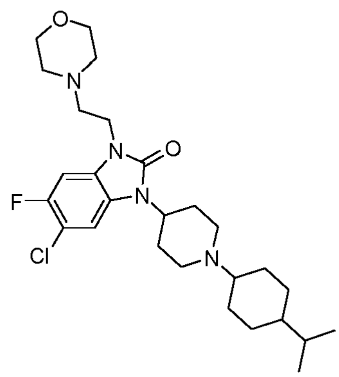

EX36

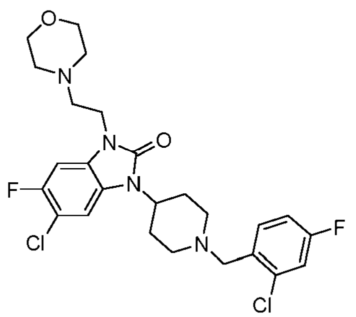

EX35

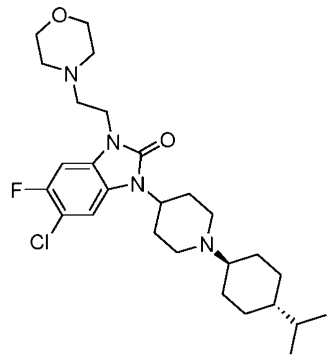

EX36A

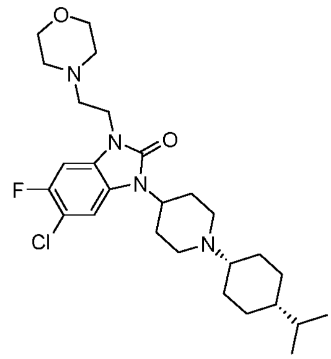

EX36B

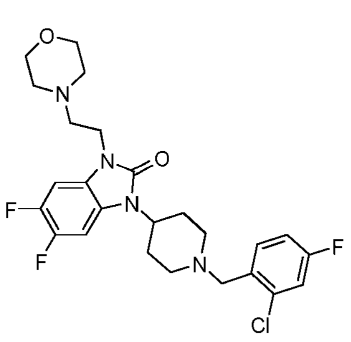

EX37

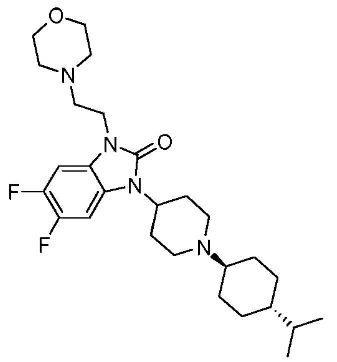

EX38A

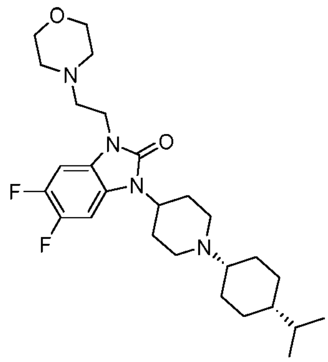

EX38B

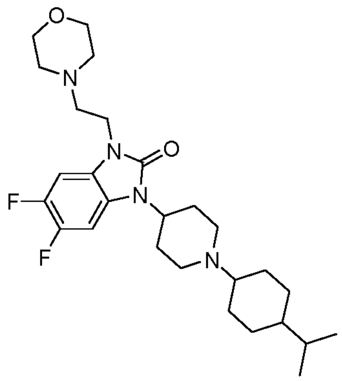

EX38

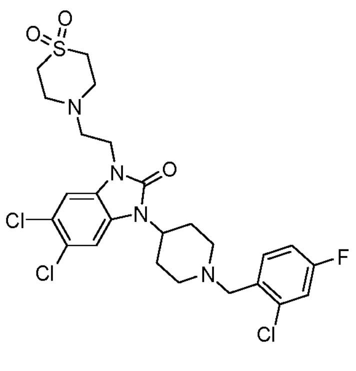

EX39

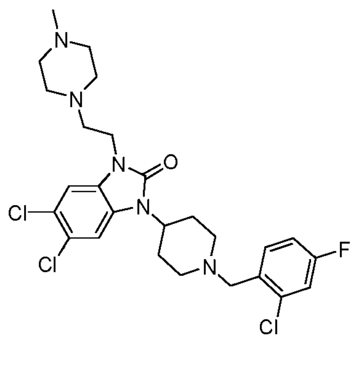

EX40

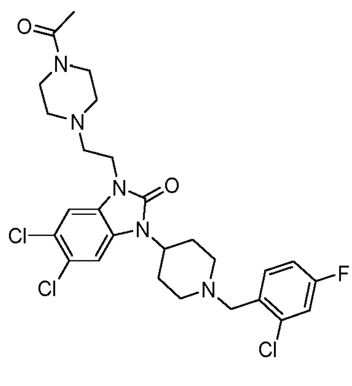

EX41

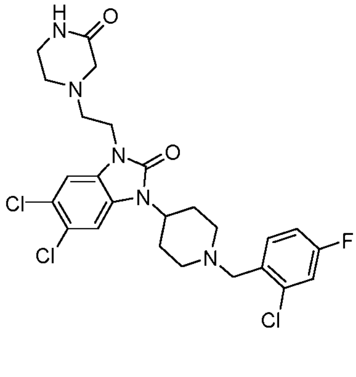

EX42

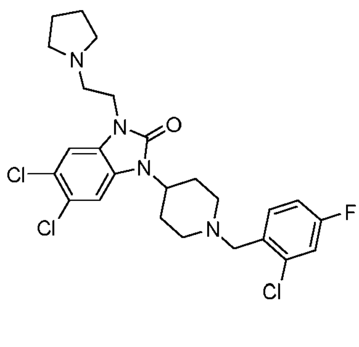

EX43

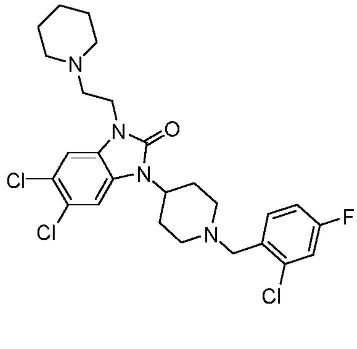

EX44

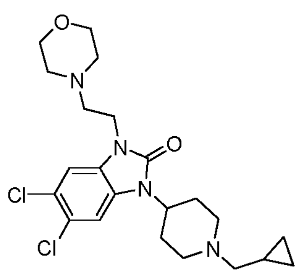

**EX45**

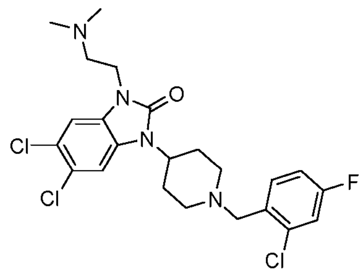

**EX46**

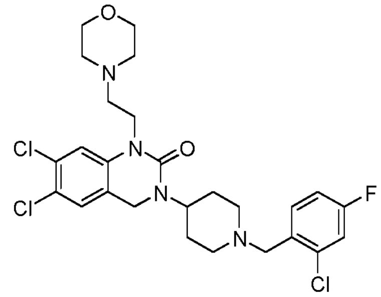

**EX47**

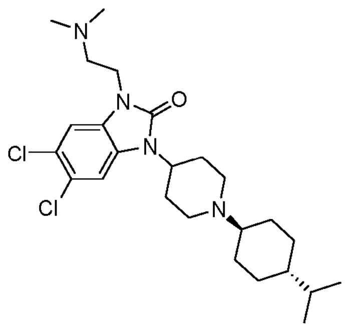

**EX48A**

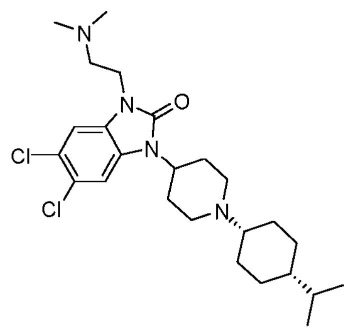

**EX48B**

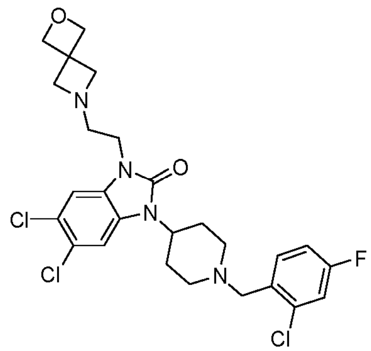

**EX49**

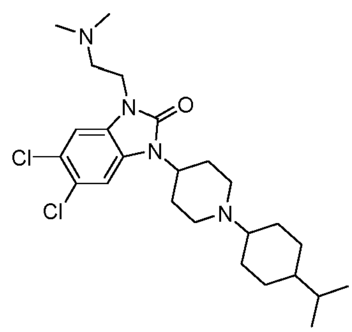

**EX48**

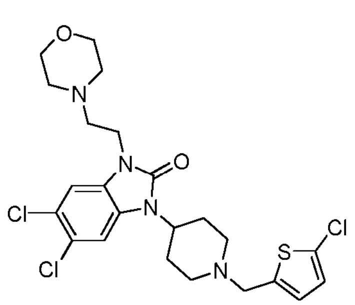

**EX50**

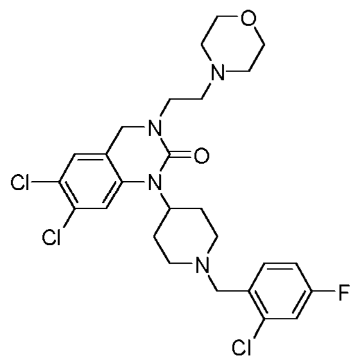

**EX51**

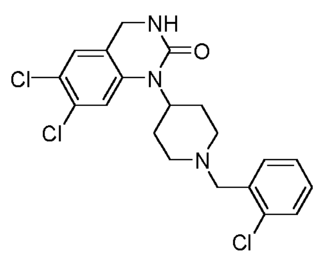

**EX52**

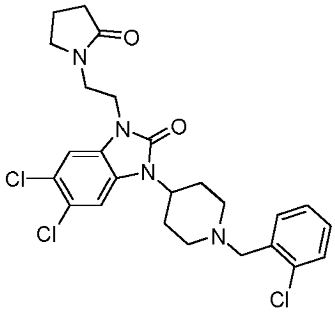

**EX53**

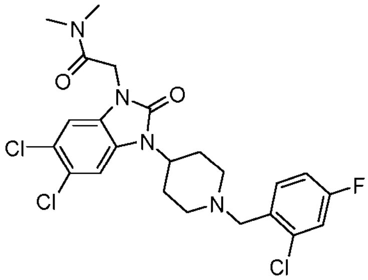

**EX54**

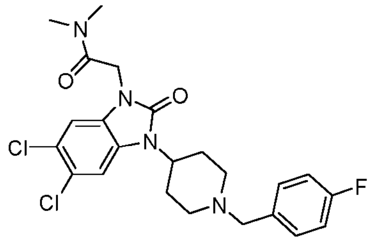

**EX55**

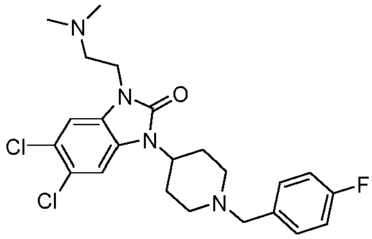

**EX56**

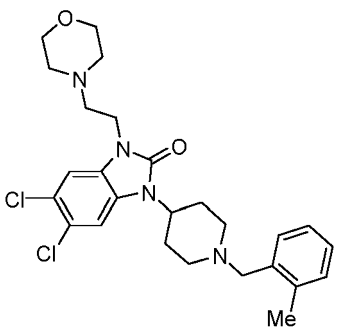

**EX57**

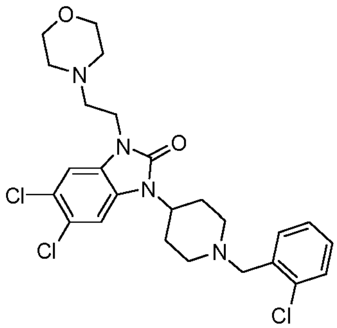

**EX58**

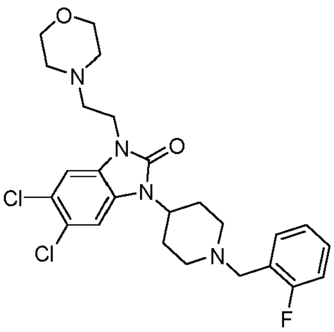

**EX59**

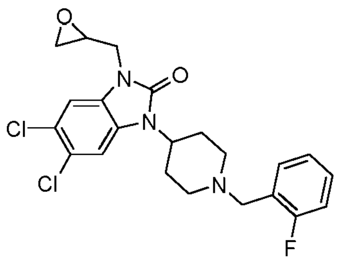

**EX60**

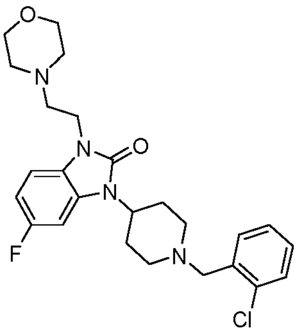

**EX61**

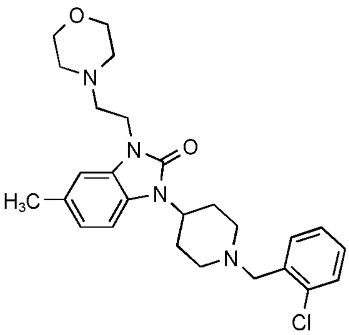

**EX62**

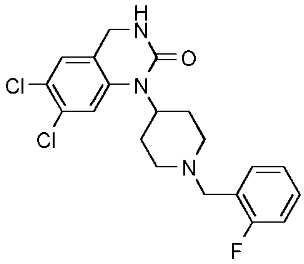

**EX63**

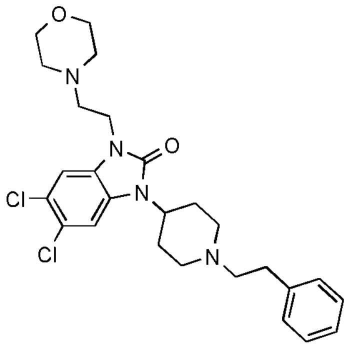

**EX64**

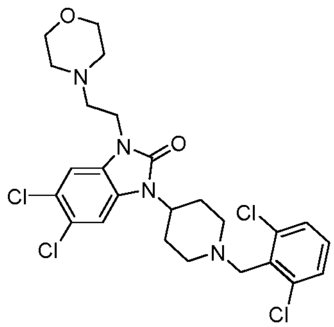

**EX65**

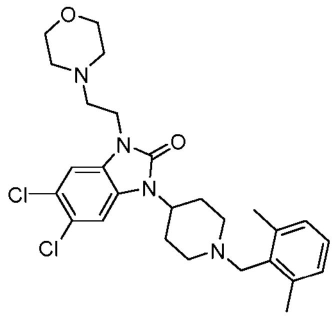

**EX66**

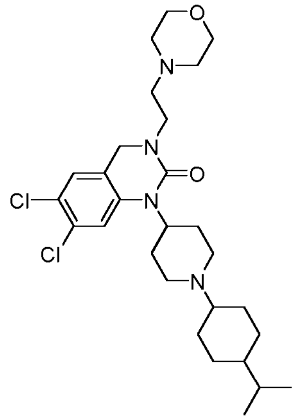

**EX67**

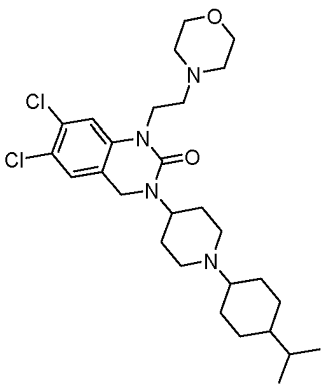

**EX68**

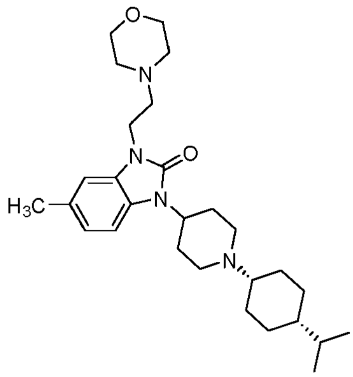

**EX69B**

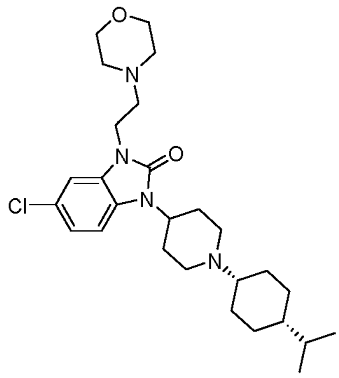

**EX70B**

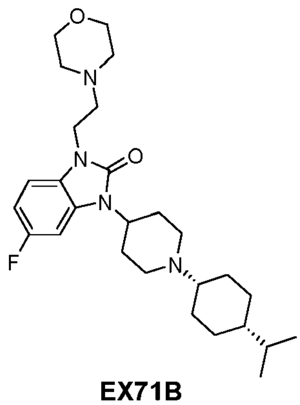

**EX71B**

**10.** Composition pharmaceutique, comprenant le composé selon l'une quelconque des revendications 1 à 9, ou un stéréoisomère, un sel pharmaceutiquement acceptable, un solvate ou un deutérate de celui-ci, et un vecteur pharmaceutiquement acceptable.

**11.** Composé selon l'une quelconque des revendications 1 à 9, ou stéréoisomère, sel pharmaceutiquement acceptable, solvate ou deutérate de celui-ci, destiné à être utilisé dans le traitement de la douleur, de l'anxiété, de la dépression, de la dépendance à l'alcool, de la toxicomanie/dépendance.

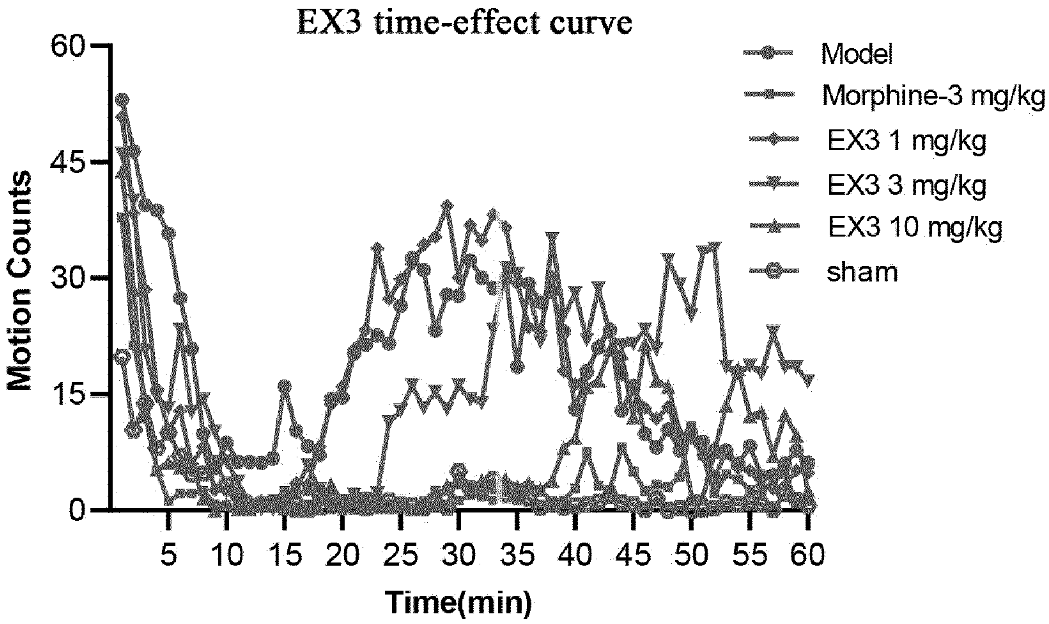
EX3 time-effect curve
Model
Morphine-3 mg/kg
EX3 1 mg/kg
EX3 3 mg/kg
EX3 10 mg/kg
sham
Motion Counts
0
15
30
45
60
5
10
15
20
25
30
35
40
45
50
55
60
Time(min)

FIG. 1A

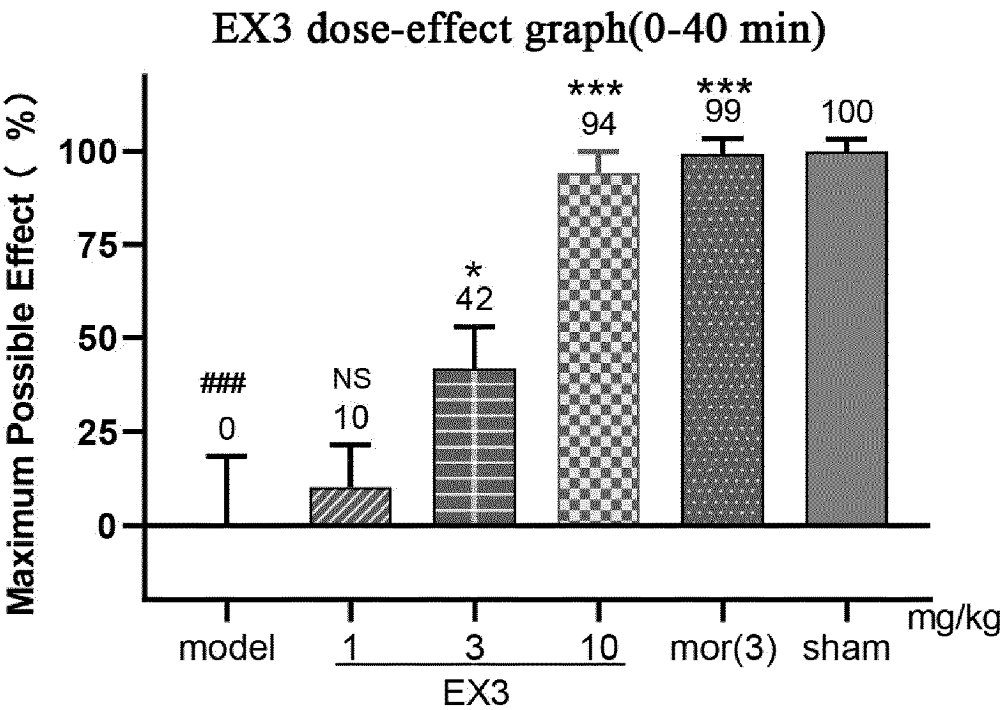
EX3 dose-effect graph(0-40 min)
Maximum Possible Effect ( %)
0
25
50
75
100
###
0
NS
10
*
42
***
94
***
99
100
model
1
3
10
mor(3)
sham
mg/kg
EX3

FIG. 1B

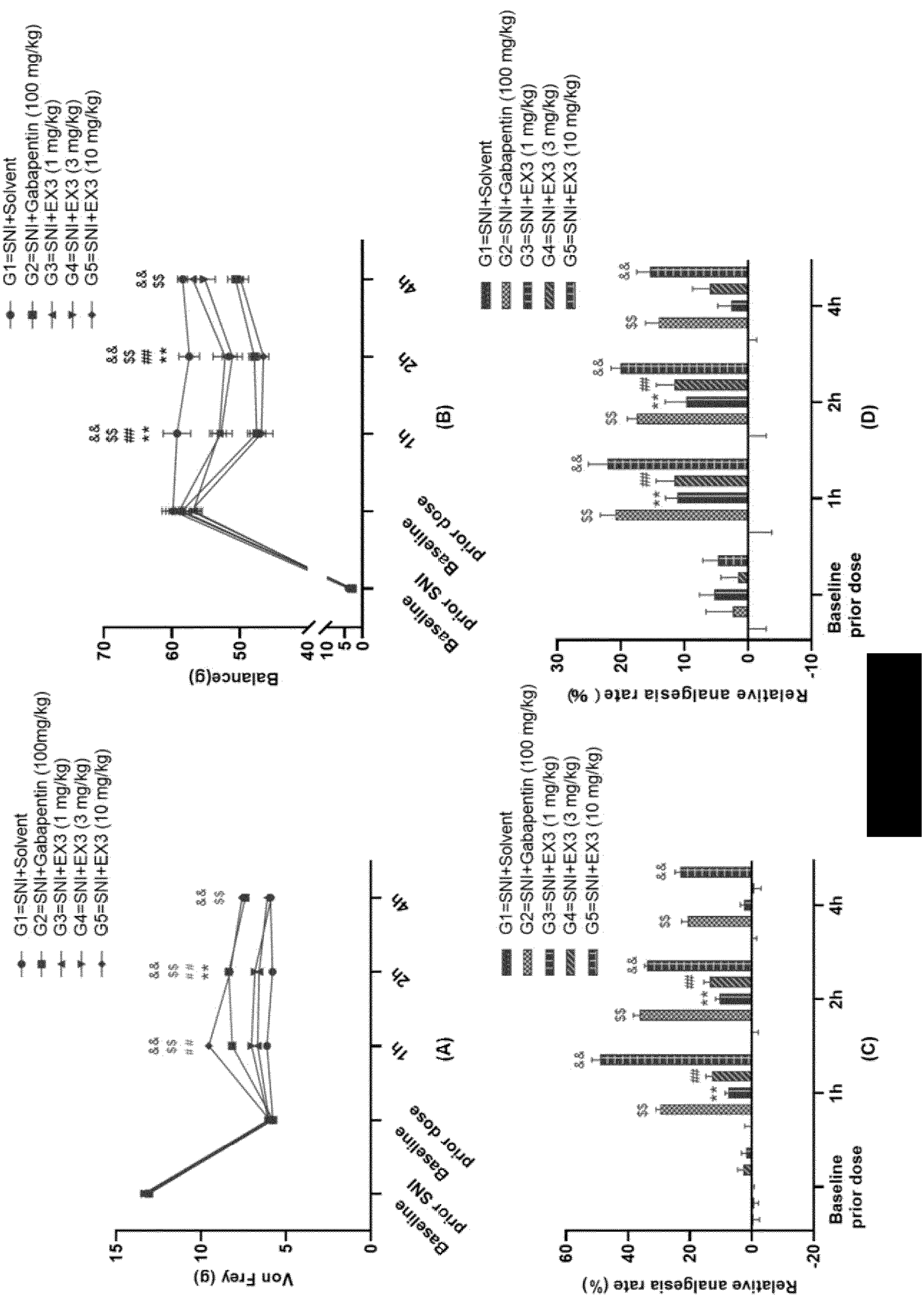

Von Frey (g)
Baseline prior SNI
Baseline prior dose
1h
2h
4h
(A)
G1=SNI+Solvent
G2=SNI+Gabapentin (100mg/kg)
G3=SNI+EX3 (1 mg/kg)
G4=SNI+EX3 (3 mg/kg)
G5=SNI+EX3 (10 mg/kg)
Balance(g)
(B)
G1=SNI+Solvent
G2=SNI+Gabapentin (100 mg/kg)
G3=SNI+EX3 (1 mg/kg)
G4=SNI+EX3 (3 mg/kg)
G5=SNI+EX3 (10 mg/kg)
Relative analgesia rate (%)
Baseline prior dose
(C)
(D)


[redacted]

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2017161017 A1
- RO 646198
- WO 0107050 A1
- WO 2017096323 A1